# EUROPEAN PATENT APPLICATION

(11) **EP 2 292 772 A1**
(43) Date of publication of application: **09.03.2011**
(21) Application number: 10180448.2
(22) Date of filing: 05.07.2002
(51) Int. Cl.: C12N 15/74, C07H 21/04, A61K 39/21

(54) **HIV vaccination with a DNA encoding a HIV polypeptide and a HIV polypeptide**

(30) Priority: 05.07.2001 US 303192 P; 31.08.2001 US 316860 P; 16.01.2002 US 349793 P; 16.01.2002 US 349728 P; 16.01.2002 US 349871 P
(62) Divisional of application: 09178990.9
(71) Applicant: Novartis Vaccines and Diagnostics, Inc., Emeryville, CA 94608 (US)
(72) Inventor: Zur Megede, Jan, Emeryville, CA 94608 (US); Barnett, Susan W., Emeryville, CA 94608 (US); Lian, Ying, Emeryville, CA 94608 (US)
(74) Representative: Hallybone, Huw George

(57) **Abstract**

The present invention relates to polynucleotides encoding immunogenic HIV polypeptides. Use of the polynucleotides in applications including immunization, generation of packaging cell lines, and production of HIV polypeptides are also described. Polynucleotides encoding antigenic HIV polypeptides are described, as are uses of these polynucleotides and polypeptide products therefrom, including formulations of immunogenic compositions and uses thereof.

## Description

### TECHNICAL FIELD

Polynucleotides encoding antigenic HIV polypeptides (*e.g*., those shown in Table C) are described, as are uses of these polynucleotides and polypeptide products including formulations of immunogenic compositions and uses thereof.

### BACKGROUND OF THE INVENTION

Acquired immune deficiency syndrome (AIDS) is recognized as one of the greatest health threats facing modern medicine. There is, as yet, no cure for this disease.

In 1983-1984, three groups independently identified the suspected etiological agent of AIDS. See, e.g., Barre-Sinoussi et al. (1983) Science 220:868-871; Montagnier et al., in Human T-Cell Leukemia Viruses (Gallo, Essex & Gross, eds., 1984); Vilmer et al. (1984) The Lancet 1:753; Popovic et al. (1984) Science 224:497-500; Levy et al. (1984) Science 225:840-842. These isolates were variously called lymphadenopathy-associated virus (LAV), human T-cell lymphotropic virus type III (HTLV-III), or AIDS-associated retrovirus (ARV). All of these isolates are strains of the same virus, and were later collectively named Human Immunodeficiency Virus (HTV). With the isolation of a related AIDS-causing virus, the strains originally called HIV are now termed HIV-1 and the related virus is called HIV-2 See, e.g., Guyader et al. (1987) Nature 326:662-669; Brun-Vezinet et al. (1986) Science 233:343-346; Clavel et al. (1986) Nature 324:691-695.

A great deal of information has been gathered about the HIV virus, however, to date an effective vaccine has not been identified. Several targets for vaccine development have been examined including the *env* and *Gag* gene products encoded by HIV. Gag gene products include, but are not limited to, Gag-polymerase and Gag-protease. Env gene products include, but are not limited to, monomeric gp120 polypeptides, oligomeric gp140 polypeptides and gp 160 polypeptides.

Haas, et al., (Current Biology 6(3):315-324, 1996) suggested that selective codon usage by HIV-1 appeared to account for a substantial fraction of the inefficiency of viral protein synthesis. Andre, et al., (J. Virol. 72(2):1497-1503, 1998) described an increased immune response elicited by DNA vaccination employing a synthetic gp120 sequence with modified codon usage. Schneider, et al., (J Virol. 71(7):4892-4903, 1997) discuss inactivation of inhibitory (or instability) elements (INS) located within the coding sequences of the Gag and Gag-protease coding sequences.

The *Gag* proteins of HIV-1 are necessary for the assembly of virus-like particles. HIV-1 *Gag* proteins are involved in many stages of the life cycle of the virus including, assembly, virion maturation after particle release, and early post-entry steps in virus replication. The roles of HIV-1 *Gag* proteins are numerous and complex (Freed, E.O., Virology 251:1-15, 1998).

Wolf, et al., (PCT International Application, WO 96/30523, published 3 October 1996; European Patent Application, Publication No. 0 449 116 A1, published 2 October 1991) have described the use of altered pr55 *Gag* of HIV-1 to act as a noninfectious retroviral-like particulate carrier, in particular, for the presentation of immunologically important epitopes. Wang, et al., (Virology 200:524-534, 1994) describe a system to study assembly of HIV Gag-β-galactosidase fusion proteins into virions. They describe the construction of sequences encoding HIV Gag-β-galactosidase fusion proteins, the expression of such sequences in the presence of HIV Gag proteins, and assembly of these proteins into virus particles.

Shiver, et al., (PCT International Application, WO 98/34640, published 13 August 1998) described altering HIV-1 (CAM1) *Gag* coding sequences to produce synthetic DNA molecules encoding HIV *Gag* and modifications of HIV *Gag.* The codons of the synthetic molecules were codons preferred by a projected host cell.

Recently, use of HIV Env polypeptides in immunogenic compositions has been described. (see, U.S. Patent No. 5,846,546 to Hurwitz et al., issued December 8, 1998, describing immunogenic compositions comprising a mixture of at least four different recombinant virus that each express a different HIV env variant; and U.S. Patent No. 5,840,313 to Vahlne et al., issued November 24, 1998, describing peptides which correspond to epitopes of the HIV-1 gp120 protein). In addition, U.S. Patent No. 5,876,731 to Sia et al, issued March 2, 1999 describes candidate vaccines against HIV comprising an amino acid sequence of a T-cell epitope of Gag linked directly to an amino acid sequence of a B-cell epitope of the V3 loop protein of an HIV-1 isolate containing the sequence GPGR.

### SUMMARY OF THE INVENTION

Described herein are novel HIV sequences, polypeptides encoded by these novel sequences, and synthetic expression cassettes generated from these and other HIV sequences. In one aspect, the present invention relates to improved HIV expression cassettes. In a second aspect, the present invention relates to generating an immune response in a subject using the expression cassettes of the present invention. In a further aspect, the present invention relates to generating an immune response in a subject using the expression cassettes of the present invention, as well as, polypeptides encoded by the expression cassettes of the present invention. In another aspect, the present invention relates to enhanced vaccine technologies for the induction of potent neutralizing antibodies and/or cellular immune responses against HIV in a subject.

In certain embodiments, the present invention relates synthetic polynucleotides and/or expression cassettes encoding HIV polypeptides, including, but not limited to, Env, Gag, Pol, Prot, Vpr, Vpu, Vif, Nef, Tat, Rev and/or fragments thereof. In addition, the present invention also relates to improved expression of HIV polypeptides and production of virus-like particles. Synthetic expression cassettes encoding the HIV polypeptides (*e.g*., Gag-, pol-, protease (prot)-, reverse transcriptase, integrase, RNAseH, Tat, Rev, Nef, Vpr, Vpu, Vif and/or Env-containing polypeptides) are described, as are uses of the expression cassettes. Mutations in some of the genes are described that reduce or eliminate the activity of the gene product without adversely affecting the ability of the gene product to generate an immune response. Exemplary synthetic polynucleotides include, but are not limited to, synthetic polynucleotides comprising at least one polynucleotide encoding a polypeptide comprising a Type B antigen and at least one polynucleotide encoding a polypeptide comprising a Type C antigen, wherein said synthetic polynucleotide sequences comprises sequences selected from, but not limited to, the following: gagCpolInaTatRevNef.opt_B (SEQ ID NO:9), GagProtInaRTmutTatRevNef.opt_B (SEQ ID NO:10), GagTatRevNef.opt_B (SEQ ID NO:11), GagComplPolmutInaTatRevNef_C (SEQ ID NO:12), GagProtInaRTmutTatRevNef_C (SEQ ID NO:13), GagRTmutTatRevNef_C (SEQ ID NO:14), GagTatRevNef_C (SEQ ID NO:15), int.opt.mut.SF2 (SEQ ID NO:16), int.opt.SF2 (SEQ ID NO:17), int.opt.mut_C (SEQ ID NO:18), int.opt_C (SEQ ID NO:19), nef.D125G.-myr.opt.SF162 (SEQ ID NO:20), nef.D107G.-myr18.opt.SF162 (SEQ ID NO:21), nef.opt.D125G.SF162 (SEQ ID NO:22), nef.opt.SF162 (SEQ ID NO:23), Nef_TV1_C_ZAopt (SEQ ID NO:24), Nef_TV2_C_ZAopt (SEQ ID NO:25), NefD124G_TV1_C_ZAopt (SEQ ID NO:26), NefD124G_TV2_C_ZAopt (SEQ ID NO:27), NefD124G-Myr_TV1_C_ZAopt (SEQ ID NO:28), nef.D106G.-myr19.opt_C (SEQ ID NO:29), p15RnaseH.opt.SF2 (SEQ ID NO:30), p15RnaseH.opt_C (SEQ ID NO:31), p2Pol.opt.YMWM.SF2 (SEQ ID NO:32), p2PolInaopt.YM.SF2 (SEQ ID NO:33), p2Polopt.SF2 (SEQ ID NO:34), p2PolTatRevNef.opt.native_B (SEQ ID NO:35), p2PolTatRevNef.opt_B (SEQ ID NO:36), p2Po1.opt.YMWM_C (SEQ ID NO:37), p2Polopt.YM_C (SEQ ID NO:38), p2Polopt_C (SEQ ID NO:39), p2PolTatRevNef opt C (SEQ ID NO:40), p2PolTatRevNef.opt.native_C (SEQ ID NO:41), p2PolTatRevNef.opt_C (SEQ ID NO:42), pol.opt.SF2 (SEQ ID NO:43), Pol_TVl1C_ZAopt (SEQ ID NO:44), Pol_TV2_C_ZAopt (SEQ ID NO:45), prot.opt.SF2 (SEQ ID NO:46), protIna.opt.SF2 (SEQ ID NO:47), protInaRT.YM.opt.SF2 (SEQ ID NO:48), protInaRT.YMWM.opt.SF2 (SEQ ID NO:49), ProtInaRTmut.SF2 (SEQ ID NO:50), protRT.opt.SF2 (SEQ ID NO:51), ProtRT.TatRevNef.opt_B (SEQ ID NO:52), ProtRTTatRevNef.opt_B (SEQ ID NO:53), protInaRT.YM.opt_C (SEQ ID NO:54), protInaRT.YMWM.opt_C (SEQ ID NO:55), ProtRT.TatRevNef.opt_C (SEQ ID NO:56), rev.exonl_2.M5-10.optSF162 (SEQ ID NO:57), rev.exon1_2.optSF162 (SEQ ID NO:58), rev.exon1_2.M5-10.opt_C (SEQ ID NO:59), revexon1_2 TV1 C ZAopt (SEQ ID NO:60), RT.opt.SF2 (mutant) (SEQ ID NO:61), RT.opt.SF2 (native) (SEQ ID NO:62), RTmut.SF2 (SEQ ID NO:63), tat.exon1_2.opt.C22-37.SF2 (SEQ ID NO:64), tat.exon1_2.opt.C37.SF2 (SEQ ID NO:65), tat.exon1_2.opt.C22-37_C (SEQ ID NO:66), tat.exon1_2.opt.C37_C (SEQ ID NO:67), TAT_CYS22_SF162_OPT (SEQ ID NO:68), tat_sf162_opt (SEQ ID NO:69), TatC22Exon1_2_TV1_C_ZAopt (SEQ ID NO:70), TatExonl_2_TV1_C_ZAopt (SEQ ID NO:71), TatRevNef.optnative.SF162 (SEQ ID NO:72), TatRevNef.opt.SF162 (SEQ ID NO:73), TatRevNefGag B (SEQ ID NO:74), TatRevNefgagCpolIna B (SEQ ID NO:75), TatRevNefGagProtInaRTmut B (SEQ ID NO:76), TatRevNefp2Pol.opt_B (SEQ ID NO:77), TatRevNefprotRTopt B (SEQ ID NO:78), TatRevNef.opt.native_ZA (SEQ ID NO:79), TatRevNef.opt_ZA (SEQ ID NO:80), TatRevNefGag C (SEQ ID NO:81), TatRevNefgagCpolIna C (SEQ ID NO:82), TatRevNefGagProtInaRTmut C (SEQ ID NO:83), TatRevNefProtRT opt C (SEQ ID NO:84), vif.opt.SF2 (SEQ ID NO:85), vpr.opt.SF2 (SEQ ID NO:86), vpu.opt.SF162 (SEQ ID NO:87), Vif_TV1_C_ZAopt (SEQ ID NO:88), Vif_TV2_C_ZAopt (SEQ ID NO:89), Vpr_TV1_C_ZAopt (SEQ ID NO:90), Vpr_TV2_C_ZAopt (SEQ ID NO:91), Vpu_TV1_C_ZAopt (SEQ ID NO:92), Vpu_TV2_C_ZAopt (SEQ ID NO:93), and fragments thereof.

Thus, one aspect of the present invention relates to expression cassettes and polynucleotides contained therein. The expression cassettes typically include an HIV-polypeptide encoding sequence inserted into an expression vector backbone. In one embodiment, an expression cassette comprises a polynucleotide sequence encoding one or more polypeptides, wherein the polynucleotide sequence comprises a sequence having between about 85% to 100% and any integer values therebetween, for example, at least about 85%, preferably about 90%, more preferably about 95%, and more preferably about 98% sequence identity to the sequences taught in the present specification.

The polynucleotides encoding the HIV polypeptides of the present invention may also include sequences encoding additional polypeptides. Such additional polynucleotides encoding polypeptides may include, for example, coding sequences for other viral proteins (e.g., hepatitis B or C or other HIV proteins, such as, polynucleotide sequences encoding an HIV *Gag* polypeptide, polynucleotide sequences encoding an HIV *Env* polypeptide and/or polynucleotides encoding one or more of vif, vpr, tat, rev, vpu and nef); cytokines or other transgenes.

In one embodiment, the sequence encoding the HIV Pol polypeptide(s) can be modified by deletions of coding regions corresponding to reverse transcriptase and integrase. Such deletions in the polymerase polypeptide can also be made such that the polynucleotide sequence preserves T-helper cell and CTL epitopes. Other antigens of interest may be inserted into the polymerase as well.

In another embodiment, an expression cassette comprises a synthetic polynucleotide comprises at least one polynucleotide encoding a polypeptide comprising a Type B antigen and at least one polynucleotide encoding a polypeptide comprising a Type C antigen, wherein said synthetic polynucleotide sequences comprises coding sequences selected from, but not limited to, the following: gagCpolInaTatRevNef.opt_B (SEQ ID NO:9), GagProtInaRTmutTatRevNef.opt_B (SEQ ID NO:10), GagTatRevNef.opt_B (SEQ ID NO:11), GagcomplPolmutInaTatRevNef_C (SEQ ID NO:12), GagProtInaRTmutTatRevNef_C (SEQ ID NO:13), GagRTmutTatRevNef_C (SEQ ID NO:14), GagTatRevNef_C (SEQ ID NO:15), int.opt.mut.SF2 (SEQ ID NO:16), int.opt.SF2 (SEQ ID NO:17), int.opt.mut_C (SEQ ID NO:18), int.opt_C (SEQ ID NO:19), nef.D125G.-myr.opt.SF162 (SEQ ID NO:20), nef.D107G.-myr18.opt.SF162 (SEQ ID NO:21), nef.opt.D125G.SF162 (SEQ ID NO:22), nef.opt.SF162 (SEQ ID NO:23), Nef_TV1_C_ZAopt (SEQ ID NO:24), Nef_TV2_C_ZAopt (SEQ ID NO:25), NefD124G_TV1_C_ZAopt (SEQ ID NO:26), NefD124G_TV2_C_ZAopt (SEQ ID NO:27), NefD124G-Myr_TV1_C_ZAopt (SEQ ID NO:28), nef.D106G.-myr19.opt_C (SEQ ID NO:29), p15RnaseH.optSF2 (SEQ ID NO:30), p15RnaseH.opt_C (SEQ ID NO:31), p2Pol.opt.YMWM.SF2 (SEQ ID NO:32), p2PolInaopt.YM.SF2 (SEQ ID NO:33), p2Polopt.SF2 (SEQ ID NO:34), p2PolTatRevNef.opt.native_B (SEQ ID NO:35), p2PolTatRevNef.opt_B (SEQ ID NO:36), p2Pol.opt.YMWM_C (SEQ ID NO:37), p2Polopt.YM_C (SEQ ID NO:38), p2Polopt_C (SEQ ID NO:39), p2PolTatRevNef opt C (SEQ ID NO:40), p2PoITatRevNef.optnative_C (SEQ ID NO:41), p2PolTatRevNef.opt_C (SEQ ID NO:42), pol.opt.SF2 (SEQ ID NO:43), Poi_TV1_C_ZAopt (SEQ ID NO:44), Pol_TV2_C_ZAopt (SEQ ID NO:45), prot.opt.SF2 (SEQ ID NO:46), protIna.opt.SF2 (SEQ ID NO:47), protInaRT.YM.opt.SF2 (SEQ ID NO:48), protInaRT.YMWM.opt.SF2 (SEQ ID NO:49), ProtInaRTmut.SF2 (SEQ ID NO:50), protRT.opt.SF2 (SEQ ID NO:51), ProtRT.TatRevNef.opt_B (SEQ ID NO:52), ProtRTTatRevNef.opt_B (SEQ ID NO:53), protInaRT.YM,opt_C (SEQ ID NO:54), protInaRT.YMWM.opt_C (SEQ ID NO:55), ProtRT.TatRevNef.opt_C (SEQ ID NO:56), rev.exonl_2.M5-10.optSF162 (SEQ ID NO:57), rev.exon1_2.opt.SF162 (SEQ ID NO:58), rev.exonl_2.M5-10.opt_C (SEQ ID NO:59), revexon1_2 TV1 C ZAopt (SEQ ID NO:60), RT.opt.SF2 (mutant) (SEQ ID NO:61), RT.opt.SF2 (native) (SEQ ID NO:62), RTmut.SF2 (SEQ ID NO:63), tat.exon1_2.opt.C22-37.SF2 (SEQ ID NO:64), tat.exon1_2.opt.C37.SF2 (SEQ ID NO:65), tat.exon1_2.opt.C22-37_C (SEQ ID NO:66), tatexon1_2.optC37_C (SEQ ID NO:67), TAT_CYS22_SF162_OPT (SEQ ID NO:68), tat_sf162_opt (SEQ ID NO:69), TatC22Exon1_2_TV1_C_ZAopt (SEQ ID NO:70), TatExon1_2_TV1_C_ZAopt (SEQ ID NO:71), TatRevNef.opt.native.SF162 (SEQ ID NO:72), TatRevNef.opt.SF162 (SEQ ID NO:73), TatRevNefGag B (SEQ ID NO:74), TatRevNefgagCpolIna B (SEQ ID NO:75), TatRevNefGagProtInaRTmut B (SEQ ID NO:76), TatRevNefp2Pol.opt_B (SEQ ID NO:77), TatRevNefprotRTopt B (SEQ ID NO:78), TatRevNef.opt.native_ZA (SEQ ID NO:79), TatRevNef.opt_ZA (SEQ ID NO:80), TatRevNefGag C (SEQ ID NO:81), TatRevNefgagCpolIna C (SEQ ID NO:82), TatRevNefGagProtInaRTmut C (SEQ ID NO:83), TatRevNefProtRT opt C (SEQ ID NO:84), vif.opt.SF2 (SEQ ID NO:85), vpr.opt.SF2 (SEQ ID NO:86), vpu.opt.SF162 (SEQ ID NO:87), Vif_TV1_C_ZAopt (SEQ ID NO:88), Vif_TV2_C_ZAopt (SEQ ID NO:89), Vpr_TV1_C_ZAopt (SEQ ID NO:90), Vpr_TV2_C ZAopt (SEQ ID NO:91), Vpu_TV1_C_ZAopt (SEQ ID NO:92), Vpu_TV2_C_ZAopt (SEQ ID NO:93), and fragments thereof, wherein the polynucleotide sequence encoding the polypeptide comprises a sequence having between about 85% to 100% and any integer values therebetween, for example, at least about 85%, preferably about 90%, more preferably about 95%, and more preferably about 98% sequence identity to the sequences taught in the present specification.

The native and synthetic polynucleotide sequences encoding the HIV polypeptides of the present invention typically have between about 85% to 100% and any integer values therebetween, for example, at least about 85%, preferably about 90%, more preferably about 95%, and most preferably about 98% sequence identity to the sequences taught herein. Further, in certain embodiments, the polynucleotide sequences encoding the HIV polypeptides of the invention will exhibit 100% sequence identity to the sequences taught herein.

The polynucleotides of the present invention can be produced by recombinant techniques, synthetic techniques, or combinations thereof.

The present invention further includes recombinant expression systems for use in selected host cells, wherein the recombinant expression systems employ one or more of the polynucleotides and expression cassettes of the present invention. In such systems, the polynucleotide sequences are operably linked to control elements compatible with expression in the selected host cell. Numerous expression control elements are known to those in the art, including, but not limited to, the following: transcription promoters, transcription enhancer elements, transcription termination signals, polyadenylation sequences, sequences for optimization of initiation of translation, and translation termination sequences. Exemplary transcription promoters include, but are not limited to those derived from CMV, CMV+intron A, SV40, RSV, HIV-Ltr, MMLV-ltr, and metallothionein.

In another aspect the invention includes cells comprising one or more of the expression cassettes of the present invention where the polynucleotide sequences are operably linked to control elements compatible with expression in the selected cell. In one embodiment such cells are mammalian cells. Exemplary mammalian cells include, but are not limited to, BHK, VERO, HT1080, 293, RD, COS-7, and CHO cells. Other cells, cell types, tissue types, etc., that may be useful in the practice of the present invention include, but are not limited to, those obtained from the following: insects (e.g., *Trichoplusia ni* (Tn5) and Sf9), bacteria, yeast, plants, antigen presenting cells (e.g., macrophage, monocytes, dendritic cells, B-cells, T-cells, stem cells, and progenitor cells thereof), primary cells, immortalized cells, tumor-derived cells.

In a further aspect, the present invention includes compositions for generating an immunological response, where the composition typically comprises at least one of the expression cassettes of the present invention and may, for example, contain combinations of expression cassettes such as one or more expression cassettes carrying a Pol-derived-polypeptide-encoding polynucleotide, one or more expression cassettes carrying a Gag-derived-polypeptide-encoding polynucleotide, one or more expression cassettes carrying accessory polypeptide-encoding polynucleotides (e.g., native or synthetic vpu, vpr, nef, vif, tat, rev), and/or one or more expression cassettes carrying an Env-derived-polypeptide-encoding polynucleotide. Such compositions may further contain an adjuvant or adjuvants. The compositions may also contain one or more HIV polypeptides. The HIV polypeptides may correspond to the polypeptides encoded by the expression cassette(s) in the composition, or may be different from those encoded by the expression cassettes. In compositions containing both expression cassettes (or polynucleotides of the present invention) and polypeptides, various expression cassettes of the present invention can be mixed and/or matched with various HIV polypeptides described herein.

In another aspect the present invention includes methods of immunization of a subject. In the method any of the above described compositions are into the subject under conditions that are compatible with expression of the expression cassette(s) in the subject. In one embodiment, the expression cassettes (or polynucleotides of the present invention) can be introduced using a gene delivery vector. The gene delivery vector can, for example, be a non-viral vector or a viral vector. Exemplary viral vectors include, but are not limited to eucaryotic layered vector initiation systems, Sindbis-virus derived vectors, retroviral vectors, and lentiviral vectors. Other exemplary vectors include, but are not limited to, pCMVKm2, pCMV6a, pCMV-link, and pCMVPLEdhfr. Compositions useful for generating an immunological response can also be delivered using a particulate carrier (e.g., PLG or CTAB-PLG microparticles). Further, such compositions can be coated on, for example, gold or tungsten particles and the coated particles delivered to the subject using, for example, a gene gun. The compositions can also be formulated as liposomes. In one embodiment of this method, the subject is a mammal and can, for example, be a human.

In a further aspect, the invention includes methods of generating an immune response in a subject. Any of the expression cassettes described herein can be expressed in a suitable cell to provide for the expression of the HIV polypeptides encoded by the polynucleotides of the present invention. The polypeptide(s) are then isolated (e.g., substantially purified) and administered to the subject in an amount sufficient to elicit an immune response. In certain embodiments, the methods comprise administration of one or more of the expression cassettes or polynucleotides of the present invention, using any of the gene delivery techniques described herein. In other embodiments, the methods comprise co-administration of one or more of the expression cassettes or polynucleotides of the present invention and one or more polypeptides, wherein the polypeptides can be expressed from these polynucleotides or can be other HIV polypeptides. In other embodiments, the methods comprise co-administration of multiple expression cassettes or polynucleotides of the present invention. In still further embodiments, the methods comprise co-administration of multiple polypeptides, for example polypeptides expressed from the polynucleotides of the present invention and/or other HIV polypeptides.

The invention further includes methods of generating an immune response in a subject, where cells of a subject are transfected with any of the above-described expression cassettes or polynucleotides of the present invention, under conditions that permit the expression of a selected polynucleotide and production of a polypeptide of interest (e.g., encoded by any expression cassette of the present invention). By this method an immunological response to the polypeptide is elicited in the subject. Transfection of the cells may be performed *ex vivo* and the transfected cells are reintroduced into the subject. Alternately, or in addition, the cells may be transfected *in vivo* in the subject. The immune response may be humoral and/or cell-mediated (cellular). In a further embodiment, this method may also include administration of an HIV polypeptides before, concurrently with, and/or after introduction of the expression cassette into the subject.

The polynucleotides of the present invention may be employed singly or in combination. The polynucleotides of the present invention, encoding HIV-derived polypeptides, may be expressed in a variety of ways, including, but not limited to the following: a polynucleotide encoding a single gene product (or portion thereof) expressed from a promoter; multiple polynucleotides encoding a more than one gene product (or portion thereof) (e.g., polycistronic coding sequences); multiple polynucleotides in-frame to produce a single polyprotein; and, multiple polynucleotides in-frame to produce a single polyprotein wherein the polyprotein has protein cleavage sites between one or more of the polypeptides comprising the polyprotein.

In one aspect the present invention includes a synthetic polynucleotide encoding two or more immunogenic HIV polypeptides, wherein at least two of said polypeptides are derived from different HIV subtypes, for example, HIV subtypes B and C. In addition other HIV subtypes may be used in combination as well, for example, Type A, Type B, Type C, Type D, Type E, Type F, Type G, Type O, etc.

The HIV polypeptides may encode antigens or epitopes from any HIV polypeptide, including but not limited to HIV polypeptides are selected from the following group: *Gag, Env, Pol, Tat, Rev, Nef, Vpr, Vpu, Vif* and combinations thereof. Other HIV polypeptides comprising antigens or epitopes are described herein (see, for example, Table A). In one embodiment the synthetic polynucleotide encodes *Tat, Rev* and *Nef* polypeptides. In another embodiment, the synthetic polynucleotide encodes *Vif, Vpr* and *Vpu* polypeptides.

The HIV polypeptides encoded by a synthetic polynucleotide may comprise one or more mutations affecting polypeptide activity or function that, for example, reduce (relative to wild-type), attenuate, inactivate, eliminate, or render non-functional the activity or function of the gene product(s) encoded the synthetic polynucleotide. For example, the synthetic polynucleotide may encode HIV polypeptides that comprise *Pol.* The mutations may, for example, be selected from the group consisting of mutations that reduce or eliminate protease function, mutations that delete the catalytic center of primer grip region of reverse transcriptase, mutations that inactive the catalytic center of DNA binding domain of integrase. In another example, the synthetic polynucleotide may encode HIV polypeptides that comprise *Env*. The mutations may, for example, comprise mutations in the cleavage site or mutations in the glycosylation site. In another example, the synthetic polynucleotide may encode HIV polypeptides that comprise *Tat.* The mutations may, for example, comprise mutations in the transactivation domain. In another example, the synthetic polynucleotide may encode HIV polypeptides that comprise *Rev.* The mutations may, for example, comprise mutations in the RNA binding-nuclear localization region or mutations in the activation domain. In another example, the synthetic polynucleotide may comprise HIV polypeptides that comprise *Nef.* The mutations may, for example, comprise mutations of myristoylation signal, mutations in oligomerization, mutations affecting infectivity and mutations affecting CD4 down regulation. In yet a further example, the synthetic polynucleotide may encode HIV polypeptides that comprise vif, vpr, and/or vpu. These polypeptides may also comprise mutations.

In a further aspect of the present invention, the synthetic polynucleotide may comprise a sequence encoding an additional antigenic polypeptide or epitope derived from an antigenic polypeptide.

The present invention also includes expression cassettes comprising the above synthetic polynucleotides. The expression cassettes may be used in recombinant expression systems. Control elements to be employed in expression cassettes may include, but are not limited to, a transcription promoter, a transcription enhancer element, a transcription termination signal, polyadenylation sequences, sequences for optimization of initiation of translation, and translation termination sequences. Exemplary transcription promoters include, but are not limited to CMV, CMV+intron A, SV40, RSV, HIV-Ltr, MMLV-ltr, and metallothionein.

In another aspect the present invention includes cells comprising the above-described synthetic polynucleotides, where typically expression cassettes comprise the synthetic polynucleotide(s). Exemplary cells include, but are not limited to mammalian cells (e.g, BHK, VERO, HT1080, 293, RD, COS-7, and CHO cells), insect cells (e.g., *Trichoplusia ni* (Tn5) or Sf9 insect cells), bacterial cells, yeast cells, plant cells, antigen presenting cells (e.g., macrophage, monocytes, dendritic cells, B-cells, T-cells, stem cells, and progenitor cells thereof), primary cells, immortalized cells, and tumor-derived cells.

In another aspect the present invention includes a method for producing a polypeptide including two or more HIV polypeptides from different subtypes, where the method may include, for example, incubating cells comprising expression cassettes encoding the polypeptide under conditions for producing the polypeptide.

In another aspect the present invention include gene delivery vectors for use in a mammalian subject, for example, where the gene delivery vector comprises an expression cassette which encodes a polypeptide including two or more HIV polypeptides from different subtypes. The expression cassette typically comprises a synthetic polynucleotide sequence operably linked to control elements compatible with expression in the subject. The present invention also includes a method of DNA immunization of a subject. Typically the method includes introducing a gene delivery vector of the present invention into the subject under conditions that are compatible with expression of the expression cassette in the subject. Exemplary gene delivery vectors include, but are not limited to, nonviral vectors, particulate carriers, and viral vectors (e.g., retroviral or lentiviral vectors). The gene delivery vectors may, for example, be coated on a gold or tungsten particle and the coated particle delivered to the subject using a gene gun, or the vector may be encapsulated in a liposome preparation. The subject may be a mammal, e.g., a human.

In another aspect the present invention includes, a method of generating an immune response in a subject. Typically the method comprises transfecting cells of the subject using a gene delivery vector (e.g., as described above), under conditions that permit the expression of the polynucleotide and production of the polypeptide including two or more HIV polypeptides from different subtypes, thereby eliciting an immunological response to the polypeptide. Exemplary gene delivery vectors include, but are not limited to, nonviral vectors, particulate carriers, and viral vectors (e.g., retroviral or lentiviral vectors). The gene delivery vectors may, for example, be coated on a gold or tungsten particle and the coated particle delivered to the subject using a gene gun, or the vector may be encapsulated in a liposome preparation. The subject may be a mammal, e.g., a human. Cells of the subject may be transfected *ex vivo* and the transfected cells reintroduced into the subject. Alternately, the transfecting may be done *in vivo* in the subject. The immune response that is generated may, for example, be a humoral immune response and/or a cellular immune response.

Gene delivery vectors may be administered, for example, intramuscularly, intramucosally, intranasally, subcutaneously, intradermally, transdermally, intravaginally, intrarectally, orally or intravenously.

These and other embodiments of the present invention will readily occur to those of ordinary skill in the art in view of the disclosure herein.

### BRIEF DESCRIPTION OF THE FIGURES

Figures 1A to 1D depict the nucleotide sequence of HIV Type C 8_5_TV1_C.ZA (SEQ ID NO:1; referred to herein as TV1). Various regions are shown in Table A.
Figures 2A-C depicts an alignment of Env polypeptides from various HIV isolates (SF162, SEQ ID NO:2; TV1.8_2, SEQ ID NO:3; TV1.8_5, SEQ ID NO:4; TV2.12-5/1, SEQ ID NO:5; Consensus Sequence, SEQ ID NO:6). The regions between the arrows indicate regions (of TV and TV2 clones, both HIV Type C isolates) in the beta and/or bridging sheet region(s) that can be deleted and/or truncated. The "*" denotes N-linked glycosylation sites (of TV1 and TV2 clones), one or more of which can be modified (e.g., deleted and/or mutated).
Figure 3 presents a schematic diagram showing the relationships between the following forms of the HIV Env polypeptide: gp 160, gp140, gp 120, and gp41.
Figure 4 presents exemplary data concerning transactivation activity of Tat mutants on LTR-CAT plasmid expression in 293 cells.
Figure 5 presents exemplary data concerning export activity of Rev mutants monitored by CAT expression.

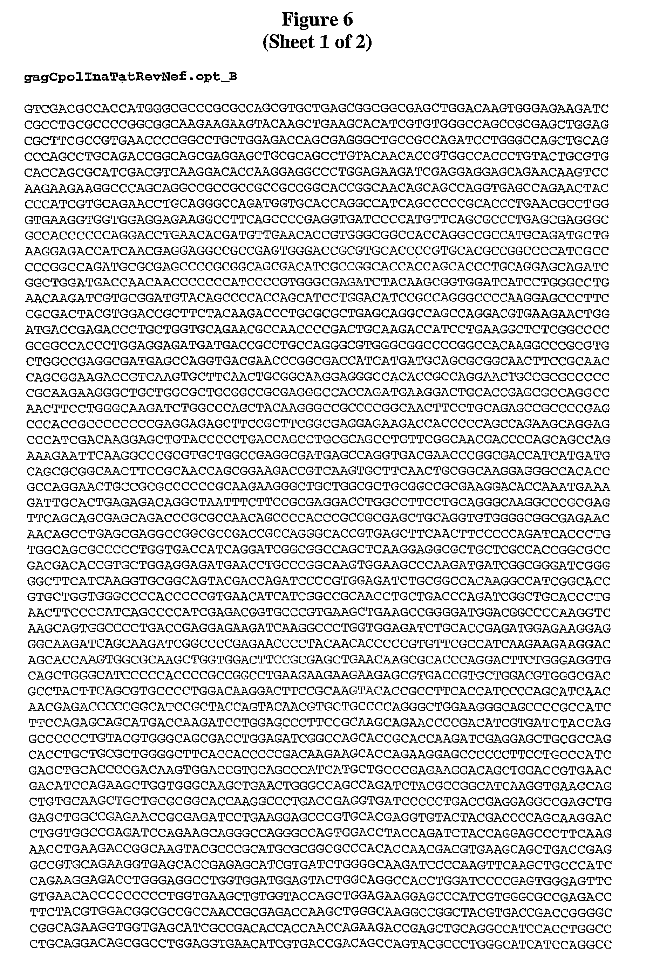

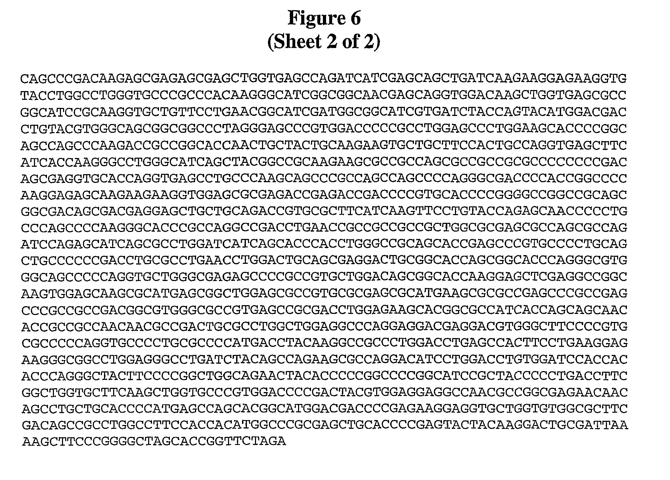

Figure 6, sheets 1 and 2, presents the sequence of the construct gagCpolInaTatRevNef.opt_B (SEQ ID NO:9).

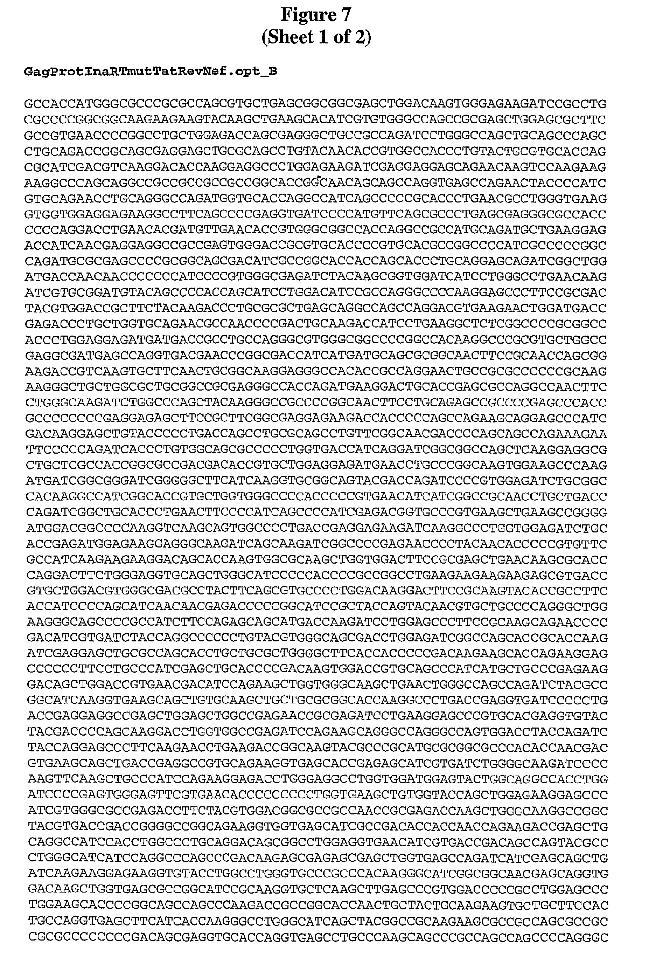

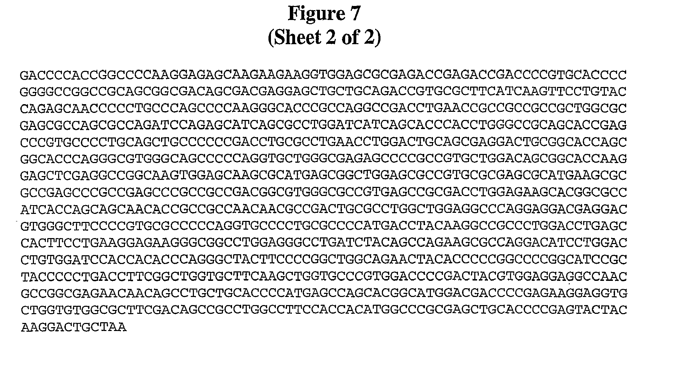

Figure 7, sheets 1 and 2, presents the sequence of the construct GagProtInaRTmutTatRevNef.opt_B (SEQ ID NO:10).

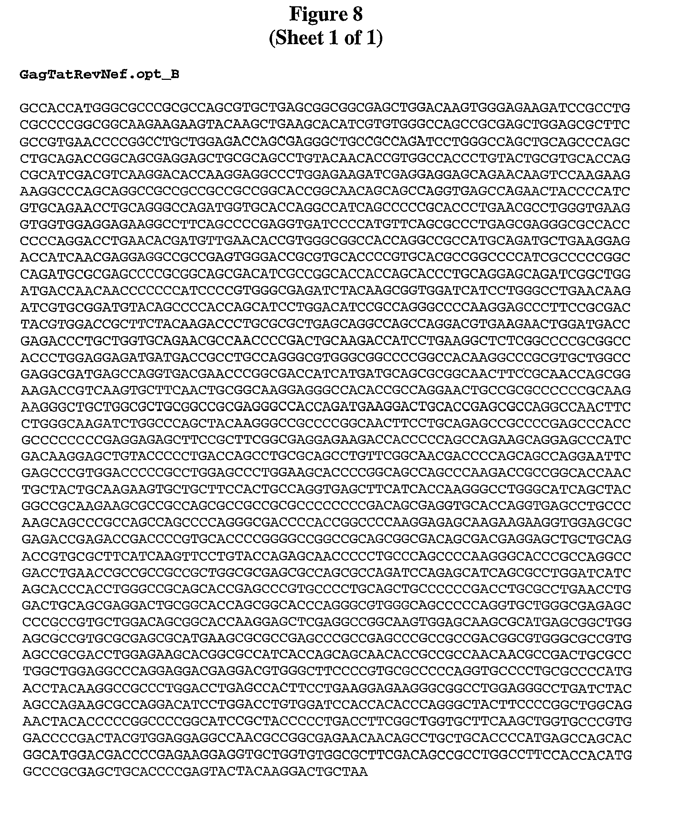

Figure 8, presents the sequence of the construct GagTatRevNef.opt_B (SEQ ID NO:11)*.*

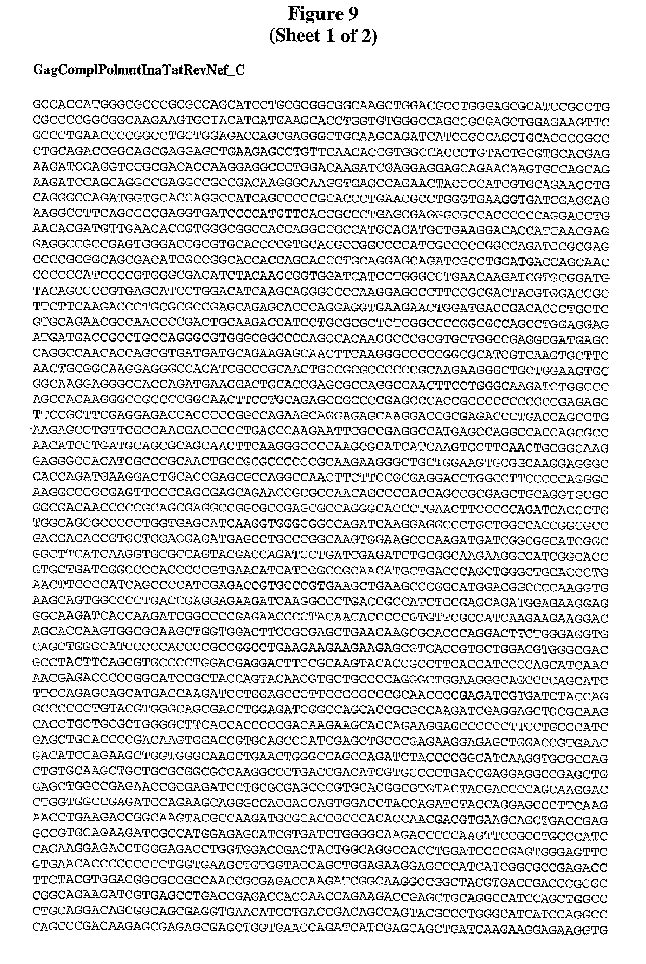

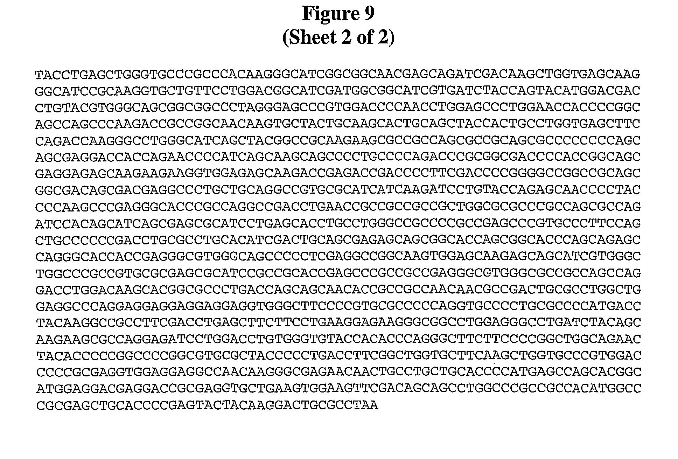

Figure 9, sheets 1 and 2, presents the sequence of the construct GagComplPolmutInaTatRevNef_C (SEQ ID NO:12).

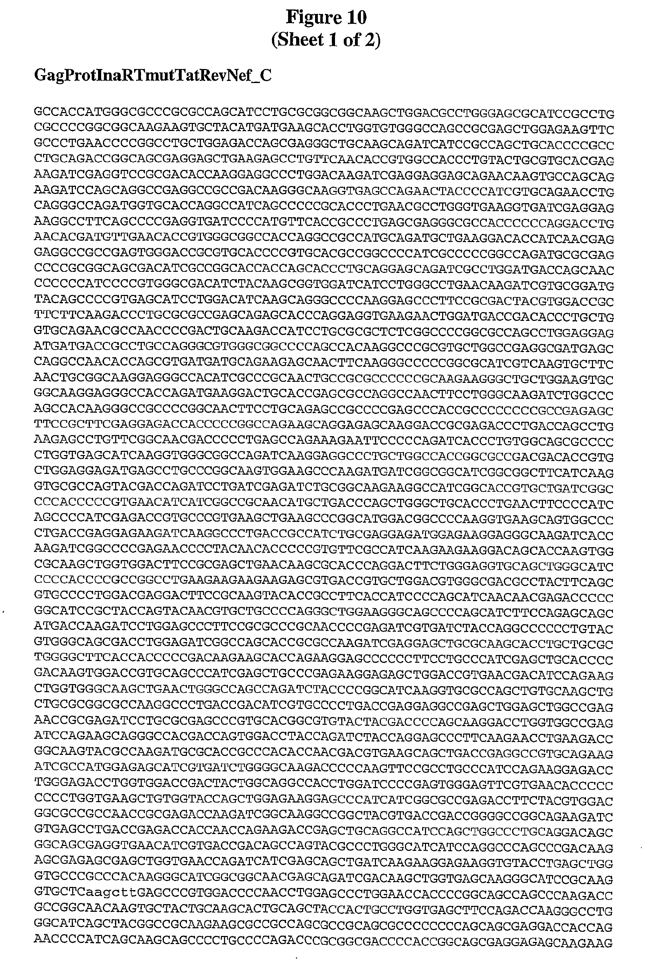

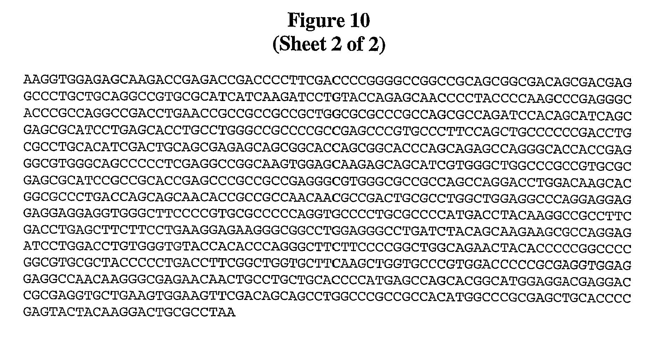

Figure 10, sheets 1 and 2, presents the sequence of the construct GagProtInaRTmutTatRevNef_C (SEQ ID NO:13).

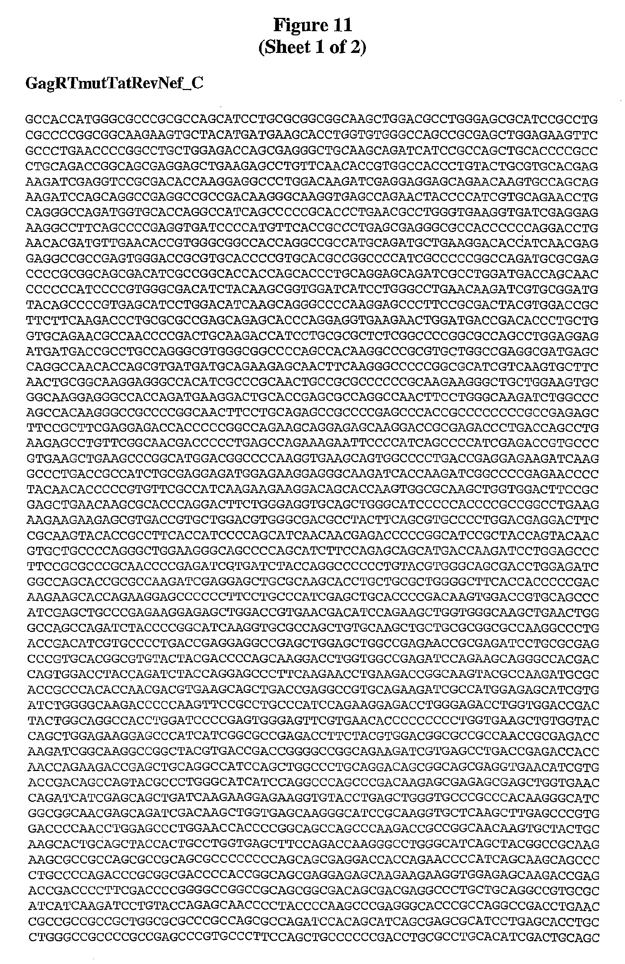

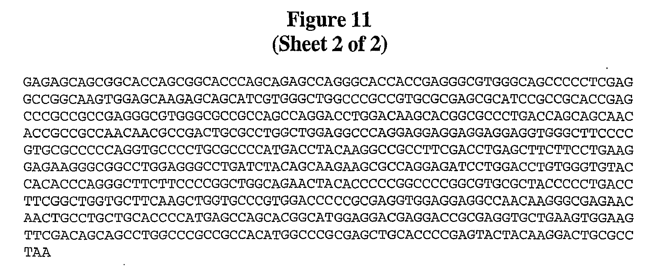

Figure 11, sheets 1 and 2, presents the sequence of the construct GagRTmutTatRevNef_C (SEQ ID NO:14).

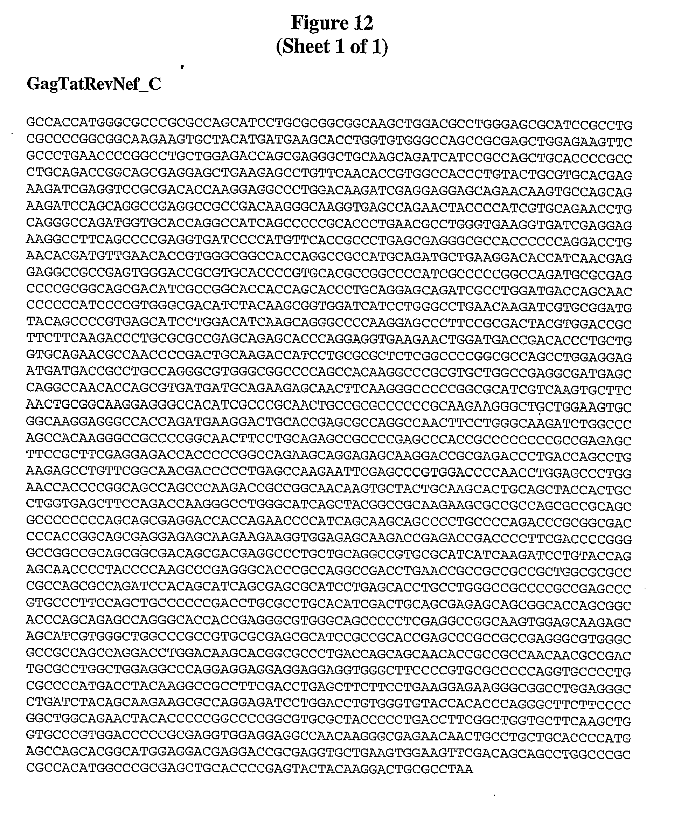

Figure 12, presents the sequence of the construct GagTatRevNef_C (SEQ ID NO:15).

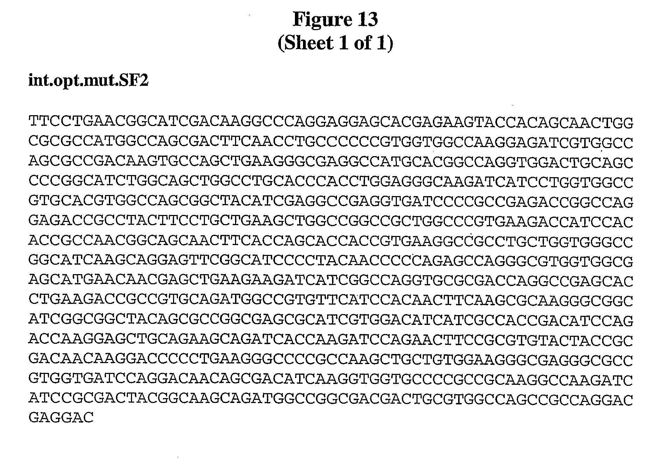

Figure 13, presents the sequence of the construct int.opt.mut.SF2 (SEQ ID NO:16).

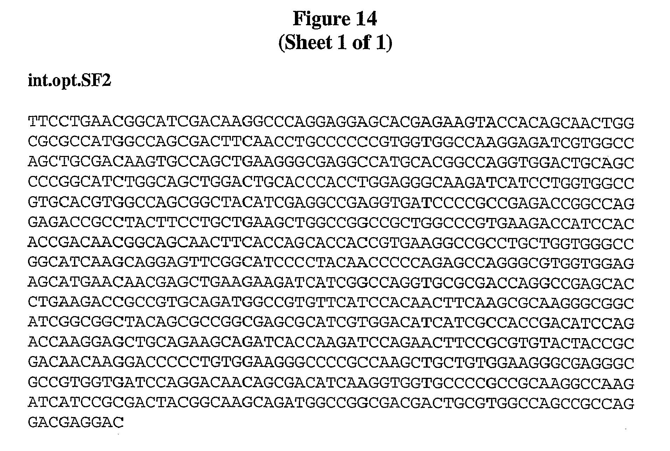

Figure 14, presents the sequence of the construct int.opt.SF2 (SEQ ID NO:17).

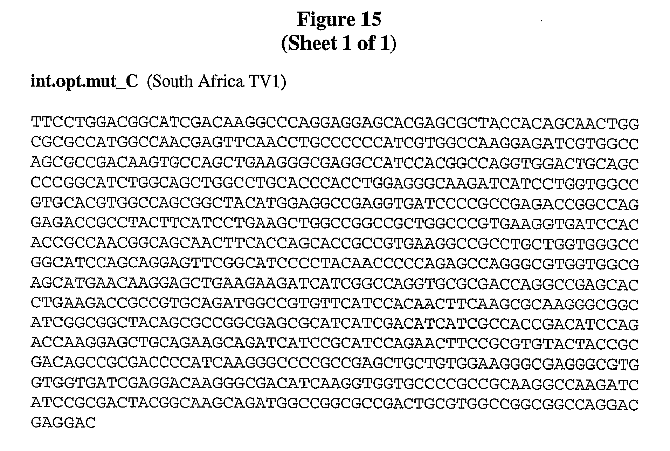

Figure 15, presents the sequence of the construct int.opt.mut_C (SEQ ID NO:18).

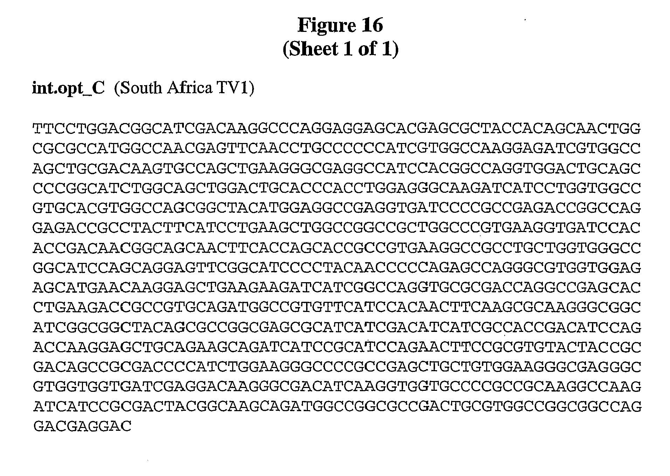

Figure 16, presents the sequence of the construct int.opt_C (SEQ ID NO:19).

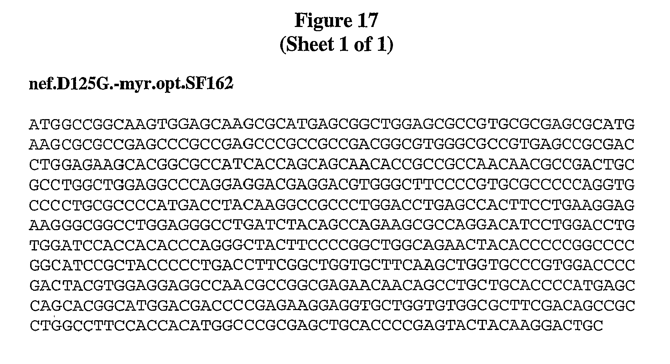

Figure 17, presents the sequence of the construct nef.D125G.-myr.opt.SF162 (SEQ ID NO:20).

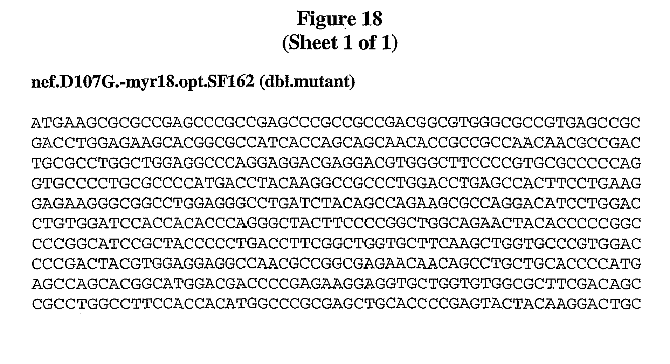

Figure 18, presents the sequence of the construct nef.D107G.-myr18.opt.SF162 (SEQ ID NO:21).

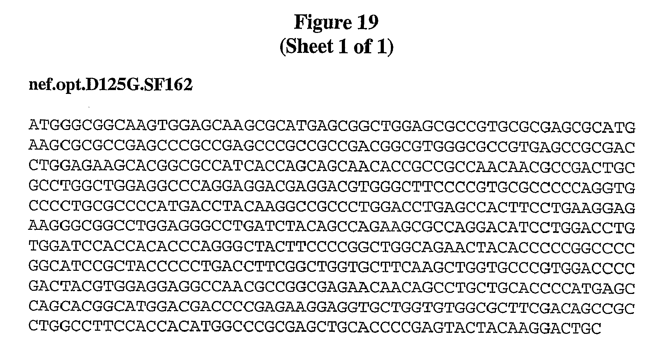

Figure 19, presents the sequence of the construct nef.opt.D125G.SF162 (SEQ ID NO:22).

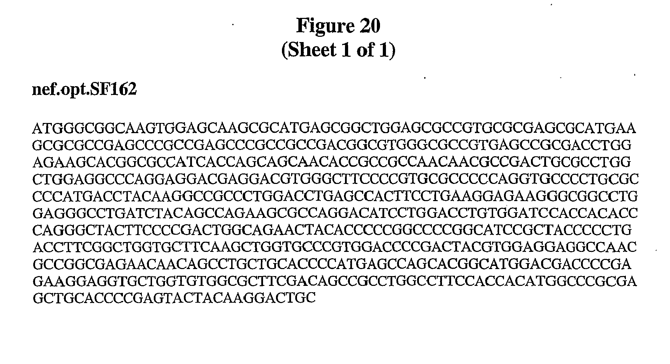

Figure 20, presents the sequence of the construct nef.opt.SF162 (SEQ ID NO:23).

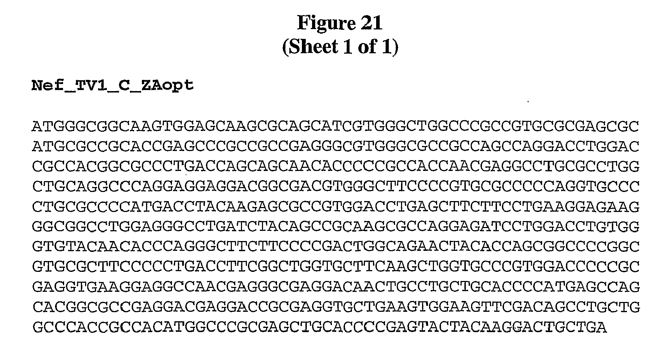

Figure 21, presents the sequence of the construct Nef_TV1_C_ZAopt (SEQ ID NO:24).

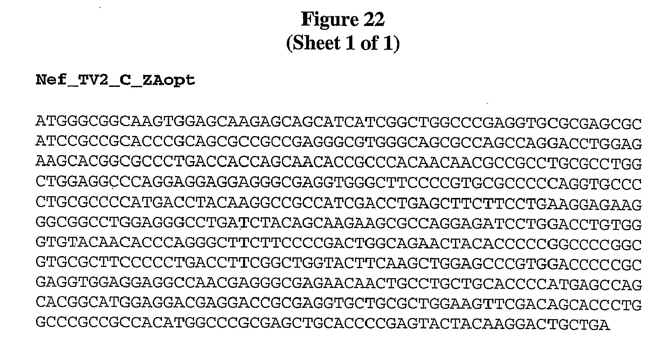

Figure 22, presents the sequence of the construct Nef_TV2_C_ZAopt (SEQ ID NO:25).

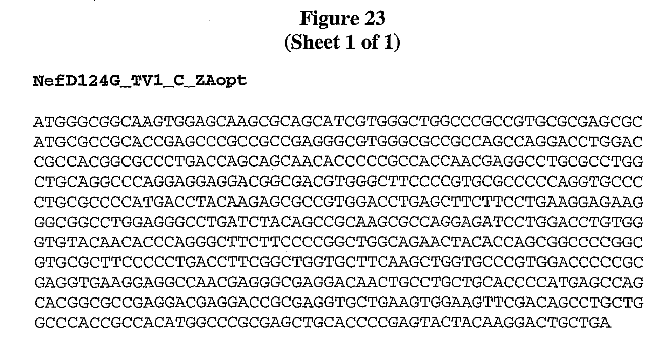

Figure 23, presents the sequence of the construct NefD124G_TV1_C_ZAopt (SEQ ID NO:26).

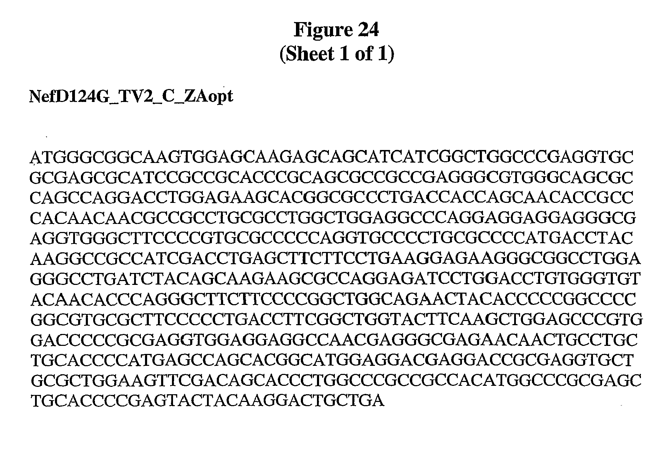

Figure 24, presents the sequence of the construct NefD124G_TV2_C_ZAopt (SEQ ID NO:27).

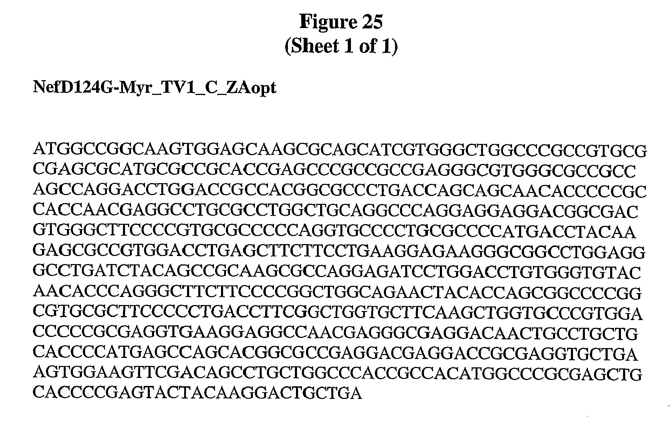

Figure 25, presents the sequence of the construct NefD124G-Myr_TV1_C_ZAopt (SEQ ID NO:28).

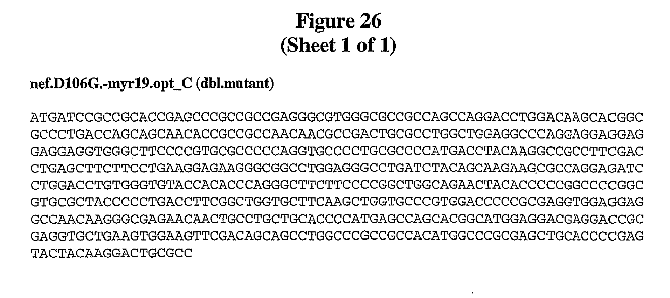

Figure 26, presents the sequence of the construct nef.D106G.-myr19.opt_C (SEQ ID NO:29).

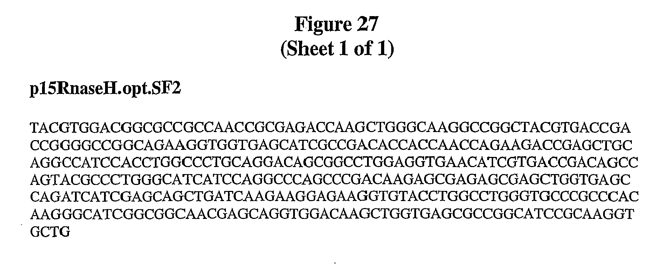

Figure 27, presents the sequence of the construct p15RnaseH.opt.SF2 (SEQ ID NO:30).

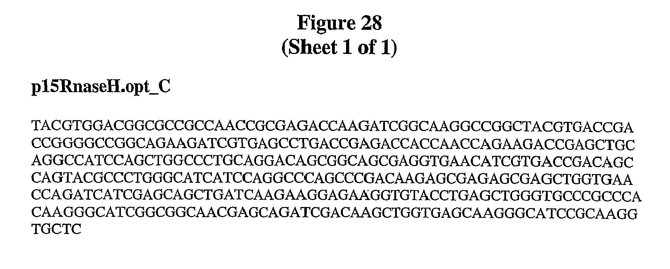

Figure 28, presents the sequence of the construct p15RnaseH.opt_C (SEQ ID NO:31).

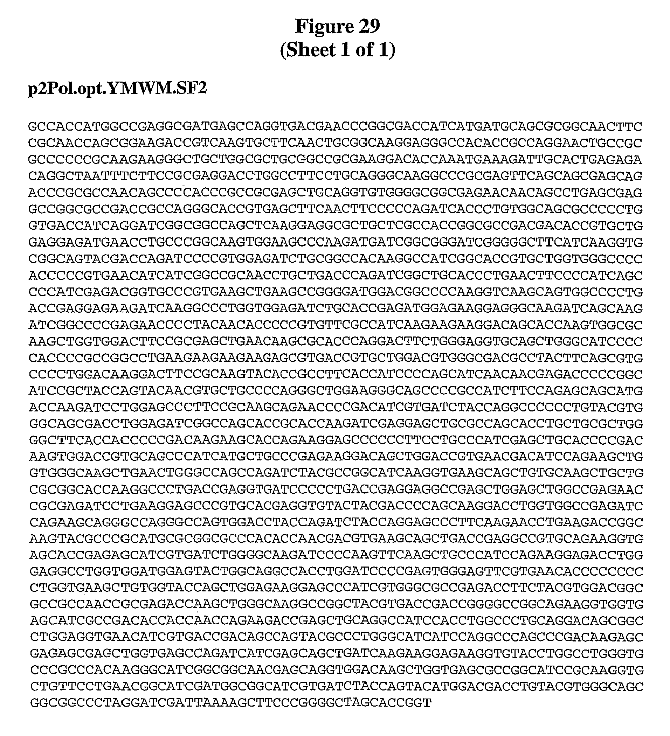

Figure 29, presents the sequence of the construct p2Pol.opt.YMWM.SF2 (SEQ ID NO:32).

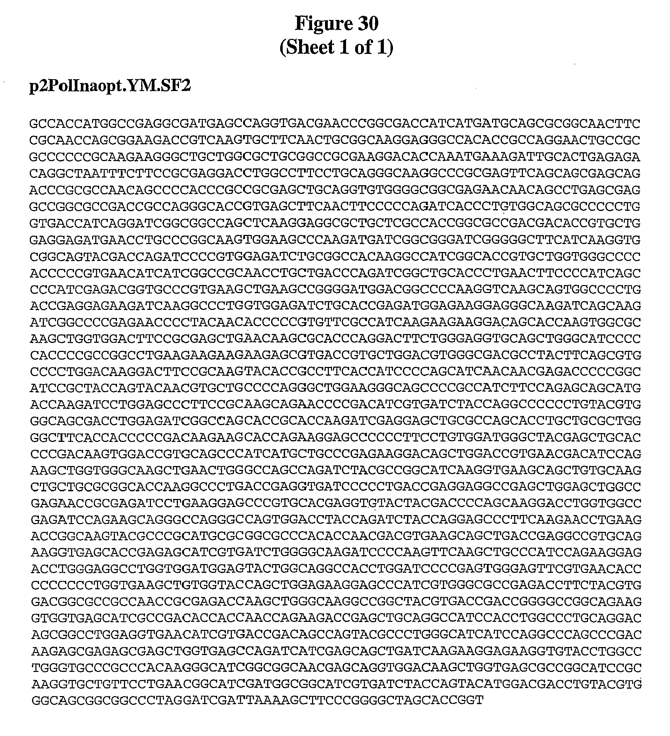

Figure 30, presents the sequence of the construct p2PolInaopt.YM.SF2 (SEQ ID NO:33).

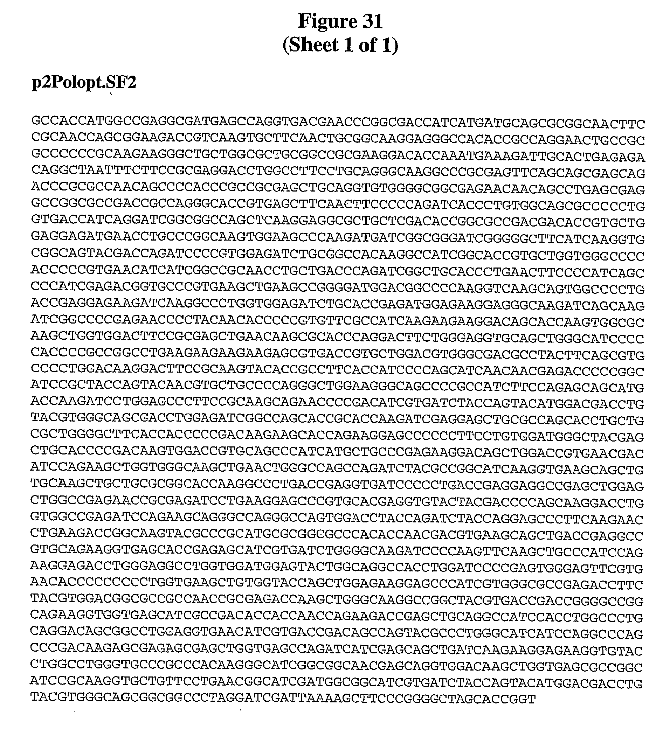

Figure 31, presents the sequence of the construct p2Polopt.SF2 (SEQ ID NO34).

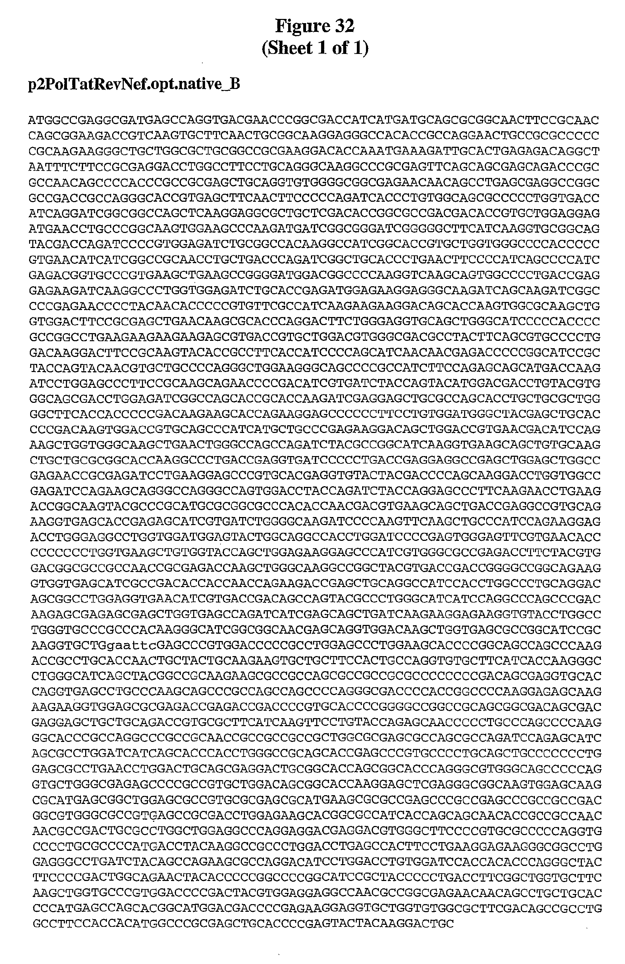

Figure 32, presents the sequence of the construct p2PolTatRevNef.opt.native_B (SEQ ID NO:35).

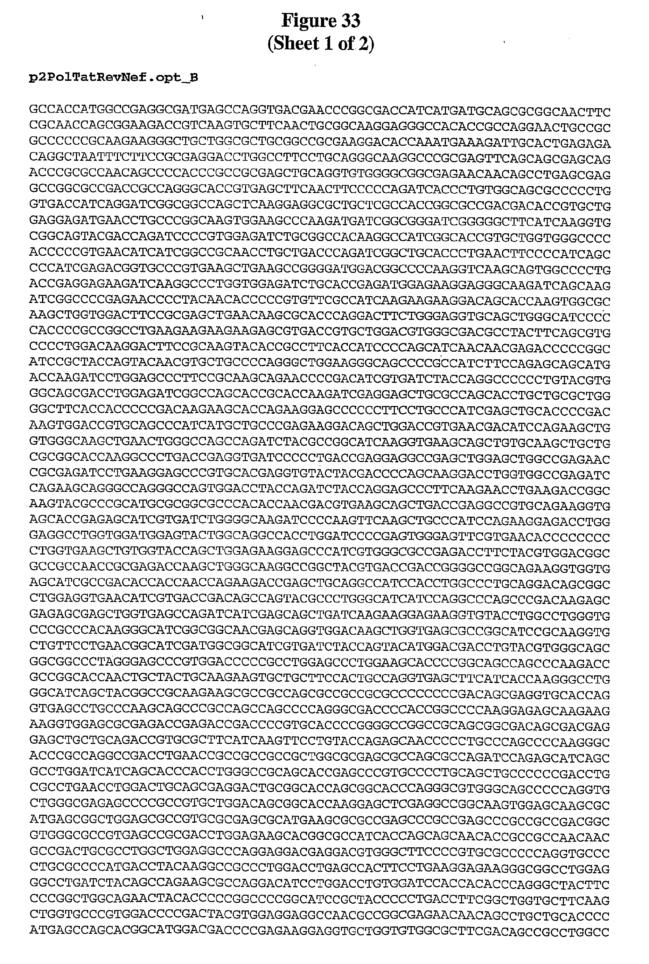

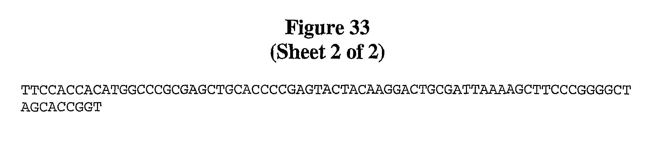

Figure 33, sheets 1 and 2, presents the sequence of the construct p2PolTatRevNef.opt_B (SEQ ID NO:36).

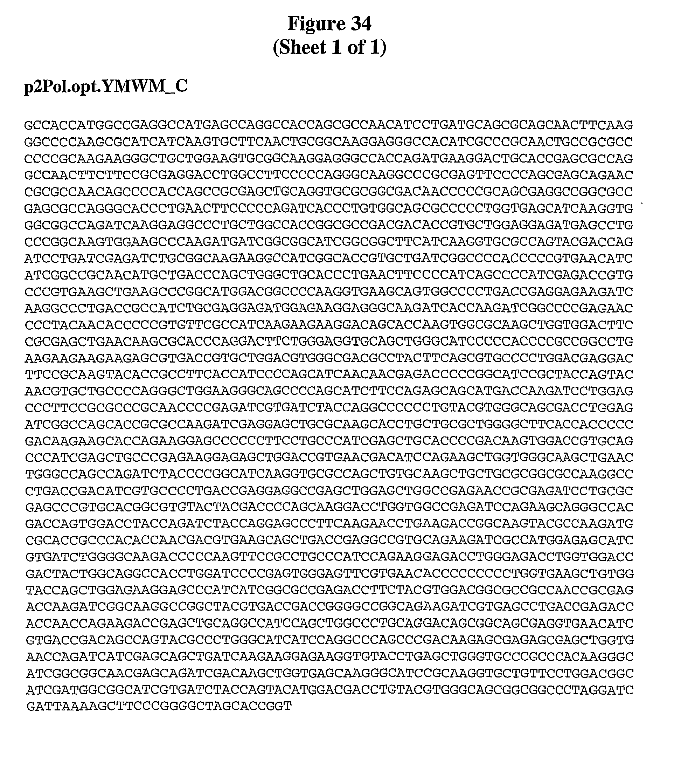

Figure 34, presents the sequence of the construct p2Pol.opt.YMWM_C (SEQ ID NO:37).

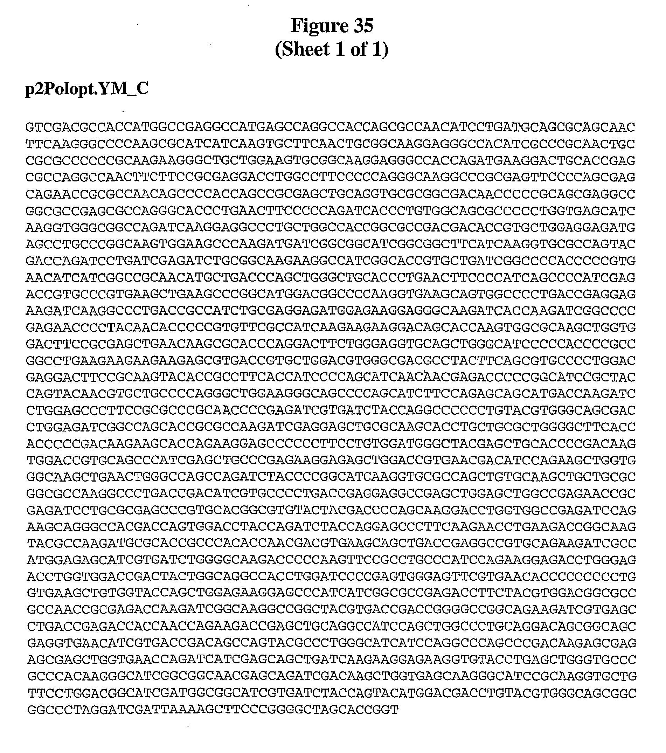

Figure 35, presents the sequence of the construct p2Polopt.YM_C (SEQ ID NO:38).

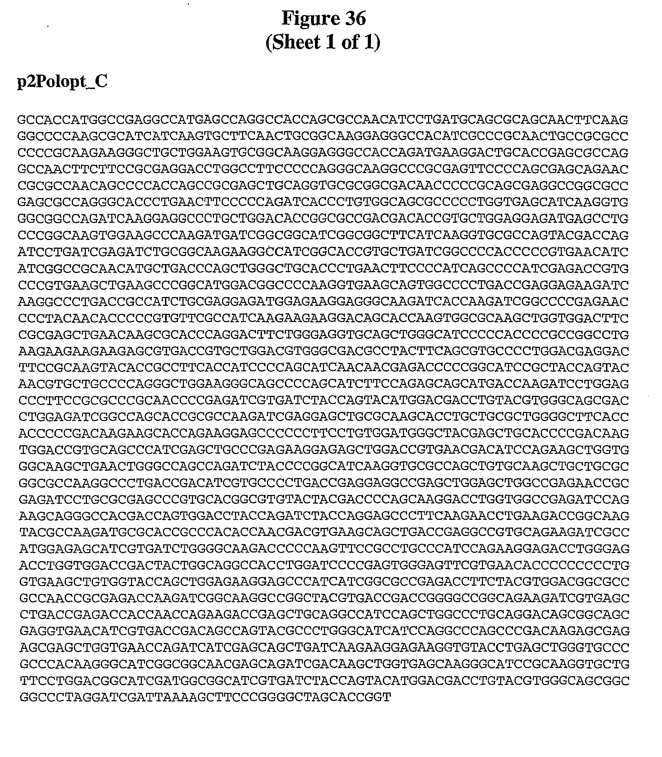

Figure 36, presents the sequence of the construct p2Polopt_C (SEQ ID NO:39).

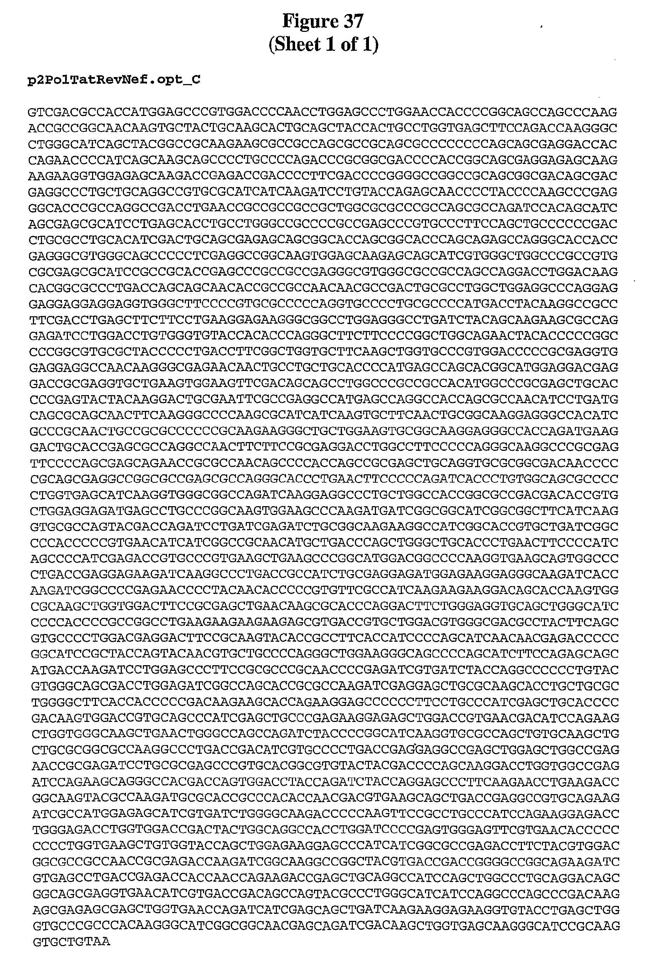

Figure 37, presents the sequence of the construct p2PolTatRevNef opt C (SEQ ID NO:40).

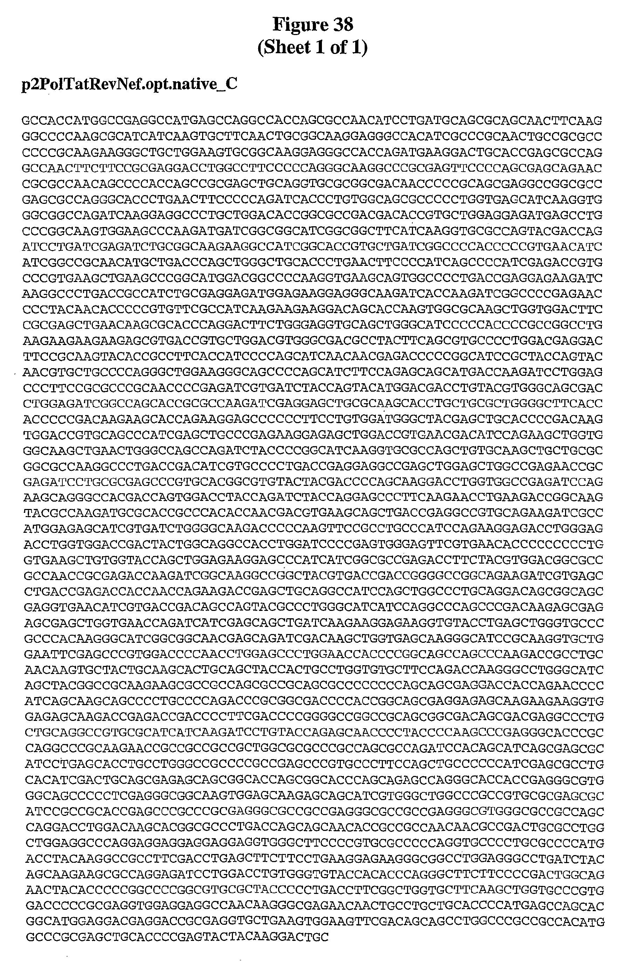

Figure 38, presents the sequence of the construct p2PolTatRevNef.opt.native_C (SEQ ID NO:41).

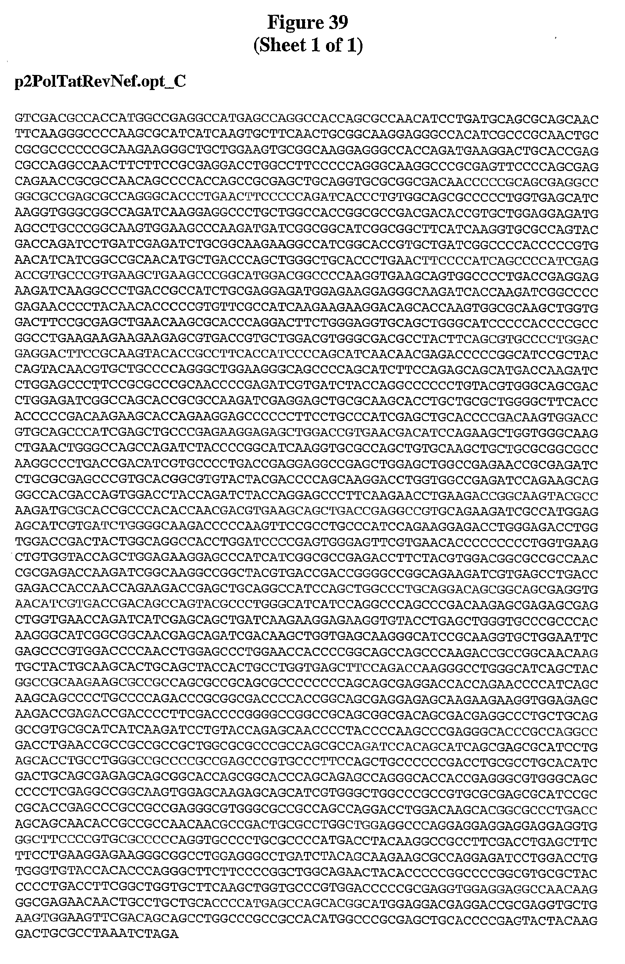

Figure 39, presents the sequence of the construct p2PolTatRevNef.opt_C (SEQ ID NO:42).

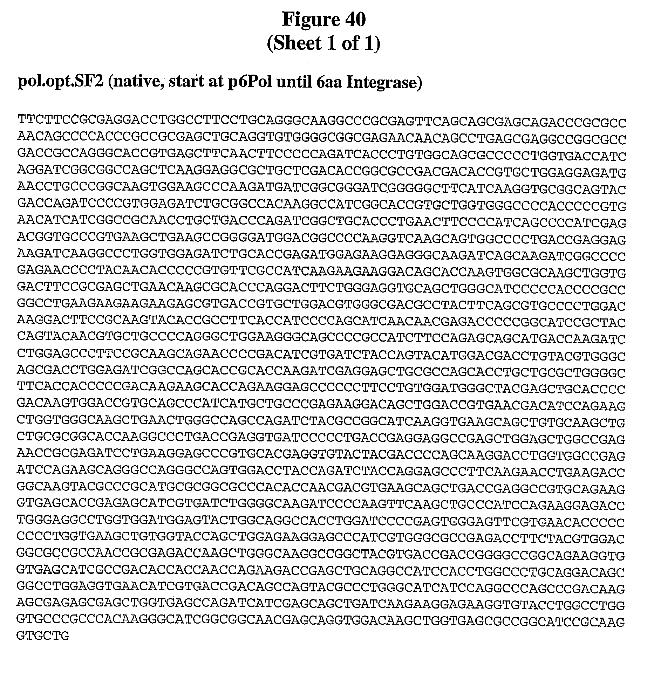

Figure 40, presents the sequence of the construct pol.opt.SF2 (SEQ ID NO:43).

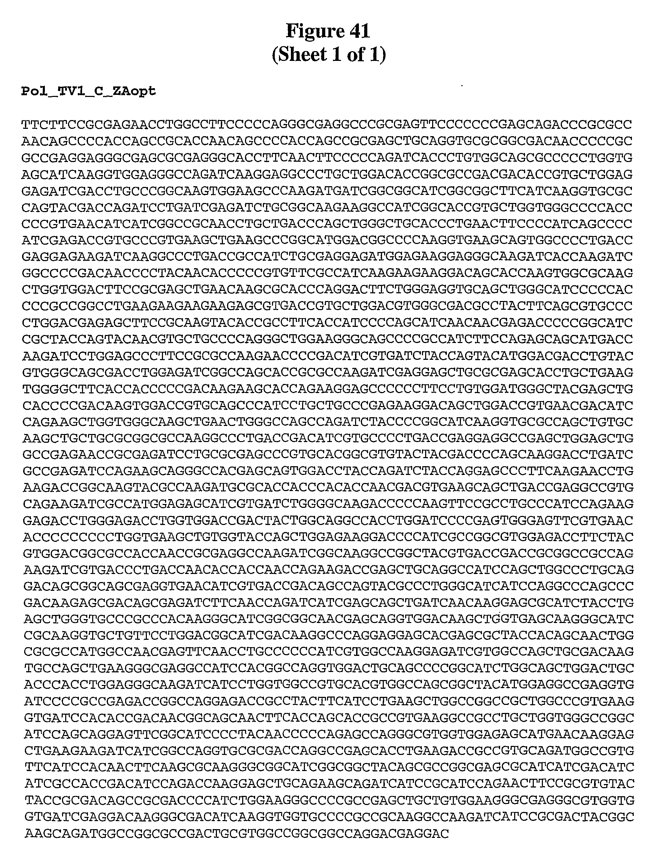

Figure 41, presents the sequence of the construct Pol_TV1_C_ZAopt (SEQ ID NO:44).

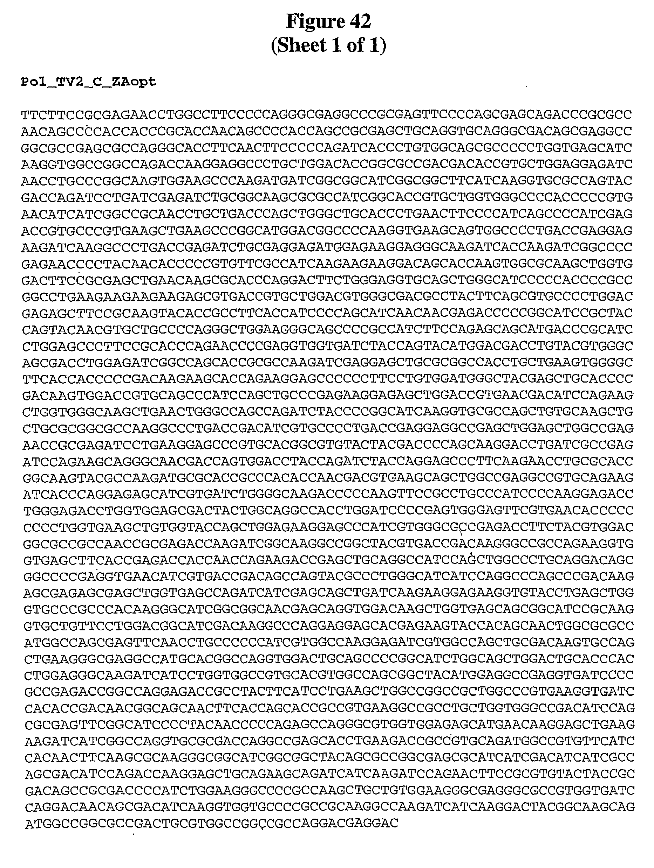

Figure 42, presents the sequence of the construct Pol_TV2_C_ZAopt (SEQ ID NO:45).

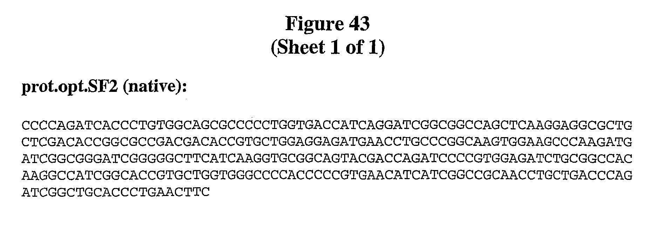

Figure 43, presents the sequence of the construct prot.opt.SF2 (SEQ ID NO:46).

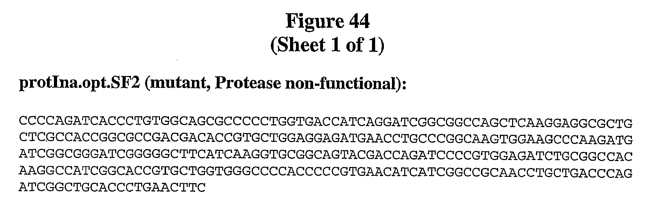

Figure 44, presents the sequence of the construct protIna.opt.SF2 (SEQ ID NO:47).

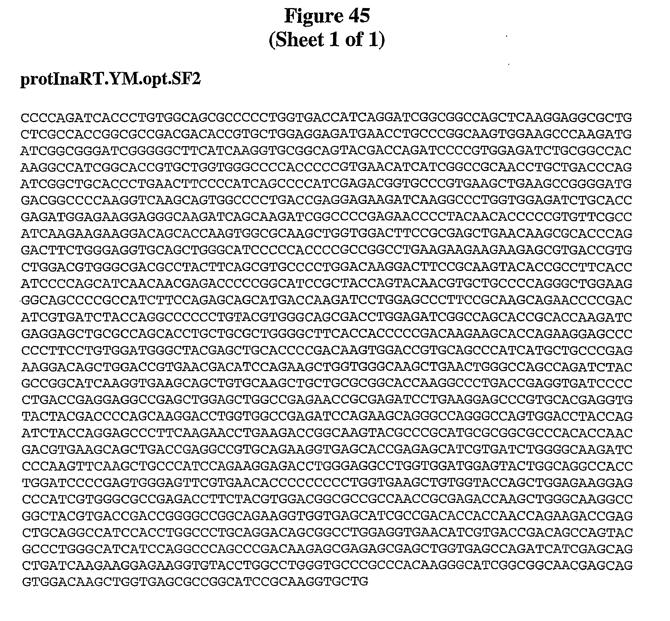

Figure 45, presents the sequence of the construct protInaRT.YM.opt.SF2 (SEQ ID NO:48).

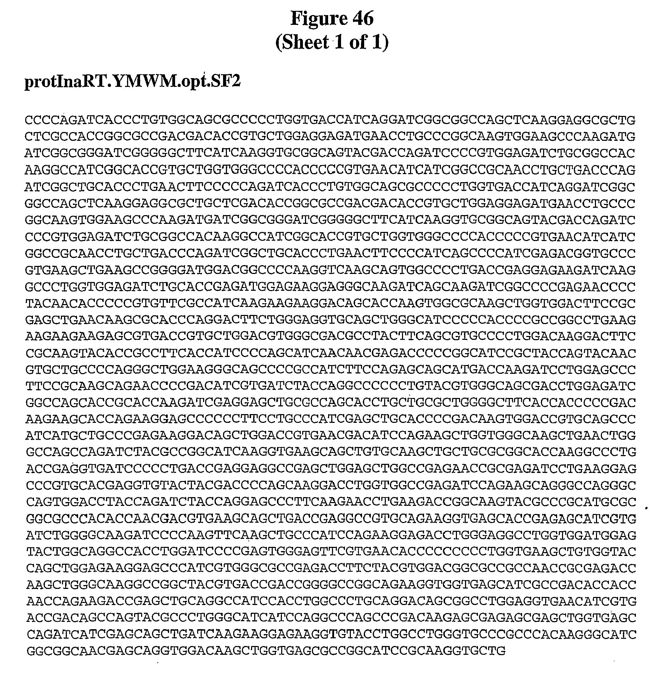

Figure 46, presents the sequence of the construct protInaRT.YMWM.opt.SF2 (SEQ ID NO:49).

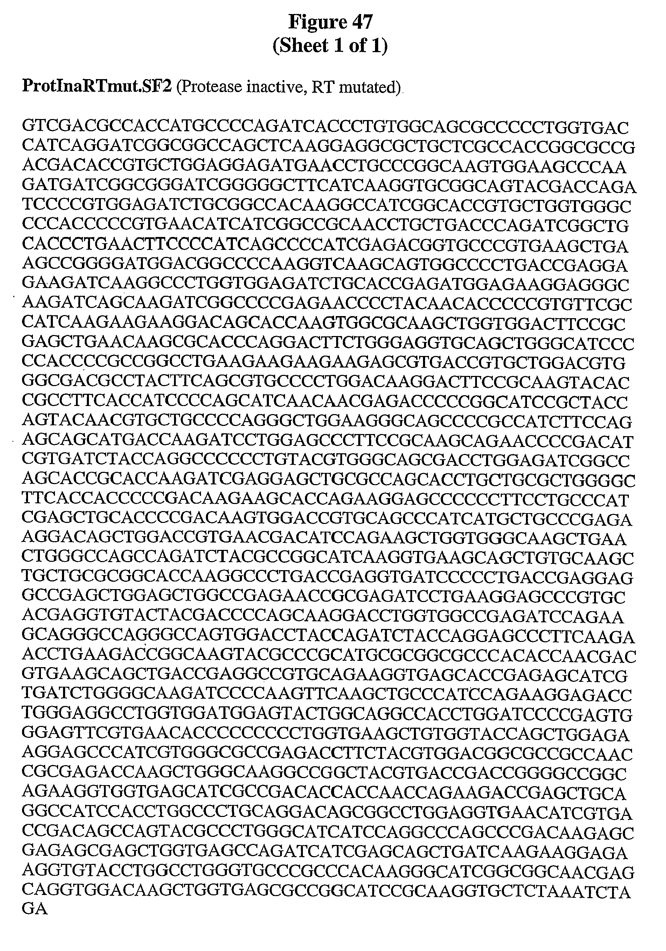

Figure 47, presents the sequence of the construct ProtInaRTmut.SF2 (SEQ ID NO:50).

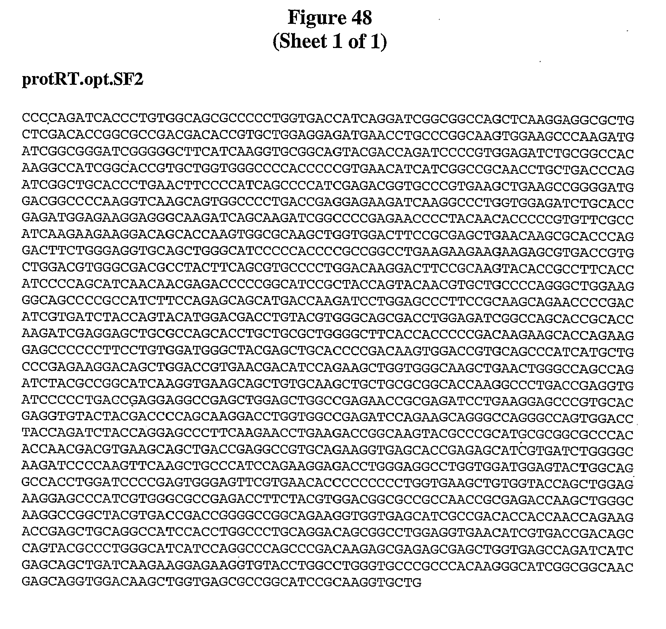

Figure 48, presents the sequence of the construct protRT.opt.SF2 (SEQ ID NO:51).

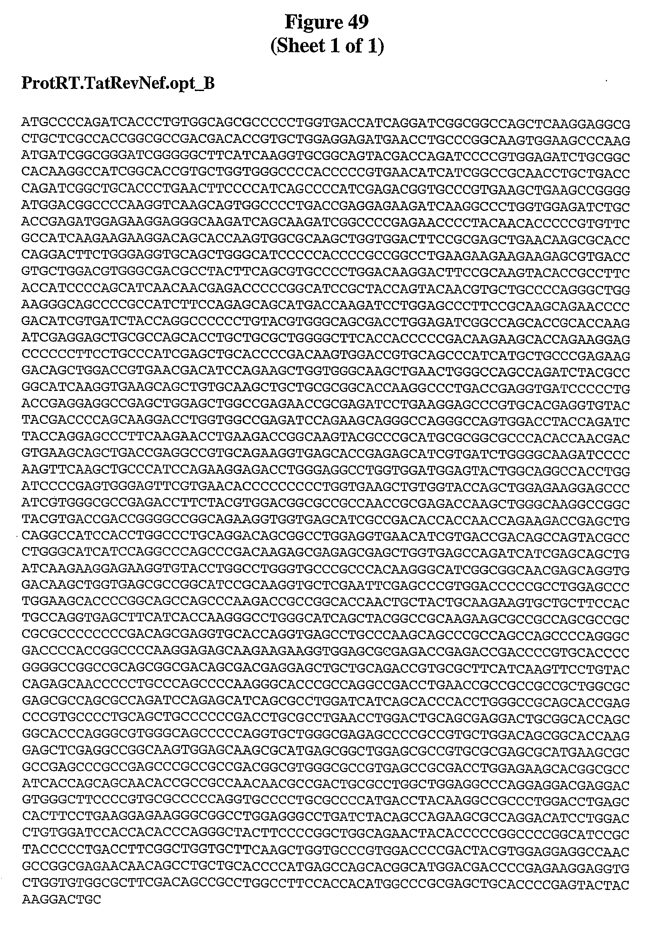

Figure 49, presents the sequence of the construct ProtRT.TatRevNef.opt_B (SEQ ID NO:52).

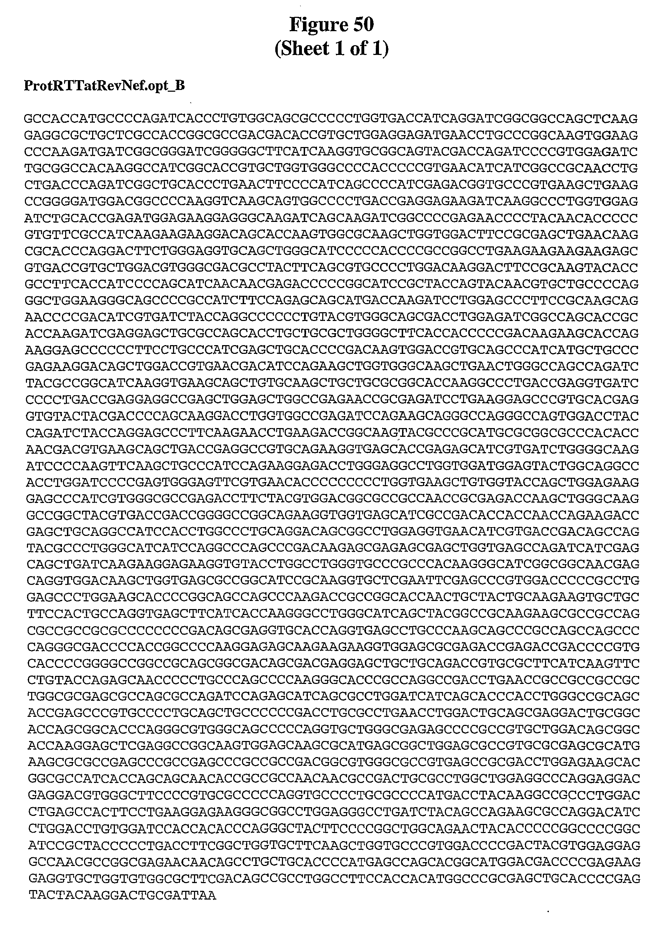

Figure 50, presents the sequence of the construct ProtRTTatRevNef.opt_B (SEQ ID NO:53).

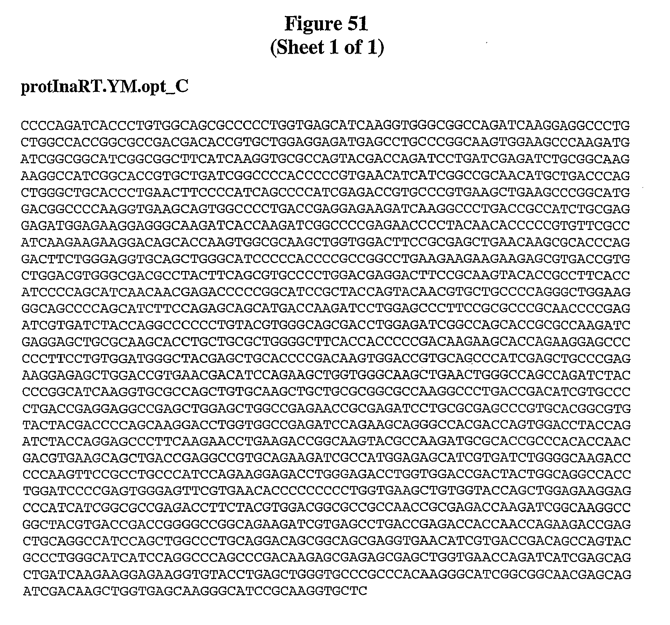

Figure 51, presents the sequence of the construct protInaRT.YM.opt_C (SEQ ID NO:54).

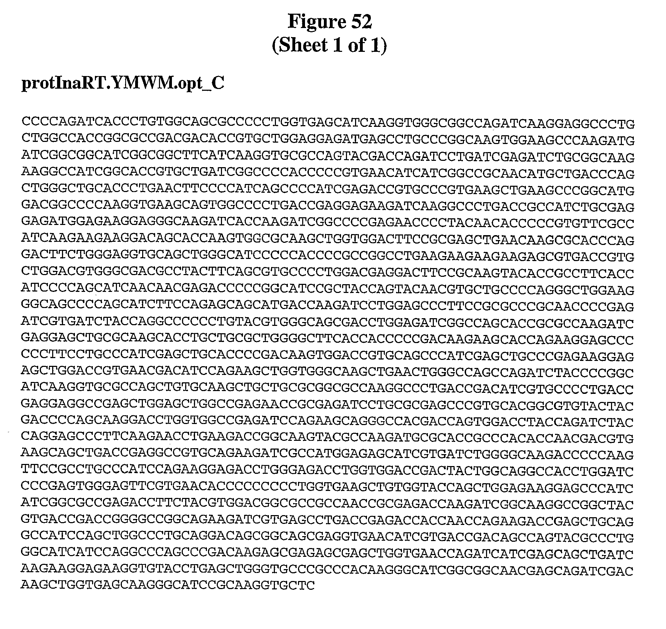

Figure 52, presents the sequence of the construct protInaRT.YMWM.opt_C (SEQ ID NO:55).

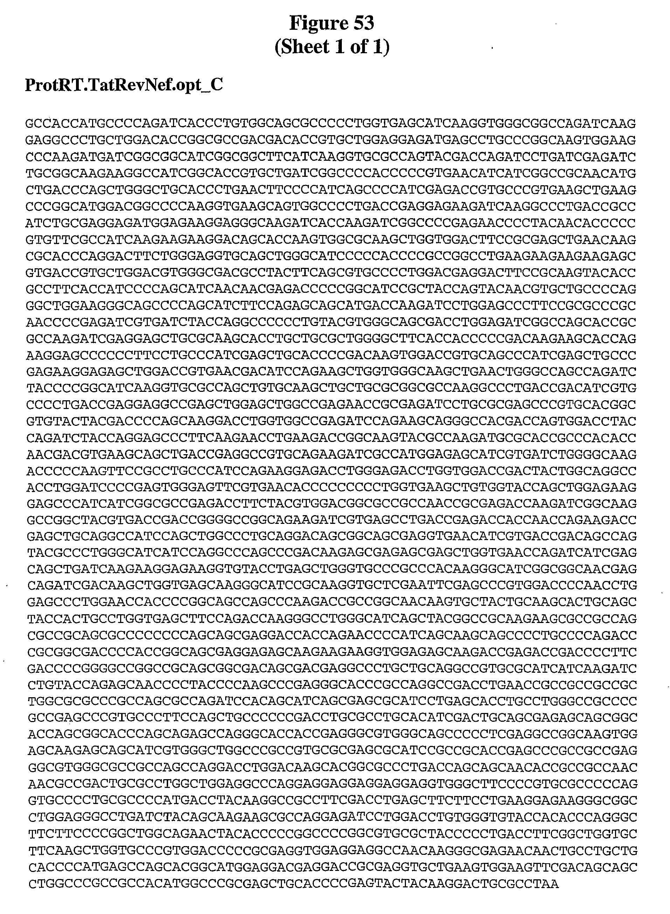

Figure 53, presents the sequence of the construct ProtRT.TatRevNef.opt_C (SEQ ID NO:56).

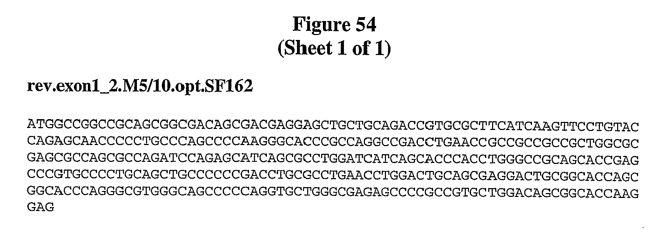

Figure 54, presents the sequence of the construct rev.exon1_2.M5-10.opt.SF162 (SEQ ID NO:57).

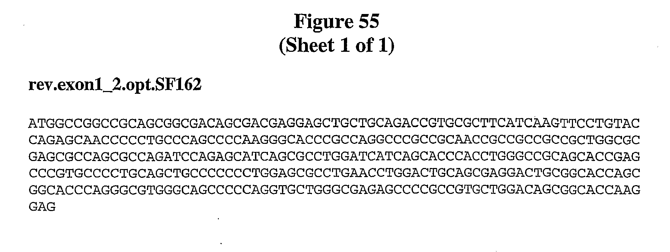

Figure 55, presents the sequence of the construct rev.exon1_2.opt.SF162 (SEQ ID NO:58).

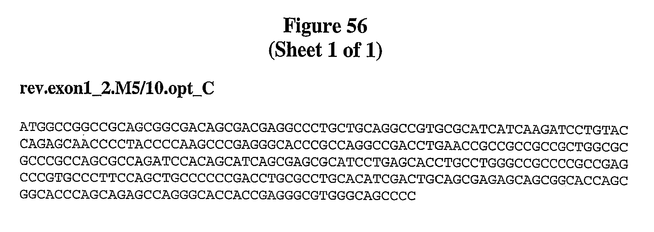

Figure 56, presents the sequence of the construct rev.exon1_2.M5-10.opt_C (SEQ ID NO:59).

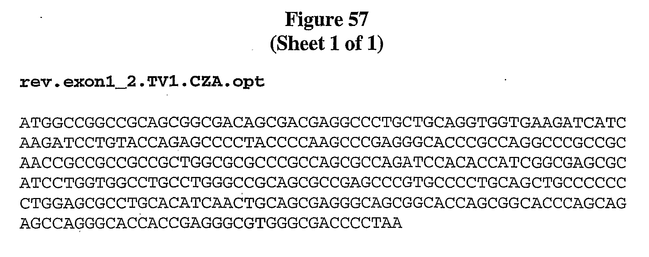

Figure 57, presents the sequence of the construct revexon1_2 TV1 CZAopt (SEQ ID NO:60).

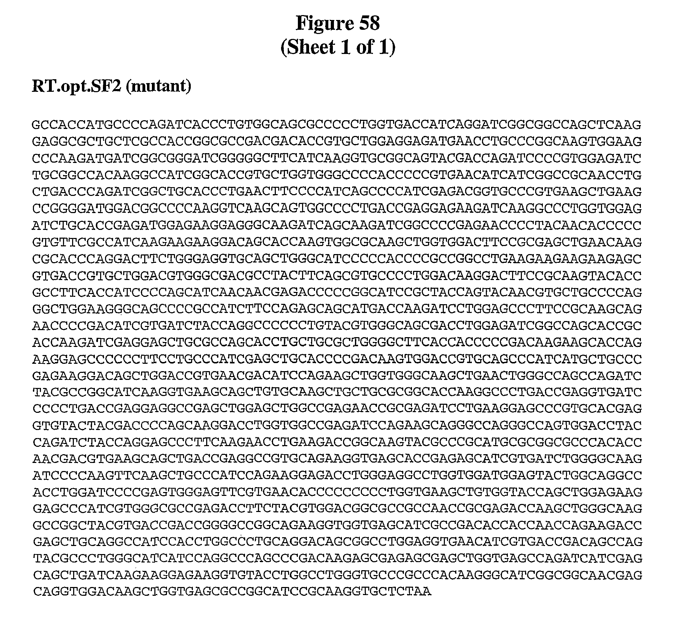

Figure 58, presents the sequence of the construct RT.opt.SF2 (mutant) (SEQ ID NO: 61).

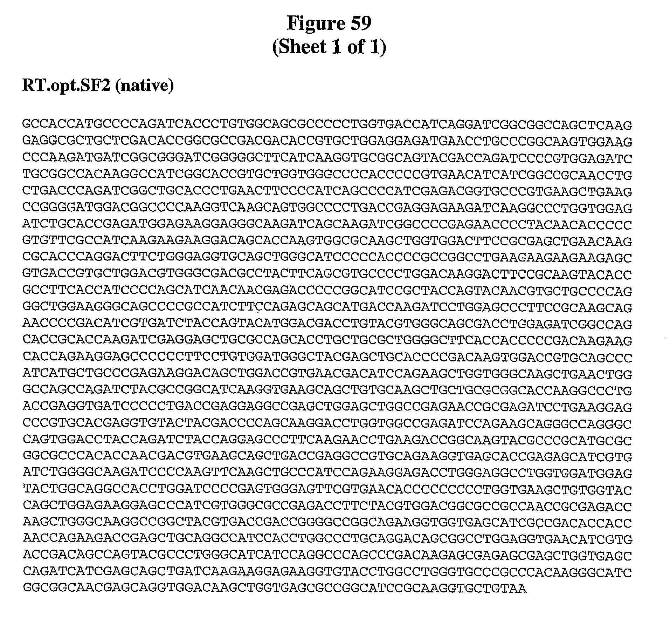

Figure 59, presents the sequence of the construct RT.opt.SF2 (native) (SEQ ID NO:62).

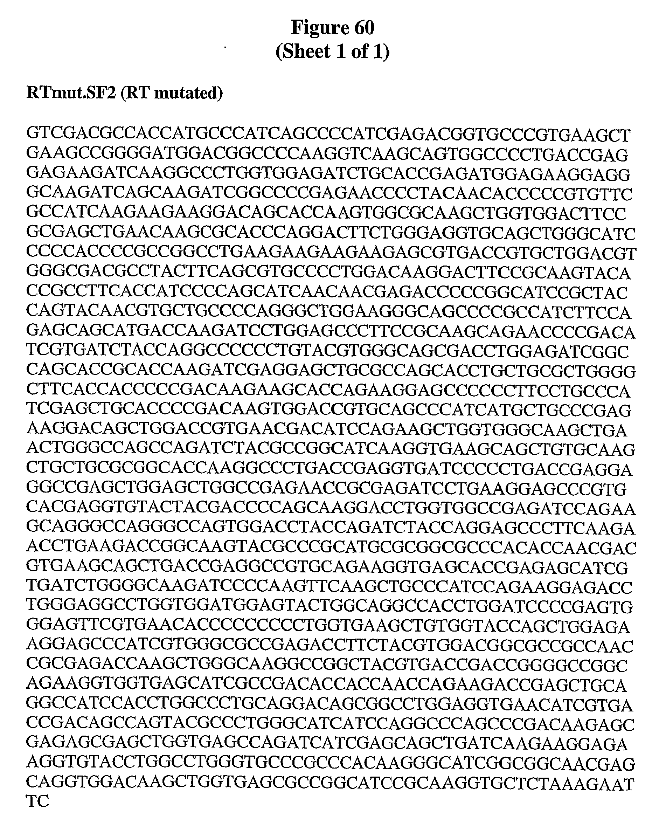

Figure 60, presents the sequence of the construct RTmut.SF2 (SEQ ID NO:63).

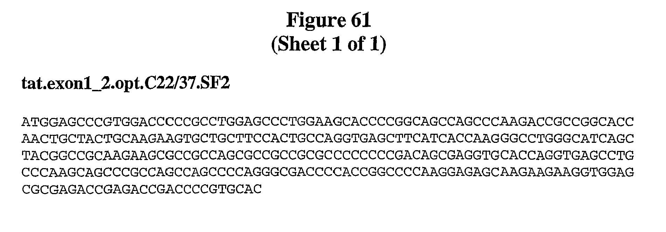

Figure 61, presents the sequence of the construct tat.exon1_2.opt.C22-37.SF2 (SEQ ID NO:64).

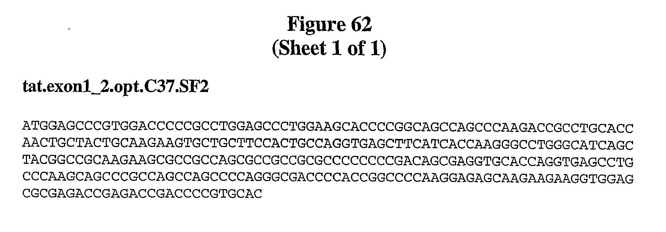

Figure 62, presents the sequence of the construct tat.exon1_2.opt.C37.SF2 (SEQ ID NO:65).

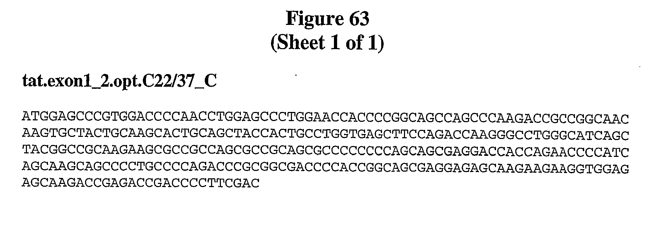

Figure 63, presents the sequence of the construct tat.exon1_2.opt.C22-37_C (SEQ ID NO:66).

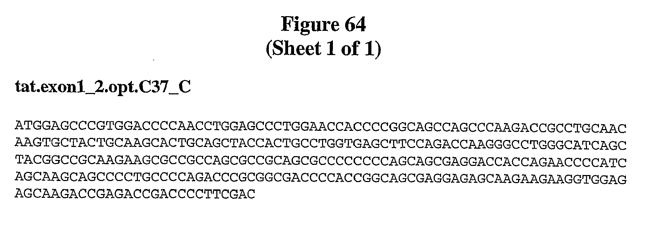

Figure 64, presents the sequence of the construct tat.exon1_2.opt.C37_C (SEQ ID NO:67).

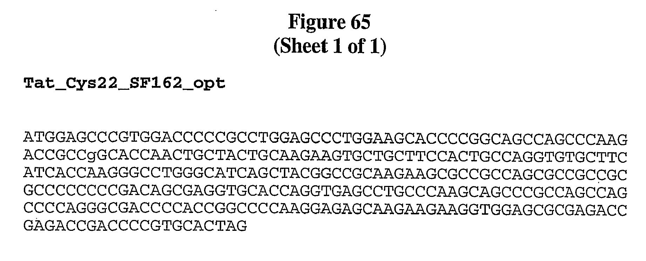

Figure 65, presents the sequence of the construct TAT_CYS22_SF162_OPT (SEQ ID NO:68).

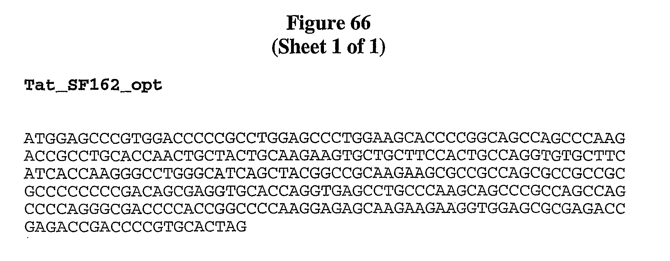

Figure 66, presents the sequence of the construct tat_sf162_opt (SEQ ID NO69).

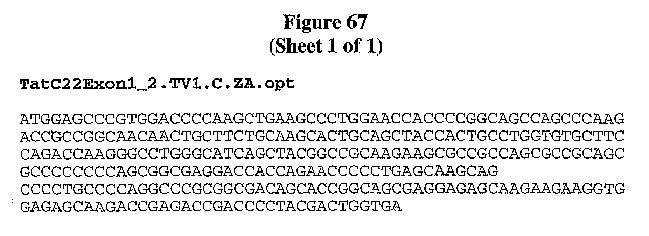

Figure 67, presents the sequence of the construct TatC22Exon1_2_TV1_C_ZAopt (SEQ ID NO:70).

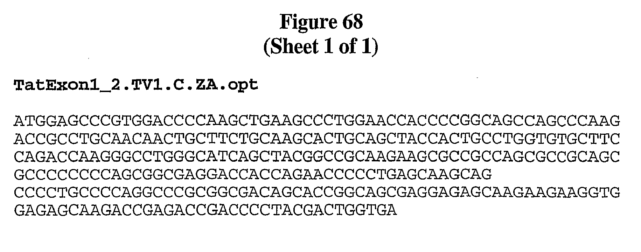

Figure 68, presents the sequence of the construct TatExon1_2_TV1_C_ZAopt (SEQ ID NO:71).

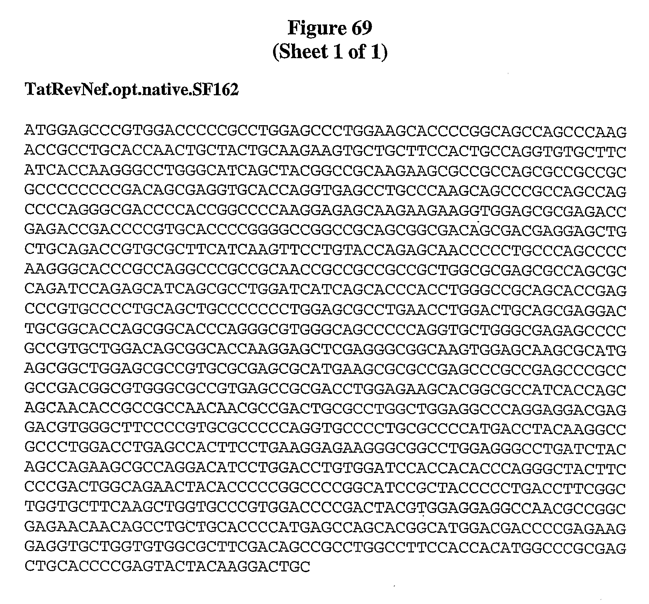

Figure 69, presents the sequence of the construct TatRevNef.opt.native.SF162 (SEQ ID NO:72).

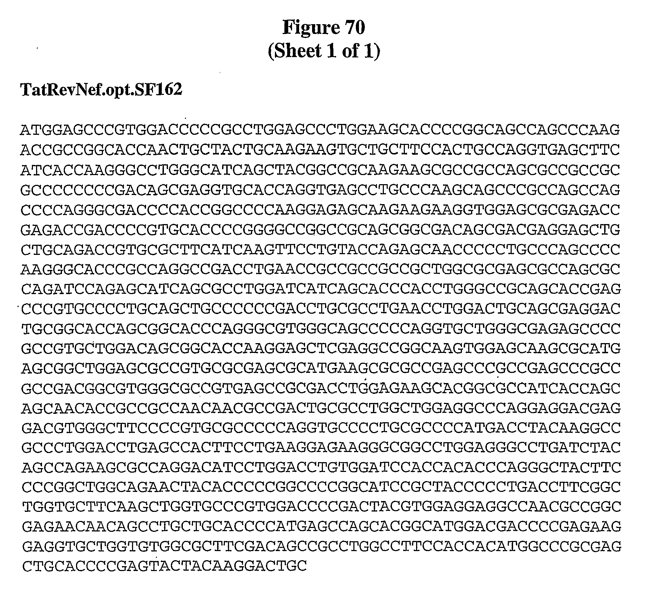

Figure 70, presents the sequence of the construct TatRevNef.opt.SF162 (SEQ ID NO:73).

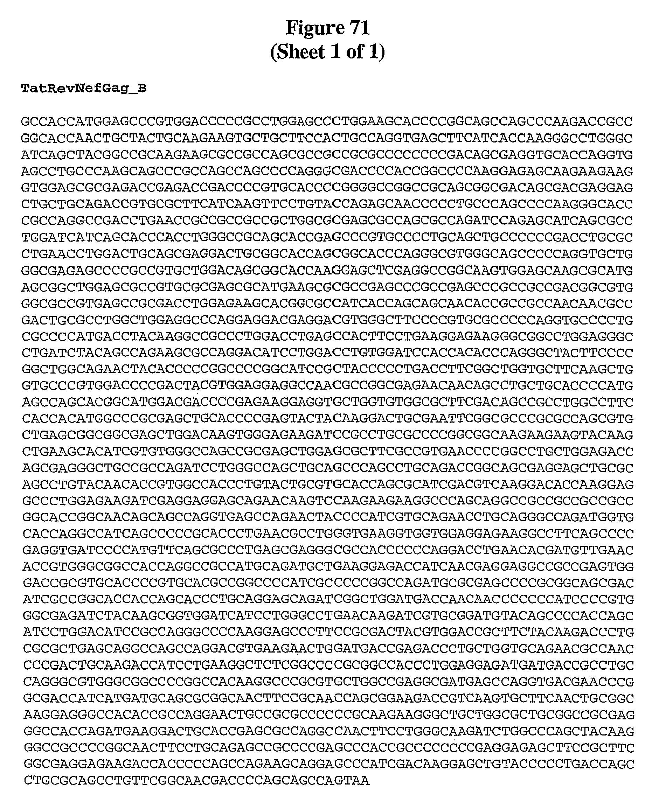

Figure 71, presents the sequence of the construct TatRevNefGag B (SEQ ID NO:74).

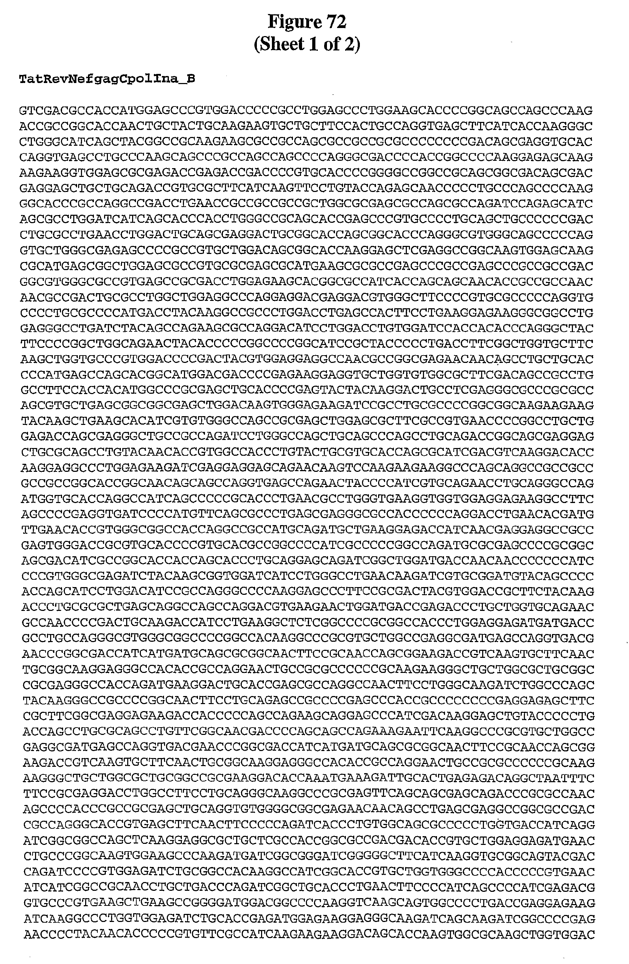

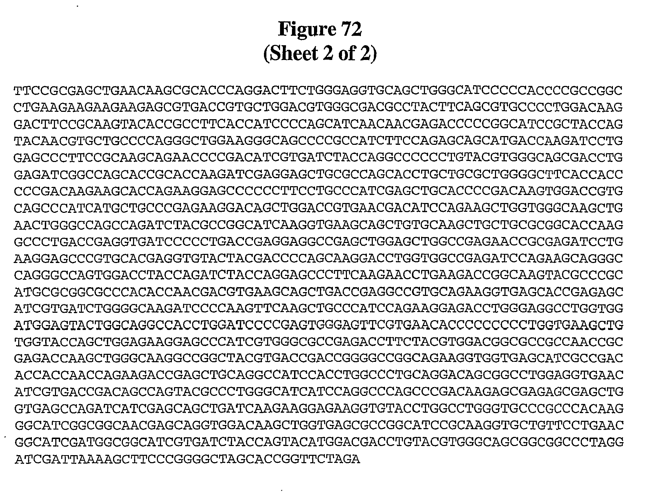

Figure 72, sheets 1 and 2, presents the sequence of the construct TatRevNefgagCpolIna B (SEQ ID NO:75).

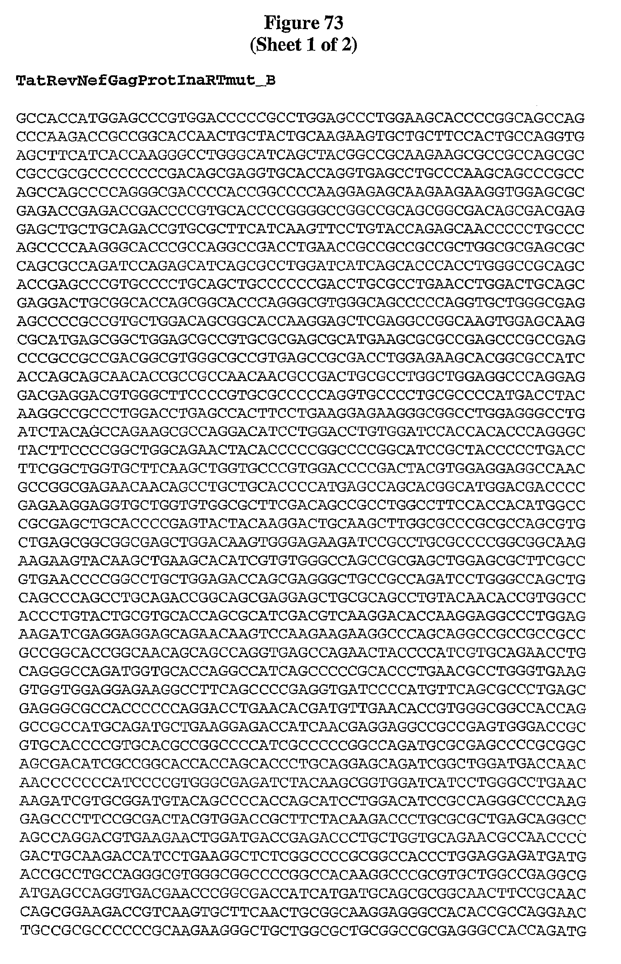

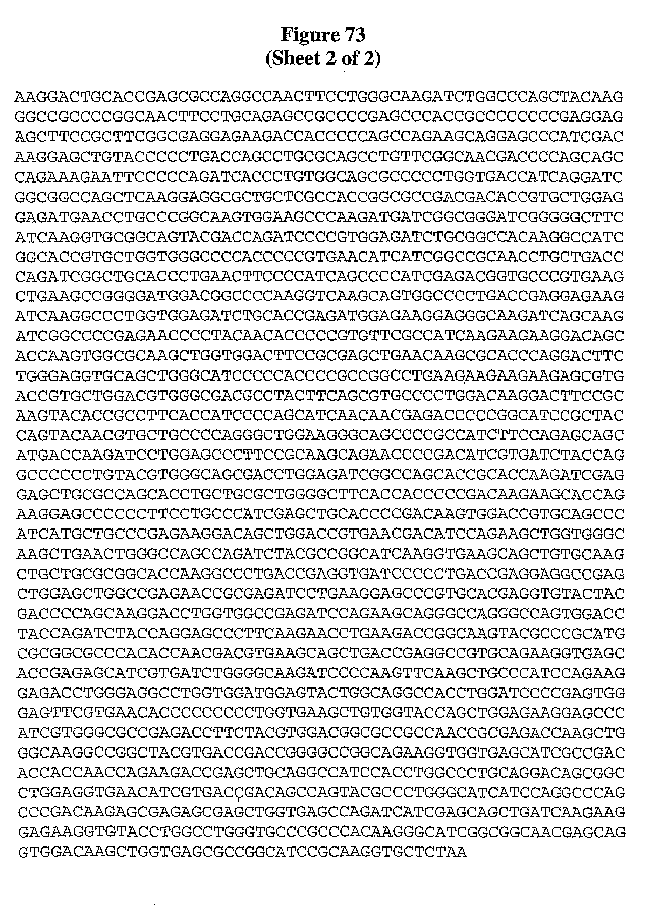

Figure 73, sheets 1 and 2, presents the sequence of the construct TatRevNefGagProtInaRTmut B (SEQ ID NO:76).

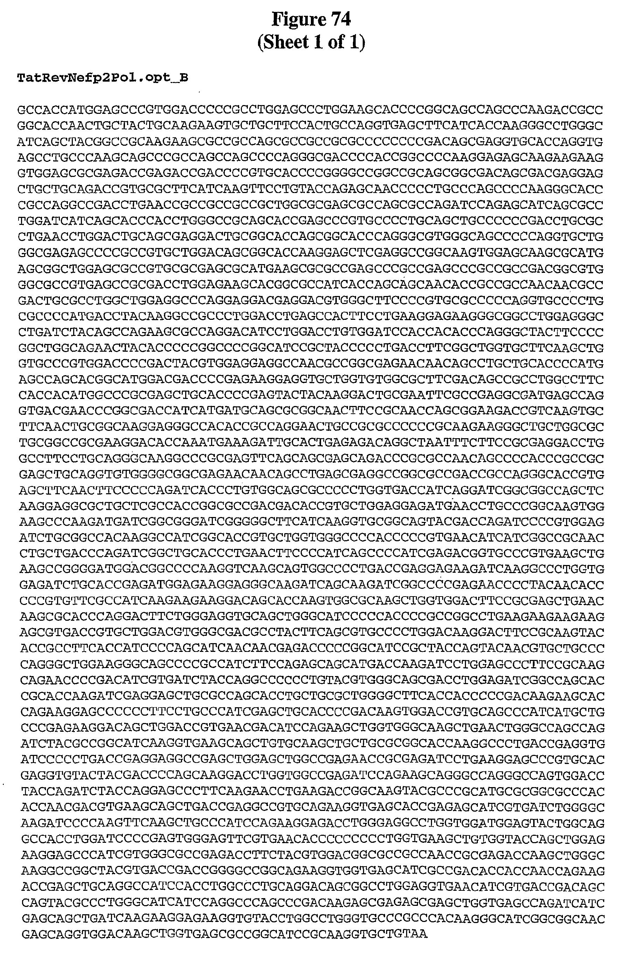

Figure 74, presents the sequence of the construct TatRevNefp2Pol.opt_B (SEQ ID NO:77).

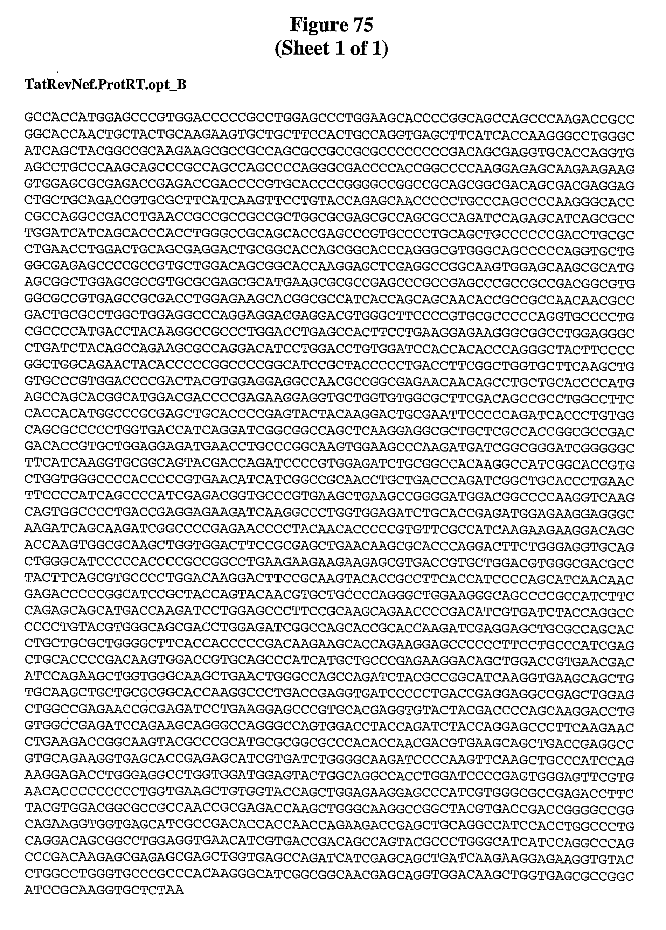

Figure 75, presents the sequence of the construct TatRevNefprotRTopt B (SEQ ID NO:78).

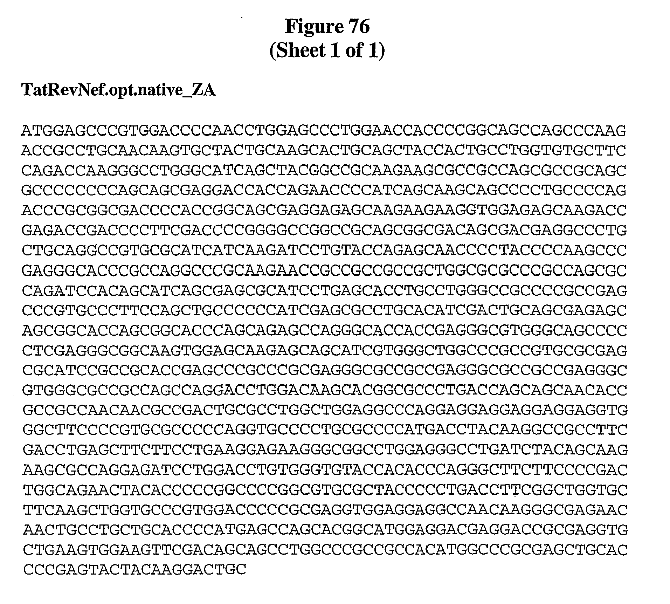

Figure 76, presents the sequence of the construct TatRevNef.opt.native_ZA (SEQ ID NO:79).

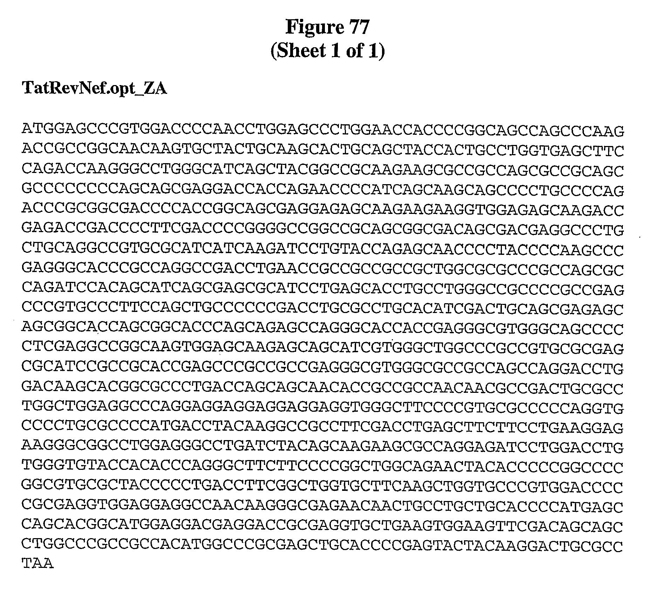

Figure 77, presents the sequence of the construct TatRevNef.opt_ZA (SEQ ID NO:80).

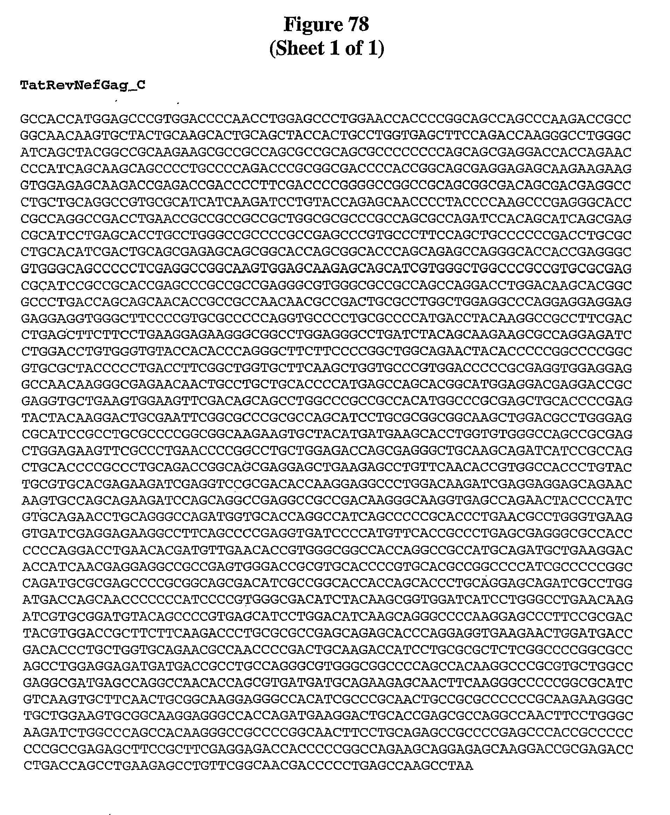

Figure 78, presents the sequence of the construct TatRevNefGag C (SEQ ID NO:81).

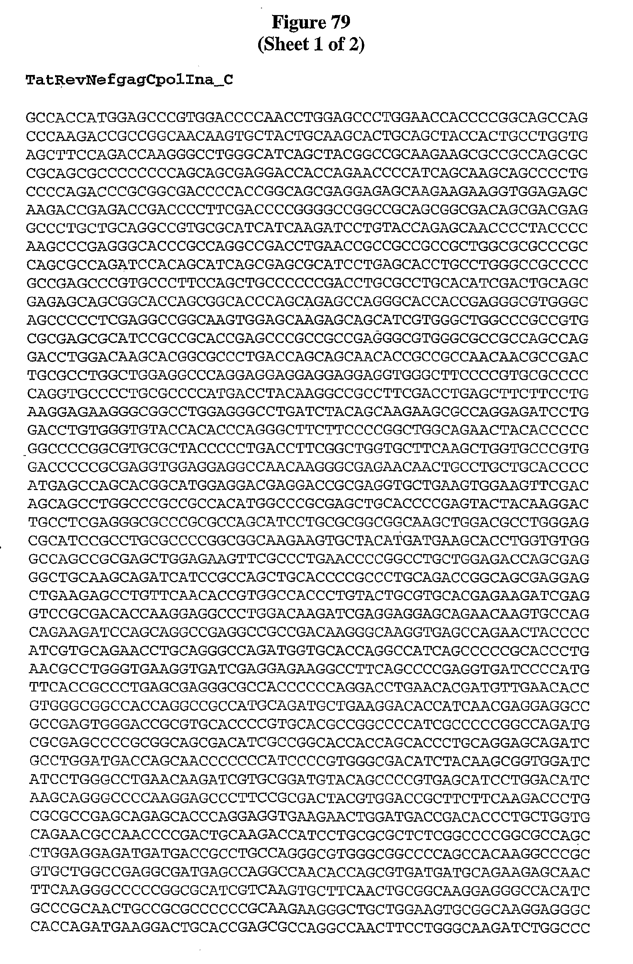

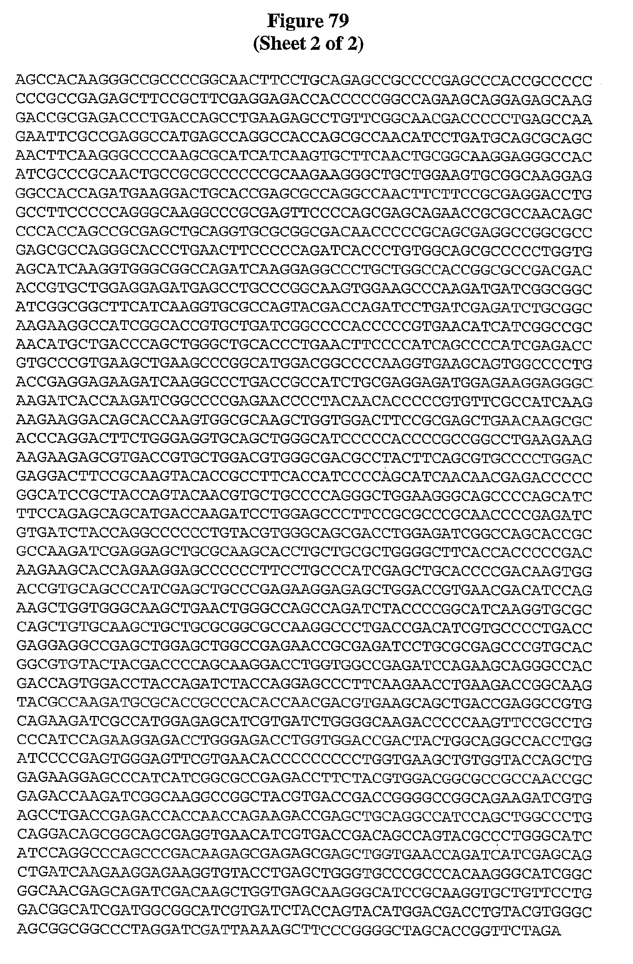

Figure 79, sheets 1 and 2, presents the sequence of the construct TatRevNefgagCpolIna C (SEQ ID NO:82).

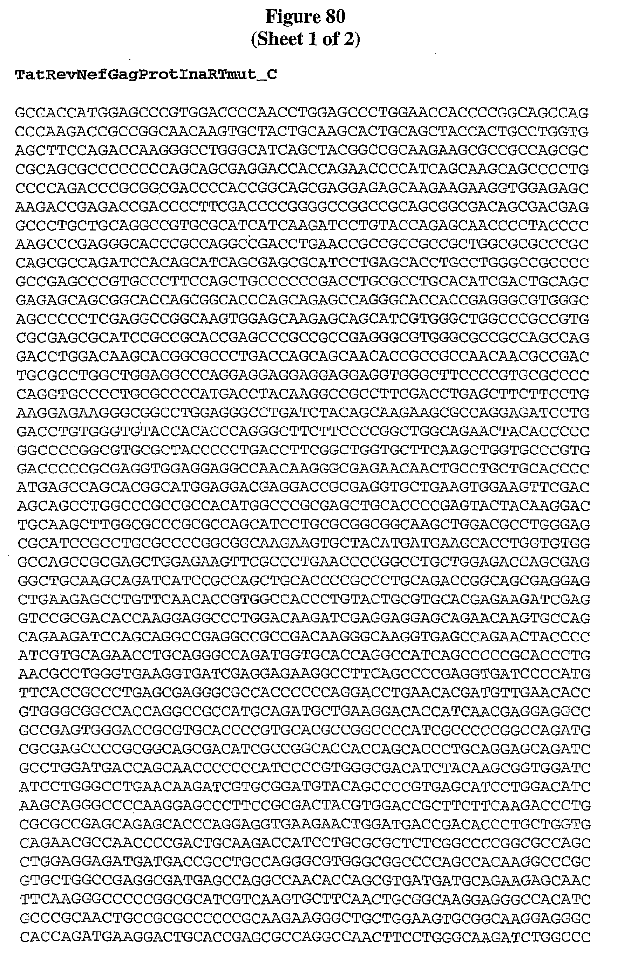

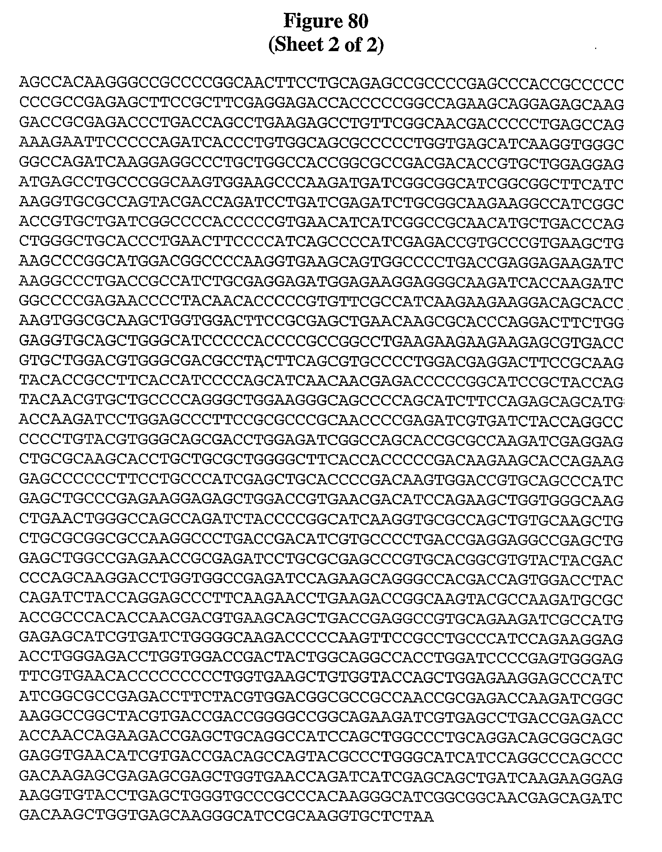

Figure 80, sheets 1 and 2, presents the sequence of the construct TatRevNefGagProtInaRTmut C (SEQ ID NO:83).

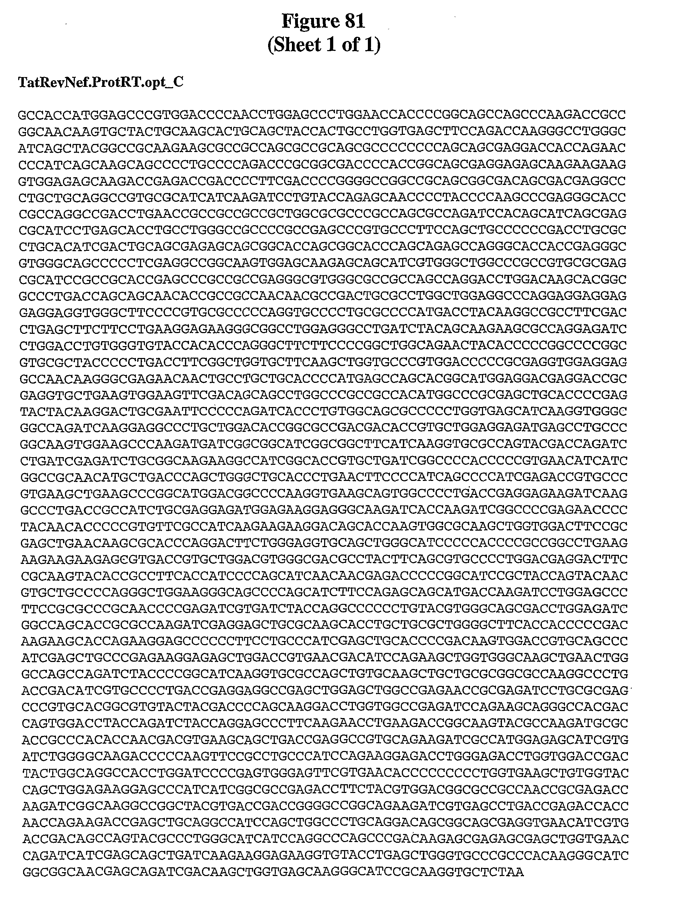

Figure 81, presents the sequence of the construct TatRevNefProtRT opt C (SEQ ID NO:84).

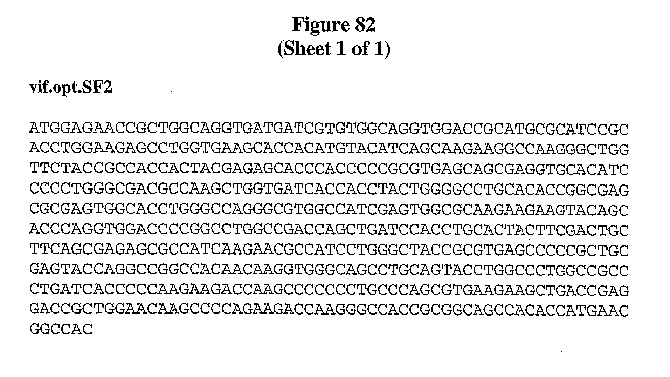

Figure 82, presents the sequence of the construct vif.opt.SF2 (SEQ ID NO:85).

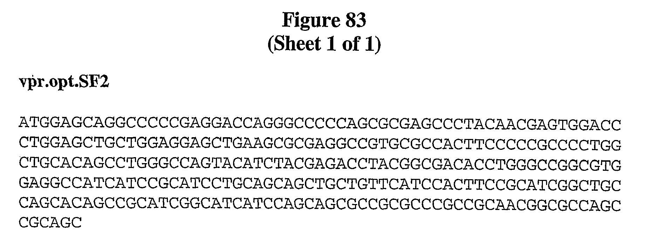

Figure 83, presents the sequence of the construct vpr.opt.SF2 (SEQ ID NO:86).

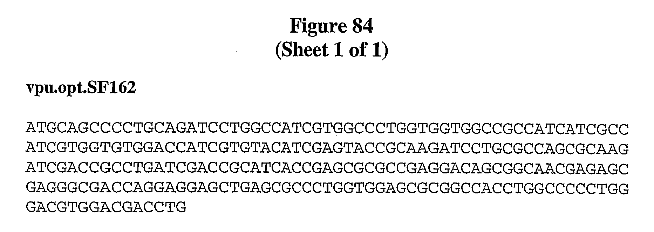

Figure 84, presents the sequence of the construct vpu.opt.SF162 (SEQ ID NO:87).

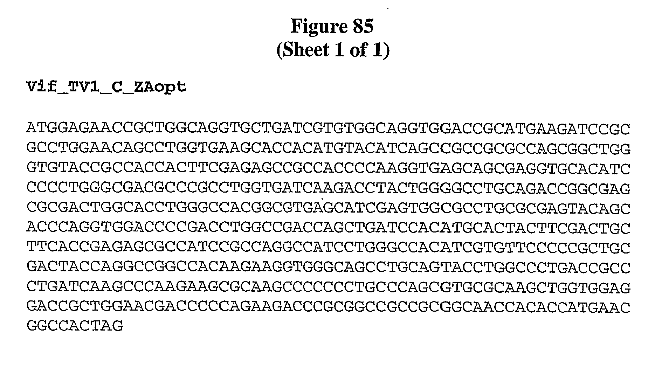

Figure 85, presents the sequence of the construct Vif_TV1_C_ZAopt (SEQ ID NO:88).

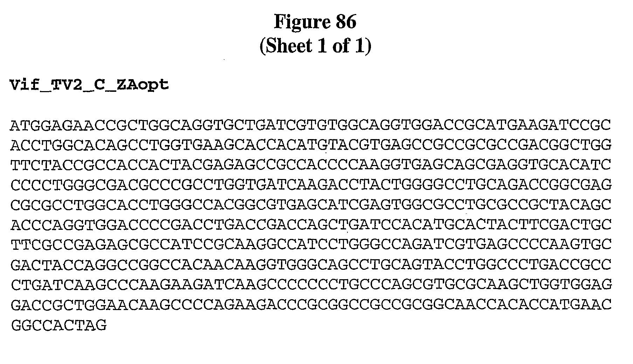

Figure 86, presents the sequence of the construct Vif_TV2_C_ZAopt (SEQ ID NO:89).

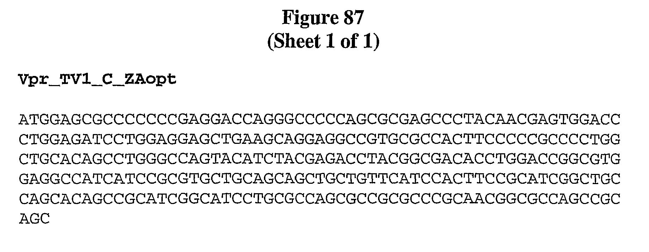

Figure 87, presents the sequence of the construct Vpr_TV_1_C_ZAopt (SEQ ID NO:90).

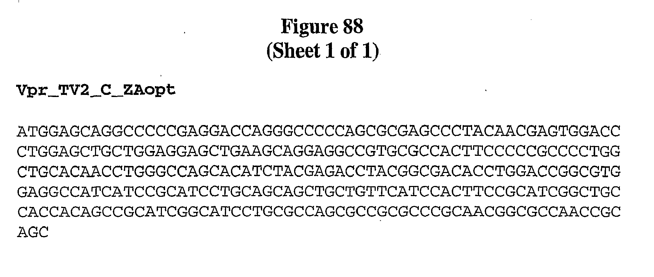

Figure 88, presents the sequence of the construct Vpr_TV2_C_ZAopt (SEQ ID NO:91).

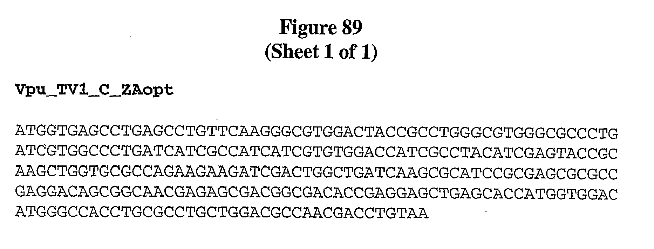

Figure 89, presents the sequence of the construct Vpu_TV1_C_ZAopt (SEQ ID NO:92).

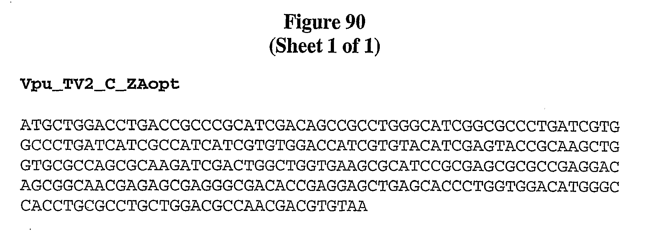

Figure 90, presents the sequence of the construct Vpu_TV2_C_ZAopt (SEQ ID NO:93).
Figure 91 presents an overview of genome organization of HIV-1 and useful subgenomic fragments.
Figure 92 presents antibody titer data from immunized rabbits following immunization with HIV Envelope DNA constructs and protein.
Figure 93 presents a comparison of ELISA titers against subtype B and C Envelope proteins in rabbit sera collected after three DNA immunizations and a single protein boost.
Figure 94 presents data of neutralizing antibody responses against subtype B SF162 EnvdV2 strain in rabbits immunized with subtype C TV1 Env in a DNA prime protein boost regimen.
Figure 95 presents data of neutralizing antibody responses against subtype C primary strains, TV1 and TV2 in 5.25 reporter cell assay after a single protein boost.
Figure 96 presents data of neutralizing antibody responses against subtype C, TV1 and Du174, and subtype B, SF162 after a single protein boost (as measured by Duke PBMC assay).

### DETAILED DESCRIPTION OF THE INVENTION

The practice of the present invention will employ, unless otherwise indicated, conventional methods of chemistry, biochemistry, molecular biology, immunology and pharmacology, within the skill of the art. Such techniques are explained fully in the literature. See, e.g., Remington's Pharmaceutical Sciences, 18th Edition (Easton, Pennsylvania: Mack Publishing Company, 1990); Methods In Enzymology (S. Colowick and N. Kaplan, eds., Academic Press, Inc.); and Handbook of Experimental Immunology, Vols. I-IV (D.M. Weir and C.C. Blackwell, eds., 1986, Blackwell Scientific Publications); Sambrook, et al., Molecular Cloning: A Laboratory Manual (2nd Edition, 1989); Short Protocols in Molecular Biology, 4th ed. (Ausubel et al. eds., 1999, John Wiley & Sons); Molecular Biology Techniques: An Intensive Laboratory Course, (Ream et al., eds., 1998, Academic Press); PCR (Introduction to Biotechniques Series), 2nd ed. (Newton & Graham eds., 1997, Springer Verlag).

As used in this specification, the singular forms "a," "an" and "the" include plural references unless the content clearly dictates otherwise. Thus, for example, reference to "an antigen" includes a mixture of two or more such agents.

### 1. DEFINITIONS

In describing the present invention, the following terms will be employed, and are intended to be defined as indicated below.

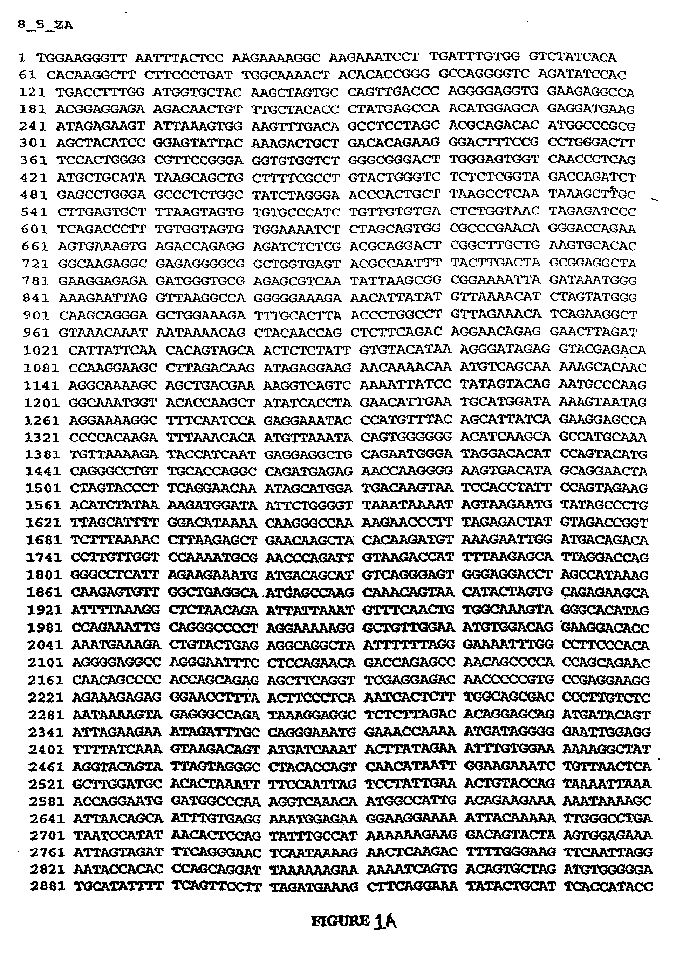

"Synthetic" sequences, as used herein, refers to HIV polypeptide-encoding polynucleotides whose expression has been modified as described herein, for example, by codon substitution, altered activities, and/or inactivation of inhibitory sequences. "Wild-type" or "native" sequences, as used herein, refers to polypeptide encoding sequences that are essentially as they are found in nature, e.g., Gag, Pol, Vif, Vpr, Tat, Rev, Vpu, Env and/or Nef encoding sequences as found in HIV isolates, *e.g.,* SF162, SF2, AF110965, AF110967, AF110968, AF110975, 8_5_TV1_C.ZA, 8_2_TV1_C.ZA or 12-5_1_TV2_C.ZA. The various regions of the HIV genome are shown in Table A, with numbering relative to 8_5_TV1_C.ZA (Figures 1A-1D). Thus, the term "Pol" refers to one or more of the following polypeptides: polymerase (p6Pol); protease (prot); reverse transcriptase (p66RT or RT); RNAseH (p15RNAseH); and/or integrase (p31Int or Int). Identification of gene regions for any selected HIV isolate can be performed by one of ordinary skill in the art based on the teachings presented herein and the information known in the art, for example, by performing alignments relative to 8_5_TV1_C.ZA (Figures 1A-1D) or alignment to other known HIV isolates, for example, Subtype B isolates with gene regions (e.g., SF2, GenBank Accession number K02007; SF162, GenBank Accession Number M38428) and Subtype C isolates with gene regions (e.g., GenBank Accession Number AF110965 and GenBank Accession Number AF110975).

As used herein, the term "virus-like particle" or "VLP" refers to a nonreplicating, viral shell, derived from any of several viruses discussed further below. VLPs are generally composed of one or more viral proteins, such as, but not limited to those proteins referred to as capsid, coat, shell, surface and/or envelope proteins, or particle-forming polypeptides derived from these proteins. VLPs can form spontaneously upon recombinant expression of the protein in an appropriate expression system. Methods for producing particular VLPs are known in the art and discussed more fully below. The presence of VLPs following recombinant expression of viral proteins can be detected using conventional techniques known in the art, such as by electron microscopy, X-ray crystallography, and the like. See, e.g., Baker et al., Biophys. J. (1991) 60:1445-1456; Hagensee et al., J. Virol. (1994) 68:4503-4505. For example, VLPs can be isolated by density gradient centrifugation and/or identified by characteristic density banding. Alternatively, cryoelectron microscopy can be performed on vitrified aqueous samples of the VLP preparation in question, and images recorded under appropriate exposure conditions.

By "particle-forming polypeptide" derived from a particular viral protein is meant a full-length or near full-length viral protein, as well as a fragment thereof, or a viral protein with internal deletions, which has the ability to form VLPs under conditions that favor VLP formation. Accordingly, the polypeptide may comprise the full-length sequence, fragments, truncated and partial sequences, as well as analogs and precursor forms of the reference molecule. The term therefore intends deletions, additions and substitutions to the sequence, so long as the polypeptide retains the ability to form a VLP. Thus, the term includes natural variations of the specified polypeptide since variations in coat proteins often occur between viral isolates. The term also includes deletions, additions and substitutions that do not naturally occur in the reference protein, so long as the protein retains the ability to form a VLP. Preferred substitutions are those which are conservative in nature, i.e., those substitutions that take place within a family of amino acids that are related in their side chains. Specifically, amino acids are generally divided into four families: (1) acidicaspartate and glutamate; (2) basic -- lysine, arginine, histidine; (3) non-polar -- alanine, valine, leucine, isoleucine, proline, phenylalanine, methionine, tryptophan; and (4) uncharged polar -- glycine, asparagine, glutamine, cystine, serine threonine, tyrosine. Phenylalanine, tryptophan, and tyrosine are sometimes classified as aromatic amino acids.

The term "HIV polypeptide" refers to any amino acid sequence that exhibits sequence homology to native HIV polypeptides (*e.g*., Gag, Env, Prot, Pol, RT, Int, vif, vpr, vpu, tat, rev, nef and/or combinations thereof) and/or which is functional. Non-limiting examples of functions that may be exhibited by HIV polypeptides include, use as immunogens (e.g., to generate a humoral and/or cellular immune response), use in diagnostics (*e.g*, bound by suitable antibodies for use in ELISAs or other immunoassays) and/or polypeptides which exhibit one or more biological activities associated with the wild type or synthetic HIV polypeptide. For example, as used herein, the term "Gag polypeptide" may refer to a polypeptide that is bound by one or more anti-Gag antibodies; elicits a humoral and/or cellular immune response; and/or exhibits the ability to form particles.

The term "HIV polypeptide" refers to any amino acid sequence that exhibits sequence homology to native HIV polypeptides (*e.g*., Gag, Env, Prot, Pol, RT, Int, vif, vpr, vpu, tat, rev, nef and/or combinations thereof) and/or which is functional. Non-limiting examples of functions that may be exhibited by HIV polypeptides include, use as immunogens (*e.g*., to generate a humoral and/or cellular immune response), use in diagnostics (*e.g*, bound by suitable antibodies for use in ELISAs or other immunoassays) and/or polypeptides which exhibit one or more biological activities associated with the wild type or synthetic HIV polypeptide. For example, as used herein, the term "Gag polypeptide" may refer to a polypeptide that is bound by one or more anti-Gag antibodies; elicits a humoral and/or cellular immune response; and/or exhibits the ability to form particles.

An "antigen" refers to a molecule containing one or more epitopes (either linear, conformational or both) that will stimulate a host's immune system to make a humoral and/or cellular antigen-specific response. The term is used interchangeably with the term "immunogen." Normally, a B-cell epitope will include at least about 5 amino acids but can be as small as 3-4 amino acids. A T-cell epitope, such as a CTL epitope, will include at least about 7-9 amino acids, and a helper T-cell epitope at least about 12-20 amino acids. Normally, an epitope will include between about 7 and 15 amino acids, such as, 9, 10, 12 or 15 amino acids. The term "antigen" denotes both subunit antigens, (i.e., antigens which are separate and discrete from a whole organism with which the antigen is associated in nature), as well as, killed, attenuated or inactivated bacteria, viruses, fungi, parasites or other microbes. Antibodies such as anti-idiotype antibodies, or fragments thereof, and synthetic peptide mimotopes, which can mimic an antigen or antigenic determinant, are also captured under the definition of antigen as used herein. Similarly, an oligonucleotide or polynucleotide which expresses an antigen or antigenic determinant *in vivo,* such as in gene therapy and DNA immunization applications, is also included in the definition of antigen herein.

For purposes of the present invention, antigens can be derived from any of several known viruses, bacteria, parasites and fungi, as described more fully below. The term also intends any of the various tumor antigens. Furthermore, for purposes of the present invention, an "antigen" refers to a protein which includes modifications, such as deletions, additions and substitutions (generally conservative in nature), to the native sequence, so long as the protein maintains the ability to elicit an immunological response, as defined herein. These modifications may be deliberate, as through site-directed mutagenesis, or may be accidental, such as through mutations of hosts which produce the antigens.

An "immunological response" to an antigen or composition is the development in a subject of a humoral and/or a cellular immune response to an antigen present in the composition of interest. For purposes of the present invention, a "humoral immune response" refers to an immune response mediated by antibody molecules, while a "cellular immune response" is one mediated by T-lymphocytes and/or other white blood cells. One important aspect of cellular immunity involves an antigen-specific response by cytolytic T-cells ("CTL"s). CTLs have specificity for peptide antigens that are presented in association with proteins encoded by the major histocompatibility complex (MHC) and expressed on the surfaces of cells. CTLs help induce and promote the destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells.

A composition or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular antigen to stimulate a cell-mediated immunological response may be determined by a number of assays, such as by lymphoproliferation (lymphocyte activation) assays, CTL cytotoxic cell assays, or by assaying for T-lymphocytes specific for the antigen in a sensitized subject. Such assays are well known in the art. See, e.g., Erickson et al., J. Immunol. (1993) 151:4189-4199; Doe et al., Eur. J. Immunol. (1994) 24:2369-2376. Recent methods of measuring cell-mediated immune response include measurement of intracellular cytokines or cytokine secretion by T-cell populations, or by measurement of epitope specific T-cells (e.g., by the tetramer technique)(reviewed by McMichael, A.J., and O'Callaghan, C.A., J. Exp. Med. 187(9)1367-1371, 1998; Mcheyzer-Williams, M.G., et al, Immunol. Rev. 150:5-21, 1996; Lalvani, A., et al, J. Exp. Med. 186:859-865, 1997).

Thus, an immunological response as used herein may be one which stimulates the production of CTLs, and/or the production or activation of helper T- cells. The antigen of interest may also elicit an antibody-mediated immune response. Hence, an immunological response may include one or more of the following effects: the production of antibodies by B-cells; and/or the activation of suppressor T-cells and/or γδ T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

An "immunogenic composition" is a composition that comprises an antigenic molecule where administration of the composition to a subject results in the development in the subject of a humoral and/or a cellular immune response to the antigenic molecule of interest. The immunogenic composition can be introduced directly into a recipient subject, such as by injection, inhalation, oral, intranasal and mucosal (*e.g*., intra-rectally or intra-vaginally) administration.

By "subunit vaccine" is meant a vaccine composition which includes one or more selected antigens but not all antigens, derived from or homologous to, an antigen from a pathogen of interest such as from a virus, bacterium, parasite or fungus. Such a composition is substantially free of intact pathogen cells or pathogenic particles, or the lysate of such cells or particles. Thus, a "subunit vaccine" can be prepared from at least partially purified (preferably substantially purified) immunogenic polypeptides from the pathogen, or analogs thereof. The method of obtaining an antigen included in the subunit vaccine can thus include standard purification techniques, recombinant production, or synthetic production.

"Substantially purified" general refers to isolation of a substance (compound, polynucleotide, protein, polypeptide, polypeptide composition) such that the substance comprises the majority percent of the sample in which it resides. Typically in a sample a substantially purified component comprises 50%, preferably 80%-85%, more preferably 90-95% of the sample. Techniques for purifying polynucleotides and polypeptides of interest are well-known in the art and include, for example, ion-exchange chromatography, affinity chromatography and sedimentation according to density.

A "coding sequence" or a sequence which "encodes" a selected polypeptide, is a nucleic acid molecule which is transcribed (in the case of DNA) and translated (in the case of mRNA) into a polypeptide *in vivo* when placed under the control of appropriate regulatory sequences (or "control elements"). The boundaries of the coding sequence are determined by a start codon at the 5' (amino) terminus and a translation stop codon at the 3' (carboxy) terminus. A coding sequence can include, but is not limited to, cDNA from viral, procaryotic or eucaryotic mRNA, genomic DNA sequences from viral or procaryotic DNA, and even synthetic DNA sequences. A transcription termination sequence such as a stop codon may be located 3' to the coding sequence.

Typical "control elements", include, but are not limited to, transcription promoters, transcription enhancer elements, transcription termination signals, polyadenylation sequences (located 3' to the translation stop codon), sequences for optimization of initiation of translation (located 5' to the coding sequence), and translation termination sequences. For example, the sequences and/or vectors described herein may also include one or more additional sequences that may optimize translation and/or termination including, but not limited to, a Kozak sequence *(e.g.,* GCCACC placed in front (5') of the ATG of the codon-optimized wild-type leader or any other suitable leader sequence (*e.g*., tpa1, tpa2, wtLnat (native wild-type leader)) or a termination sequence *(e.g.,* TAA or, preferably, TAAA placed after (3') the coding sequence.

A "polynucleotide coding sequence" or a sequence which "encodes" a selected polypeptide, is a nucleic acid molecule which is transcribed (in the case of DNA) and translated (in the case of mRNA) into a polypeptide *in vivo* when placed under the control of appropriate regulatory sequences (or "control elements"). The boundaries of the coding sequence are determined by a start codon, for example, at or near the 5' terminus and a translation stop codon, for example, at or near the 3' terminus. Exemplary coding sequences are the modified viral polypeptide-coding sequences of the present invention. The coding regions of the polynucleotide sequences of the present invention are identifiable by one of skill in the art and may, for example, be easily identified by performing translations of all three frames of the polynucleotide and identifying the frame corresponding to the encoded polypeptide, for example, a synthetic nef polynucleotide of the present invention encodes a nef-derived polypeptide. A transcription termination sequence may be located 3' to the coding sequence. Typical "control elements", include, but are not limited to, transcription regulators, such as promoters, transcription enhancer elements, transcription termination signals, and polyadenylation sequences; and translation regulators, such as sequences for optimization of initiation of translation, e.g., Shine-Dalgarno (ribosome binding site) sequences, Kozak sequences (i.e., sequences for the optimization of translation, located, for example, 5' to the coding sequence), leader sequences, translation initiation codon (e.g., ATG), and translation termination sequences. In certain embodiments, one or more translation regulation or initiation sequences (*e.g*., the leader sequence) are derived from wild-type translation initiation sequences, *i.e.,* sequences that regulate translation of the coding region in their native state. Wild-type leader sequences that have been modified, using the methods described herein, also find use in the present invention. Promoters can include inducible promoters (where expression of a polynucleotide sequence operably linked to the promoter is induced by an analyte, cofactor, regulatory protein, *etc*.), repressible promoters (where expression of a polynucleotide sequence operably linked to the promoter is induced by an analyte, cofactor, regulatory protein, *etc*.), and constitutive promoters.

A "nucleic acid" molecule can include, but is not limited to, procaryotic sequences, eucaryotic mRNA, cDNA from eucaryotic mRNA, genomic DNA sequences from eucaryotic (*e.g*., mammalian) DNA, and even synthetic DNA sequences. The term also captures sequences that include any of the known base analogs of DNA and RNA.

"Operably linked" refers to an arrangement of elements wherein the components so described are configured so as to perform their usual function. Thus, a given promoter operably linked to a coding sequence is capable of effecting the expression of the coding sequence when the proper enzymes are present. The promoter need not be contiguous with the coding sequence, so long as it functions to direct the expression thereof. Thus, for example, intervening untranslated yet transcribed sequences can be present between the promoter sequence and the coding sequence and the promoter sequence can still be considered "operably linked" to the coding sequence.

"Recombinant" as used herein to describe a nucleic acid molecule means a polynucleotide of genomic, cDNA, semisynthetic, or synthetic origin which, by virtue of its origin or manipulation: (1) is not associated with all or a portion of the polynucleotide with which it is associated in nature; and/or (2) is linked to a polynucleotide other than that to which it is linked in nature. The term "recombinant" as used with respect to a protein or polypeptide means a polypeptide produced by expression of a recombinant polynucleotide. "Recombinant host cells," "host cells," "cells," "cell lines," "cell cultures," and other such terms denoting procaryotic microorganisms or eucaryotic cell lines cultured as unicellular entities, are used interchangeably, and refer to cells which can be, or have been, used as recipients for recombinant vectors or other transfer DNA, and include the progeny of the original cell which has been transfected. It is understood that the progeny of a single parental cell may not necessarily be completely identical in morphology or in genomic or total DNA complement to the original parent, due to accidental or deliberate mutation. Progeny of the parental cell which are sufficiently similar to the parent to be characterized by the relevant property, such as the presence of a nucleotide sequence encoding a desired peptide, are included in the progeny intended by this definition, and are covered by the above terms.

Techniques for determining amino acid sequence "similarity" are well known in the art. In general, "similarity" means the exact amino acid to amino acid comparison of two or more polypeptides at the appropriate place, where amino acids are identical or possess similar chemical and/or physical properties such as charge or hydrophobicity. A so-termed "percent similarity" then can be determined between the compared polypeptide sequences. Techniques for determining nucleic acid and amino acid sequence identity also are well known in the art and include determining the nucleotide sequence of the mRNA for that gene (usually via a cDNA intermediate) and determining the amino acid sequence encoded thereby, and comparing this to a second amino acid sequence. In general, "identity" refers to an exact nucleotide to nucleotide or amino acid to amino acid correspondence of two polynucleotides or polypeptide sequences, respectively.

Two or more polynucleotide sequences can be compared by determining their "percent identity." Two or more amino acid sequences likewise can be compared by determining their "percent identity." The percent identity of two sequences, whether nucleic acid or peptide sequences, is generally described as the number of exact matches between two aligned sequences divided by the length of the shorter sequence and multiplied by 100. An approximate alignment for nucleic acid sequences is provided by the local homology algorithm of Smith and Waterman, Advances in Applied Mathematics 2:482-489 (1981). This algorithm can be extended to use with peptide sequences using the scoring matrix developed by Dayhoff, Atlas of Protein Sequences and Structure, M.O. Dayhoff ed., 5 suppl. 3:353-358, National Biomedical Research Foundation, Washington, D.C., USA, and normalized by Gribskov, Nucl. Acids Res. 14(6):6745-6763 (1986). An implementation of this algorithm for nucleic acid and peptide sequences is provided by the Genetics Computer Group (Madison, WI) in their BestFit utility application. The default parameters for this method are described in the Wisconsin Sequence Analysis Package Program Manual, Version 8 (1995) (available from Genetics Computer Group, Madison, WI). Other equally suitable programs for calculating the percent identity or similarity between sequences are generally known in the art.

For example, percent identity of a particular nucleotide sequence to a reference sequence can be determined using the homology algorithm of Smith and Waterman with a default scoring table and a gap penalty of six nucleotide positions. Another method of establishing percent identity in the context of the present invention is to use the MPSRCH package of programs copyrighted by the University of Edinburgh, developed by John F. Collins and Shane S. Sturrok, and distributed by IntelliGenetics, Inc. (Mountain View, CA). From this suite of packages, the Smith-Waterman algorithm can be employed where default parameters are used for the scoring table (for example, gap open penalty of 12, gap extension penalty of one, and a gap of six). From the data generated, the "Match" value reflects "sequence identity." Other suitable programs for calculating the percent identity or similarity between sequences are generally known in the art, such as the alignment program BLAST, which can also be used with default parameters. For example, BLASTN and BLASTP can be used with the following default parameters: genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs can be found at the following internet address: http://www.ncbi.nlm.gov/cgi-bin/BLAST

One of skill in the art can readily determine the proper search parameters to use for a given sequence, exemplary preferred Smith Waterman based parameters are presented above. For example, the search parameters may vary based on the size of the sequence in question. Thus, for the polynucleotide sequences of the present invention the length of the polynucleotide sequence disclosed herein is searched against a selected database and compared to sequences of essentially the same length to determine percent identity. For example, a representative embodiment of the present invention would include an isolated polynucleotide comprising X contiguous nucleotides, wherein (i) the X contiguous nucleotides have at least about a selected level of percent identity relative to Y contiguous nucleotides of one or more of the sequences described herein (e.g., in Table C) or fragment thereof, and (ii) for search purposes X equals Y, wherein Y is a selected reference polynucleotide of defined length (for example, a length of from 15 nucleotides up to the number of nucleotides present in a selected full-length sequence).

The sequences of the present invention can include fragments of the sequences, for example, from about 15 nucleotides up to the number of nucleotides present in the full-length sequences described herein (e.g., see the Figures), including all integer values falling within the above-described range. For example, fragments of the polynucleotide sequences of the present invention may be 30-60 nucleotides, 60-120 nucleotides, 120-240 nucleotides, 240-480 nucleotides, 480-1000 nucleotides, and all integer values therebetween.

The synthetic expression cassettes (and purified polynucleotides) of the present invention include related polynucleotide sequences having about 80% to 100%, greater than 80-85%, preferably greater than 90-92%, more preferably greater than 95%, and most preferably greater than 98% up to 100% (including all integer values falling within these described ranges) sequence identity to the synthetic expression cassette and/or polynucleotide sequences disclosed herein (for example, to the sequences of the present invention) when the sequences of the present invention are used as the query sequence against, for example, a database of sequences.

Two nucleic acid fragments are considered to "selectively hybridize" as described herein. The degree of sequence identity between two nucleic acid molecules affects the efficiency and strength of hybridization events between such molecules. A partially identical nucleic acid sequence will at least partially inhibit a completely identical sequence from hybridizing to a target molecule. Inhibition of hybridization of the completely identical sequence can be assessed using hybridization assays that are well known in the art (e.g., Southern blot, Northern blot, solution hybridization, or the like, see Sambrook, et al., *supra* or Ausubel et al., *supra).* Such assays can be conducted using varying degrees of selectivity, for example, using conditions varying from low to high stringency. If conditions of low stringency are employed, the absence of non-specific binding can be assessed using a secondary probe that lacks even a partial degree of sequence identity (for example, a probe having less than about 30% sequence identity with the target molecule), such that, in the absence of non-specific binding events, the secondary probe will not hybridize to the target.

When utilizing a hybridization-based detection system, a nucleic acid probe is chosen that is complementary to a target nucleic acid sequence, and then by selection of appropriate conditions the probe and the target sequence "selectively hybridize," or bind, to each other to form a hybrid molecule. A nucleic acid molecule that is capable of hybridizing selectively to a target sequence under "moderately stringent" typically hybridizes under conditions that allow detection of a target nucleic acid sequence of at least about 10-14 nucleotides in length having at least approximately 70% sequence identity with the sequence of the selected nucleic acid probe. Stringent hybridization conditions typically allow detection of target nucleic acid sequences of at least about 10-14 nucleotides in length having a sequence identity of greater than about 90-95% with the sequence of the selected nucleic acid probe. Hybridization conditions useful for probe/target hybridization where the probe and target have a specific degree of sequence identity, can be determined as is known in the art (see, for example, Nucleic Acid Hybridization: A Practical Approach, editors B.D. Hames and S.J. Higgins, (1985) Oxford; Washington, DC; IRL Press).

With respect to stringency conditions for hybridization, it is well known in the art that numerous equivalent conditions can be employed to establish a particular stringency by varying, for example, the following factors: the length and nature of probe and target sequences, base composition of the various sequences, concentrations of salts and other hybridization solution components, the presence or absence of blocking agents in the hybridization solutions (e.g., formamide, dextran sulfate, and polyethylene glycol), hybridization reaction temperature and time parameters, as well as, varying wash conditions. The selection of a particular set of hybridization conditions is selected following standard methods in the art (see, for example, Sambrook, et al., *supra* or Ausubel et al., *supra*)*.*

A first polynucleotide is "derived from" second polynucleotide if it has the same or substantially the same basepair sequence as a region of the second polynucleotide, its cDNA, complements thereof, or if it displays sequence identity as described above.

A first polypeptide is "derived from" a second polypeptide if it is (i) encoded by a first polynucleotide derived from a second polynucleotide, or (ii) displays sequence identity to the second polypeptides as described above.

Generally, a viral polypeptide is "derived from" a particular polypeptide of a virus (viral polypeptide) if it is (i) encoded by an open reading frame of a polynucleotide of that virus (viral polynucleotide), or (ii) displays sequence identity to polypeptides of that virus as described above.

"Encoded by" refers to a nucleic acid sequence which codes for a polypeptide sequence, wherein the polypeptide sequence or a portion thereof contains an amino acid sequence of at least 3 to 5 amino acids, more preferably at least 8 to 10 amino acids, and even more preferably at least 15 to 20 amino acids from a polypeptide encoded by the nucleic acid sequence. Also encompassed are polypeptide sequences which are immunologically identifiable with a polypeptide encoded by the sequence. Further, polyproteins can be constructed by fusing in-frame two or more polynucleotide sequences encoding polypeptide or peptide products. Further, polycistronic coding sequences may be produced by placing two or more polynucleotide sequences encoding polypeptide products adjacent each other, typically under the control of one promoter, wherein each polypeptide coding sequence may be modified to include sequences for internal ribosome binding sites.

"Purified polynucleotide" refers to a polynucleotide of interest or fragment thereof which is essentially free, e.g., contains less than about 50%, preferably less than about 70%, and more preferably less than about 90%, of the protein with which the polynucleotide is naturally associated. Techniques for purifying polynucleotides of interest are well-known in the art and include, for example, disruption of the cell containing the polynucleotide with a chaotropic agent and separation of the polynucleotide(s) and proteins by ion-exchange chromatography, affinity chromatography and sedimentation according to density.

By "nucleic acid immunization" is meant the introduction of a nucleic acid molecule encoding one or more selected antigens into a host cell, for the *in vivo* expression of an antigen, antigens, an epitope, or epitopes. The nucleic acid molecule can be introduced directly into a recipient subject, such as by injection, inhalation, oral, intranasal and mucosal administration, or the like, or can be introduced *ex vivo,* into cells which have been removed from the host. In the latter case, the transformed cells are reintroduced into the subject where an immune response can be mounted against the antigen encoded by the nucleic acid molecule.

"Gene transfer" or "gene delivery" refers to methods or systems for reliably inserting DNA of interest into a host cell. Such methods can result in transient expression of non-integrated transferred DNA, extrachromosomal replication and expression of transferred replicons (e.g., episomes), or integration of transferred genetic material into the genomic DNA of host cells. Gene delivery expression vectors include, but are not limited to, vectors derived from alphaviruses, pox viruses and vaccinia viruses. When used for immunization, such gene delivery expression vectors may be referred to as vaccines or vaccine vectors.

"T lymphocytes" or "T cells" are non-antibody producing lymphocytes that constitute a part of the cell-mediated arm of the immune system. T cells arise from immature lymphocytes that migrate from the bone marrow to the thymus, where they undergo a maturation process under the direction of thymic hormones. Here, the mature lymphocytes rapidly divide increasing to very large numbers. The maturing T cells become immunocompetent based on their ability to recognize and bind a specific antigen. Activation of immunocompetent T cells is triggered when an antigen binds to the lymphocyte's surface receptors.

The term "transfection" is used to refer to the uptake of foreign DNA by a cell. A cell has been "transfected" when exogenous DNA has been introduced inside the cell membrane. A number of transfection techniques are generally known in the art. *See, e.g*., Graham et al. (1973) Virogy, 52:456, Sambrook et al. (1989) Molecular Cloning, a laboratory manual, Cold Spring Harbor Laboratories, New York, Davis et al. (1986) Basic Methods in Molecular Biology, Elsevier, and Chu et al. (1981) Gene 13:197. Such techniques can be used to introduce one or more exogenous DNA moieties into suitable host cells. The term refers to both stable and transient uptake of the genetic material, and includes uptake of peptide- or antibody-linked DNAs.

A "vector" is capable of transferring gene sequences to target cells (e.g., viral vectors, non-viral vectors, particulate carriers, and liposomes). Typically, "vector construct," "expression vector," and "gene transfer vector," mean any nucleic acid construct capable of directing the expression of a gene of interest and which can transfer gene sequences to target cells. Thus, the term includes cloning and expression vehicles, as well as viral vectors.

Transfer of a "suicide gene" (e.g., a drug-susceptibility gene) to a target cell renders the cell sensitive to compounds or compositions that are relatively nontoxic to normal cells. Moolten, F.L. (1994) Cancer Gene Ther. 1:279-287. Examples of suicide genes are thymidine kinase of herpes simplex virus (HSV-tk), cytochrome P450 (Manome et al. (1996) Gene Therapy 3:513-520), human deoxycytidine kinase (Manome et al. (1996) Nature Medicine 2(5):567-573) and the bacterial enzyme cytosine deaminase (Dong et al. (1996) Human Gene Therapy 7:713-720). Cells which express these genes are rendered sensitive to the effects of the relatively nontoxic prodrugs ganciclovir (HSV-tk), cyclophosphamide (cytochrome P450 2B1), cytosine arabinoside (human deoxycytidine kinase) or 5-fluorocytosine (bacterial cytosine deaminase). Culver et al. (1992) Science 256:1550-1552, Huber et al. (1994) Proc. Natl. Acad. Sci. USA 91:8302-8306.

A "selectable marker" or "reporter marker" refers to a nucleotide sequence included in a gene transfer vector that has no therapeutic activity, but rather is included to allow for simpler preparation, manufacturing, characterization or testing of the gene transfer vector.

A "specific binding agent" refers to a member of a specific binding pair of molecules wherein one of the molecules specifically binds to the second molecule through chemical and/or physical means. One example of a specific binding agent is an antibody directed against a selected antigen.

By "subject" is meant any member of the subphylum chordata, including, without limitation, humans and other primates, including non-human primates such as rhesus macaque, chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term does not denote a particular age. Thus, both adult and newborn individuals are intended to be covered. The system described above is intended for use in any of the above vertebrate species, since the immune systems of all of these vertebrates operate similarly.

By "pharmaceutically acceptable" or "pharmacologically acceptable" is meant a material which is not biologically or otherwise undesirable, i.e., the material may be administered to an individual in a formulation or composition without causing any undesirable biological effects or interacting in a deleterious manner with any of the components of the composition in which it is contained.

By "physiological pH" or a "pH in the physiological range" is meant a pH in the range of approximately 7.0 to 8.0 inclusive, more typically in the range of approximately 7.2 to 7.6 inclusive.

As used herein, "treatment" refers to any of (i) the prevention of infection or reinfection, as in a traditional vaccine, (ii) the reduction or elimination of symptoms, and (iii) the substantial or complete elimination of the pathogen in question. Treatment may be effected prophylactically (prior to infection) or therapeutically (following infection).

By "co-administration" is meant administration of more than one composition or molecule. Thus, co-administration includes concurrent administration or sequentially administration (in any order), via the same or different routes of administration. Non-limiting examples of co-administration regimes include, co-administration of nucleic acid and polypeptide; co-administration of different nucleic acids (*e.g*., different expression cassettes as described herein and/or different gene delivery vectors); and co-administration of different polypeptides (*e.g*., different HIV polypeptides and/or different adjuvants). The term also encompasses multiple administrations of one of the co-administered molecules or compositions (*e.g.*, multiple administrations of one or more of the expression cassettes described herein followed by one or more administrations of a polypeptide-containing composition). In cases where the molecules or compositions are delivered sequentially, the time between each administration can be readily determined by one of skill in the art in view of the teachings herein.

"Lentiviral vector", and "recombinant lentiviral vector" refer to a nucleic acid construct which carries, and within certain embodiments, is capable of directing the expression of a nucleic acid molecule of interest. The lentiviral vector include at least one transcriptional promoter/enhancer or locus defining element(s), or other elements which control gene expression by other means such as alternate splicing, nuclear RNA export, post-translational modification of messenger, or post-transcriptional modification of protein. Such vector constructs must also include a packaging signal, long terminal repeats (LTRS) or portion thereof, and positive and negative strand primer binding sites appropriate to the retrovirus used (if these are not already present in the retroviral vector). Optionally, the recombinant lentiviral vector may also include a signal which directs polyadenylation, selectable markers such as Neo, TK, hygromycin, phleomycin, histidinol, or DHFR, as well as one or more restriction sites and a translation termination sequence. By way of example, such vectors typically include a 5' LTR, a tRNA binding site, a packaging signal, an origin of second strand DNA synthesis, and a 3'LTR or a portion thereof

"Lentiviral vector particle" as utilized within the present invention refers to a lentivirus which carries at least one gene of interest. The retrovirus may also contain a selectable marker. The recombinant lentivirus is capable of reverse transcribing its genetic material (RNA) into DNA and incorporating this genetic material into a host cell's DNA upon infection. Lentiviral vector particles may have a lentiviral envelope, a non-lentiviral envelope (e.g., an ampho or VSV-G envelope), or a chimeric envelope.

"Nucleic acid expression vector" or "Expression cassette" refers to an assembly which is capable of directing the expression of a sequence or gene of interest. The nucleic acid expression vector includes a promoter which is operably linked to the sequences or gene(s) of interest. Other control elements may be present as well. Expression cassettes described herein may be contained within a plasmid construct. In addition to the components of the expression cassette, the plasmid construct may also include a bacterial origin of replication, one or more selectable markers, a signal which allows the plasmid construct to exist as single-stranded DNA (*e.g*., a M13 origin of replication), a multiple cloning site, and a "mammalian" origin of replication (e.g., a SV40 or adenovirus origin of replication).

"Packaging cell" refers to a cell which contains those elements necessary for production of infectious recombinant retrovirus which are lacking in a recombinant retroviral vector. Typically, such packaging cells contain one or more expression cassettes which are capable of expressing proteins which encode *Gag, pol and* env proteins.

"Producer cell" or "vector producing cell" refers to a cell which contains all elements necessary for production of recombinant retroviral vector particles.

### 2. MODES OF CARRYING OUT THE INVENTION

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein.

### 2.1. THE HIV GENOME

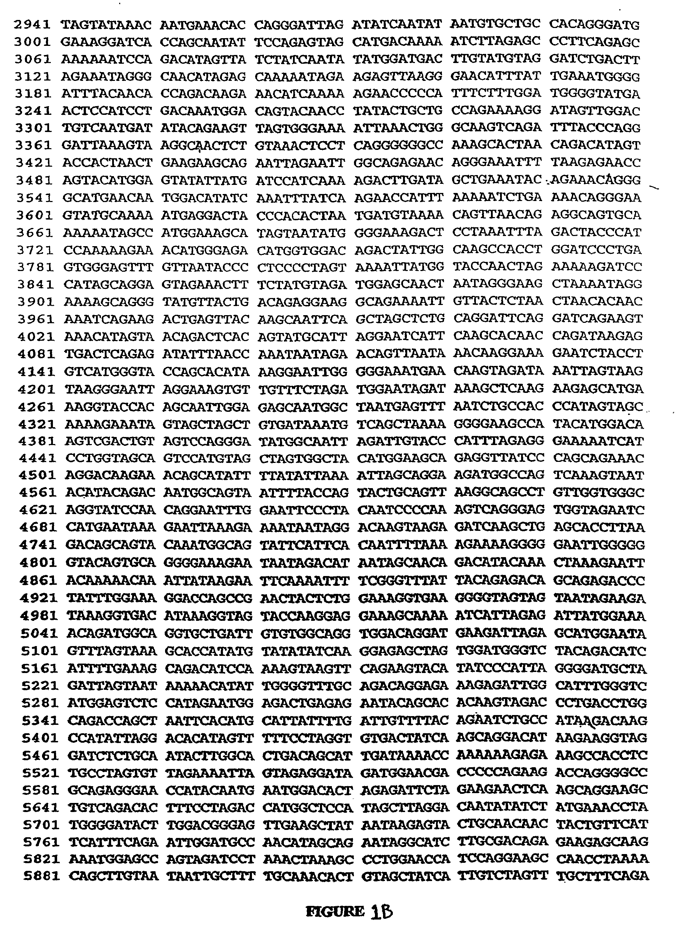

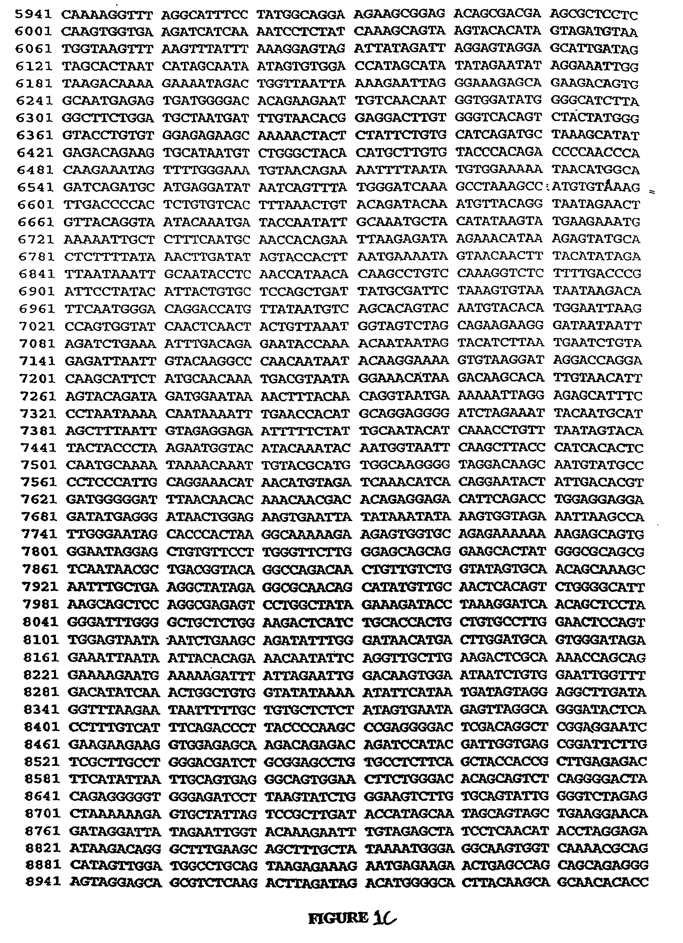

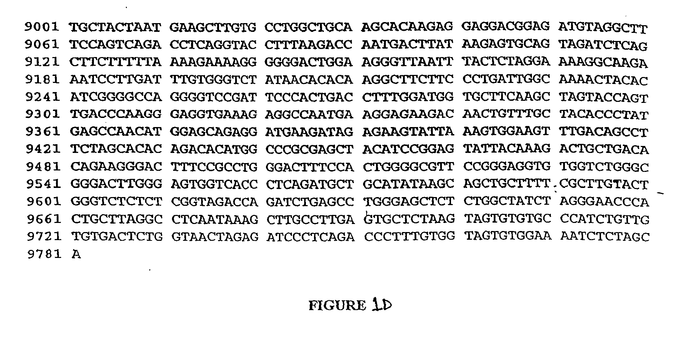

The HIV genome and various polypeptide-encoding regions are shown in Table A. The nucleotide positions are given relative to 8_5_TV1_C.ZA (Figure 1; an HIV Type C isolate). However, it will be readily apparent to one of ordinary skill in the art in view of the teachings of the present disclosure how to determine corresponding regions in-other HIV strains or variants (*e.g.,* isolates HIV_{IIIb}, HIV_{SF2}, HIV-1_{SF162}, HIV-1_{SF170}, HIV_{LAV}, HIV_{LAI}, HIV_{MN}, HIV-1_{CM235},, HIV-1_{US4}, other HIV-1 strains from diverse subtypes(e.g., subtypes, A through G, and O), HIV-2 strains and diverse subtypes (e.g., HIV-2_{UC1} and HIV-2_{UC2}), and simian immunodeficiency virus (SIV). (See, e.g., Virology, 3rd Edition (W.K. Joklik ed. 1988); Fundamental Virology, 2nd Edition (B.N. Fields and D.M. Knipe, eds. 1991); Virology, 3rd Edition (Fields, BN, DM Knipe, PM Howley, Editors, 1996, Lippincott-Raven, Philadelphia, PA; for a description of these and other related viruses), using for example, sequence comparison programs (*e.g*., BLAST and others described herein) or identification and alignment of structural features (*e.g*., a program such as the "ALB" program described herein that can identify the various regions).

**Table A: Regions of the HIV Genome relative to 8_5_TV1_C.ZA**

| **Region** | **Position in nucleotide sequence** |
|---|---|
| **5'LTR** | **1-636** |
| U3 | 1-457 |
| R | 458-553 |
| U5 | 554-636 |
| NFkB II | 340-348 |
| NFkB I | 354-362 |
| Sp1 III | 379-388 |
| Sp1 II | 390-398 |
| Sp1 I | 400-410 |
| TATA Box | 429-433 |
| TAR | 474-499 |
| Poly A signal | 529-534 |
| | |
| **PBS** | **638-655** |

| **p7 binding region, packaging signal** | **685-791** |
|---|---|
| | |
| **Gag:** | **792-2285** |
| p17 | 792-1178 |
| p24 | 1179-1871 |
| Cyclophilin A bdg. | 1395-1505 |
| MHR | 1632-1694 |
| p2 | 1872-1907 |
| p7 | 1908-2072 |
| Frameshift slip | 2072-2078 |
| p1 | 2073-2120 |
| p6Gag | 2121-2285 |
| Zn-motif I | 1950-1991 |
| Zn-motif II | 2013-2054 |
| | |

| **PoI:** | **2072-5086** |
|---|---|
| p6Pol | 2072-2245 |
| Prot | 2246-2542 |
| p66RT | 2543-4210 |
| p15RNaseH | 3857-4210 |
| p31Int | 4211-5086 |
| | |

| **Vif:** | **5034-5612** |
|---|---|
| Hydrophilic region | 5292-5315 |
| | |

| **Vpr:** | **5552-5839** |
|---|---|
| Oligomerization | 5552-5677 |
| Amphipathic a-helix | 5597-5653 |
| | |

| **Tat:** | **5823-6038 and 8417-8509** |
|---|---|
| Tat-1 exon | 5823-6038 |
| Tat-2 exon | 8417-8509 |
| N-terminal domain | 5823-5885 |
| Trans-activation domain | 5886-5933 |
| Transduction domain | 5961-5993 |
| | |

| **Rev:** | **5962-6037 and 8416-8663** |
|---|---|
| Rev-1 exon | 5962-6037 |
| Rev-2 exon | 8416-8663 |
| High-affinity bdg. site | 8439-8486 |
| Leu-rich effector domain | 8562-8588 |
| | |

| **Vpu:** | **6060-6326** |
|---|---|
| Transmembrane domain | 6060-6161 |
| Cytoplasmic domain | 6162-6326 |
| | |

| **Env (gp160):** | **6244-8853** |
|---|---|
| Signal peptide | 6244-6324 |
| gp120 | 6325-7794 |
| V1 | 6628-6729 |
| V2 | 6727-6852 |
| V3 | 7150-7254 |
| V4 | 7411-7506 |
| V5 | 7663-7674 |
| C1 | 6325-6627 |
| C2 | 6853-7149 |
| C3 | 7255-7410 |
| C4 | 7507-7662 |
| C5 | 7675-7794 |
| CD4 binding | 7540-7566 |
| gp41 | 7795-8853 |
| Fusion peptide | 7789-7842 |
| Oligomerization domain | 7924-7959 |
| N-terminal heptad repeat | 7921-8028 |
| C-terminal heptad repeat | 8173-8280 |
| Immunodominant region | 8023-8076 |
| | |

| **Nef:** | **8855-9478** |
|---|---|
| Myristoylation | 8858-8875 |
| SH3 binding | 9062-9091 |
| Polypurine tract | 9128-9154 |
| SH3 binding | 9296-9307 |

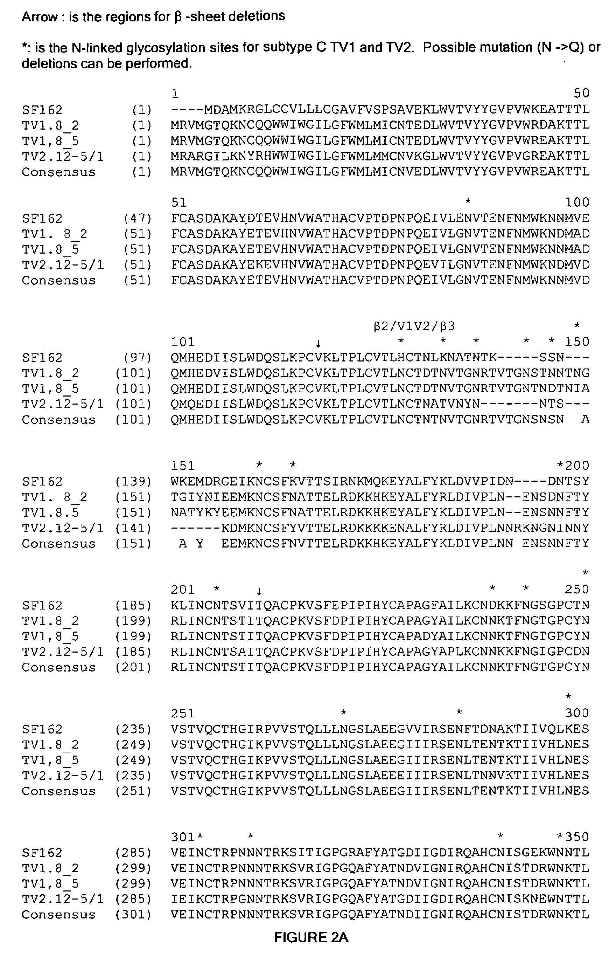

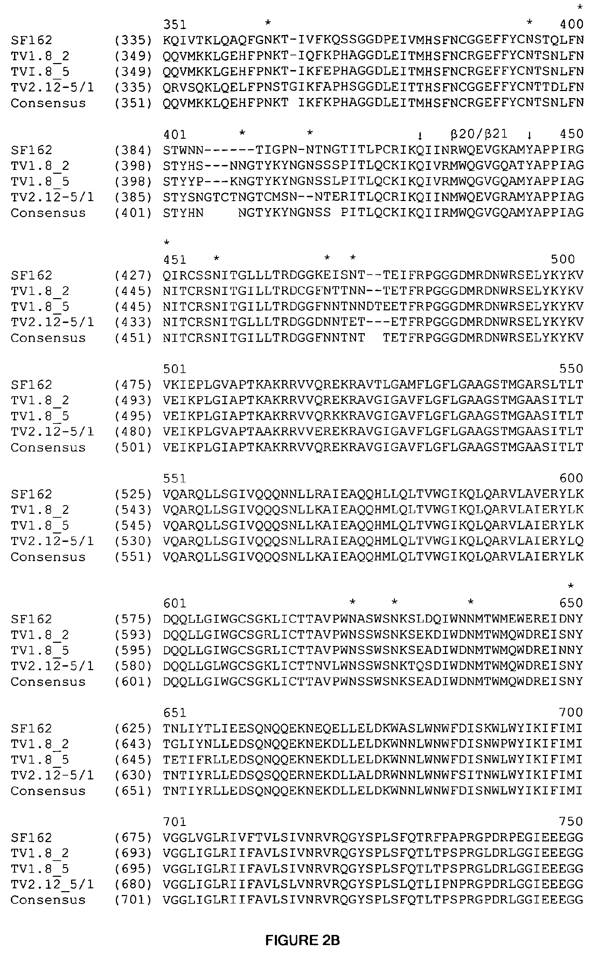

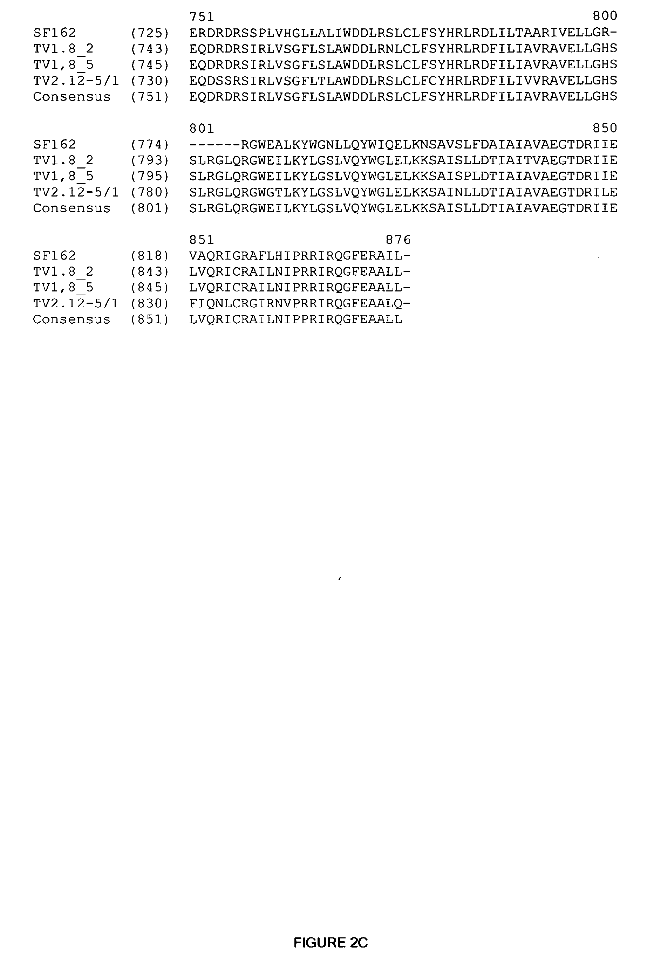

It will be readily apparent that one of skill in the art can readily align any sequence to that shown in Table A to determine relative locations of any particular HIV gene. For example, using one of the alignment programs described herein (*e.g*., BLAST), other HIV genomic sequences can be aligned with 8_5_TV1_C.ZA (Table A) and locations of genes determined. Polypeptide sequences can be similarly aligned. For example, Figures 2A-2C shows the alignment of Env polypeptide sequences from various strains, relative to SF-162. As described in detail in co-owned WO/39303, Env polypeptides (*e.g*., gp120, gp 140 and gp160) include a "bridging sheet" comprised of 4 anti-parallel β-strands (β-2, β-3, β-20 and β-21) that form a β-sheet. Extruding from one pair of the β-strands (β-2 and β-3) are two loops, V1 and V2. The β-2 sheet occurs at approximately amino acid residue 113 (Cys) to amino acid residue 117 (Thr) while β-3 occurs at approximately amino acid residue 192 (Ser) to amino acid residue 194 (Ile), relative to SF-162. The "V1/V2 region" occurs at approximately amino acid positions 120 (Cys) to residue 189 (Cys), relative to SF-162. Extruding from the second pair of β-strands (β-20 and β-21) is a "small-loop" structure, also referred to herein as "the bridging sheet small loop." The locations of both the small loop and bridging sheet small loop can be determined relative to HXB-2 following the teachings herein and in WO/39303. Also shown by arrows in Figure 2A-C are approximate sites for deletions sequence from the beta sheet region. The "*" denotes N-glycosylation sites that can be mutated following the teachings of the present specification.

### 2.2.0 SYNTHETIC EXPRESSION CASSETTES

One aspect of the present invention is the generation of HIV-1 coding sequences, and related sequences, for example having improved expression relative to the corresponding wild-type sequences.

### 2.2.1 MODIFICATION OF HIV-1 NUCLEIC ACID CODING SEQUENCES

One aspect of the present invention is the generation of HIV-1 coding sequences, and related sequences, having improved expression relative to the corresponding wild-type sequences.

First, the HIV-1 codon usage pattern was modified so that the resulting nucleic acid coding sequence was comparable to codon usage found in highly expressed human genes. The HIV codon usage reflects a high content of the nucleotides A or T of the codon-triplet. The effect of the HIV-1 codon usage is a high AT content in the DNA sequence that results in a decreased translation ability and instability of the mRNA. In comparison, highly expressed human codons prefer the nucleotides G or C. The HIV coding sequences were modified to be comparable to codon usage found in highly expressed human genes.

Second, there are inhibitory (or instability) elements (INS) located within the coding sequences of, for example, the Gag coding sequences. The RRE is a secondary RNA structure that interacts with the HIV encoded Rev-protein to overcome the expression down-regulating effects of the INS. To overcome the post-transcriptional activating mechanisms of RRE and Rev, the instability elements can be inactivated by introducing multiple point mutations that do not alter the reading frame of the encoded proteins.

Third, for some genes the coding sequence has been altered such that the polynucleotide coding sequence encodes a gene product that is inactive or non-functional (e.g., inactivated polymerase, protease, tat, rev, nef, vif, vpr, and/or vpu gene products). Example 1 describes some exemplary mutations. Example 8 presents information concerning functional analysis of mutated Tat, Rev and Nef antigens.

The synthetic coding sequences are assembled by methods known in the art, for example by companies such as the Midland Certified Reagent Company (Midland, Texas).

Modification of the Gag polypeptide coding sequences results in improved expression relative to the wild-type coding sequences in a number of mammalian cell lines (as well as other types of cell lines, including, but not limited to, insect cells).

Some exemplary polynucleotide sequences encoding Gag-containing polypeptides are gagCpollnaTatRevNefopt_B, GagProtInaRTmutTatRevNef.opt_B, GagTatRevNef.opt_B, GagComplPolmutInaTatRevNef_C, GagProtInaRTmutTatRevNef_C, GagRTmutTatRevNef_C, and GagTatRevNef_C.

Similarly, the present invention may also includes synthetic Env-encoding polynucleotides and modified Env proteins, for example, those described in WO 00/39303, WO 00/39302, WO 00/39304, WO 02/04493.

The codon usage pattern for Env was modified as described above for Gag so that the resulting nucleic acid coding sequence was comparable to codon usage found in highly expressed human genes. Experiments performed in support of the present invention show that the synthetic Env sequences were capable of higher level of protein production relative to the native Env sequences.

Modification of the Env polypeptide coding sequences results in improved expression relative to the wild-type coding sequences in a number of mammalian cell lines (as well as other types of cell lines, including, but not limited to, insect cells). Similar Env polypeptide coding sequences can be obtained, modified and tested for improved expression from a variety of isolates, including those described above for Gag.

Further modifications of Env include, but are not limited to, generating polynucleotides that encode Env polypeptides having mutations and/or deletions therein. For instance, the hypervariable regions, V1 and/or V2, can be deleted as described herein. Additionally, other modifications, for example to the bridging sheet region and/or to N-glycosylation sites within Env can also be performed following the teachings of the present specification. (see, Figure 2A-C, as well as WO 00/39303, WO 00/39302, WO 00/39304, WO 02/04493). Various combinations of these modifications can be employed to generate synthetic expression cassettes as described herein.

The present invention also includes expression cassettes which include synthetic Pol sequences. As noted above, "Pol" includes, but is not limited to, the protein-encoding regions comprising polymerase, protease, reverse transcriptase and/or integrase-containing sequences (Wan et et al (1996) Biochem. J. 316:569-573; Kohl et al. (1988) PNAS USA 85:4686-4690; Krausslich et al. (1988) J. Viral. 62:4393-4397; Coffin, "Retroviridae and their Replication" in Virology, pp1437-1500 (Raven, New York, 1990); Patel et. al. (1995) Biochemistry 34:5351-5363). Thus, the synthetic expression cassettes exemplified herein include one or more of these regions and one or more changes to the resulting amino acid sequences. Some exemplary polynucleotide sequences encoding Pol-derived polypeptides are presented in Table C.

The codon usage pattern for Pol was modified as described above for Gag and Env so that the resulting nucleic acid coding sequence was comparable to codon usage found in highly expressed human genes.

Constructs may be modified in various ways. For example, the expression constructs may include a sequence that encodes the first 6 amino acids of the integrase polypeptide. This 6 amino acid region is believed to provide a cleavage recognition site recognized by HIV protease (*see, e.g.,* McCornack et al. (1997) FEBS Letts 414:84-88). Constructs may include a multiple cloning site (MCS) for insertion of one or more transgenes, typically at the 3' end of the construct. In addition, a cassette encoding a catalytic center epitope derived from the catalytic center in RT is typically included 3' of the sequence encoding 6 amino acids of integrase. This cassette encodes Ile178 through Serine 191 of RT and may be added to keep this well conserved region as a possible CTL epitope. Further, the constructs contain an insertion mutations to preserve the reading frame. (see, e.g., Park et al. (1991) J. Virol. 65:5111).

In certain embodiments, the catalytic center and/or primer grip region of RT are modified. The catalytic center and primer grip regions of RT are described, for example, in Patel et al. (1995) Biochem. 34:5351 and Palaniappan et al. (1997) J. Biol. Chem. 272(17):11157. For example, wild type sequence encoding the amino acids YMDD at positions 183-185 of p66 RT, numbered relative to AF110975, may be replaced with sequence encoding the amino acids "AP". Further, the primer grip region (amino acids WMGY, residues 229-232 of p66RT, numbered relative to AF110975) may be replaced with sequence encoding the amino acids "PI."

For the Pol sequence, the changes in codon usage are typically restricted to the regions up to the -1 frameshift and starting again at the end of the Gag reading frame; however, regions within the frameshift translation region can be modified as well. Finally, inhibitory (or instability) elements (INS) located within the coding sequences of the protease polypeptide coding sequence can be altered as well.

Experiments can be performed in support of the present invention to show that the synthetic Pol sequences were capable of higher level of protein production relative to the native Pol sequences. Modification of the Pol polypeptide coding sequences results in improved expression relative to the wild-type coding sequences in a number of mammalian cell lines (as well as other types of cell lines, including, but not limited to, insect cells). Similar Pol polypeptide coding sequences can be obtained, modified and tested for improved expression from a variety of isolates, including those described above for Gag and Env.

The present invention also includes expression cassettes which include synthetic sequences derived HIV genes other than Gag, Env and Pol, including but not limited to, regions within Gag, Env, Pol, as well as, tat, rev, nef, vif, vpr, and vpu. Further, the present invention includes synthetic polynucleotides and/or expression cassettes comprising at least two antigenic polypeptides, wherein the antigenic peptides are selected from at least two different HIV types, for example, Type A, Type B, Type C, Type D, Type E, Type F, Type G, Type O, etc. The synthetic polynucleotide sequences of the present invention (comprising at least one polynucleotide encoding a polypeptide comprising a Type B antigen and at least one polynucleotide encoding a polypeptide comprising a Type C antigen) may be, for example, selected from the following sequences: gagCpolInaTatRevNef.opt_B, GagProtInaRTmutTatRevNef.opt_B, GagTatRevNef.opt_B, GagCompIPohnutlnaTatRevNef_C, GagProtInaRTmutTatRevNef_C, GagRTmutTatRevNef_C, GagTatRevNef_C, int.opt.mut.SF2, int.opt.SF2, int.opt.mut_C, int.opt_C, nef.D125G.-myr.opt.SF162, nef.D107G.-myr18.opt.SF162, nef.opt.D125G.SF162, nef.opt.SF162, Nef_TV1_C_ZAopt, Nef_TV2_C_ZAopt, NefD124G_TV1_C_ZAopt, NefD124G_TV2_C_ZAopt, NefD 124G-Myr_TV1_C_ZAopt, nef.D106G.-myr19.opt_C, p15RnaseH.opt.SF2, p15RnaseH.opt_C, p2Pol.opt.YMWM.SF2, p2PolInaopt.YM.SF2, p2Polopt.SF2, p2PolTatRevNef.opt.native_B, p2PolTatRevNef.op_B, p2Pol.opt.YMWM_C, p2Polopt.YM_C, p2Polopt_C, p2PolTatRevNef opt C, p2PolTatRevNef,opt,native_C, p2PolTatRevNef.opt_C, pol.opt.SF2, Pol_TVl_C_ZAopt, Pol_TV2_C_ZAopt, prot.opt.SF2, protIna.opt.SF2, protInaRT.YM.opt.SF2, protInaRT.YMWM.opt.SF2, ProtInaRTmut.SF2, protRT.opt.SF2, ProtRT.TatRevNefopt_B, ProtRTTatRevNef.opt_B, protInaRT.YM.opt_C, protInaRT.YMWM.opt_C, ProtRT.TatRevNef.opt_C, rev.exonl_2.M5-10.opt.SF162, rev.exonl_2.opt.SF162, rev.exon1_2.M5-10.opt_C, revexon1_2 TV1 C ZAopt, RT.opt.SF2 (mutant), RT.opt.SF2 (native), RTmut.SF2, tat.exon1_2.opt.C22-37.SF2, tat.exon1_2.optC37.SF2, tat.exon1_2. opt.C22-37_C, tat.exon1_2.opt.C37_C, TAT_CYS22_SF162_OPT, tat_sf162_opt, TatC22Exon1_2_TV1_C_ZAopt, TatExon1.2_TV1_C_ZAopt, TatRevNef.opt.native.SF162, TatRevNef.opt.SF162, TatRevNefGag B, TatRevNefgagCpo1Ina B, TatRevNefGagProtInaRTmut B, TatRevNefp2Po1.op_B, TatRevNefprotRTopt B, TatRevNef.opt.native_ ZA, TatRevNef.opt_ZA, TatRevNefGag C, TatRevNefgagCpolIna C, TatRevNefGagProtInaRTmut C, TatRevNefProtRT opt C, vif.opt.SF2, vpr.opt.SF2, vpu.opt.SF162, Vif_TV1_C_ZAopt, Vif_TV2_C_ZAopt, Vpr_TV1_C_ZAopt, Vpr_TV2 C_ZAopt, Vpu_TV1_C_ZAopt, Vpu_TV2_C_ZAopt, and fragments thereof. Such sequences may be used, for example, in their entirety or sequences encoding specific epitopes or antigens may be selected from the synthetic coding sequences following the teachings of the present specification and information known in the art. For example, the polypeptide sequences encoded by the polynucleotides may be subjected to computer analysis to predict antigenic peptide fragments within the full-length sequences. The corresponding polynucleotide coding fragments may then be used in the constructs of the present invention. Exemplary algorithms useful for such analysis include, but are not limited to, the following:
AMPHI. This program has been used to predict T-cell epitopes (Gao, et al., (1989) J. Immunol. 143:3007; Roberts, et al, (1996) AIDS Res Hum Retrovir 12:593; Quakyi, et al., (1992) Scand J Immunol suppl. 11:9). The AMPHI algorithm is available int the Protean package of DNASTAR, Inc. (Madison, WI, USA).

ANTIGENIC INDEX. This algorithm is useful for predicting antigenic determinants (Jameson & Wolf, (1998) CABIOS 4:181:186; Sherman, KE, et al., Hepatology 1996 Apr;23(4):688-94; Kasturi, KN, et al, J Exp Med 1995 Mar 1;181(3):1027-36; van Kampen V, et al., Mol Immunol 1994 Oct;31(15):1133-40; Ferroni P, et al., J Clin Microbiol 1993 Jun;31(6):1586-91; Beattie J, et al., Eur J Biochem 1992 Nov 15;210(1):59-66; Jones GL, et al, Mol Biochem Parasitol 1991 Sep;48(1):1-9).

HYDROPHILICITY. One algorithm useful for determining antigenic determinants from amino acid sequences was disclosed by Hopp & Woods (1981) PNAS USA 78:3824-3828.

Default parameters, for the above-recited algorithms, may be used to determine antigenic sites. Further, the results of two or more of the above analyses may be combined to identify particularly preferred fragments.

Sequences obtained from other strains can be manipulated in similar fashion following the teachings of the present specification. As noted above, the codon usage pattern is modified as described above for Gag, Env and Pol so that the resulting nucleic acid coding sequence is comparable to codon usage found in highly expressed human genes. Typically these synthetic sequences are capable of higher level of protein production relative to the native sequences and that modification of the wild-type polypeptide coding sequences results in improved expression relative to the wild-type coding sequences in a number of mammalian cell lines (as well as other types of cell lines, including, but not limited to, insect cells). Furthermore, the nucleic acid sequence can also be modified to introduce mutations into one or more regions of the gene, for instance to alter the function of the gene product (e.g., render the gene product non-functional) and/or to eliminate site modifications (e.g., the myristoylation site in Nef).

Synthetic expression cassettes, comprising at least one polynucleotide encoding a polypeptide comprising a Type B antigen and at least one polynucleotide encoding a polypeptide comprising a Type C antigen, may be, for example, derived from HIV Type B and Type C coding sequences, exemplified herein including, but not limited to, the following: gagCpolInaTatRevNef.opt_B, GagProtInaRTmutTatRevNef.opt_B, GagTatRevNef.op_B, GagComplPolmutInaTatRevNef_C, GagProtInaRTmutTatRevNef_C, GagRTmutTatRevNef_C, GagTatRevNef_C, int.opt.mut.SF2, int.opt.SF2, int.opt.mut_C, int.opt_C, nef.D125G.-myr.opt.SF162, nef.D107G.-myr18.opt.SF162, nef,opt.D125G.SF162, nef.opt.SF162, Nef_TV1_C_ZAopt, Nef_TV2_C_ZAopt, NefD124G_TV1_C_ZAopt, NefD124G_TV2_C_ZAopt, NefD124G-Myr_TV1_C_ZAopt, nef.D106G.-myr19.opt_C, p15RnaseH.opt.SF2, p15RnaseH.opt_C, p2Pol.opt.YMWM.SF2, p2PolInaopt.YM.SF2, p2Polopt.SF2, p2PolTatRevNef.opt.native_B, p2PolTatRevNef.opt_B, p2Po1.opt.YMWM_C, p2Polopt.YM_C, p2Polopt_C, p2PolTatRevNef opt C, p2PolTatRevNef.opt.native_C, p2PolTatRevNef.opt_C, pol.opt.SF2, Pol_TV1_C_ZAopt, Pol_TV2_C_ZAopt, prot.opt.SF2, protIna.opt.SF2, protInaRT.YM-opt.SF2, protlnaRT.YMWM.opt.SF2, ProtInaRTmut.SF2, protRT.opt.SF2, ProtRT.TatRevNef.opt_B, ProtRTTatRevNef.opt_B, protInaRT.YM.opt_C, protlnaRT.YMWM.opt_C, ProtRT.TatRevNef.opt_C, rev.exonl_2.M5-10.opt.SF162, rev.exon1_2.optSF162, rev.exonl_2.M5-10.op_C, revexon1_2 TV1 C ZAopt, RT.opt.SF2 (mutant), RT.opt.SF2 (native), RTmut.SF2, tatexonl_2.optC22-37.SF2, tat.exon1_2.opt.C37.SF2, tat.exon1_2.opt.C22-37_C, tat.exon1_2.opt.C37_C, TAT_CYS22_SF162_OPT, tat_sf162_opt, TatC22Exon1_2-TV1_C_ZAopt, TatExonl_2_TV1_C_ZAopt, TatRevNef.opt.native.SF162, TatRevNef.opt.SF162, TatRevNefGag B, TatRevNefgagCpolIna B, TatRevNefGagProtInaRTmut B, TatRevNefp2Pol,opt_B, TatRevNefprotRTopt B, TatRevNef.optnative_ZA, TatRevNef.opt_ZA, TatRevNefGag C, TatRevNefgagCpolIna C, TatRevNefGagProtInaRTmut C, TatRevNefProtRT opt C, vif.opt.SF2, vpr.opt.SF2, vpu.opt.SF162, Vif_TV1_c_ZAopt, Vif_TV2_C_ZAopt, Vpr_TV1_C_ZAopt, Vpr_TV2_C_ZAopt, Vpu_TV1_C_ZAopt, Vpu_TV2_C_ZAopt, and fragments thereof.

Gag-complete refers to in-frame polyproteins comprising, e.g., Gag and pol, wherein the p6 portion of Gag is present.

Additional sequences that may be employed in some aspects of the present invention have been described in WO 00/39302, WO 00/39303, WO 00/39304, and WO 02/04493.

### 2.2.2 FURTHER MODIFICATION OF SEQUENCES INCLUDING HIV NUCLEIC ACID CODING SEQUENCES

The HIV polypeptide-encoding expression cassettes described herein may also contain one or more further sequences encoding, for example, one or more transgenes. Further sequences (*e.g*., transgenes) useful in the practice of the present invention include, but are not limited to, further sequences are those encoding further viral epitopes/antigens {including but not limited to, HCV antigens (e.g., E1, E2; Houghton, M.., et al., U.S. Patent No. 5,714,596, issued February 3, 1998; Houghton, M.., et al., U.S. Patent No. 5,712,088, issued January 27, 1998; Houghton, M.., et al., U.S. Patent No. 5,683,864, issued November 4, 1997; Weiner, A.J., et al., U.S. Patent No. 5,728,520, issued March 17, 1998; Weiner, A.J., et al., U.S. Patent No. 5,766,845, issued June 16, 1998; Weiner, A.J., et al., U.S. Patent No. 5,670,152, issued September 23,1997), HIV antigens (e.g., derived from one or more HIV isolate); and sequences encoding tumor antigens/epitopes. Further sequences may also be derived from non-viral sources, for instance, sequences encoding cytokines such interleukin-2 (IL-2), stem cell factor (SCF), interleukin 3 (IL-3), interleukin 6 (IL-6), interleukin 12 (IL-12), G-CSF, granulocyte macrophage-colony stimulating factor (GM-CSF), interleukin-1 alpha (IL-lI), interleukin-11 (IL-11), MIP-lI, tumor necrosis factor (TNF), leukemia inhibitory factor (LIF), c-kit ligand, thrombopoietin (TPO) and flt3 ligand, commercially available from several vendors such as, for example, Genzyme (Framingham, MA), Genentech (South San Francisco, CA), Amgen (Thousand Oaks, CA), R&D Systems and Immunex (Seattle, WA). Additional sequences are described below. Also, variations on the orientation of the Gag and other coding sequences, relative to each other, are described below.

HIV polypeptide coding sequences can be obtained from other HIV isolates, see, e.g., Myers et al. Los Alamos Database, Los Alamos National Laboratory, Los Alamos, New Mexico (1992); Myers et al., Human Retroviruses and Aids, 1997, Los Alamos, New Mexico: Los Alamos National Laboratory. Synthetic expression cassettes can be generated using such coding sequences as starting material by following the teachings of the present specification.

Further, the synthetic expression cassettes of the present invention include related polypeptide sequences having greater than 85%, preferably greater than 90%, more preferably greater than 95%, and most preferably greater than 98% sequence identity to the polypeptides encoded by the synthetic expression cassette sequences disclosed herein.

Exemplary expression cassettes and modifications are set forth in Example 1.

### 2.2.3 EXPRESSION OF SYNTHETIC SEQUENCES ENCODING HIV-1 POLYPEPTIDES AND RELATED POLYPEPTIDES

Synthetic HIV-encoding sequences (expression cassettes) of the present invention can be cloned into a number of different expression vectors to evaluate levels of expression and, in the case of Gag-containing constructs, production of VLPs. The synthetic DNA fragments for HIV polypeptides can be cloned into eucaryotic expression vectors, including, a transient expression vector, CMV-promoter-based mammalian vectors, and a shuttle vector for use in baculovirus expression systems. Corresponding wild-type sequences can also be cloned into the same vectors.

These vectors can then be transfected into a several different cell types, including a variety of mammalian cell lines (293, RD, COS-7, and CHO, cell lines available, for example, from the A.T.C.C.). The cell lines are then cultured under appropriate conditions and the levels of any appropriate polypeptide product can be evaluated in supernatants. (see, Table A). For example, p24 can be used to evaluate Gag expression; gp160, gp140 or gp120 can be used to evaluate Env expression; p6pol can be used to evaluate Pol expression; prot can be used to evaluate protease; p15 for RNAseH; p31 for Integrase; and other appropriate polypeptides for Vif, Vpr, Tat, Rev, Vpu and Nef. Further, modified polypeptides can also be used, for example, other Env polypeptides include, but are not limited to, for example, native gp160, oligomeric gp140, monomeric gp120 as well as modified and/or synthetic sequences of these polypeptides. The results of these assays demonstrate that expression of synthetic HIV polypeptide-encoding sequences are significantly higher than corresponding wild-type sequences.

Further, Western Blot analysis can be used to show that cells containing the synthetic expression cassette produce the expected protein at higher per-cell concentrations than cells containing the native expression cassette. The HIV proteins can be seen in both cell lysates and supernatants. The levels of production are significantly higher in cell supernatants for cells transfected with the synthetic expression cassettes of the present invention.

Fractionation of the supernatants from mammalian cells transfected with the synthetic expression cassette can be used to show that the cassettes provide superior production of HIV proteins and, in the case of Gag, VLPs, relative to the wild-type sequences.

Efficient expression of these HIV-containing polypeptides in mammalian cell lines provides the following benefits: the polypeptides are free of baculovirus contaminants; production by established methods approved by the FDA; increased purity; greater yields (relative to native coding sequences); and a novel method of producing the Sub HIV-containing polypeptides in CHO cells which is not feasible in the absence of the increased expression obtained using the constructs of the present invention. Exemplary Mammalian cell lines include, but are not limited to, BHK, VERO, HT1080, 293, 293T, RD, COS-7, CHO, Jurkat, HUT, SUPT, C8166, MOLT4/clone8, MT-2, MT-4, H9, PM1, CEM, and CEMX174 (such cell lines are available, for example, from the A.T.C.C.).

A synthetic Gag expression cassette of the present invention will also exhibit high levels of expression and VLP production when transfected into insect cells. Synthetic expression cassettes described herein also demonstrate high levels of expression in insect cells. Further, in addition to a higher total protein yield, the final product from the synthetic polypeptides consistently contains lower amounts of contaminating baculovirus proteins than the final product from the native sequences.

Further, synthetic expression cassettes of the present invention can also be introduced into yeast vectors which, in turn, can be transformed into and efficiently expressed by yeast cells (*Saccharomyces cerevisea;* using vectors as described in Rosenberg, S. and Tekamp-Olson, P., U.S. Patent No. RE35,749, issued, March 17, 1998).

In addition to the mammalian and insect vectors, the synthetic expression cassettes of the present invention can be incorporated into a variety of expression vectors using selected expression control elements. Appropriate vectors and control elements for any given cell an be selected by one having ordinary skill in the art in view of the teachings of the present specification and information known in the art about expression vectors.

For example, a synthetic expression cassette can be inserted into a vector which includes control elements operably linked to the desired coding sequence, which allow for the expression of the gene in a selected cell-type. For example, typical promoters for mammalian cell expression include the SV40 early promoter, a CMV promoter such as the CMV immediate early promoter (a CMV promoter can include intron A), RSV, HIV-Ltr, the mouse mammary tumor virus LTR promoter (MMLV-ltr), the adenovirus major late promoter (Ad MLP), and the herpes simplex virus promoter, among others. Other nonviral promoters, such as a promoter derived from the murine metallothionein gene, will also find use for mammalian expression. Typically, transcription termination and polyadenylation sequences will also be present, located 3' to the translation stop codon. Preferably, a sequence for optimization of initiation of translation, located 5' to the coding sequence, is also present. Examples of transcription terminator/polyadenylation signals include those derived from SV40, as described in Sambrook, et al., *supra,* as well as a bovine growth hormone terminator sequence. Introns, containing splice donor and acceptor sites, may also be designed into the constructs for use with the present invention (Chapman et al., Nuc. Acids Res. (1991) 19:3979-3986).

Enhancer elements may also be used herein to increase expression levels of the mammalian constructs. Examples include the SV40 early gene enhancer, as described in Dijkema et al., EMBO J. (1985) 4:761, the enhancer/promoter derived from the long terminal repeat (LTR) of the Rous Sarcoma Virus, as described in Gorman et al., Proc. Natl. Acad. Sei. USA (1982b) 79:6777 and elements derived from human CMV, as described in Boshart et al., Cell (1985) 41:521, such as elements included in the CMV intron A sequence (Chapman et al., Nuc. Acids Res. (1991) 19:3979-3986).

The desired synthetic polypeptide encoding sequences can be cloned into any number of commercially available vectors to generate expression of the polypeptide in an appropriate host system. These systems include, but are not limited to, the following: baculovirus expression {Reilly, P.R., et al., BACULOVIRUS EXPRESSION VECTORS: A LABORATORY MANUAL (1992); Beames, et al., Biotechniques 11:378 (1991); Pharmingen; Clontech, Palo Alto, CA)}, vaccinia expression {Earl, P. L., et al., "Expression of proteins in mammalian cells using vaccinia" In Current Protocols in Molecular Biology (F. M. Ausubel, et al. Eds.), Greene Publishing Associates & Wiley Interscience, New York (1991); Moss, B., et al., U.S. Patent Number 5,135,855, issued 4 August 1992}, expression in bacteria {Ausubel, F.M., et al., CURRENT PROTOCOLS IN MOLECULAR BIOLOGY, John Wiley and Sons, Inc., Media PA; Clontech}, expression in yeast {Rosenberg, S. and Tekamp-Olson, P., U.S. Patent No. RE35,749, issued, March 17, 1998; Shuster, J.R., U.S. Patent No. 5,629,203, issued May 13, 1997; Gellissen, G., et al., Antonie Van Leeuwenhoek, 62(1-2):79-93 (1992); Romanos, M.A., et al., Yeast 8(6):423-488 (1992); Goeddel, D.V., Methods in Enzymology 185 (1990); Guthrie, C., and G.R. Fink, Methods in Enzymology 194 (1991)}, expression in mammalian cells {Clontech; Gibco-BRL, Ground Island, NY; *e.g*., Chinese hamster ovary (CHO) cell lines (Haynes, J., et al., Nuc. Acid. Res. 11:687-706 (1983); 1983, Lau, Y.F., et al., Mol. Cell. Biol. 4:1469-1475 (1984); Kaufman, R. J., "Selection and coamplification of heterologous genes in mammalian cells," in Methods in Enzymology, vol. 185, pp537-566. Academic Press, Inc., San Diego CA (1991)}, and expression in plant cells {plant cloning vectors, Clontech Laboratories, Inc., Palo Alto, CA, and Pharmacia LKB Biotechnology, Inc., Pistcataway, NJ; Hood, E., et al., J. Bacteriol. 168:1291-1301 (1986); Nagel, R., et al., FEMS Microbiol. Lett. 67:325 (1990); An, et al., "Binary Vectors", and others in Plant Molecular Biology Manual A3:1-19 (1988); Miki, B.L.A., et al., pp.249-265, and others in Plant DNA Infectious Agents (Hohn, T., et al., eds.) Springer-Verlag, Wien, Austria, (1987); Plant Molecular Biology: Essential Techniques, P.G. Jones and J.M. Sutton, New York, J. Wiley, 1997; Miglani, Gurbachan Dictionary of plant Genetics and Molecular Biology, New York, Food Products Press, 1998; Henry, R. J., Practical Applications of Plant Molecular Biology, New York, Chapman & Hall, 1997}.

Also included in the invention is an expression vector, containing coding sequences and expression control elements which allow expression of the coding regions in a suitable host. The control elements generally include a promoter, translation initiation codon, and translation and transcription termination sequences, and an insertion site for introducing the insert into the vector. Translational control elements have been reviewed by M. Kozak (e.g., Kozak, M., Mamm. Genome 7(8):563-574, 1996; Kozak, M., Biochimie 76(9):815-821, 1994; Kozak, M., J Cell Biol 108(2):229-241, 1989; Kozak, M., and Shatkin, A.J., Methods Enzymol 60:360-375, 1979).

Expression in yeast systems has the advantage of commercial production. Recombinant protein production by vaccinia and CHO cell line have the advantage of being mammalian expression systems. Further, vaccinia virus expression has several advantages including the following: (i) its wide host range; (ii) faithful post-transcriptional modification, processing, folding, transport, secretion, and assembly of recombinant proteins; (iii) high level expression of relatively soluble recombinant proteins; and (iv) a large capacity to accommodate foreign DNA.

The recombinantly expressed polypeptides from synthetic HIV polypeptide-encoding expression cassettes are typically isolated from lysed cells or culture media. Purification can be carried out by methods known in the art including salt fractionation, ion exchange chromatography, gel filtration, size-exclusion chromatography, size-fractionation, and affinity chromatography. Immunoaffinity chromatography can be employed using antibodies generated based on, for example, HIV antigens.

Advantages of expressing the proteins of the present invention using mammalian cells include, but are not limited to, the following: well-established protocols for scale-up production; the ability to produce VLPs; cell lines are suitable to meet good manufacturing process (GMP) standards; culture conditions for mammalian cells are known in the art.

Synthetic HIV 1 polynucleotides are described herein, see, for example, the figures. Various forms of the different embodiments of the invention, described herein, may be combined.

Exemplary expression assays are set forth in Example 2. Exemplary conditions for Western Blot analysis are presented in Example 3.

### 2.3.0 PRODUCTION OF VIRUS-LIKE PARTICLES AND USE OF THE CONSTRUCTS OF THE PRESENT INVENTION TO CREATE PACKAGING CELL LINES.

The group-specific antigens (Gag) of human immunodeficiency virus type-1 (HIV-1) self-assemble into noninfectious virus-like particles (VLP) that are released from various eucaryotic cells by budding (reviewed by Freed, E.O., Virology 251:1-15, 1998). The Gag-containing synthetic expression cassettes of the present invention provide for the production of HIV-Gag virus-like particles (VLPs) using a variety of different cell types, including, but not limited to, mammalian cells.

Viral particles can be used as a matrix for the proper presentation of an antigen entrapped or associated therewith to the immune system of the host.

### 2.3.1 VLP PRODUCTION USING THE SYNTHETIC EXPRESSION CASSETTES OF THE PRESENT INVENTION

The Gag-containing synthetic expression cassettes of the present invention may provide superior production of both Gag proteins and VLPs, relative to native Gag coding sequences. Further, electron microscopic evaluation of VLP production can be used to show that free and budding immature virus particles of the expected size are produced by cells containing the synthetic expression cassettes.

Using the synthetic expression cassettes of the present invention, rather than native Gag coding sequences, for the production of virus-like particles provide several advantages. First, VLPs can be produced in enhanced quantity making isolation and purification of the VLPs easier. Second, VLPs can be produced in a variety of cell types using the synthetic expression cassettes, in particular, mammalian cell lines can be used for VLP production, for example, CHO cells. Production using CHO cells provides (i) VLP formation; (ii) correct myristoylation and budding; (iii) absence of non-Macmillian cell contaminants (e.g., insect viruses and/or cells); and (iv) ease of purification. The synthetic expression cassettes of the present invention are also useful for enhanced expression in cell-types other than mammalian cell lines. For example, infection of insect cells with baculovirus vectors encoding the synthetic expression cassettes results in higher levels of total Gag protein yield and higher levels of VLP production (relative to wild-type coding sequences). Further, the final product from insect cells infected with the baculovirus-Gag synthetic expression cassettes consistently contains lower amounts
of contaminating insect proteins than the final product when wild-coding sequences are used.

VLPs can spontaneously form when the particle-forming polypeptide of interest is recombinantly expressed in an appropriate host cell. Thus, the VLPs produced using the synthetic expression cassettes of the present invention are conveniently prepared using recombinant techniques. As discussed below, the Gag polypeptide encoding synthetic expression cassettes of the present invention can include other polypeptide coding sequences of interest (for example, HIV protease, HIV polymerase, Env; synthetic Env). Expression of such synthetic expression cassettes yields VLPs comprising the Gag polypeptide, as well as, the polypeptide of interest.

Once coding sequences for the desired particle-forming polypeptides have been isolated or synthesized, they can be cloned into any suitable vector or replicon for expression. Numerous cloning vectors are known to those of skill in the art, and the selection of an appropriate cloning vector is a matter of choice. See, generally, Sambrook et al, *supra.* The vector is then used to transform an appropriate host cell. Suitable recombinant expression systems include, but are not limited to, bacterial, mammalian, baculovirus/insect, vaccinia, Semliki Forest virus (SFV), Alphaviruses (such as, Sindbis, Venezuelan Equine Encephalitis (VEE)), mammalian, yeast and Xenopus expression systems, well known in the art. Particularly preferred expression systems are mammalian cell lines, vaccinia, Sindbis, eucaryotic layered vector initiation systems (e.g., US Patent No. 6,015,686, US Patent No. 5, 814,482, US Patent No. 6,015,694, US Patent No. 5,789,245, EP 1029068A2, WO 9918226A2/A3, EP 00907746A2, WO 9738087A2), insect and yeast systems.

The synthetic DNA fragments for the expression cassettes of the present invention, e.g., Pol, Gag, Env, Tat, Rev, Nef, Vif, Vpr, and/or Vpu, may be cloned into the following eucaryotic expression vectors: pCMVKm2, for transient expression assays and DNA immunization studies, the pCMVKm2 vector is derived from pCMV6a (Chapman et al., Nuc. Acids Res. (1991) 19:3979-3986) and comprises a kanamycin selectable marker, a ColE1 origin of replication, a CMV promoter enhancer and Intron A, followed by an insertion site for the synthetic sequences described below followed by a polyadenylation signal derived from bovine growth hormone -- the pCMVKm2 vector differs from the pCMV-link vector only in that a polylinker site is inserted into pCMVKm2 to generate pCMV-link; pESN2dhfr and pCMVPLEdhfr, for expression in Chinese Hamster Ovary (CHO) cells; and, pAcC13, a shuttle vector for use in the Baculovirus expression system (pAcC13, is derived from pAcC12 which is described by Munemitsu S., et al., Mol Cell Biol. 10(11):5977-5982, 1990).

Briefly, construction of pCMVPLEdhfr was as follows.

To construct a DHFR cassette, the EMCV IRES (internal ribosome entry site) leader was PCR-amplified from pCite-4a+ (Novagen, Inc., Milwaukee, WI) and inserted into pET-23d (Novagen, Inc., Milwaukee, WI) as an *Xba-Nco* fragment to give pET-EMCV. The *dhfr* gene was PCR-amplified from pESN2dhfr to give a product with a Gly-Gly-Gly-Ser spacer in place of the translation stop codon and inserted as an *Nco-Bam*H1 fragment to give pET-E-DHFR. Next, the attenuated *neo* gene was PCR amplified from a pSV2Neo (Clontech, Palo Alto, CA) derivative and inserted into the unique *Bam*H1 site of pET-E-DHFR to give pET-B-DHFR/Neo₍ₘ₂₎. Finally the bovine growth hormone terminator from pCDNA3 (Invitrogen, Inc., Carlsbad, CA) was inserted downstream of the *neo* gene to give pET-E-DHFR/Neo₍ₘ₂)BGHt. The EMCV*-dhfr*/*neo* selectable marker cassette fragment was prepared by cleavage of pET-E-DHFR/Neo₍ₘ₂₎BGHt.

In one vector construct the CMV enhancer/promoter plus Intron A was transferred from pCMV6a (Chapman et al., *Nuc. Acids Res.* (1991) 19:3979-3986) as a *Hiti*dIII*-Sal*1 fragment into pUC19 (New England Biolabs, Inc., Beverly, MA). The vector backbone of pUC19 was deleted from the Nde1 to the Sap1 sites. The above described DHFR cassette was added to the construct such that the EMCV IRES followed the CMV promoter. The vector also contained an amp^{r} gene and an SV40 origin of replication.

A number of mammalian cell lines are known in the art and include immortalized cell lines available from the American Type Culture Collection (A.T.C.C.), such as, but not limited to, Chinese hamster ovary (CHO) cells, HeLa cells, baby hamster kidney (BHK) cells, monkey kidney cells (COS), as well as others. Similarly, bacterial hosts such as *E. coli, Bacillus subtilis,* and *Streptococcus spp.,* will find use with the present expression constructs. Yeast hosts useful in the present invention include *inter alia, Saccharomyces cerevisiae, Candida albicans, Candida maltosa, Hansenula polymorpha, Kluyveromyces fragilis, Kluyveromyces lactis, Pichia guillerimondii, Pichia pastoris, Schizosaccharomyces pombe* and *Yarrowia lipolytica.* Insect cells for use with baculovirus expression vectors include, *inter alia, Aedes aegypti, Autographa californica, Bombyx mori, Drosophila melanogaster, Spodoptera frugiperda,* and *Trichoplusia ni.* See, e.g., Summers and Smith, Texas Agricultural Experiment Station Bulletin No. 1555 (1987).

Viral vectors can be used for the production of particles in eucaryotic cells, such as those derived from the pox family of viruses, including vaccinia virus and avian poxvirus. Additionally, a vaccinia based infection/transfection system, as described in Tomei et al., J. Virol. (1993) 67:4017-4026 and Selby et al., J. Gen. Virol. (1993) 74:1103-1113, will also find use with the present invention. In this system, cells are first *infected in vitro* with a vaccinia virus recombinant that encodes the bacteriophage T7 RNA polymerase. This polymerase displays exquisite specificity in that it only transcribes templates bearing T7 promoters. Following infection, cells are transfected with the DNA of interest, driven by a T7 promoter. The polymerase expressed in the cytoplasm from the vaccinia virus recombinant transcribes the transfected DNA into RNA which is then translated into protein by the host translational machinery. Alternately, T7 can be added as a purified protein or enzyme as in the "Progenitor" system (Studier and Moffatt, J. Mol. Biol. (1986) 189:113-130). The method provides for high level, transient, cytoplasmic production of large quantities of RNA and its translation product(s).

Depending on the expression system and host selected, the VLPS are produced by growing host cells transformed by an expression vector under conditions whereby the particle-forming polypeptide is expressed and VLPs can be formed. The selection of the appropriate growth conditions is within the skill of the art. If the VLPs are formed intracellularly, the cells are then disrupted, using chemical, physical or mechanical means, which lyse the cells yet keep the VLPs substantially intact. Such methods are known to those of skill in the art and are described in, e.g., Protein Purification Applications: A Practical Approach, (E.L.V. Harris and S. Angal, Eds., 1990).

The particles are then isolated (or substantially purified) using methods that preserve the integrity thereof, such as, by gradient centrifugation, e.g., cesium chloride (CsCl) sucrose gradients, pelleting and the like (see, e.g., Kirnbauer et al. J. Virol. (1993) 67:6929-6936), as well as standard purification techniques including, e.g., ion exchange and gel filtration chromatography.

VLPs produced by cells containing the synthetic expression cassettes of the present invention can be used to elicit an immune response when administered to a subject. One advantage of the present invention is that VLPs can be produced by mammalian cells carrying the synthetic expression cassettes at levels previously not possible. As discussed above, the VLPs can comprise a variety of antigens in addition to the Gag polypeptide (e.g., Gag-protease, Gag-polymerase, Env, synthetic Env, etc.). Purified VLPs, produced using the synthetic expression cassettes of the present invention, can be administered to a vertebrate subject, usually in the form of vaccine compositions. Combination vaccines may also be used, where such vaccines contain, for example, an adjuvant subunit protein (e.g., Env). Administration can take place using the VLPs formulated alone or formulated with other antigens. Further, the VLPs can be administered prior to, concurrent with, or subsequent to, delivery of the synthetic expression cassettes for DNA immunization (see below) and/or delivery of other vaccines. Also, the site of VLP administration may be the same or different as other vaccine compositions that are being administered. Gene delivery can be accomplished by a number of methods including, but are not limited to, immunization with DNA, alphavirus vectors, pox virus vectors, and vaccinia virus vectors.

VLP immune-stimulating (or vaccine) compositions can include various excipients, adjuvants, carriers, auxiliary substances, modulating agents, and the like. The immune stimulating compositions will include an amount of the VLP/antigen sufficient to mount an immunological response. An appropriate effective amount can be determined by one of skill in the art. Such an amount will fall in a relatively broad range that can be determined through routine trials and will generally be an amount on the order of about 0.1 µg to about 1000 µg, more preferably about 1 µg to about 300 ug, of VLP/antigen.

A carrier is optionally present which is a molecule that does not itself induce the production of antibodies harmful to the individual receiving the composition. Suitable carriers are typically large, slowly metabolized macromolecules such as proteins, polysaccharides, polylactic acids, polyglycollic acids, polymeric amino acids, amino acid copolymers, lipid aggregates (such as oil droplets or liposomes), and inactive virus particles. Examples of particulate carriers include those derived from polymethyl methacrylate polymers, as well as microparticles derived from poly(lactides) and poly(lactide-co-glycolides), known as PLG. See, e.g., Jeffery et al., Pharm. Res. (1993) 10:362-368; McGee JP, et al., J Microencapsul. 14(2):197-210, 1997; O'Hagan DT, et al., Vaccine 11(2): 149-54, 1993. Such carriers are well known to those of ordinary skill in the art. Additionally, these carriers may function as immunostimulating agents ("adjuvants"). Furthermore, the antigen may be conjugated to a bacterial toxoid, such as toxoid from diphtheria, tetanus, cholera, etc., as well as toxins derived from *E. coli.*

Adjuvants may also be used to enhance the effectiveness of the compositions. Such adjuvants include, but are not limited to: (1) aluminum salts (alum), such as aluminum hydroxide, aluminum phosphate, aluminum sulfate, etc.; (2) oil-in-water emulsion formulations (with or without other specific immunostimulating agents such as muramyl peptides (see below) or bacterial cell wall components), such as for example (a) MF59 (International Publication No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particle generated therefrom such as ISCOMs (immunostimulating complexes); (4) Complete Freunds Adjuvant (CFA) and Incomplete Freunds Adjuvant (IFA); (5) cytokines, such as interleukins (IL-1, IL-2, etc.), macrophage colony stimulating factor (M-CSF), tumor necrosis factor (TNF), etc.; (6) oligonucleotides or polymeric molecules encoding immunostimulatory CpG motifs (Davis, H.L., et al., J. Immunology 160:870-876, 1998; Sato, Y. et al., Science 273:352-354, 1996) or complexes of antigens/oligonucleotides {Polymeric molecules include double and single stranded RNA and DNA, and backbone modifications thereof, for example, methylphosphonate linkages; or (7) detoxified mutants of a bacterial ADP-ribosylating toxin such as a cholera toxin (CT), a pertussis toxin (PT), or an *E. coli* heat-labile toxin (LT), particularly LT-K63 (where lysine is substituted for the wild-type amino acid at position 63) LT-R72 (where arginine is substituted for the wild-type amino acid at position 72), CT-S109 (where serine is substituted for the wild-type amino acid at position 109), and PT-K9/G129 (where lysine is substituted for the wild-type amino acid at position 9 and glycine substituted at position 129) (see, e.g., International Publication Nos. W093/13202 and W092/19265); and (8) other substances that act as immunostimulating agents to enhance the effectiveness of the composition. Further, such polymeric molecules include alternative polymer backbone structures such as, but not limited to, polyvinyl backbones (Pitha, Biochem Biophys Acta, 204:39, 1970a; Pitha, Biopolymers, 9:965, 1970b), and morpholino backbones (Summerton, J., et al., U.S. Patent No. 5,142,047, issued 08/25/92; Summerton, J., et al., U.S. Patent No. 5,185,444 issued 02/09/93). A variety of other charged and uncharged polynucleotide analogs have been reported. Numerous backbone modifications are known in the art, including, but not limited to, uncharged linkages (*e.g*., methyl phosphonates, phosphotriesters, phosphoamidates, and carbamates) and charged linkages (*e.g*., phosphorothioates and phosphorodithioates).}; and (7) other substances that act as immunostimulating agents to enhance the effectiveness of the VLP immune-stimulating (or vaccine) composition. Alum, CpG oligonucleotides, and MF59 are preferred.

Muramyl peptides include, but are not limited to, N-acetyl-muramyl-L-threonyl-D-isoglutamine (thr-MDP), N-acteyl-normuramyl-L-alanyl-D-isogluatme (nor-MDP), N-acetylmuramyl-L-alanyl-D-isogluatminyl-L-alanine-2-(1'-2-dipalmitoyl-*sn*-glycero-3-huydroxyphosphoryloxy)-ethylamine (MTP-PE), etc.

Dosage treatment with the VLP composition may be a single dose schedule or a multiple dose schedule. A multiple dose schedule is one in which a primary course of vaccination may be with 1-10 separate doses, followed by other doses given at subsequent time intervals, chosen to maintain and/or reinforce the immune response, for example at 1-4 months for a second dose, and if needed, a subsequent dose(s) after several months. The dosage regimen will also, at least in part, be determined by the need of the subject and be dependent on the judgment of the practitioner.

If prevention of disease is desired, the antigen carrying VLPs are generally administered prior to primary infection with the pathogen of interest. If treatment is desired, e.g., the reduction of symptoms or recurrences, the VLP compositions are generally administered subsequent to primary infection.

### 2.3.2 USING THE SYNTHETIC EXPRESSION CASSETTES OF THE PRESENT INVENTION TO CREATE PACKAGING CELL LINES

A number of viral based systems have been developed for use as gene transfer vectors for mammalian host cells. For example, retroviruses (in particular, lentiviral vectors) provide a convenient platform for gene delivery systems. A coding sequence of interest (for example, a sequence useful for gene therapy applications) can be inserted into a gene delivery vector and packaged in retroviral particles using techniques known in the art. Recombinant virus can then be isolated and delivered to cells of the subject either *in vivo* or *ex vivo.* A number of retroviral systems have been described, including, for example, the following: (U.S. Patent No. 5,219,740; Miller et al. (1989) BioTechniques 7:980; Miller, A.D. (1990) Human Gene Therapy 1:5*;* Scarpa et al. (1991) Virology 180:849; Burns et al. (1993) Proc. Natl. Acad. Sci. USA 90:8033; Boris-Lawrie et al. (1993) Cur. Opin. Genet. Develop. 3:102; GB 2200651; EP 0415731; EP 0345242; WO 89/02468; WO 89/05349; WO 89/09271; WO 90/02806; WO 90/07936; WO 90/07936; WO 94/03622; WO 93/25698; WO 93/25234; WO 93/11230; WO 93/10218; WO 91/02805; in U.S. 5,219,740; U.S. 4,405,712; U.S. 4,861,719; U.S. 4,980,289 and U.S. 4,777,127; in U.S. Serial No. 07/800,921; and in Vile (1993) Cancer Res 53:3860-3864; Vile (1993) Cancer Res 53:962-967; Ram (1993) Cancer Res 53:83-88; Takamiya (1992) J Neurosci Res 33:493-503; Baba (1993) J Neurosurg 79:729-735; Mann (1983) Cell 33:153; Cane (1984) Proc Natl Acad Sci USA 81;6349; and Miller (1990) Human Gene Therapy 1*.*

In other embodiments, gene transfer vectors can be constructed to encode a cytokine or other immunomodulatory molecule. For example, nucleic acid sequences encoding native IL-2 and gamma-interferon can be obtained as described in US Patent Nos. 4,738,927 and 5,326,859, respectively, while useful muteins of these proteins can be obtained as described in U.S. Patent No. 4,853,332. Nucleic acid sequences encoding the short and long forms of mCSF can be obtained as described in US Patent Nos. 4,847,201 and 4,879,227, respectively. In particular aspects of the invention, retroviral vectors expressing cytokine or immunomodulatory genes can be produced as described herein (for example, employing the packaging cell lines of the present invention) and in International Application No. PCT US 94/02951, entitled "Compositions and Methods for Cancer Immunotherapy."

Examples of suitable immunomodulatory molecules for use herein include the following: IL-1 and IL-2 (Karupiah et al. (1990) J. Immunology 144:290-298, Weber et al. (1987) J. Exp. Med. 166:1716-1733, Gansbacher et al. (1990) J. Exp. Med. 172:1217-1224, and U.S. Patent No. 4,738,927); IL-3 and IL-4 (Tepper et al. (1989) Cell 57:503-512, Golumbek et al. (1991) Science 254:713-716, and U.S. Patent No. 5,017,691); IL-5 and IL-6 (Brakenhof et al. (1987) J. Immunol. 139:4116-4121, and International Publication No. WO 90/06370); IL-7 (U.S. Patent No. 4,965,195); IL-8, IL-9, IL-10, IL-11, IL-12, and IL-13 *(*Cytokine Bulletin, Summer 1994); IL-14 and IL-15; alpha interferon (Finter et al. (1991) Drugs 42:749-765, U.S. Patent Nos. 4,892,743 and 4,966,843, International Publication No. WO 85/02862, Nagata et al. (1980) Nature 284:316-320, Familletti et al. (1981) Methods in Enz. 78:387-394, Twu et al. (1989) Proc. Natl. Acad. Sci. USA 86:2046-2050, and Faktor et al. (1990) Oncogene 5:867-872); beta-interferon (Seif et al. (1991) J. Virol. 65:664-671); gamma-interferons (Radford et al. (1991) The American Society of Hepatology 20082015, Watanabe et al. (1989) Proc. Natl. Acad. Sci. USA 86:9456-9460, Gansbacher et al. (1990) Cancer Research 50:7820-7825, Maio et al. (1989) Can. Immunol. Immunother. 30:34-42, and U.S. Patent Nos. 4,762,791 and 4,727,138); G-CSF (U.S. Patent Nos. 4,999,291 and 4,810,643); GM-CSF (International Publication No. WO 85/04188).

Immunomodulatory factors may also be agonists, antagonists, or ligands for these molecules. For example, soluble forms of receptors can often behave as antagonists for these types of factors, as can mutated forms of the factors themselves.

Nucleic acid molecules that encode the above-described substances, as well as other nucleic acid molecules that are advantageous for use within the present invention, may be readily obtained from a variety of sources, including, for example, depositories such as the American Type Culture Collection, or from commercial sources such as British Bio-Technology Limited (Cowley, Oxford England). Representative examples include BBG 12 (containing the GM-CSF gene coding for the mature protein of 127 amino acids), BBG 6 (which contains sequences encoding gamma interferon), A.T.C.C. Deposit No. 39656 (which contains sequences encoding TNF), A.T.C.C. Deposit No. 20663 (which contains sequences encoding alpha-interferon), A.T.C.C. Deposit Nos. 31902, 31902 and 39517 (which contain sequences encoding beta-interferon), A.T.C.C. Deposit No. 67024 (which contains a sequence which encodes Interleukin-1b), A.T.C.C. Deposit Nos. 39405, 39452, 39516, 39626 and 39673 (which contain sequences encoding Interleukin-2), A.T.C.C. Deposit Nos. 59399, 59398, and 67326 (which contain sequences encoding Interleukin-3), A.T.C.C. Deposit No. 57592 (which contains sequences encoding Interleukin-4), A.T.C.C. Deposit Nos. 59394 and 59395 (which contain sequences encoding Interleukin-5), and A.T.C.C. Deposit No. 67153 (which contains sequences encoding Interleukin-6).

Plasmids containing cytokine genes or immunomodulatory genes (International Publication Nos. WO 94/02951 and WO 96/21015) can be digested with appropriate restriction enzymes, and DNA fragments containing the particular gene of interest can be inserted into a gene transfer vector using standard molecular biology techniques. (*See, e.g.,* Sambrook et al., *supra.,* or Ausubel et al. (eds) Current Protocols in Molecular Biology, Greene Publishing and Wiley-Interscience).

Polynucleotide sequences coding for the above-described molecules can be obtained using recombinant methods, such as by screening cDNA and genomic libraries from cells expressing the gene, or by deriving the gene from a vector known to include the same. For example, plasmids which contain sequences that encode altered cellular products may be obtained from a depository such as the A.T.C.C., or from commercial sources. Plasmids containing the nucleotide sequences of interest can be digested with appropriate restriction enzymes, and DNA fragments containing the nucleotide sequences can be inserted into a gene transfer vector using standard molecular biology techniques.

Alternatively, cDNA sequences for use with the present invention may be obtained from cells which express or contain the sequences, using standard techniques, such as phenol extraction and PCR of cDNA or genomic DNA. See, e.g., Sambrook et al., *supra,* for a description of techniques used to obtain and isolate DNA. Briefly, mRNA from a cell which expresses the gene of interest can be reverse transcribed with reverse transcriptase using oligo-dT or random primers. The single stranded cDNA may then be amplified by PCR (see U.S. Patent Nos. 4,683,202, 4,683,195 and 4,800,159, see also PCR Technology: Principles and Applications for DNA Amplification, Erlich (ed.), Stockton Press, 1989)) using oligonucleotide primers complementary to sequences on either side of desired sequences.

The nucleotide sequence of interest can also be produced synthetically, rather than cloned, using a DNA synthesizer (*e.g*., an Applied Biosystems Model 392 DNA Synthesizer, available from ABI, Foster City, California). The nucleotide sequence can be designed with the appropriate codons for the expression product desired. The complete sequence is assembled from overlapping oligonucleotides prepared by standard methods and assembled into a complete coding sequence. See, e.g., Edge (1981) Nature 292:756; Nambair et al. (1984) Science 223:1299; Jay et al. (1984) J. Biol. Chem. 259:6311.

The synthetic expression cassettes of the present invention can be employed in the construction of packaging cell lines for use with retroviral vectors.

One type of retrovirus, the murine leukemia virus, or "MLV", has been widely utilized for gene therapy applications (see generally Mann et al. (Cell 33:153, 1993), Cane and Mulligan (Proc, Nat'l. Acad. Sci. USA 81:6349, 1984), and Miller et al., Human Gene Therapy 1:5-14,1990.

Lentiviral vectors typically, comprise a 5' lentiviral LTR, a tRNA binding site, a packaging signal, a promoter operably linked to one or more genes of interest, an origin of second strand DNA synthesis and a 3' lentiviral LTR, wherein the lentiviral vector contains a nuclear transport element. The nuclear transport element may be located either upstream (5') or downstream (3') of a coding sequence of interest (for example, a synthetic Gag or Env expression cassette of the present invention). Within certain embodiments, the nuclear transport element is not RRE. Within one embodiment the packaging signal is an extended packaging signal. Within other embodiments the promoter is a tissue specific promoter, or, alternatively, a promoter such as CMV. Within other embodiments, the lentiviral vector further comprises an internal ribosome entry site.

A wide variety of lentiviruses may be utilized within the context of the present invention, including for example, lentiviruses selected from the group consisting of HIV, HIV-1, HIV-2, FIV and SIV.

Within yet another aspect of the invention, host cells (e.g., packaging cell lines) are provided which contain any of the expression cassettes described herein. For example, within one aspect packaging cell line are provided comprising an expression cassette that comprises a sequence encoding synthetic Gag-polymerase, and a nuclear transport element, wherein the promoter is operably linked to the sequence encoding Gag-polymerase. Packaging cell lines may further comprise a promoter and a sequence encoding tat, rev, or an envelope, wherein the promoter is operably linked to the sequence encoding tat, rev, Env or sequences encoding modified versions of these proteins. The packaging cell line may further comprise a sequence encoding any one or more of other HIV gene encoding sequences.

In one embodiment, the expression cassette (carrying, for example, the synthetic Gag-polymerase) is stably integrated. The packaging cell line, upon introduction of a lentiviral vector, typically produces particles. The promoter regulating expression of the synthetic expression cassette may be inducible. Typically, the packaging cell line, upon introduction of a lentiviral vector, produces particles that are essentially free of replication competent virus.

Packaging cell lines are provided comprising an expression cassette which directs the expression of a synthetic *Gag-polymerase* gene or comprising an expression cassette which directs the expression of a synthetic Env genes described herein. (See, also, Andre, S., et al., Journal of Virology 72(2):1497-1503, 1998; Haas, J., et al., Current Biology 6(3):315-324, 1996) for a description of other modified Env sequences). A lentiviral vector is introduced into the packaging cell line to produce a vector producing cell line.

As noted above, lentiviral vectors can be designed to carry or express a selected gene(s) or sequences of interest. Lentiviral vectors may be readily constructed from a wide variety of lentiviruses *(see* RNA Tumor Viruses, Second Edition, Cold Spring Harbor Laboratory, 1985). Representative examples of lentiviruses included HIV, HIV-1, HIV-2, FIV and SIV. Such lentiviruses may either be obtained from patient isolates, or, more preferably, from depositories or collections such as the American Type Culture Collection, or isolated from known sources using available techniques.

Portions of the lentiviral gene delivery vectors (or vehicles) may be derived from different viruses. For example, in a given Recombinant lentiviral vector, LTRs may be derived from an HIV, a packaging signal from SIV, and an origin of second strand synthesis from HrV-2. Lentiviral vector constructs may comprise a 5' lentiviral LTR, a tRNA binding site, a packaging signal, one or more heterologous sequences, an origin of second strand DNA synthesis and a 3' LTR, wherein said lentiviral vector contains a nuclear transport element that is not RRE.

Briefly, Long Terminal Repeats ("LTRs") are subdivided into three elements, designated U5, R and U3. These elements contain a variety of signals which are responsible for the biological activity of a retrovirus, including for example, promoter and enhancer elements which are located within U3. LTRs may be readily identified in the provirus (integrated DNA form) due to their precise duplication at either end of the genome. As utilized herein, a 5' LTR should be understood to include a 5' promoter element and sufficient LTR sequence to allow reverse transcription and integration of the DNA form of the vector. The 3' LTR should be understood to include a polyadenylation signal, and sufficient LTR sequence to allow reverse transcription and integration of the DNA form of the vector.

The tRNA binding site and origin of second strand DNA synthesis are also important for a retrovirus to be biologically active, and may be readily identified by one of skill in the art. For example, retroviral tRNA binds to a tRNA binding site by Watson-Crick base pairing, and is carried with the retrovirus genome into a viral particle. The tRNA is then utilized as a primer for DNA synthesis by reverse transcriptase. The tRNA binding site may be readily identified based upon its location just downstream from the 5'LTR. Similarly, the origin of second strand DNA synthesis is, as its name implies, important for the second strand DNA synthesis of a retrovirus. This region, which is also referred to as the poly-purine tract, is located just upstream of the 3'LTR.

In addition to a 5' and 3' LTR, tRNA binding site, and origin of second strand DNA synthesis, recombinant retroviral vector constructs may also comprise a packaging signal, as well as one or more genes or coding sequences of interest. In addition, the lentiviral vectors have a nuclear transport element which, in preferred embodiments is not RRE. Representative examples of suitable nuclear transport elements include the element in Rous sarcoma virus (Ogert, et al., J ViroL 70, 3834-3843, 1996), the element in Rous sarcoma virus (Liu & Mertz, Genes & Dev., 9, 1766-1789, 1995) and the element in the genome of simian retrovirus type I (Zolotukhin, et al., J Virol. 68, 7944-7952, 1994). Other potential elements include the elements in the histone gene (Kedes, Annu. Rev. Biochem. 48, 837-870, 1970), the α-interferon gene (Nagata et al., Nature 287, 401-408, 1980), the β-adrenergic receptor gene (Koilka, et al., Nature 329, 75-79, 1987), and the c-Jun gene (Hattorie, et al., Proc. Natl. Acad. Sci. USA 85, 9148-9152, 1988).

Recombinant lentiviral vector constructs typically lack both *Gag-polymerase* and *Env* coding sequences. Recombinant lentiviral vector typically contain less than 20, preferably 15, more preferably 10, and most preferably 8 consecutive nucleotides found in *Gag-polymerase* and *Env* genes. One advantage of the present invention is that the synthetic *Gag-polymerase* expression cassettes, which can be used to construct packaging cell lines for the recombinant retroviral vector constructs, have little homology to wild-type Gag-polymerase sequences and thus considerably reduce or eliminate the possibility of homologous recombination between the synthetic and wild-type sequences.

Lentiviral vectors may also include tissue-specific promoters to drive expression of one or more genes or sequences of interest.

Lentiviral vector constructs may be generated such that more than one gene of interest is expressed. This may be accomplished through the use of di- or oligo-cistronic cassettes (e.g., where the coding regions are separated by 80 nucleotides or less, *see generally* Levin et al., Gene 108:167-174, 1991), or through the use of Internal Ribosome Entry Sites ("IRES").

Packaging cell lines suitable for use with the above described recombinant retroviral vector constructs may be readily prepared given the disclosure provided herein. Briefly, the parent cell line from which the packaging cell line is derived can be selected from a variety of mammalian cell lines, including for example, 293, RD, COS-7, CHO, BHK, VERO, HT1080, and myeloma cells.

After selection of a suitable host cell for the generation of a packaging cell line, one or more expression cassettes are introduced into the cell line in order to complement or supply in *trans* components of the vector which have been deleted.

Representative examples of suitable synthetic HIV polynucleotide sequences have been described herein for use in expression cassettes of the present invention. As described above, the native and/or synthetic coding sequences may also be utilized in these expression cassettes.

Utilizing the above-described expression cassettes, a wide variety of packaging cell lines can be generated. For example, within one aspect packaging cell line are provided comprising an expression cassette that comprises a sequence encoding synthetic Gag-polymerase, and a nuclear transport element, wherein the promoter is operably linked to the sequence encoding Gag-polymerase. Within other aspects, packaging cell lines are provided comprising a promoter and a sequence encoding tat, rev, Env, or other HIV antigens or epitopes derived therefrom, wherein the promoter is operably linked to the sequence encoding tat, rev, Env, or the HIV antigen or epitope. Within further embodiments, the packaging cell line may comprise a sequence encoding any one or more of tat, rev, nef, vif, vpu or vpr. For example, the packaging cell line may contain only tat, rev, nef, vif, vpu, or vpr alone, tat rev and nef, nef and vif, nef and vpu, nef and vpr, vif and vpu, vif and vpr, vpu and vpr, nef vif and vpu, nef vif and vpr, nef vpu and vpr, vif vpu and vpr, all four of nef, vif, vpu, and vpr, etc.

In one embodiment, the expression cassette is stably integrated. Within another embodiment, the packaging cell line, upon introduction of a lentiviral vector, produces particles. Within further embodiments the promoter is inducible. Within certain preferred embodiments of the invention, the packaging cell line, upon introduction of a lentiviral vector, produces particles that are free of replication competent virus.

The synthetic cassettes containing modified coding sequences are transfected into a selected cell line. Transfected cells are selected that (i) carry, typically, integrated, stable copies of the HIV coding sequences, and (ii) are expressing acceptable levels of these polypeptides (expression can be evaluated by methods known in the prior art in view of the teachings of the present disclosure). The ability of the cell line to produce VLPs may also be verified.

A sequence of interest is constructed into a suitable viral vector as discussed above. This defective virus is then transfected into the packaging cell line. The packaging cell line provides the viral functions necessary for producing virus-like particles into which the defective viral genome, containing the sequence of interest, are packaged. These VLPs are then isolated and can be used, for example, in gene delivery or gene therapy.

Further, such packaging cell lines can also be used to produce VLPs alone, which can, for example, be used as adjuvants for administration with other antigens or in vaccine compositions. Also, co-expression of a selected sequence of interest encoding a polypeptide (for example, an antigen) in the packaging cell line can also result in the entrapment and/or association of the selected polypeptide in/with the VLPs.

Various forms of the different embodiments of the present invention (*e*.*g*., synthetic constructs) may be combined.

### 2.4.0 DNA IMMUNIZATION AND GENE DELIVERY

A variety of HIV polypeptide antigens, particularly HIV antigens, can be used in the practice of the present invention. HIV antigens can be included in DNA immunization constructs containing, for example, a synthetic Env expression cassettes, a synthetic Gag expression cassette, a synthetic pol-derived polypeptide expression cassette, a synthetic expression cassette comprising sequences encoding one or more accessory or regulatory genes (e.g., tat, rev, nef, vif, vpu, vpr), and/or a synthetic Gag expression cassette fused in-frame to a coding sequence for the polypeptide antigen (synthetic or wild-type), where expression of the construct results in VLPs presenting the antigen of interest.

HIV antigens of particular interest to be used in the practice of the present invention include pol, tat, rev, nef, vif, vpu, vpr, and other HIV-1 (also known as HTLV-III, LAV, ARV, etc.) antigens or epitopes derived therefrom, including, but not limited to, antigens such as gp120, gp41, gp 160 (both native and modified); Gag; and pol from a variety of isolates including, but not limited to, HIV_{IIIb}, HIV_{SF2}, HIV-1_{SF162}, HIV-1_{SF170}, HIV_{LAV}, HIV_{LAI}, HIV_{MN}, HIV-1_{CM235},, HIV-1_{US4}, other HIV-1 strains from diverse subtypes(e.g., subtypes, A through G, and O), HIV-2 strains and diverse subtypes (e.g., HIV-2_{UC1} and HIV-2_{UC2}). See, e.g., Myers, et al., Los Alamos Database, Los Alamos National Laboratory, Los Alamos, New Mexico; Myers, et al., Human Retroviruses and Aids, 1990, Los Alamos, New Mexico: Los Alamos National Laboratory. These antigens may be synthetic (as described herein) or wild-type.

To evaluate efficacy, DNA immunization using synthetic expression cassettes of the present invention can be performed, for example, as follows. Mice are immunized with a tat/rev/nef synthetic expression cassette. Other mice are immunized with a tat/rev/nef wild type expression cassette. Mouse immunizations with plasmid-DNAs typically show that the synthetic expression cassettes provide a clear improvement of immunogenicity relative to the native expression cassettes. Also, a second boost immunization will induce a secondary immune response, for example, after approximately two weeks. Further, the results of CTL assays typically show increased potency of synthetic expression cassettes for induction of cytotoxic T-lymphocyte (CTL) responses by DNA immunization.

Exemplary primate studies directed at the evaluation of neutralizing antibodies and cellular immune responses against HIV are described below.

It is readily apparent that the subject invention can be used to mount an immune response to a wide variety of antigens and hence to treat or prevent infection, particularly HIV infection.

### 2.4.1 DELIVERY OF THE SYNTHETIC EXPRESSION CASSETTES OF THE PRESENT INVENTION

Polynucleotide sequences coding for the above-described molecules can be obtained using recombinant methods, such as by screening cDNA and genomic libraries from cells expressing the gene, or by deriving the gene from a vector known to include the same. Furthermore, the desired gene can be isolated directly from cells and tissues containing the same, using standard techniques, such as phenol extraction and PCR of cDNA or genomic DNA. See, e.g., Sambrook et al., *supra,* for a description of techniques used to obtain and isolate DNA. The gene of interest can also be produced synthetically, rather than cloned. The nucleotide sequence can be designed with the appropriate codons for the particular amino acid sequence desired. In general, one will select preferred codons for the intended host in which the sequence will be expressed. The complete sequence is assembled from overlapping oligonucleotides prepared by standard methods and assembled into a complete coding sequence. See, e.g., Edge, Nature (1981) 292:756; Nambair et al., Science (1984) 223:1299; Jay et al., J. Biol. Chem. (1984) 259:6311; Stemmer, W.P.C., (1995) Gene 164:49-53.

Next, the gene sequence encoding the desired antigen can be inserted into a vector containing a synthetic expression cassette of the present invention. In one embodiment, polynucleotides encoding selected antigens are separately cloned into expression vectors (e.g., Env-coding polynucleotide in a first vector, Gag-coding polynucleotide in a second vector, Pol-derived polypeptide-coding polynucleotide in a third vector, tat-, rev-, nef-, vif-, vpu-, vpr-coding polynucleotides in further vectors, etc.). In certain embodiments, the antigen is inserted into or adjacent a synthetic Gag coding sequence such that when the combined sequence is expressed it results in the production of VLPs comprising the Gag polypeptide and the antigen of interest, *e*.*g*., Env (native or modified) or other antigen(s) (native or modified) derived from HIV. Insertions can be made within the coding sequence or at either end of the coding sequence (5', amino terminus of the expressed Gag polypeptide; or 3', carboxy terminus of the expressed Gag polypeptide)(Wagner, R., et al., Arch Virol. 127:117-137,1992; Wagner, R., et al., Virology 200:162-175, 1994; Wu, X., et al., J. Virol. 69(6):3389-3398, 1995; Wang, C-T., et al., Virology 200:524-534, 1994; Chazal, N., et al., Virology 68(1):111-122, 1994; Griffiths, J.C., et al., J. Virol. 67(6):3191-3198, 1993; Reicin, A.S., et al., J. Virol. 69(2):642-650, 1995).

Up to 50% of the coding sequences of p55Gag can be deleted without affecting the assembly to virus-like particles and expression efficiency (Borsetti, A., et al, J. Virol. 72(11):9313-9317, 1998; Gamier, L., et al., J Virol 72(6):4667-4677, 1998; Zhang, Y., et al., J Virol 72(3):1782-1789, 1998; Wang, C., et al., J Virol 72(10): 7950-7959, 1998). In one embodiment of the present invention, immunogenicity of the high level expressing synthetic Gag expression cassettes can be increased by the insertion of different structural or non-structural HIV antigens, multi-epitope cassettes, or cytokine sequences into deleted regions of Gag sequence. Such deletions may be generated following the teachings of the present invention and information available to one of ordinary skill in the art. One possible advantage of this approach, relative to using full-length sequences fused to heterologous polypeptides, can be higher expression/secretion efficiency of the expression product.

When sequences are added to the amino terminal end of Gag, the polynucleotide can contain coding sequences at the 5' end that encode a signal for addition of a myristic moiety to the Gag-containing polypeptide (e.g., sequences that encode Met-Gly).

The ability of Gag-containing polypeptide constructs to form VLPs can be empirically determined following the teachings of the present specification.

The synthetic expression cassettes can also include control elements operably linked to the coding sequence, which allow for the expression of the gene *in vivo* in the subject species. For example, typical promoters for mammalian cell expression include the SV40 early promoter, a CMV promoter such as the CMV immediate early promoter, the mouse mammary tumor virus LTR promoter, the adenovirus major late promoter (Ad MLP), and the herpes simplex virus promoter, among others. Other nonviral promoters, such as a promoter derived from the murine metallothionein gene, will also find use for mammalian expression. Typically, transcription termination and polyadenylation sequences will also be present, located 3' to the translation stop codon. Preferably, a sequence for optimization of initiation of translation, located 5' to the coding sequence, is also present. Examples of transcription terminator/polyadenylation signals include those derived from SV40, as described in Sambrook et al., *supra,* as well as a bovine growth hormone terminator sequence.

Enhancer elements may also be used herein to increase expression levels of the mammalian constructs. Examples include the SV40 early gene enhancer, as described in Dijkema et al., EMBO J. (1985) 4:761, the enhancer/promoter derived from the long terminal repeat (LTR) of the Rous Sarcoma Virus, as described in Gorman et al., Proc. Natl. Acad. Sci. USA (1982b) 79:6777 and elements derived from human CMV, as described in Boshart et al., Cell (1985) 41:521, such as elements included in the CMV intron A sequence.

Furthermore, plasmids can be constructed which include a chimeric antigen-coding gene sequences, encoding, e.g., multiple antigens/epitopes of interest, for example derived from more than one viral isolate.

Typically the antigen coding sequences precede or follow the synthetic coding sequence and the chimeric transcription unit will have a single open reading frame encoding both the antigen of interest and the synthetic coding sequences. Alternatively, multi-cistronic cassettes (e.g., bi-cistronic cassettes) can be constructed allowing expression of multiple antigens from a single mRNA using the EMCV IRES, or the like (Example 7).

In one embodiment of the present invention, a nucleic acid immunizing composition may comprise, for example, the following: a first expression vector comprising a Gag expression cassette, a second vector comprising an Env expression cassette, and a third expression vector comprising a Pol expression cassette, or one or more coding region of Pol (e.g., Prot, RT, RNase, Int), wherein further antigen coding sequences may be associated with the Pol expression, such antigens may be obtained, for example, from accessory genes (e.g., vpr, vpu, vif), regulatory genes (e.g., nef, tat, rev), or portions of the Pol sequences (e.g., Prot, RT, RNase, Int)). In another embodiment, a nucleic acid immunizing composition may comprise, for example, an expression cassette comprising any of the synthetic polynucleotide sequences of the present invention. In another embodiment, a nucleic acid immunizing composition may comprise, for example, an expression cassette comprising coding sequences for a number of HIV genes (or sequences derived from such genes) wherein the coding sequences are in-frame and under the control of a single promoter, for example, Gag-Env constructs, Tat-Rev-Nef constructs, P2Pol-tat-rev-nef constructs, etc. The synthetic coding sequences of the present invention may be combined in any number of combinations depending on the coding sequence products (i.e., HIV polypeptides) to which, for example, an immunological response is desired to be raised. In yet another embodiment, synthetic coding sequences for multiple HIV-derived polypeptides may be constructed into a polycistronic message under the control of a single promoter wherein IRES are placed adjacent the coding sequence for each encoded polypeptide.

Exemplary synthetic polynucleotides and/or expression cassettes of the present invention may comprise, for example, the following: Tat_{(Type B)}Rev_{(Type B)}Nef_{(Type B})Tat_{(Type C)}Rev_{(TYPe C)}Nef_{(Type C)}; p2Pol_{(Type B)}Tat(_{Type B)}Rev_{(Type B)}Nef_{(Type B)}Tat_{(Type C)}Rev_{(Type C)}Nef_{(Type C)}; P2pol_{(Type C)}Tat_{(Type}, _{B})Rev_{(Type B)}Nef_{(Type B)}Tat(_{Type C)}ReV_{(Type C)}Nef_{(Type C)}; Gag complPol Tat_{(Type B)}Rev_{(Type B)}Nef_{(Type B)}Tat_{(Type)}Rev_{(Type C)}Nef_{(Type C)} (where GagcomplPol may be, for example, derived from any subtype or based on a consensus sequence derived from a variety of subtypes); p2PolTat_{(Typ B)}ReV_{(Type B)}Nef_{(Typ B)}Tat_{(Typ C)}ReV_{(Type C)}Nef_{(Type C)} (where p2Pol may be, for example, derived from any subtype or based on a consensus sequence derived from a variety of subtypes); PolTat_{(Type B)}Rev_{(Type B)}Nef_{(Type B)}Tat_{(Type C)}Rev_{(Type C)}Nef_{(Type C)} (where Pol may be, for example, derived from any subtype or based on a consensus sequence derived from a variety of subtypes); ProtRTTat_{(Type B)}Rev_{(Type B)}Nef_{(Type B)}Tat_{(Type C)}Rev_{(Type C)}Nef_{(Type C}) (where ProtRT may be, for example, derived from any subtype or based on a consensus sequence derived from a variety of subtypes); ProtTat_{(Type B})Rev_{(Type B)}Nef_{(Type B)}Tat_{(Type C)}Rev(_{TyPe C)}Nef_{(Type C)} (where Prot may be, for example, derived from any subtype or based on a consensus sequence derived from a variety of subtypes); and GagTat_{(Type B)}ReV_{(Type B)}Nef_{(Type B)}Tat_{(Type C)}Rev_{(Type C)}Nef_{(Type C)} (where Gag may be, for example, derived from any subtype or based on a consensus sequence derived from a variety of subtypes).

Further, synthetic polynucleotides and/or expression cassettes of the present invention may comprise, for example, the following: Vif_{(Type B)}Vpr_{(Type B)}VPu_{(Type B)}Vif_{(TypeC)}Vpr_{(Type C)}Vpu_{(Type C)}; p2Pol_{(Type B)}Vif_{(Type B)}Vpr_{(Type B)}VPU_{(Type B)}Vif_{(Type C})Vpr_{(Type C)}Vpu_{(Type C)}; p2Pol_{(TyPe C)}Vif_{(Type B)}Vpr_{(Type B)}Vpu_{(Type B)}Vif_{(Type C})Vpr_{(Type C)}Vpu_{(Type C}); Gag complPol Vif_{(Type B)}Vpr_{(Type B)}Vpu_{(Type B)}Vif_{(Type C)}Vpr_{(Type C)}Vpu_{(TyPe C)} (where GagcomplPol may be, for example, derived from any subtype or based on a consensus sequence derived from a variety of subtypes); p2Pol Vif_{(Type B)}Vpr_{(Type B)}Vpu_{(Type B)}Vif_{(Type C)}Vpr_{(Type C)}Vpu_{(Type C)} (where p2Pol may be, for example, derived from any subtype or based on a consensus sequence derived from a variety of subtypes); Pol Vif_{(Type B)}Vpr_{(Type B)}Vpu_{(Type B)}Vif_{(Type C)}Vpr_{(Type C)}Vpu_{(Type C)} (where Pol may be, for example, derived from any subtype or based on a consensus sequence derived from a variety of subtypes); ProtRTVif(_{Type B)}Vpr_{(Type B)}Vpu_{(Type B)}Vif_{(Type C)}Vpr_{(Type C)}Vpu_{(Type C)} (where ProtRT may be, for example, derived from any subtype or based on a consensus sequence derived from a variety of subtypes); Prot Vif_{(Type B)}Vpr_{(Type B)}Vpu_{(Type B)}Vif_{(Typec)}Vpr_{(Type C)}Vpu_{(Type C)} (where Prot may be, for example, derived from any subtype or based on a consensus sequence derived from a variety of subtypes); and Gag Vif_{(Type B)}Vpr_{(Type B)}Vpu_{(Type B)}Vif_{(Type C)}Vpr_{(Type C)}Vpu_{(Type C)} (where Gag may be, for example, derived from any subtype or based on a consensus sequence derived from a variety of subtypes).

In one general embodiment, synthetic polynucleotides and/or expression cassettes of the present invention may comprise, for example, the following: tandem repeats of Int, wherein at least two of the gene product coding sequences are derived from different HIV Types (e.g, A-G, O); Tat-Rev-Nef, wherein at least two of the gene product coding sequences are derived from different HIV Types (e.g, A-G, O); tandem repeats of Tat-Rev-Nef coding sequences, wherein at least two of the gene product coding sequences are derived from different HIV Types (e.g, A-G, O); Vif-Vpr-Vpu, wherein at least two of the gene product coding sequences are derived from different HIV Types (e.g, A-G, O); tandem repeats of Vif-Vpr-Vpu coding sequences, wherein at least two of the gene product coding sequences are derived from different HIV Types (e.g, A-G, O); and Tat-Rev-Nef-Vif-Vpr-Vpu, wherein at least two of the gene product coding sequences are derived from different HIV Types (e.g, A-G, O); and tandem repeats of Tat-Rev-Nef-Vif-Vpr-Vpu coding sequences, wherein at least two of the gene product coding sequences are derived from different HIV Types (e.g, A-G, O).

Such synthetic polynucleotide coding sequences (for example, as described herein above) may encode functional gene products or be mutated to reduce (relative to wild-type), attenuate, inactivate, eliminate, or render non-functional the activity of the gene product(s) encoded the synthetic polynucleotide. The orders of the coding sequences within the synthetic polynucleotide may vary. An optimal order may be determined empirically based, for example, on obtaining desired expression levels of the products in a target cell type.

Once complete, the constructs are used for nucleic acid immunization using standard gene delivery protocols. Methods for gene delivery are known in the art. See, e.g., U.S. Patent Nos. 5,399,346, 5,580,859, 5,589,466. Genes can be delivered either directly to the vertebrate subject or, alternatively, delivered *ex vivo,* to cells derived from the subject and the cells reimplanted in the subject.

A number of viral based systems have been developed for gene transfer into mammalian cells. For example, retroviruses provide a convenient platform for gene delivery systems. Selected sequences can be inserted into a vector and packaged in retroviral particles using techniques known in the art. The recombinant virus can then be isolated and delivered to cells of the subject either *in vivo* or *ex vivo.* A number of retroviral systems have been described (U.S. Patent No. 5,219,740; Miller and, Rosman, BioTechniques (1989) 7:980-990; Miller, A.D., Human Gene Therapy (1990) 1:5-14; Scarpa et al., Virology (1991) 180:849-852; Burns et al., Proc. Natl. Acad. Sci. USA (1993) 90:8033-8037; and Boris-Lawrie and Temin, Cur. Opin. Genet. Develop. (1993) 3:102-109.

A number of adenovirus vectors have also been described. Unlike retroviruses which integrate into the host genome, adenoviruses persist extrachromosomally thus minimizing the risks associated with insertional mutagenesis (Haj-Ahmad and Graham, J. Virol. (1986) 57:267-274; Bett et al., J. Virol. (1993) 67:5911-5921; Mittereder et al., Human Gene Therapy (1994) 5:717-729; Seth et al., J. Virol. (1994) 68:933-940; Barr et al., Gene Therapy (1994) 1:51-58; Berkner, K.L. BioTechniques (1988) 6:616-629; and Rich et al., Human Gene Therapy (1993) 4:461-476).

Additionally, various adeno-associated virus (AAV) vector systems have been developed for gene delivery. AAV vectors can be readily constructed using techniques well known in the art. See, e.g., U.S. Patent Nos. 5,173,414 and 5,139,941; International Publication Nos. WO 92/01070 (published 23 January 1992) and WO 93/03769 (published 4 March 1993); Lebkowski et al., Molec. Cell. Biol. (1988) 8:3988-3996; Vincent et al., Vaccines 90 (1990) (Cold Spring Harbor Laboratory Press); Carter, B.J. Current Opinion in Biotechnology(1992) 3:533-539; Muzyczka, N. Current Topics in Microbiol. and Immunol. (1992) 158:97-129; Kotin, R.M. Human Gene Therapy (1994) 5:793-801; Shelling and Smith, Gene Therapy (1994) 1:165-169; and Zhou et al., J. Exp. Med. (1994) 179:1867-1875.

Another vector system useful for delivering the polynucleotides of the present invention is the enterically administered recombinant poxvirus vaccines described by Small, Jr., P.A., et al. (U.S. Patent No. 5,676,950, issued October 14, 1997).

Additional viral vectors which will fmd use for delivering the nucleic acid molecules encoding the antigens of interest include those derived from the pox family of viruses, including vaccinia virus and avian poxvirus. By way of example, vaccinia virus recombinants expressing the genes can be constructed as follows. The DNA encoding the particular synthetic HIV polypeptide coding sequence is first inserted into an appropriate vector so that it is adjacent to a vaccinia promoter and flanking vaccinia DNA sequences, such as the sequence encoding thymidine kinase (TK). This vector is then used to transfect cells which are simultaneously infected with vaccinia. Homologous recombination serves to insert the vaccinia promoter plus the gene encoding the coding sequences of interest into the viral genome. The resulting TK⁻ recombinant can be selected by culturing the cells in the presence of 5-bromodeoxyuridine and picking viral plaques resistant thereto.

Alternatively, avipoxviruses, such as the fowlpox and canarypox viruses, can also be used to deliver the genes. Recombinant avipox viruses, expressing immunogen from mammalian pathogens, are known to confer protective immunity when administered to non-avian species. The use of an avipox vector is particularly desirable in human and other mammalian species since members of the avipox genus can only productively replicate in susceptible avian species and therefore are not infective in mammalian cells. Methods for producing recombinant avipoxviruses are known in the art and employ genetic recombination, as described above with respect to the production of vaccinia viruses. See, e.g., WO 91/12882; WO 89/03429; and WO 92/03545.

Molecular conjugate vectors, such as the adenovirus chimeric vectors described in Michael et al., J. Biol. Chem. (1993) 268:6866-6869 and Wagner et al., Proc. Natl. Acad. Sci. USA (1992) 89:6099-6103, can also be used for gene delivery.

Members of the Alphavirus genus, such as, but not limited to, vectors derived from the Sindbis, Semliki Forest, and Venezuelan Equine Encephalitis viruses, will also find use as viral vectors for delivering the polynucleotides of the present invention (for example, a synthetic Gag-polypeptide encoding expression cassette). For a description of Sindbis-virus derived vectors useful for the practice of the instant methods, see, Dubensky et al., J. Virol. (1996) 70:508-519; and International Publication Nos. WO 95/07995 and WO 96/17072; as well as, Dubensky, Jr., T.W., et al., U.S. Patent No. 5,843,723, issued December 1, 1998, and Dubensky, Jr., T.W., U.S. Patent No. 5,789,245, issued August 4, 1998. Preferred expression systems include, but are not limited to, eucaryotic layered vector initiation systems (e.g., US Patent No. 6,015,686, US Patent No. 5, 814,482, US Patent No. 6,015,694, US Patent No. 5,789,245, EP 1029068A2, WO 9918226A2/A3, EP 00907746A2, WO 9738087A2).

A vaccinia based infection/transfection system can be conveniently used to provide for inducible, transient expression of the coding sequences of interest in a host cell. In this system, cells are first infected *in vitro* with a vaccinia virus recombinant that encodes the bacteriophage T7 RNA polymerase. This polymerase displays exquisite specificity in that it only transcribes templates bearing T7 promoters. Following infection, cells are transfected with the polynucleotide of interest, driven by a T7 promoter. The polymerase expressed in the cytoplasm from the vaccinia virus recombinant transcribes the transfected DNA into RNA which is then translated into protein by the host translational machinery. The method provides for high level, transient, cytoplasmic production of large quantities of RNA and its translation products. See, e.g., Elroy-Stein and Moss, Proc. Natl. Acad. Sci. USA (1990) 87:6743-6747; Fuerst et al., Proc. Natl. Acad. Sci. USA (1986) 83:8122-8126.

As an alternative approach to infection with vaccinia or avipox virus recombinants, or to the delivery of genes using other viral vectors, an amplification system can be used that will lead to high level expression following introduction into host cells. Specifically, a T7 RNA polymerase promoter preceding the coding region for T7 RNA polymerase can be engineered. Translation of RNA derived from this template will generate T7 RNA polymerase which in turn will transcribe more template. Concomitantly, there will be a cDNA whose expression is under the control of the T7 promoter. Thus, some of the T7 RNA polymerase generated from translation of the amplification template RNA will lead to transcription of the desired gene. Because some T7 RNA polymerase is required to initiate the amplification, T7 RNA polymerase can be introduced into cells along with the template(s) to prime the transcription reaction. The polymerase can be introduced as a protein or on a plasmid encoding the RNA polymerase. For a further discussion of T7 systems and their use for transforming cells, see, e.g., International Publication No. WO 94/26911; Studier and Moffatt, J. Mol. Biol. (1986) 189:113-130; Deng and Wolff, Gene (1994) 143:245-249; Gao et al., Biochem. Biophys. Res. Commun. (1994) 200:1201-1206; Gao and Huang, Nuc. Acids Res. (1993) 21:2867-2872; Chen et al., Nuc. Acids Res. (1994) 22:2114-2120; and U.S. Patent No. 5,135,855.

Delivery of the expression cassettes of the present invention can also be accomplished using eucaryotic expression vectors comprising CMV-derived elements, such vectors include, but are not limited to, the following: pCMVKm2, pCMV-link pCMVPLEdhfr, and pCMV6a (all described above).

Synthetic expression cassettes of interest can also be delivered without a viral vector. For example, the synthetic expression cassette can be packaged in liposomes prior to delivery to the subject or to cells derived therefrom. Lipid encapsulation is generally accomplished using liposomes which are able to stably bind or entrap and retain nucleic acid. The ratio of condensed DNA to lipid preparation can vary but will generally be around 1:1 (mg DNA:micromoles lipid), or more of lipid. For a review of the use of liposomes as carriers for delivery of nucleic acids, see, Hug and Sleight, Biochim. Biophys. Acta. (1991) 1097:1-17; Straubinger et al., in Methods of Enzymology (1983), Vol. 101, pp. 512-527.

Liposomal preparations for use in the present invention include cationic (positively charged), anionic (negatively charged) and neutral preparations, with cationic liposomes particularly preferred. Cationic liposomes have been shown to mediate intracellular delivery of plasmid DNA (Felgner et al., Proc. Natl. Acad. Sci. USA (1987) 84:7413-7416); mRNA (Malone et al., Proc. Natl. Acad. Sci. USA (1989) 86:6077-6081); and purified transcription factors (Debs et al., J. Biol. Chem. (1990) 265:10189-10192), in functional form.

Cationic liposomes are readily available. For example, N[1-2,3-dioleyloxy)propyl]-N,N,N-triethylammonium (DOTMA) liposomes are available under the trademark Lipofectin, from GIBCO BRL, Grand Island, NY. (See, also, Felgner et al., Proc. Natl. Acad. Sci. USA (1987) 84:7413-7416). Other commercially available lipids include (DDAB/DOPE) and DOTAP/DOPE (Boerhinger). Other cationic liposomes can be prepared from readily available materials using techniques well known in the art. See, e.g., Szoka et al., Proc. Natl. Acad. Sci. USA (1978) 75:4194-4198; PCT Publication No. WO 90/11092 for a description of the synthesis of DOTAP (1,2-bis(oleoyloxy)-3-(trimethylammonio)propane) liposomes.

Similarly, anionic and neutral liposomes are readily available, such as, from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See, e.g., Straubinger et al., in METHODS OF IMMUNOLOGY (1983), Vol. 101, pp. 512-527; Szoka et al., Proc. Natl. Acad. Sci. USA (1978) 75:4194-4198; Papahadjopoulos et al., Biochim. Biophys. Acta (1975) 394:483; Wilson et al., Cell (1979) 17:77); Deamer and Bangham, Biochim. Biophys. Acta (1976) 443:629; Ostro et al., Biochem. Biophys. Res. Commun. (1977) 76:836; Fraley et al., Proc. Natl. Acad. Sci. USA (1979) 76:3348); Enoch and Strittmatter, Proc. Natl. Acad. Sci. USA (1979) 76:145); Fraley et al., J. Biol. Chem. (1980) 255:10431; Szoka and Papahadjopoulos, Proc. Natl. Acad. Sci. USA (1978) 75:145; and Schaefer-Ridder et al., Science (1982) 215:166.

The DNA and/or protein antigen(s) can also be delivered in cochleate lipid compositions similar to those described by Papahadjopoulos et al., Biochem. Biophys. Acta. (1975) 394:483-491. See, also, U.S. Patent Nos. 4,663,161 and 4,871,488.

The synthetic expression cassette of interest may also be encapsulated, adsorbed to, or associated with, particulate carriers. Such carriers present multiple copies of a selected antigen to the immune system and promote trapping and retention of antigens in local lymph nodes. The particles can be phagocytosed by macrophages and can enhance antigen presentation through cytokine release. Examples of particulate carriers include those derived from polymethyl methacrylate polymers, as well as microparticles derived from poly(lactides) and poly(lactide-co-glycolides), known as PLG. See, e.g., Jeffery et al., Pharm. Res. (1993) 10:362-368; McGee JP, et al., J Microencapsul. 14(2):197-210, 1997; O'Hagan DT, et al., Vaccine 11(2):149-54, 1993. Suitable microparticles may also be manufactured in the presence of charged detergents, such as anionic or cationic detergents, to yield microparticles with a surface having a net negative or a net positive charge. For example, microparticles manufactured with anionic detergents, such as hexadecyltrimethylammonium bromide (CTAB), i.e. CTAB-PLG microparticles, adsorb negatively charged macromolecules, such as DNA. (see, e.g., Int'1 Application Number PCT/US99/17308).

Furthermore, other particulate systems and polymers can be used for the *in vivo* or *ex vivo* delivery of the gene of interest. For example, polymers such as polylysine, polyarginine, polyornithine, spermine, spermidine, as well as conjugates of these molecules, are useful for transferring a nucleic acid of interest. Similarly, DEAE dextran-mediated transfection, calcium phosphate precipitation or precipitation using other insoluble inorganic salts, such as strontium phosphate, aluminum silicates including bentonite and kaolin, chromic oxide, magnesium silicate, talc, and the like, will find use with the present methods. See, e.g., Felgner, P.L., Advanced Drug Delivery Reviews (1990) 5:163-187, for a review of delivery systems useful for gene transfer. Peptoids (Zuckerman, R.N., et al., U.S. Patent No. 5,831,005, issued November 3, 1998) may also be used for delivery of a construct of the present invention.

Additionally, biolistic delivery systems employing particulate carriers such as gold and tungsten, are especially useful for delivering synthetic expression cassettes of the present invention. The particles are coated with the synthetic expression cassette(s) to be delivered and accelerated to high velocity, generally under a reduced atmosphere, using a gun powder discharge from a "gene gun." For a description of such techniques, and apparatuses useful therefore, see, e.g., U.S. Patent Nos. 4,945,050; 5,036,006; 5,100,792; 5,179,022; 5,371,015; and 5,478,744. Also, needle-less injection systems can be used (Davis, H.L., et al, Vaccine 12:1503-1509, 1994; Bioject, Inc., Portland, OR).

Recombinant vectors carrying a synthetic expression cassette of the present invention are formulated into compositions for delivery to the vertebrate subject. These compositions may either be prophylactic (to prevent infection) or therapeutic (to treat disease after infection). The compositions will comprise a "therapeutically effective amount" of the gene of interest such that an amount of the antigen can be produced *in vivo* so that an immune response is generated in the individual to which it is administered. The exact amount necessary will vary depending on the subject being treated; the age and general condition of the subject to be treated; the capacity of the subject's immune system to synthesize antibodies; the degree of protection desired; the severity of the condition being treated; the particular antigen selected and its mode of administration, among other factors. An appropriate effective amount can be readily determined by one of skill in the art. Thus, a "therapeutically effective amount" will fall in a relatively broad range that can be determined through routine trials.

The compositions will generally include one or more "pharmaceutically acceptable excipients or vehicles" such as water, saline, glycerol, polyethyleneglycol, hyaluronic acid, ethanol, etc. Additionally, auxiliary substances, such as wetting or emulsifying agents, pH buffering substances, and the like, may be present in such vehicles. Certain facilitators of nucleic acid uptake and/or expression can also be included in the compositions or coadministered, such as, but not limited to, bupivacaine, cardiotoxin and sucrose.

Once formulated, the compositions of the invention can be administered directly to the subject (e.g., as described above) or, alternatively, delivered *ex vivo,* to cells derived from the subject, using methods such as those described above. For example, methods for the *ex vivo* delivery and reimplantation of transformed cells into a subject are known in the art and can include, e.g., dextran-mediated transfection, calcium phosphate precipitation, polybrene mediated transfection, lipofectamine and LT-1 mediated transfection, protoplast fusion, electroporation, encapsulation of the polynucleotide(s) (with or without the corresponding antigen) in liposomes, and direct microinjection of the DNA into nuclei.

Direct delivery of synthetic expression cassette compositions *in vivo* will generally be accomplished with or without viral vectors, as described above, by injection using either a conventional syringe or a gene gun, such as the Accell® gene delivery system (PowderJect Technologies, Inc., Oxford, England). The constructs can be injected either subcutaneously, epidermally, intradermally, intramucosally such as nasally, rectally and vaginally, intraperitoneally, intravenously, orally or intramuscularly. Delivery of DNA into cells of the epidermis is particularly preferred as this mode of administration provides access to skin-associated lymphoid cells and provides for a transient presence of DNA in the recipient. Other modes of administration include oral and pulmonary administration, suppositories, needle-less injection, transcutaneous and transdermal applications. Dosage treatment may be a single dose schedule or a multiple dose schedule. Administration of nucleic acids may also be combined with administration of peptides or other substances.

Exemplary immunogenicity studies are presented in Examples 4, 5, 6, 9, 10, 11, and 12.

### 2.4.2 Ex VIVO DELIVERY OF THE SYNTHETIC EXPRESSION CASSETTES OF THE PRESENT INVENTION

In one embodiment, T cells, and related cell types (including but not limited to antigen presenting cells, such as, macrophage, monocytes, lymphoid cells, dendritic cells, B-cells, T-cells, stem cells, and progenitor cells thereof), can be used for *ex vivo* delivery of the synthetic expression cassettes of the present invention. T cells can be isolated from peripheral blood lymphocytes (PBLs) by a variety of procedures known to those skilled in the art. For example, T cell populations can be "enriched" from a population of PBLs through the removal of accessory and B cells. In particular, T cell enrichment can be accomplished by the elimination of non-T cells using anti-MHC class II monoclonal antibodies. Similarly, other antibodies can be used to deplete specific populations of non-T cells. For example, anti-Ig antibody molecules can be used to deplete B cells and anti-MacI antibody molecules can be used to deplete macrophages.

T cells can be further fractionated into a number of different subpopulations by techniques known to those skilled in the art. Two major subpopulations can be isolated based on their differential expression of the cell surface markers CD4 and CD8. For example, following the enrichment of T cells as described above, CD4⁺ cells can be enriched using antibodies specific for CD4 (see Coligan et al., *supra).* The antibodies may be coupled to a solid support such as magnetic beads. Conversely, CD8+ cells can be enriched through the use of antibodies specific for CD4 (to remove CD4⁺ cells), or can be isolated by the use of CD8 antibodies coupled to a solid support. CD4 lymphocytes from HIV-1 infected patients can be expanded *ex vivo,* before or after transduction as described by Wilson et. al. (1995) J. Infect. Dis. 172:88.

Following purification of T cells, a variety of methods of genetic modification known to those skilled in the art can be performed using non-viral or viral-based gene transfer vectors constructed as described herein. For example, one such approach involves transduction of the purified T cell population with vector-containing supernatant of cultures derived from vector producing cells. A second approach involves co-cultivation of an irradiated monolayer of vector-producing cells with the purified T cells. A third approach involves a similar co-cultivation approach; however, the purified T cells are pre-stimulated with various cytokines and cultured 48 hours prior to the co-cultivation with the irradiated vector producing cells. Pre-stimulation prior to such transduction increases effective gene transfer (Nolta et al. (1992) Exp. Hematol. 20:1065). Stimulation of these cultures to proliferate also provides increased cell populations for re-infusion into the patient. Subsequent to co-cultivation, T cells are collected from the vector producing cell monolayer, expanded, and frozen in liquid nitrogen.

Gene transfer vectors, containing one or more synthetic expression cassette of the present invention (associated with appropriate control elements for delivery to the isolated T cells) can be assembled using known methods and following the guidance of the present specification.

Selectable markers can also be used in the construction of gene transfer vectors. For example, a marker can be used which imparts to a mammalian cell transduced with the gene transfer vector resistance to a cytotoxic agent. The cytotoxic agent can be, but is not limited to, neomycin, aminoglycoside, tetracycline, chloramphenicol, sulfonamide, actinomycin, netropsin, distamycin A, anthracycline, or pyrazinamide. For example, neomycin phosphotransferase II imparts resistance to the neomycin analogue geneticin (G418).

The T cells can also be maintained in a medium containing at least one type of growth factor prior to being selected. A variety of growth factors are known in the art which sustain the growth of a particular cell type. Examples of such growth factors are cytokine mitogens such as rIL-2, IL-10, IL-12, and IL-15, which promote growth and activation of lymphocytes. Certain types of cells are stimulated by other growth factors such as hormones, including human chorionic gonadotropin (hCG) and human growth hormone. The selection of an appropriate growth factor for a particular cell population is readily accomplished by one of skill in the art.

For example, white blood cells such as differentiated progenitor and stem cells are stimulated by a variety of growth factors. More particularly, IL-3, IL-4, IL-5, IL-6, IL-9, GM-CSF, M-CSF, and G-CSF, produced by activated T_{H} and activated macrophages, stimulate myeloid stem cells, which then differentiate into pluripotent stem cells, granulocyte-monocyte progenitors, eosinophil progenitors, basophil progenitors, megakaryocytes, and erythroid progenitors. Differentiation is modulated by growth factors such as GM-CSF, IL-3, IL-6, IL-11, and EPO.

Pluripotent stem cells then differentiate into lymphoid stem cells, bone marrow stromal cells, T cell progenitors, B cell progenitors, thymocytes, T_{H} Cells, T_{C} cells, and B cells. This differentiation is modulated by growth factors such as IL-3, IL-4, IL-6, IL-7, GM-CSF, M-CSF, G-CSF, IL-2, and IL-5.

Granulocyte-monocyte progenitors differentiate to monocytes, macrophages, and neutrophils. Such differentiation is modulated by the growth factors GM-CSF, M-CSF, and TL-8. Eosinophil progenitors differentiate into eosinophils. This process is modulated by GM-CSF and IL-5.

The differentiation of basophil progenitors into mast cells and basophils is modulated by GM-CSF, IL-4, and IL-9. Megakaryocytes produce platelets in response to GM-CSF, EPO, and IL-6. Erythroid progenitor cells differentiate into red blood cells in response to EPO.

Thus, during activation by the CD3-binding agent, T cells can also be contacted with a mitogen, for example a cytokine such as IL-2. In particularly preferred embodiments, the IL-2 is added to the population of T cells at a concentration of about 50 to 100 µg/ml. Activation with the CD3-binding agent can be carried out for 2 to 4 days.

Once suitably activated, the T cells are genetically modified by contacting the same with a suitable gene transfer vector under conditions that allow for transfection of the vectors into the T cells. Genetic modification is carried out when the cell density of the T cell population is between about 0.1 x 10⁶ and 5 x 10⁶, preferably between about 0.5 x 10⁶ and 2 x 10⁶. A number of suitable viral and nonviral-based gene transfer vectors have been described for use herein.

After transduction, transduced cells are selected away from non-transduced cells using known techniques. For example, if the gene transfer vector used in the transduction includes a selectable marker which confers resistance to a cytotoxic agent, the cells can be contacted with the appropriate cytotoxic agent, whereby non-transduced cells can be negatively selected away from the transduced cells. If the selectable marker is a cell surface marker, the cells can be contacted with a binding agent specific for the particular cell surface marker, whereby the transduced cells can be positively selected away from the population. The selection step can also entail fluorescence-activated cell sorting (FACS) techniques, such as where FACS is used to select cells from the population containing a particular surface marker, or the selection step can entail the use of magnetically responsive particles as retrievable supports for target cell capture and/or background removal.

More particularly, positive selection of the transduced cells can be performed using a FACS cell sorter (e.g. a FACSVantage™ Cell Sorter, Becton Dickinson Immunocytometry Systems, San Jose, CA) to sort and collect transduced cells expressing a selectable cell surface marker. Following transduction, the cells are stained with fluorescent-labeled antibody molecules directed against the particular cell surface marker. The amount of bound antibody on each cell can be measured by passing droplets containing the cells through the cell sorter. By imparting an electromagnetic charge to droplets containing the stained cells, the transduced cells can be separated from other cells. The positively selected cells are then harvested in sterile collection vessels. These cell sorting procedures are described in detail, for example, in the FACSVantage™ Training Manual, with particular reference to sections 3-11 to 3-28 and 10-1 to 10-17.

Positive selection of the transduced cells can also be performed using magnetic separation of cells based on expression or a particular cell surface marker. In such separation techniques, cells to be positively selected are first contacted with specific binding agent (e.g., an antibody or reagent the interacts specifically with the cell surface marker). The cells are then contacted with retrievable particles (e.g., magnetically responsive particles) which are coupled with a reagent that binds the specific binding agent (that has bound to the positive cells). The cell-binding agent-particle complex can then be physically separated from non-labeled cells, for example using a magnetic field. When using magnetically responsive particles, the labeled cells can be retained in a container using a magnetic filed while the negative cells are removed. These and similar separation procedures are known to those of ordinary skill in the art.

Expression of the vector in the selected transduced cells can be assessed by a number of assays known to those skilled in the art. For example, Western blot or Northern analysis can be employed depending on the nature of the inserted nucleotide sequence of interest. Once expression has been established and the transformed T cells have been tested for the presence of the selected synthetic expression cassette, they are ready for infusion into a patient via the peripheral blood stream.

The invention includes a kit for genetic modification of an *ex vivo* population of primary mammalian cells. The kit typically contains a gene transfer vector coding for at least one selectable marker and at least one synthetic expression cassette contained in one or more containers, ancillary reagents or hardware, and instructions for use of the kit.

### 2.4.3 FURTHER DELIVERY REGIMES

Any of the polynucleotides (*e.g*., expression cassettes) or polypeptides described herein (delivered by any of the methods described above) can also be used in combination with other DNA delivery systems and/or protein delivery systems. Non-limiting examples include co-administration of these molecules, for example, in prime-boost methods where one or more molecules are delivered in a "priming" step and, subsequently, one or more molecules are delivered in a "boosting" step. In certain embodiments, the delivery of one or more nucleic acid-containing compositions and is followed by delivery of one or more nucleic acid-containing compositions and/or one or more polypeptide-containing compositions (*e.g*., polypeptides comprising HIV antigens). In other embodiments, multiple nucleic acid "primes" (of the same or different nucleic acid molecules) can be followed by multiple polypeptide "boosts" (of the same or different polypeptides). Other examples include multiple nucleic acid administrations and multiple polypeptide administrations.

In any method involving co-administration, the various compositions can be delivered in any order. Thus, in embodiments including delivery of multiple different compositions or molecules, the nucleic acids need not be all delivered before the polypeptides. For example, the priming step may include delivery of one or more polypeptides and the boosting comprises delivery of one or more nucleic acids and/or one more polypeptides. Multiple polypeptide administrations can be followed by multiple nucleic acid administrations or polypeptide and nucleic acid administrations can be performed in any order. In any of the embodiments described herein, the nucleic acid molecules can encode all, some or none of the polypeptides. Thus, one or more or the nucleic acid molecules (*e.g*., expression cassettes) described herein and/or one or more of the polypeptides described herein can be co-administered in any order and via any administration routes. Therefore, any combination of polynucleotides and/or polypeptides described herein can be used to generate elicit an immune reaction.

### 3.0 IMPROVED HIV-1 GAG AND POL EXPRESSION CASSETTES

While not desiring to be bound by any particular model, theory, or hypothesis, the following information is presented to provide a more complete understanding of the present invention.

The world health organization (WHO) estimated the number of people worldwide that are infected with HIV-1 to exceed 36.1 million. The development of a safe and effective HIV vaccine is therefore essential at this time. Recent studies have demonstrated the importance of CTL in controlling the HIV-1 replication in infected patients. Furthermore, CTL reactivity with multiple HIV antigens will be necessary for the effective control of virus replication. Experiments performed in support of the present invention suggest that the inclusion of HIV-1 Gag and Pol, beside Env for the induction of neutralizing antibodies, into the vaccine is useful.

To increase the potency of HIV-1 vaccine candidates, codon modified Gag and Pol expression cassettes were designed, either for Gag alone or Gag plus Pol. To evaluate possible differences in expression and potency, the expression of these constructs was analyzed and immunogenicity studies carried out in mice.

Several expression cassettes encoding Gag and Pol were designed, including, but not limited to, the following: GagProtease, GagPolAintegrase with frameshift (gagFSpol), and GagPolAintegrase in-frame (gagpol). Versions of GagPolAintegrase in-frame were also designed with attenuated (Att) or non-functional Protease (Ina). The nucleic acid sequences were codon modified to correspond to the codon usage of highly expressed human genes. Mice were immunized with titrated DNA doses and humoral and cellular immune responses evaluated by ELISA and intracellular cytokine staining (Example 10).

The immune responses in mice has been seen to be correlated with relative levels of expression *in vitro.* Vaccine studies in rhesus monkeys will further address immune responses and expression levels in vivo.

### 4.0 ENHANCED VACCINE TECHNOLOGIES FOR THE INDUCTION OF POTENT NEUTRALIZING ANTIBODIES AND CELLULAR IMMUNE RESPONSES AGAINST HIV.

While not desiring to be bound by any particular model, theory, or hypothesis, the following information is presented to provide a more complete understanding of the present invention.

Protection against HIV infection will likely require potent and broadly reactive pre-existing neutralizing antibodies in vaccinated individuals exposed to a virus challenge. Although cellular immune responses are desirable to control viremia in those who get infected, protection against infection has not been demonstrated for vaccine approaches that rely exclusively on the induction of these responses. For this reason, experiments performed in support of the present invention use prime-boost approaches that employ novel V-deleted envelope antigens from primary HIV isolates (e.g., R5 subtype B (HIV-1_{SF162}) and subtype C (HIV-1_{TVI}) strains). These antigens were delivered by enhanced DNA [polyactide co-glycolide (PLG) microparticle formulations or electroporation] or alphavirus replicon particle-based vaccine approaches, followed by booster immunizations with Env proteins in MF59 adjuvant. Efficient in vivo expression of plasmid encoded genes by electrical permeabilization has been described (see, *e.g.,* Zucchelli et al. (2000) J. Virol. 74:11598-11607; Banga et al. (1998) Trends Biotechnol. 10:408-412; Heller et al. (1996) Febs Lett. 389:225-228; Mathiesen et al. (1999) Gene Ther. 4:508-514; Mir et al. (1999) Proc. Nat'l Acad Sci. USA 8:4262-4267; Nishi et al. (1996) Cancer Res.5:1050-1055). Both native and V-deleted monomeric (gp120) and oligomeric (o-gp140) forms of protein from the SF162 strain were tested as boosters. All protein preparations were highly purified and extensively characterized by biophysical and immunochemical methodologies. Results from rabbit and primate immunogenicity studies indicated that, whereas neutralizing antibody responses could be consistently induced against the parental non-V2-deleted SF162 virus, the induction of responses against heterologous HIV strains improved with deletion of the V2 loop of the immunogens. Moreover, using these prime-boost vaccine regimens, potent HIV antigen-specific CD4 + and CD8+ T-cell responses were also demonstrated.

Based on these findings, V2-deleted envelope DNA and protein vaccines were chosen for advancement toward clinical evaluation. Similar approaches for immunization may be employed using, for example, nucleic acid immunization employing the synthetic HIV polynucleotides of the present invention coupled with corresponding or heterologous HIV-derived polypeptide boosts.

One embodiment of this aspect of the present invention may be described generally as follows. Antigens are selected for the vaccine composition(s). Env polypeptides are typically employed in a first antigenic composition used to induce an immune response. Further, Gag polypeptides are typically employed in a second antigenic composition used to induce an immune response. The second antigenic composition may include further HIV-derived polypeptide sequences, including, but not limited to, Pol, Tat, Rev, Nef, Vif, Vpr, and/or Vpu sequences. A DNA prime vaccination is typically performed with the first and second antigenic compositions. Further DNA vaccinations with one or more of the antigenic compositions may also be included at selected time intervals. The prime is typically followed by at least one boost. The boost may, for example, include adjuvanted HIV-derived polypeptides (e.g., corresponding to those used for the DNA vaccinations), coding sequences for HIV-derived polypeptides (e.g., corresponding to those used for the DNA vaccinations) encoded by a viral vector, further DNA vaccinations, and/or combinations of the foregoing. In one embodiment, a DNA prime is administered with a first antigenic composition (e.g., a DNA construct encoding an Envelope polypeptide) and second antigenic composition (e.g., a DNA construct encoding a Gag polypeptide, as Pol polypeptide, a Tat polypeptide, a Nef polypeptide, and a Rev polypeptide). The DNA construct for use in the prime may, for example, comprise a CMV promoter operably linked to the polynucleotide encoding the polypeptide sequence. The DNA prime is followed by a boost, for example, an adjuvanted Envelope polypeptide boost and a viral vector boost (where the viral vector encodes, e.g., a Gag polypeptide, a Pol polypeptide, a Tat polypeptide, a Nef polypeptide, and a Rev polypeptide). Alternately (or in addition), the boost may be an adjuvanted Gag polypeptide, Pol polypeptide, Tat polypeptide, Nef polypeptide, and Rev polypeptide boost and a viral vector boost (where the viral vector encodes, e.g., an Envelope polypeptide). The boost may include all polypeptide antigens which were encoded in the DNA prime; however, this is not required. Further, different polypeptide antigens may be used in the boost relative to the initial vaccination and visa versa. Further, the initial vaccination may be a viral vector rather than a DNA construct.

Some factors that may be considered in HIV envelope vaccine design are as follows. Envelope-based vaccines have demonstrated protection against infection in non-human primate models. Passive antibody studies have demonstrated protection against HIV infection in the presence of neutralizing antibodies against the virus challenge stock. Vaccines that exclude Env generally confer less protective efficacy. Experiments performed in support of the present invention have demonstrated that monomeric gp120 protein-derived from the SF2 lab strain provided neutralization of HIV-11ab strains and protection against virus challenges in primate models. Primary gp120 protein derived from Thai E field strains provided cross-subtype neutralization of lab strains. Primary sub-type B oligomeric o-gp140 protein provided partial neutralization of subtype B primary (field) isolates. Primary sub-type B o-gp140ΔV2 DNA prime plus protein boost provided potent neutralization of diverse subtype B primary isolates and protection against virus challenge in primate models. Primary sub-type C o-gp140 and o-gp 140AV2 likely provide similar results to those just described for sub-type B.

Vaccine strategies for induction of potent, broadly reactive, neutralizing antibodies may be assisted by construction of Envelope polypeptide structures that expose conserved neutralizing epitopes, for example, variable-region deletions and de-glycosylations, envelope protein-receptor complexes, rational design based on crystal structure (e.g., β-sheet deletions), and gp41-fusion domain based immunogens.

Stable CHO cell lines for envelope protein production have been developed using optimized envelope polypeptide coding sequences, including, but not limited to, the following: gp 120, o-gp140, gp120ΔV2, o-gp140ΔV2, gp120ΔV1V2, o-gp140ΔV1V2.

In addition, following prime-boost regimes (such as those described above) appear to be beneficial to help reduce viral load in infected subjects, as well as possibly slow or prevent progression of HIV-related disease (relative to untreated subjects).

Exemplary antigenic compositions and immunogenicity studies are presented in Examples 9, 10, 11, and 12.

### EXPERIMENTAL

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

### Example 1

### Generation of Synthetic Expression Cassettes

### A. Generating Synthetic Polynucleotides

The polynucleotide sequences of the present invention were manipulated to maximize expression of their gene products. The order of the following steps may vary.

First, the HIV-1 codon usage pattern was modified so that the resulting nucleic acid coding sequence was comparable to codon usage found in highly expressed human genes. The HIV codon usage reflects a high content of the nucleotides A or T of the codon-triplet. The effect of the HIV-1 codon usage is a high AT content in the DNA sequence that results in a high AU content in the RNA and in a decreased translation ability and instability of the mRNA. In comparison, highly expressed human codons prefer the nucleotides G or C. The wild-type sequences were modified to be comparable to codon usage found in highly expressed human genes.

Second, for some genes non-functional variants were created. In the following table (Table B) mutations affecting the activity of several HIV genes are disclosed.

**Table B**

| **Gene** | **"Region"** | **Exemplary Mutations** |
|---|---|---|
| Pol | prot | **Att** = Reduced activity by attenuation of Protease |
| | | (Thr26Ser) (e.g., Konvalinka et al., 1995, J Virol 69: 7180-86) |
| | | **Ina** = Mutated Protease, nonfunctional enzyme |
| | | (Asp25A1a)(e.g., Konvalinka et al., 1995, J Virol 69: 7180-86) |
| | RT | **YM** = Deletion of catalytic center (YMDD_AP; SEQ ID NO:7) (e.g., Biochemistry, 1995, 34, 5351, Patel et. al.) |
| | | **WM** = Deletion of primer grip region (WMGY PI; SEQ ID NO: 8) (e.g., J Biol Chem, 272, 17, 11157, Palaniappan, et. al., 1997) |
| | RNase | no direct mutations, RnaseH is affected by "WM" mutation in RT |
| | Integrase | 1) Mutation of HHCC domain, Cys40Ala (e.g., Wiskerchen et. al., 1995, J Virol, 69: 376). |
| | | 2.) Inactivation catalytic center, Asp64Ala, Asp116A1a, Glu152Ala (e.g., Wiskerchen et. al., 1995, J Virol, 69: 376). |
| | | 3) Inactivation of minimal DNA binding domain (MDBD), deletion of Trp235(e.g., Ishikawa et. al., 1999, J Virol, 73: 4475). |
| | | Constructs int.opt.mut.SF2 and int.opt.mut_C (South Africa TV1) both contain all these mutations (1, 2, and 3) |
| Env | | Mutations in cleavage site (*e.g*., mut1-4,7) |
| | | |
| | | Mutations in glycosylation site (*e.g*., GM mutants, for example, change Q residue in V1 and/or V2 to N residue; may also be designated by residue altered in sequence) |
| Tat | | Mutants of Tat in transactivation domain (e.g., Caputo et al., 1996, Gene Ther. 3:235) |
| | | cys22 mutant (Cys22GIy) = **TatC22** |
| | | cys37 mutant (Cys37Ser) = **TatC37** |
| | | cys22/37 double mutant = **TatC22/37** |
| Rev | | Mutations in Rev domains (e.g., Thomas et al., 1998, J Virol. 72:2935-44) |
| | | Mutation in RNA binding-nuclear localization ArgArg38,39AspLeu = **M5** |
| | | Mutation in activation domain LeuGlu78,79AspLeu = **M10** |
| Nef | | Mutations of myristoylation signal and in oligomerization domain: |
| | | 1. Single point mutation myristoylation signal: Gly-to-Ala = **-Myr** |
| | | |
| | | 2. Deletion of N-terminal first 18 (sub-type B, e.g., SF162) or 19 (sub-type C, e.g., South Africa clones) amino acids: **-Myr18** or **-Myr19** (respectively) |
| | | |
| | | (e.g., Peng and Robert-Guroff, 2001, Immunol Letters 78: 195-200) |
| | | Single point mutation oligomerization: |
| | | (e.g., Liu et al., 2000, J Viro174: 5310-19) Asp 125Gly (sub B SF162) or Asp 124Gly (sub C South Africa clones) |
| | | |
| | | Mutations affecting (1) infectivity (replication) of HIV-virions and/or (2) CD4 down regulation. (*e.g*., Lundquist et al. (2002) J Virol. 76(9):4625-33) |
| Vif | | Mutations of Vif: |
| | | e.g., Simon et al., 1999, J Virol 73:2675-81 |
| Vpr | | Mutations of Vpr: |
| | | e.g., Singh et al., 2000, J Virol 74: 10650-57 |
| Vpu | | Mutations of Vpu: |
| | | e.g., Tiganos et al., 1998, Virology 251: 96-107 |

Constructs comprising some of these mutations are described herein. Vif, vpr and vpu synthetic constructs are described. Reducing or eliminating the function of the associated gene products can be accomplished employing the teachings set forth in the above table, in view of the teachings of the present specification.

In one embodiment of the invention, the full length coding region of the Gag-polymerase sequence is included with the synthetic Gag sequences in order to increase the number of epitopes for virus-like particles expressed by the synthetic, optimized Gag expression cassette. Because synthetic HIV-1 Gag-polymerase expresses the potentially deleterious functional enzymes reverse transcriptase (RT) and integrase (INT) (in addition to the structural proteins and protease), it is important to inactivate RT and INT functions. Several in-frame deletions in the RT and INT reading frame can be made to achieve catalytic nonfunctional enzymes with respect to their RT and INT activity. {Jay. A. Levy (Editor) (1995) The Retroviridae, Plenum Press, New York. ISBN 0-306-45033X. Pages 215-20; Grimison, B. and Laurence, J. (1995), Journal Of Acquired Immune Deficiency Syndromes and Human Retrovirology 9(1):58-68; Wakefield, J. K.,et al., (1992) Journal Of Virology 66(11):6806-6812; Esnouf, R.,et al., (1995) Nature Structural Biology 2(4):303-308; Maignan, S., et al., (1998) Journal Of Molecular Biology 282(2):359-368; Katz, R. A. and Skalka, A. M. (1994) Annual Review Of Biochemistry 73 (1994); Jacobo-Molina, A., et al., (1993) Proceedings Of the National Academy Of Sciences Of the United States Of America 90(13):6320-6324; Hickman, A. B., et al., (1994) Journal Of Biological Chemistry 269(46):29279-29287; Goldgur, Y., et al., (1998) Proceedings Of the Notional Academy Of Sciences Of the United States Of America 95(16):9150-9154; Goette, M., et al., (1998) Journal Of Biological Chemistry 273(17):10139-10146; Gorton, J. L., et al., (1998) Journal of Virology 72(6):5046-5055; Engelman, A., et al., (1997) Journal Of Virology 71(5):3507-3514; Dyda, F., et al., Science 266(5193):1981-1986; Davies, J. F., et al., (1991) Science 252(5002):88-95; Bujacz, G., et al., (1996) Febs Letters 398(2-3):175-178; Beard, W. A., et al., (1996) Journal Of Biological Chemistry 271(21):12213-12220; Kohlstaedt, L. A., et al., (1992) Science 256(5065):1783-1790; Krug, M. S. and Berger, S. L. (1991) Biochemistry 30(44):10614-10623; Mazumder, A., et al., (1996) Molecular Pharmacology 49(4):621-628; Palaniappan, C., et al., (1997) Journal Of Biological Chemistry 272(17):11157-11164; Rodgers, D. W., et al., (1995) Proceedings Of the National Academy Of Sciences Of the United States Of America 92(4):1222-1226; Sheng, N. and Dennis, D. (1993) Biochemistry 32(18):4938-4942; Spence, R. A., et al., (1995) Science 267(5200):988-993.}

Furthermore selected B- and/or T-cell epitopes can be added to the Gag-polymerase constructs within the deletions of the RT- and INT-coding sequence to replace and augment any epitopes deleted by the functional modifications of RT and INT. Alternately, selected B- and T-cell epitopes (including CTL epitopes) from RT and INT can be included in a minimal VLP formed by expression of the synthetic Gag or synthetic GagProt cassette, described above. (For descriptions of known HIV Band T-cell epitopes see, HIV Molecular Immunology Database CTL Search Interface; Los Alamos Sequence Compendia, 1987-1997 internet address: http://hiv-web.lanl.gov/immunology/index.html.)

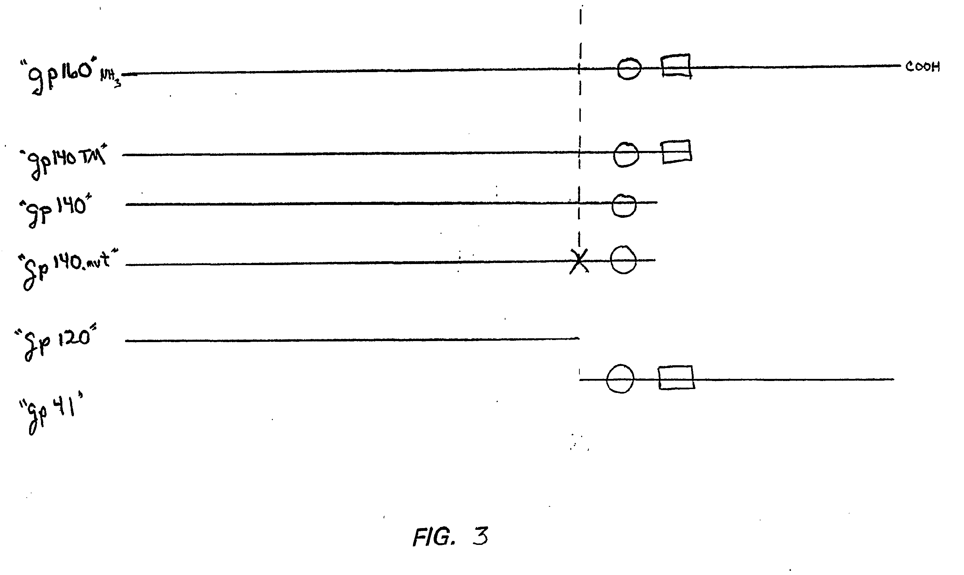

In another aspect, the present invention comprises *Env* coding sequences that include, but are not limited to, polynucleotide sequences encoding the following HIV-encoded polypeptides: gp160, gp140, and gp120 (see, e.g., U.S. Patent No. 5,792,459 for a description of the HIV-1_{SF2} ("SF2") Env polypeptide). The relationships between these polypeptides is shown schematically in Figure 3 (in the figure: the polypeptides are indicated as lines, the amino and carboxy termini are indicated on the gp 160 line; the open circle represents the oligomerization domain; the open square represents a transmembrane spanning domain (TM); and "c" represents the location of a cleavage site, in gp140.mut the "X" indicates that the cleavage site has been mutated such that it no longer functions as a cleavage site). The polypeptide gp160 includes the coding sequences for gp 120 and gp41. The polypeptide gp41 is comprised of several domains including an oligomerization domain (OD) and a transmembrane spanning domain (TM). In the native envelope, the oligomerization domain is required for the non-covalent association of three gp41 polypeptides to form a trimeric structure: through non-covalent interactions with the gp41 trimer (and itself), the gp120 polypeptides are also organized in a trimeric structure. A cleavage site (or cleavage sites) exists approximately between the polypeptide sequences for gp120 and the polypeptide sequences corresponding to gp41. This cleavage site(s) can be mutated to prevent cleavage at the site. The resulting gp140 polypeptide corresponds to a truncated form of gp160 where the transmembrane spanning domain of gp41 has been deleted. This gp140 polypeptide can exist in both monomeric and oligomeric (*i.e.* trimeric) forms by virtue of the presence of the oligomerization domain in the gp41 moiety. In the situation where the cleavage site has been mutated to prevent cleavage and the transmembrane portion of gp41 has been deleted the resulting polypeptide product is designated "mutated" gp140 (e.g., gp140.mut). As will be apparent to those in the field, the cleavage site can be mutated in a variety of ways. (See, also, WO 00/39302).

Wild-type HIV coding sequences (*e.g*., Gag, Env, Pol, tat, rev, nef, vpr, vpu, vif, etc.) can be selected from any known HIV isolate and these sequences manipulated to maximize expression of their gene products following the teachings of the present invention. The wild-type coding region maybe modified in one or more of the following ways. In one embodiment, sequences encoding hypervariable regions of Env, particularly V1 and/or V2 were deleted. In other embodiments, mutations were introduced into sequences, for example, encoding the cleavage site in Env to abrogate the enzymatic cleavage of oligomeric gp140
into gp120 monomers. (See, e.g., Earl et al. (1990) PNAS USA 87:648-652; Earl et al. (1991) J. Virol. 65:31-41). In yet other embodiments, hypervariable region(s) were deleted, N-glycosylation sites were removed and/or cleavage sites mutated. As discussed above, different mutations may be introduced into the coding sequences of different genes (see, e.g., Table B). For example, *Tat* coding sequences were modified according to the teachings of the present specification, for example to affect the transactivation domain of the gene product (*e.g*., replacing a cystein residue at position 22 with a glycine, Caputo et al. (1996) Gene Therapy 3:235).

To create the synthetic coding sequences of the present invention the gene cassettes are designed to comprise the entire coding sequence of interest. Synthetic gene cassettes are constructed by oligonucleotide synthesis and PCR amplification to generate gene fragments. Primers are chosen to provide convenient restriction sites for subcloning. The resulting fragments are then ligated to create the entire desired sequence which is then cloned into an appropriate vector. The final synthetic sequences are (i) screened by restriction endonuclease digestion and analysis,(ii) subjected to DNA sequencing in order to confirm that the desired sequence has been obtained and (iii) the identity and integrity of the expressed protein confirmed by SDS-PAGE and Western blotting. The synthetic coding sequences are assembled at Chiron Corp. (Emeryville, CA) or by the Midland Certified Reagent Company (Midland, Texas).

Percent identity to the synthetic sequences of the present invention can be determined, for example, using the Smith-Waterman search algorithm (Time Logic, Incline Village, NV), with the following exemplary parameters: weight matrix = nuc4x4hb; gap opening penalty = 20, gap extension penalty = 5, reporting threshold = 1; alignment threshold = 20.

Various forms of the different embodiments of the present invention (*e.g*., constructs) may be combined.

Exemplary embodiments of the synthetic polynucleotides of the present invention include, but are not limited to, the sequences presented in Table C.

**Table C**

| Type B and C Synthetic, Codon Optimized Polynucleotides | | |
|---|---|---|
| **Name** | **Figure Number** | **Description (encoding)** |
| gagCpolInaTatRevNef.opt_B (SEQ ID NO:9) | 6 | Gag complete, protease non-functional, RT mutated, tat mutated, rev mutated, nef mutated; all in frame {Type B} |
| GagProtInaRTmutTatRevNef.opt_B (SEQ ID NO:10) | 7 | Gag, protease non-functional, RT mutated, tat mutated, rev mutated, nef mutated; all in frame {Type B} |
| GagTatRevNef.opt-B (SEQ ID NO:11) | 8 | Gag, tat mutated, rev mutated, nef mutated; all in frame {Type B} |
| GagCompIPohnutInaTatRevNef_C (SEQ ID NO: 12) | 9 | Gag complete, Pol, RT mutated, protease non-functional, tat mutated, rev mutated, nef mutated; all in-frame {Type C} |
| GagProtInaRTmutTatRevNef_C (SEQ ID NO:13) | 10 | Gag, protease non-functional, RT mutated, tat mutated, rev mutated, nef mutated; all in-frame {Type C} |
| GagRTmutTatRevNef_C (SEQ ID NO: 14) | 11 | Gag, RT mutated, tat mutated, rev mutated, nef mutated; all in-frame { Type C} |
| GagTatRevNef_C (SEQ ID NO:15) | 12 | Gag, tat mutated, rev mutated, nef mutated; all in-frame {Type C} |
| int.opt.mut.SF2 (SEQ ID NO: 16) | 13 | integrase mutated {Type B} |
| int.opt.SF2 (SEQ ID NO:17) | 14 | integrase {Type B} |
| int.opt.mut_C (SEQ ID NO:18) | 15 | integrase mutated {Type C} |
| mt.opt_C (SEQ ID NO:19) | 16 | integrase {Type C} |
| nef.D125G.-myr.optSF162 (SEQ ID NO:20) | 17 | nef mutated, myristoyilation defective {Type B} |
| nef.D107G.-myr18.optSF162 (SEQ ID NO:21) | 18 | nef mutated, myristoyilation defective {Type B} |
| nef.opt.D125G.SF162 (SEQ ID NO:22) | 19 | nef mutated {Type B} |
| nef.opt.SF162 (SEQ ID NO:23) | 20 | nef {Type B} |
| Nef_TV1_C_ZAopt (SEQ ID NO:24) | 21 | nucleotide sequence of a synthetic Nef-encoding polynucleotide derived from 8_5_TV1_C.ZA |
| | | nef.opt.TV1 (native) {Type C} |
| Nef_TV2 C_ZAopt (SEQ ID NO:25) | 22 | nucleotide sequence of a synthetic Nef-encoding polynucleotide derived from 12-5_1_TV2_C.ZA |
| | | nef.opt.TV2 {Type C} |
| NefD124G_TV1_C_ZAopt (SEQ ID NO:26) | 23 | nucleotide sequence of a synthetic Nef-encoding polynucleotide derived from 8_5_TV1_C.ZA. The sequence includes a mutation at position 124. |
| | | nef.opt.d124G.TV1 (mutant) {Type C} |
| NefD124G_TV2_C_ZAopt (SEQ ID NO:27) | 24 | nucleotide sequence of a synthetic Nef-encoding polynucleotide derived from 12-5_1_TV2_C.ZA. The sequence includes a mutation at position 124. |
| | | nef.d124G.opt.TV2 (mutant) {Type C} |
| NefD124G-Myr_TV1_C_ZAopt (SEQ ID NO:28) | 25 | nucleotide sequence of a synthetic Nef-encoding polynucleotide derived from 8_5_TV1_C.ZA. The sequence includes a mutation at position 124 and a mutation that eliminates the myristoyilation site of the Nef gene product. |
| | | nef.db1/mutant.opt.TV1 (mutant) {Type C} |
| nef.D106G.-myr19.opt_C (SEQ ID NO:29) | 26 | nef mutated {Type C} |
| p15RnaseH.opt.SF2 (SEQ ID NO:30) | 27 | p15 RNase H; in-frame {Type B} |
| p15RnaseH.opt_C (SEQ ID NO:31) | 28 | p15 RNase H; all in-frame {Type C} |
| p2Po1.opt.YMWM.SF2 (SEQ ID NO:32) | 29 | p2 pol mutated (RT YM, WM) {Type B} |
| p2PolInaopt.YM.SF2 (SEQ ID NO:33) | 30 | p2 pol, protease non-functional, RT YM; all in frame {Type B} |
| p2Polopt.SF2 (SEQ ID NO:34) | 31 | p2 pol; all in frame {Type B} |
| p2PolTatRevNef.opt.native_B (SEQ ID NO:35) | 32 | p2 pol tat rev nef; all native; all in frame {Type B} |
| p2PolTatRevNef.opt_B (SEQ ID NO:36) | 33 | p2 pol, protease mutated, RT mutated, tat mutated, rev mutated, nef, mutated; all in frame {Type B} |
| p2Pol.opt.YMWM_C (SEQ ID NO: 37) | 34 | p2 Pol, RT mutated YM WM; all in-frame {Type C} |
| p2Polopt.YM_C (SEQ ID NO:38) | 35 | p2 pol, RT mutated YM; all in-frame {Type C} |
| p2Polopt_C (SEQ ID NO:39) | 36 | p2 Pol; all in-frame {Type C} |
| p2PolTatRevNef opt C (SEQ ID NO:40) | 37 | p2 Pol, RT mutated, protease non-functional, tat mutated, rev mutated, nef mutated; all in-frame { Type C} |
| p2PoITatRevNef.opt.native_C (SEQ ID NO:41) | 38 | p2 pol, tat native, rev native, nef native; all in-frame {Type C} |
| p2PolTatRevNef.opt_C (SEQ ID NO:42) | 39 | p2 Pol, RT mutated, protease non-functional, tat mutated, rev mutated, nef mutated; all in-frame; all in-frame { Type C} |
| po1.opt.SF2 (SEQ ID NO:43) | 40 | pol {Type B} |
| Pol_TV1_C_ZAopt (SEQ ID NO:44) | 41 | nucleotide sequence of a synthetic Pol-encoding polynucleotide derived from 8_5_TV1_C.ZA |
| | | pol.opt.TV1 (native) {Type C} |
| Pol_TV2_C_ZAopt (SEQ ID NO:45) | 42 | nucleotide sequence of a synthetic Pol-encoding polynucleotide derived from 12-5_1_TV2_C.ZA |
| | | po1.opt.TV2 {Type C} |
| prot.opt.SF2 (SEQ ID NO:46) | 43 | protease {Type B} |
| protIna.opt.SF2 (SEQ ID NO:47) | 44 | protease non-functional {Type B} |
| protInaRT.YM.opt.SF2 (SEQ ID NO:48) | 45 | protease non-functional, RT YM mutated; all in frame {Type B} |
| protInaRT.YMWM.opt.SF2 (SEQ ID NO:49) | 46 | protease non-functional, RT YM WM mutated; all in frame |
| ProtInaRTmut.SF2 (SEQ ID NO:50) | 47 | Protease inactive, RT mutated; all in frame {Type B} |
| protRT.opt.SF2 (SEQ ID NO:51) | 48 | protease RT; all in frame {Type B} |
| ProtRT.TatRevNef.opC_B (SEQ ID NO:52) | 49 | protease mutated, RT mutated, tat mutated, rev mutated, nef, mutated; all in frame { Type B} |
| ProtRTTatRevNef.opt_B (SEQ ID NO:53) | 50 | protease mutated, RT mutated, tat mutated, rev mutated, nef, mutated; all in frame {Type B} |
| protInaRT.YM.opt_C (SEQ ID NO:54) | 51 | Protease non-functional, RT mutated YM; all in-frame {Type C} |
| protInaRT.YMWM.opC_C (SEQ ID NO:55) | 52 | Protease non-functional, RT mutated YM WM; all in-frame {TypeC} |
| ProtRT.TatRevNef.opC_C (SEQ ID NO:56) | 53 | RT mutated, Protease non-functional, tat mutated, rev mutated, nef mutated; all in-frame {Type C} |
| rev.exon1_2.M5-10.optSF162 (SEQ ID NO:57) | 54 | rev exon 1 and 2 in-frame, rev mutated {Type B} |
| rev.exon1-2.opt.SF162 (SEQ ID NO:58) | 55 | rev exon 1 and 2 in-frame {Type B} |
| rev.exon1_2.M5-10.opt_C (SEQ ID NO:59) | 56 | rev exons 1 and 2 mutated; all in-frame {Type C} |
| revexon1_2 TV1 C ZAopt (SEQ ID NO:60) | 57 | nucleotide sequence of a synthetic rev-encoding polynucleotide derived from 8_5_TV1_C.ZA. The synthetic sequence depicted corresponds to exons 1 and 2 of rev in-frame. |
| | | rev.exon1_2.opt.TV1 (native) {Type C} |
| RT.opt.SF2 (mutant) (SEQ ID NO:61) | 58 | RT mutant {Type B} |
| RT.opt.SF2 (native) (SEQ ID NO:62) | 59 | RT native {Type B} |
| RTmut.SF2 (SEQ ID NO:63) | 60 | RT mutated {TypeB} |
| tat.exon1_2.opt.C22-37.SF2 (SEQ ID NO:64) | 61 | tat exon 1 and 2 in-frame, tat mutated {Type B} |
| tat.exon1_2.opt.C37.SF2 (SEQ ID NO:65) | 62 | tat exon 1 and 2 in-frame, tat mutated {Type B} |
| tat.exon1_2.optC22-37_C (SEQ ID NO:66) | 63 | tat exons 1 and 2 mutated; all in-frame {Type C} |
| tat.exon1_2.opt.C37_C (SEQ ID NO:67) | 64 | tat exon 1 and 2 mutated; all in-frame {Type C} |
| TAT_CYS22_SF162_OPT (SEQ ID NO:68) | 65 | tat construct encoding a tat polypeptide having the cystein residue at position 22 changed (cys22); SF162 derived |
| | | Tat.cys22.opt (exons 1&2 in-frame) {TypeB} |
| tat_sf162_opt (SEQ ID NO:69) | 66 | tat construct encoding a full-length tat polypeptide derived from strain SF162 |
| | | Tat.opt (exons 1&2 in-frame) {TypeB} |
| TatC22Exon2_2_TV1 C_ZAopt (SEQ ID NO:70) | 67 | nucleotide sequence of a synthetic Tat-encoding polynucleotide derived from 8_5_TV1_C.ZA. The synthetic polynucleotide includes both exons 1 and 2 of Tat, in-frame, and further includes a mutation in exon 1 |
| | | tat.exon1_2.opt.TV1 (mutant) {TypeC} |
| TatExon1_2_TV1_C_ZAopt (SEQ ID NO:71) | 68 | nucleotide sequence of a synthetic Tat-encoding polynucleotide derived from 8_5_TV1_C.ZA. The synthetic polynucleotide includes both exons 1 and 2 of Tat in-frame |
| | | tat.exon1_2.opt.TV1 (native) {Type C} |
| TatRevNef.opt.native.SF162 (SEQ ID NO:72) | 69 | tat native, rev native, nef native; all in frame {Type B} |
| TatRevNef.opt.SF162 (SEQ ID NO:73) | 70 | tat mutated, rev mutated, nef mutated; all in frame {Type B} |
| TatRevNefGag B (SEQ ID NO:74) | 71 | tat mutated, rev mutated, nef mutated, gag; all in frame {TypeB} |
| TatRevNefgagCpolIna B (SEQ ID NO:75) | 72 | tat mutated, rev mutated, nef mutated, gag complete, protease non-functional, RT mutated; all in frame {Type B } |
| TatRevNefGagProtInaRTmut B (SEQ ID NO:76) | 73 | tat mutated, rev mutated, nef mutated, gag, protease non-functional, RT mutant; all in frame {TypeB} |
| TatRevNefp2Pol.opt_B (SEQ ID NO:77) | 74 | tat mutated, rev mutated, nef mutated, p2 pol, protease mutated, RT mutated; all in frame {Type B} |
| TatRevNefprotRTopt B (SEQ ID NO:78) | 75 | tat mutated, rev mutated, nef mutated, protease mutated, RT mutated; all in frame {Type B} |
| TatRevNef.opt.native_ZA (SEQ ID NO:79) | 76 | tat native, rev native, nef native; all in-frame {Type C} |
| TatRevNef.opt_ZA (SEQ ID NO: 80) | 77 | tat mutated, rev mutated, nef mutated; all in-frame {Type C} |
| TatRevNefGag C (SEQ ID NO:81) | 78 | tat mutated, rev mutated, nef mutated, Gag; all in-frame {TypeC} |
| TatRevNefgagCpolIna C (SEQ ID NO: 82) | 79 | tat mutated, rev mutated, nef mutated, Gag complete, pol, RT mutated, protease non-functional; all in-frame {Type C} |
| TatRevNefGagProtInaRTmut C (SEQ ID NO: 83) | 80 | tat mutated, rev mutated, nef mutated, Gag, Protease non-functional, RT mutated; all in-frame {TypeC} |
| TatRevNefProtRT opt C (SEQ ID NO:84) | 81 | tat mutated, rev mutated, nef mutated, protease non-functional, RT mutated; all in-frame {Type C} |
| vif.opt.SF2 (SEQ ID NO:85) | 82 | optimized vif derived from SF2 {Type B} |
| vpr.opt.SF2 (SEQ ID NO:86) | 83 | optimized vpr derived from SF2 {Type B} |
| vpu.opt.SF162 (SEQ ID NO:87) | 84 | optimized vpu derived from SF162 {Type B} |
| Vif_TV1_C_ZAopt (SEQ ID NO: 88) | 85 | nucleotide sequence of a synthetic Vif-encoding polynucleotide derived from 8_5_TV1_C.ZA |
| | | vif.opt.TV1 (native) |
| Vif_TV2_C_ZAopt (SEQ ID NO: 89) | 86 | nucleotide sequence of a synthetic Vif-encoding polynucleotide derived from 12-5_1_TV2_C.ZA |
| | | vif.opt.TV2 (native) |
| Vpr_TV1_C_ZAopt (SEQ ID NO:90) | 87 | nucleotide sequence of a synthetic Vpr-encoding polynucleotide derived from 8_5_TV1_C.ZA |
| | | vpr.opt.TV1 (native) |
| Vpr_TV2_C_ZAopt (SEQ ID NO:91) | 88 | nucleotide sequence of a synthetic Vpr-encoding polynucleotide derived from 12-5_1_TV2_C.ZA |
| | | vpr.opt.TV2 (native) |
| Vpu_TV1_C_ZAopt (SEQ ID NO:92) | 89 | nucleotide sequence of a synthetic Vpu-encoding polynucleotide derived from 8_5_TV1_C.ZA |
| | | vpu.opt.TV1 (native) |
| Vpu_TV2_C ZAopt (SEQ ID NO:93) | 90 | nucleotide sequence of a synthetic Vpu-encoding polynucleotide derived from 12-5_1_TV2_C.ZA |
| | | vpu.opt.TV2 (native) |

### B. Creating Expression Cassettes Comprising the Synthetic Polynucleotides of the Present Invention

The synthetic DNA fragments of the present invention are cloned into the following expression vectors: pCMVKm2, for transient expression assays and DNA immunization studies, the pCMVKm2 vector was derived from pCMV6a (Chapman et al., Nuc. Acids Res. (1991) 19:3979-3986) and comprises a kanamycin selectable marker, a ColE1 origin of replication, a CMV promoter enhancer and Intron A, followed by an insertion site for the synthetic sequences described below followed by a polyadenylation signal derived from bovine growth hormone -- the pCMVKm2 vector differs from the pCMV-link vector only in that a polylinker site was inserted into pCMVKm2 to generate pCMV-link; pESN2dhfr and pCMVPLEdhfr (also known as pCMVIII), for expression in Chinese Hamster Ovary (CHO) cells; and, pAcC13, a shuttle vector for use in the Baculovirus expression system (pAcC13, was derived from pAcC 12 which was described by Munemitsu S., et al., Mol Cell Biol. 10(11):5977-5982, 1990). See, also co-owned WO 00/39302, WO 00/39303, WO 00/39304, WO 02/04493 for a description of these vectors.

Briefly, construction of pCMVPLEdhfr (pCMVIII) was as follows. To construct a DHFR cassette, the EMCV IRES (internal ribosome entry site) leader was PCR-amplified from pCite-4a+ (Novagen, Inc., Milwaukee, WI) and inserted into pET-23d (Novagen, Inc., Milwaukee, WI) as an *Xba-Nco* fragment to give pET-EMCV. The *dhfr* gene was PCR-amplified from pESN2dhfr to give a product with a Gly-Gly-Gly-Ser spacer in place of the translation stop codon and inserted as an *Nco-Bam*H1 fragment to give pET-E-DHFR. Next, the attenuated *neo* gene was PCR amplified from a pSV2Neo (Clontech, Palo Alto, CA) derivative and inserted into the unique *Bam*H1 site of pET-E-DHFR to give pET-E-DHFR/Neo₍ₘ₂₎. Then, the bovine growth hormone terminator from pCDNA3 (Invitrogen, Inc., Carlsbad, CA) was inserted downstream of the *neo* gene to give pET-E-DHFR/Neo₍ₘ₂)BGHt. The EMCV-*dhfr*/*neo* selectable marker cassette fragment was prepared by cleavage of pET-E-DHFR/Neo₍ₘ₂₎BGHt. The CMV enhancer/promoter plus Intron A was transferred from pCMV6a (Chapman et al., Nuc. Acids Res. (1991) 19:3979-3986) as a *Hind*III-*Sal*1 fragment into pUC19 (New England Biolabs, Inc., Beverly, MA). The vector backbone of pUC19 was deleted from the Nde1 to the Sap1 sites. The above described DHFR cassette was added to the construct such that the EMCV IRES followed the CMV promoter to produce the final construct. The vector also contained an amp^{r} gene and an SV40 origin of replication.

Expression vectors of the present invention contain one or more of the synthetic coding sequences disclosed herein, e.g., shown in the Figures. When the expression cassette contains more than one coding sequence the coding sequences may all be in-frame to generate one polyprotein; alternately, the more than one polypeptide coding sequences may comprise a polycistronic message where, for example, an IRES is placed 5' to each polypeptide coding sequence.

### Example 2

### Expression Assays for the Synthetic Coding Sequences

The wild-type sequences are cloned into expression vectors having the same features as the vectors into which the synthetic HIV-derived sequences were cloned.

Expression efficiencies for various vectors carrying the wild-type (any known isolated) and corresponding synthetic sequence(s) are evaluated as follows. Cells from several mammalian cell lines (293, RD, COS-7, and CHO; all obtained from the American Type Culture Collection, 10801 University Boulevard, Manassas, VA 20110-2209) are transfected with 2 µg of DNA in transfection reagent LT1 (Pan Vera Corporation, 545 Science Dr., Madison, WI). The cells are incubated for 5 hours in reduced serum medium (Opti-MEM, Gibco-BRL, Gaithersburg, MD). The medium is then replaced with normal medium as follows: 293 cells, IMDM, 10% fetal calf serum, 2% glutamine (BioWhittaker, Walkersville, MD); RD and COS-7 cells, D-MEM, 10% fetal calf serum, 2% glutamine (Opti-MEM, Gibco-BRL, Gaithersburg, MD); and CHO cells, Ham's F-12, 10% fetal calf serum, 2% glutamine (Opti-MEM, Gibco-BRL, Gaithersburg, MD). The cells are incubated for either 48 or 60 hours. Supernatants are harvested and filtered through 0.45 µm syringe filters and, optionally, stored at - 20°C.

Supernatants are evaluated using the Coulter p24-assay (Coulter Corporation, Hialeah, FL, US), using 96-well plates coated with a suitable monoclonal antibody directed against an HIV antigen (*e.g*, a murine monoclonal directed again an HIV core antigen). The appropriate HIV antigen binds to the coated wells and biotinylated antibodies against HIV recognize the bound antigen. Conjugated strepavidin-horseradish peroxidase reacts with the biotin. Color develops from the reaction of peroxidase with TMB substrate. The reaction is terminated by addition of 4N H₂SO₄. The intensity of the color is directly proportional to the amount of HIV antigen in a sample.

Chinese hamster ovary (CHO) cells are also transfected with plasmid DNA encoding the synthetic HIV polypeptides described herein (*e.g*., pESN2dhfr or pCMVIII vector backbone) using Mirus TransIT-LT1 polyamine transfection reagent (Pan Vera) according to the manufacturers instructions and incubated for 96 hours. After 96 hours, media is changed to selective media (F12 special with 250 µg/ml G418) and cells are split 1:5 and incubated for an additional 48 hours. Media is changed every 5-7 days until colonies start forming at which time the colonies are picked, plated into 96 well plates and screened by Capture ELISA. Positive clones are expanded in 24 well plates and are screened several times for HIV protein production by Capture ELISA, as described above. After reaching confluency in 24 well plates, positive clones are expanded to T25 flasks (Corning, Corning, NY). These are screened several times after confluency and positive clones are expanded to T75 flasks.

Positive T75 clones are frozen in LN2 and the highest expressing clones are amplified with 0-5 µM methotrexate (MTX)at several concentrations and plated in 100mm culture dishes. Plates are screened for colony formation and all positive closed are again expanded as described above. Clones are expanded an amplified and screened at each step capture ELISA. Positive clones are frozen at each methotrexate level. Highest producing clones are grown in perfusion bioreactors (3L, 100L) for expansion and adaptation to low serum suspension culture conditions for scale-up to larger bioreactors.

Data from experiments performed in support of the present invention show that the synthetic HIV expression cassettes provided dramatic increases in production of their protein products, relative to the native (wild-type) sequences, when expressed in a variety of cell lines and that stably transfected CHO cell lines, which express the desired HIV polypeptide(s), may be produced. Production of HIV polypeptides using CHO cells provides (i) correct glycosylation patterns and protein conformation (as determined by binding to panel of MAbs); (ii) correct binding to CD4 receptor molecules; (iii) absence of non-mammalian cell contaminants (e.g., insect viruses and/or cells); and (iv) ease of purification.

### Example 3

### Western Blot Analysis of Expression

Western blot analysis of cells transfected with the HIV expression cassettes described herein are performed essentially as described in co-owned WO 00/39302. Briefly, human 293 cells are transfected as described in Example 2 with pCMV6abased vectors containing native or synthetic HIV expression cassettes. Cells are cultivated for 60 hours post-transfection. Supernatants are prepared as described. Cell lysates are prepared as follows. The cells are washed once with phosphate-buffered saline, lysed with detergent [1% NP40 (Sigma Chemical Co., St. Louis, MO) in 0.1 M Tris-HCl, pH 7.5], and the lysate transferred into fresh tubes. SDS-polyacrylamide gels (pre-cast 8-16%; Novex, San Diego, CA) are loaded with 20 µl of supernatant or 12.5 µl of cell lysate. A protein standard is also loaded (5 µl, broad size range standard; BioRad Laboratories, Hercules, CA). Electrophoresis is carried out and the proteins are transferred using a BioRad Transfer Chamber (BioRad Laboratories, Hercules, CA) to Immobilon P membranes (Millipore Corp., Bedford, MA) using the transfer buffer recommended by the manufacturer (Millipore), where the transfer is performed at 100 volts for 90 minutes. The membranes are exposed to HIV-1-positive human patient serum and immunostained using o-phenylenediamine dihydrochloride (OPD; Sigma).

The results of the immunoblotting analysis are used to show that cells containing the synthetic HIV expression cassette produce the expected HIV-polypeptide(s) at higher per-cell concentrations than cells containing the native expression cassette.

### Example 4

### In Vivo Immunogenicity of Synthetic HIV Expression Cassettes

### A. Immunization

To evaluate the immunogenicity of the synthetic HIV expression cassettes, a mouse study may be performed. The plasmid DNA, e.g., pCMVKM2 carrying an expression cassette comprising a synthetic sequence of the present invention, is diluted to the following final concentrations in a total injection volume of 100 µl: 20 µg, 2 µg, 0.2 µg, and 0.02 µg. To overcome possible negative dilution effects of the diluted DNA, the total DNA concentration in each sample is brought up to 20 µg using the vector (pCMVKM2) alone. As a control, plasmid DNA comprising an expression cassette encoding the native, corresponding polypeptide is handled in the same manner. Twelve groups of four Balb/c mice (Charles River, Boston, MA) are intramuscularly immunized (50 µl per leg, intramuscular injection into the *tibialis anterior*) using varying dosages.

### B. Humoral Immune Response

The humoral immune response is checked with a suitable anti-HIV antibody ELISAs (enzyme-linked immunosorbent assays) of the mice sera 0 and 4 weeks post immunization (groups 5-12) and, in addition, 6 and 8 weeks post immunization, respectively, 2 and 4 weeks post second immunization (groups 1-4).

The antibody titers of the sera are determined by anti-HIV antibody ELISA. Briefly, sera from immunized mice were screened for antibodies directed against an appropriate HIV protein (*e.g*., HIV p55 for Gag). ELISA microtiter plates are coated with 0.2 µg of HIV protein per well overnight and washed four times; subsequently, blocking is done with PBS-0.2% Tween (Sigma) for 2 hours. After removal of the blocking solution, 100 µl of diluted mouse serum is added. Sera are tested at 1/25 dilutions and by serial 3-fold dilutions, thereafter. Microtiter plates are washed four times and incubated with a secondary, peroxidase-coupled anti-mouse IgG antibody (Pierce, Rockford, IL). ELISA plates are washed and 100 µl of 3, 3', 5, 5'-tetramethyl benzidine (TMB; Pierce) was added per well. The optical density of each well is measured after 15 minutes. The titers reported are the reciprocal of the dilution of serum that gave a half-maximum optical density (O.D.).

The results of the mouse immunizations with plasmid-DNAs are used to show that the synthetic expression cassettes provide improvement of immunogenicity relative to the native expression cassettes. Also, the second boost immunization induces a secondary immune response after two weeks (groups 1-3).

### C. Cellular Immune Response

The frequency of specific cytotoxic T-lymphocytes (CTL) is evaluated by a standard chromium release assay of peptide pulsed Balb/c mouse CD4 cells. HIV protein-expressing vaccinia virus infected CD-8 cells are used as a positive control (vv-protein). Briefly, spleen cells (Effector cells, E) are obtained from the BALB/c mice (immunized as described above). The cells are cultured, restimulated, and assayed for CTL activity against, e.g., Gag peptide-pulsed target cells as described (Doe, B., and Walker, C.M., AIDS 10(7):793-794, 1996). Cytotoxic activity is measured in a standard ⁵¹Cr release assay. Target (T) cells are cultured with effector (E) cells at various E:T ratios for 4 hours and the average cpm from duplicate wells is used to calculate percent specific ⁵¹Cr release.

Cytotoxic T-cell (CTL) activity is measured in splenocytes recovered from the mice immunized with HIV DNA constructs described herein. Effector cells from the DNA-immunized animals exhibit specific lysis of HIV peptide-pulsed SV-BALB (MHC matched) targets cells indicative of a CTL response. Target cells that are peptide-pulsed and derived from an MHC-unmatched mouse strain (MC57) are not lysed. The results of the CTL assays are used to show increased potency of synthetic HIV expression cassettes for induction of cytotoxic T-lymphocyte (CTL) responses by DNA immunization.

### Example 5

### In Vivo Immunogenicity of Synthetic HIV Expression Cassettes

### A. General Immunization Methods

To evaluate the immunogenicity of the synthetic HIV expression cassettes, studies using guinea pigs, rabbits, mice, rhesus macaques and baboons are performed. The studies are typically structured as follows: DNA immunization alone (single or multiple); DNA immunization followed by protein immunization (boost); DNA immunization followed by Sindbis particle immunization; immunization by Sindbis particles alone.

### B. Guinea Pigs

Experiments may be performed in guinea pigs as follows. Groups comprising six guinea pigs each are immunized intramuscularly or mucosally at 0, 4, and 12 weeks with plasmid DNAs encoding expression cassettes comprising one or more the sequences described herein. The animals are subsequently boosted at approximately 18 weeks with a single dose (intramuscular, intradermally or mucosally) of the HIV protein encoded by the sequence(s) of the plasmid boost and/or other HIV proteins. Antibody titers (geometric mean titers) are measured at two weeks following the third DNA immunization and at two weeks after the protein boost. These results are used to demonstrate the usefulness of the synthetic constructs to generate immune responses, as well as, the advantage of providing a protein boost to enhance the immune response following DNA immunization.

### C. Rabbits

Experiments may be performed in rabbits as follows. Rabbits are immunized intramuscularly, mucosally, or intradermally (using a Bioject needless syringe) with plasmid DNAs encoding the HIV proteins described herein. The nucleic acid immunizations are followed by protein boosting after the initial immunization. Typically, constructs comprising the synthetic HIV-polypeptide-encoding polynucleotides of the present invention are highly immunogenic and generate substantial antigen binding antibody responses after only 2 immunizations in rabbits.

### D. Humoral Immune Response

In any immunized animal model, the humoral immune response is checked in serum specimens from the immunized animals with an anti-HIV antibody ELISAs (enzyme-linked immunosorbent assays) at various times post-immunization. The antibody titers of the sera are determined by anti-HIV antibody ELISA as described above. Briefly, sera from immunized animals are screened for antibodies directed against the HIV polypeptide/protein(s) encoded by the DNA and/or polypeptide used to immunize the animals. Wells of ELISA microtiter plates are coated overnight with the selected *HIV* polypeptide/protein and washed four times; subsequently, blocking is done with PBS-0.2% Tween (Sigma) for 2 hours. After removal of the blocking solution, 100 µl of diluted mouse serum is added. Sera are tested at 1/25 dilutions and by serial 3-fold dilutions, thereafter. Microtiter plates are washed four times and incubated with a secondary, peroxidase-coupled anti-mouse IgG antibody (Pierce, Rockford, IL). ELISA plates are washed and 100 µl of 3, 3', 5, 5'-tetramethyl benzidine (TMB; Pierce) was added per well. The optical density of each well is measured after 15 minutes. Titers are typically reported as the reciprocal of the dilution of serum that gave a half-maximum optical density (O.D.).

Cellular immune response may also be evaluated.

### Example 6

### DNA-immunization of Baboons and Rhesus Macaques Using Expression Cassettes Comprising he Synthetic HIV Polynucleotides of the Present Invention A. Baboons

Four baboons are immunized 3 times (weeks 0, 4 and 8) bilaterally, intramuscular into the quadriceps or mucosally using the gene delivery vehicles described herein. The animals are bled two weeks after each immunization and an HIV antibody ELISA is performed with isolated plasma. The ELISA is performed essentially as described above except the second antibody-conjugate is an anti-human IgG, g-chain specific, peroxidase conjugate (Sigma Chemical Co., St. Louis, MD 63178) used at a dilution of 1:500. Fifty µg/ml yeast extract may be added to the dilutions of plasma samples and antibody conjugate to reduce non-specific background due to preexisting yeast antibodies in the baboons. Lymphoproliferative responses to are observed in baboons two weeks post-fourth immunization (at week 14), and enhanced substantially post-boosting with HIV-polypeptide (at week 44 and 76). Such proliferation results are indicative of induction of T-helper cell functions.

### B. Rhesus Macaques

The improved potency of the synthetic, codon-modified *HIV* polypeptide encoding polynucleotides of the present invention, when constructed into expression plasmids may be confirmed in rhesus macaques. Typically, the macaques have detectable HIV-specific CTL after two or three 1 mg doses of modified *HIV* polynucleotide. In sum, these results demonstrate that the synthetic HIV DNA is immunogenic in non-human primates. Neutralizing antibodies may also detected.

### Example 7

### Co-Transfection of Monocistronic and Multicistronic Constructs

The present invention includes co-transfection with multiple, monocistronic expression cassettes, as well as, co-transfection with one or more multi-cistronic expression cassettes, or combinations thereof.

Such constructs, in a variety of combinations, may be transfected into 293T cells for transient transfection studies.

For example, a bicistronic construct may be made where the coding sequences for the different HIV polypeptides are under the control of a single CMV promoter and, between the two coding sequences, an IRES (internal ribosome entry site (EMCV IRES); Kozak, M., Critical Reviews in Biochemistry and Molecular Biology 27(45):385-402, 1992; Witherell, G.W., et al., Virology 214:660-663, 1995) sequence is introduced after the first HIV coding sequence and before the second HIV coding sequence.

Supernatants collected from cell culture are tested for the presence of the HIV proteins and indicate that appropriate proteins are expressed in the transfected cells (e.g., if an Env coding sequence was present the corresponding Env protein was detected; if a Gag coding sequence was present the corresponding Gag protein was detected, etc).

The production of chimeric VLPs by these cell lines may be determined using electron microscopic analysis. (See, e.g., co-owned WO 00/39302).

### Example 8

### Accessory gene components for an HIV- vaccine: functional analysis of mutated Tat, Rev and Nef Type C antigens

The HIV-1 regulatory and accessory genes have received increased attention as components of HIV vaccines due to their role in viral pathogenesis, the high ratio of highly conserved CTL epitopes and their early expression in the viral life cycle. Because of various undesirable properties of these genes, questions regarding their safety and suitability as vaccine components have been raised. Experiments performed in support of the present invention have analyzed candidate HIV-1 subtype C *tat, rev,* and *nef* mutants for efficient expression and inactivation of potential deleterious functions. Other HIV subtype accessory genes may be evaluated similarly.

Sequence-modified, mutant *tat, rev,* and *nef* genes coding for consensus Tat, Rev and Nef proteins of South African HIV-1 subtype C were constructed using overlapping synthetic oligonucleotides and PCR-based site-directed mutagenesis. Constructs of the wild-type genes of the isolates closely resembling the respective consensus sequences were also made by PCR. *In vitro* expression of the constructs was analyzed by western blotting. The *trans*-activation activity of the Tat mutants and nuclear RNA export activity of the Rev mutants were studied after transfection of various cell lines using reporter-gene-based functionality assays.

*In vitro* expression of all constructs was demonstrated by western blotting using antigen specific mouse serum generated by DNA vaccination of mice with Tat, Rev, or Nef-expression plasmids. Expression levels of the sequence-modified genes were significantly higher than the wild-type genes.

Subtype B and C Tat cDNA was mutated to get TatC22, TatC37, and TatC22/37. Tat activity assays in three cell lines (RD, HeLa and 293). In the background of the subtype C consensus Tat, a single mutation at C22 was insufficient to inactivate LTR-dependent CAT expression. In contrast, this activity was significantly impaired in RD, 293 and HeLa cells using the single mutation, C37, or the double mutation, C22C37 (see Table B). Corresponding results were obtained for Tat mutants derived from subtype B strains.

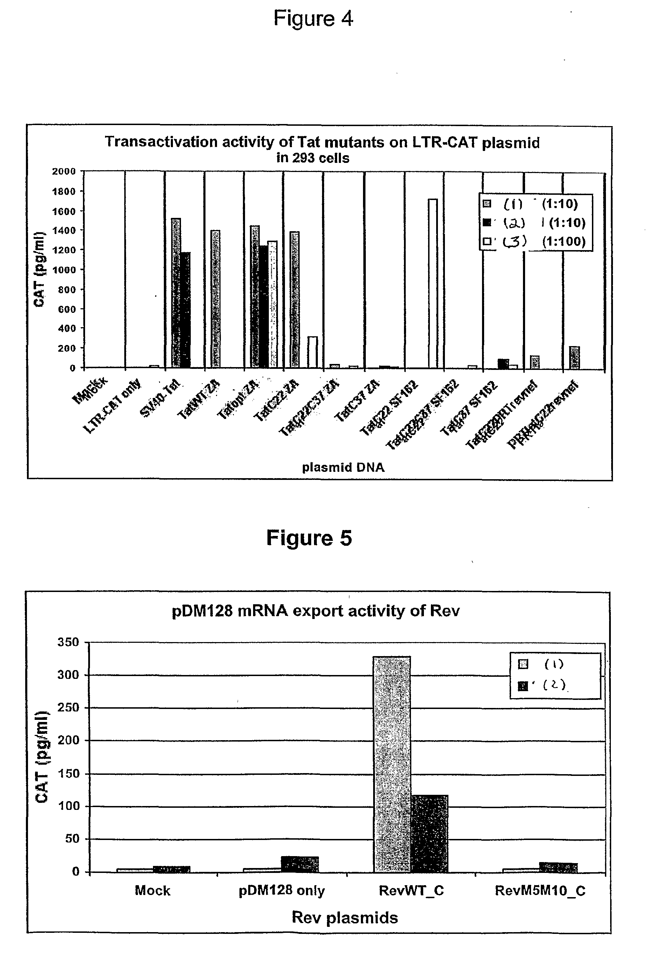

Exemplary results are presented in Figure 4 for transactivation activity of Tat mutants on LTR-CAT plasmid in 293 cells. Three independent assays were performed for each construct (Figure 4, legend (1), (2), (3)).

The subtype C constructs TatC22ProtRTTatRevNef and ProtRTTatC22RevNef showed reduced Tat activity when compared to TatC22 alone, probably due to structural changes caused by the fusion protein.

For Rev constructs, to test for the loss of function, a CAT assay with a reporter plasmid including native or mutated Rev was used. As shown in Figure 5, compared to wild-type Rev, the mRNA export function of the subtype C Rev with a double mutation, M5M10 (see Table B), was significantly lower. The background levels are shown in the "mock" data and the pDM128 reporter plasmid without Rev date. Two independent assays were performed for each construct (Figure 5, legend (1), (2)).

Assays to measure Nef-specific functions may also be performed (Nef mutations are described in Table B). For example, FACs analysis is used to look for the presence of MHC1 and CD4 on cell surfaces. Cells are assayed in the presence and absence of Nef expression (for controls), as well as using the synthetic polynucleotides of the present invention that encode native nef protein and mutated nef protein. Down-regulation of MHC1 and CD4 expression indicates that the nef gene product is not functional, i.e., if nef is non-functional there is no down regulation.

These data demonstrate the impaired functionality of *tat* and *rev* DNA immunogens that may form part of a multi-component HIV-1 subtype C vaccine. In contrast to previous published data by other groups, the C22 mutation did not sufficiently inactivate the transactivation function of Tat. The C37 mutation appeared to be required for inactivation of subtype C and subtype B Tat proteins.

### Example 9

### Evaluation of immunogenicity of various HIV polypeptide encoding plasmids

As noted above, the immunogenicity of any of the polynucleotides or expression cassettes described herein is readily evaluated. In the following table (Table D) are exemplified procedures involving a comparison of the immunogenicity of subtype B and C envelope plasmids, both individually and as a mixed-subtype vaccine, using electroporation, in rabbits. It will be apparent that such methods are equally applicable to any other HIV polypeptide.

**Table D**

| **Grp** | **Animal** | **Imm'n** # | **Adjuvant** | **Immunogen** | **Total Dose** | **Vol/ Site** | **Sites/ Animal** | **Route** |
|---|---|---|---|---|---|---|---|---|
| 1 | 1-4 | 1, 2 | - | pCMV 160 **TV1** DNA | 1.0mg | 0.5ml | 2 | IM/Quad (Electro) |
| | | 3 | - | pCMV 160 **TV1** DNA | 1.0mg | 0.5ml | 2 | IM/Quad (Electro) |
| | | | **MF59C** | Protein TBD | 0.05mg | 0.5ml | 2 | IM/Glut |
| 2 | 5-8 | 1, 2 | - | pCMV 160 dV2 **TV1** DNA | 1.0mg | 0.5ml | 2 | IM/Quad (Electro) |
| | | 3 | - | pCMV 160 dV2 **TV1** DNA | 1.0mg | 0.5ml | 2 | DvVQuad (Electro) |
| | | | | | 0.05mg | 0.5ml | 2 | IM/Glut |
| | | | **MF59C** | Protein TBD | | | | |
| 3 | 9-12 | 1, 2 | - | pCMV 160 dV1/V2 **TV1** DNA | 1.0mg | 0.5ml | 2 | IM/Quad (Electro) |
| | | 3 | - | pCMV 160 dV1/V2 **TV1** DNA | 1.0mg | 0.5ml | 2 | IM/Quad (Electro) |
| | | | | | 0.05mg | 0.5ml | 2 | IM/Glut |
| | | | **MF59C** | Protein TBD | | | | |
| 4 | 13-16 | 1, 2 | - | pCMV 140 **TV1** DNA | 1.0mg | 0.5ml | 2 | IM/Quad (Electro) |
| | | 3 | - | pCMV 140 **TV1** DNA | 1.0mg | 0.5ml | 2 | IM/Quad (Electro) |
| | | | **MF59C** | Protein TBD | 0.05mg | 0.5ml | 2 | IM/Glut |
| 5 | 17-20 | 1, 2 | - | pCMV140dV2**TV1** DNA | 1.0mg | 0.5ml | 2 | IM/Quad (Electro) |
| | | 3 | - | pCMV140dV2**TV1** DNA | 1.0mg | 0.5ml | 2 | IM/Quad (Electro) |
| | | | **MF59C** | Protein TBD | 0.05mg | 0.5ml | 2 | IM/Glut |
| 6 | 21-24 | 1, 2 | - | pCMV 140 dV1/V2 **TV1** DNA | 1.0mg | 0.5ml | 2 | IM/Quad (Electro) |
| | | 3 | - | pCMV 140 dV1/V2 **TV1** DNA | 1.0mg | 0.5ml | 2 | IM/Quad (Electro) |
| | | | | | 0.05mg | 0.5ml | 2 | IM/Glut |
| | | | **MF59C** | Protein TBD | | | | |
| 7 | 25-28 | 1, 2 | - | pSIN140dV2**SF162** DNA | 1.0mg | 0.5ml | 2 | IM/Quad (Electro) |
| | | 3 | - | pSIN 140 dV2 **SF162** DNA | 1.0mg | 0.5ml | 2 | IM/Quad (Electro) |
| | | | **MF59C** | Protein TBD | 0.05mg | 0.5ml | 2 | IM/Glut |
| 8 | 29-32 | 1, 2 | - | pCMV 140 dV2 **SF162** DNA | 1.0mg | 0.5ml | 2 | IM/Quad (Electro) |
| | | 3 | - | pCMV 140 dV2 **SF162** DNA | 1.0mg | 0.5ml | 2 | IM/Quad (Electro) |
| | | | **MF59C** | Protein TBD | 0.05mg | 0.5ml | 2 | IM/Glut |
| 9 | 33-36 | 1, 2 | - | pCMV 140 Q154 **SF162** DNA | 1.0mg | 0.5ml | 2 | IM/Quad (Electro) |
| | | 3 | - | pCMV 140 Q154 **SF162** DNA | 1.0mg | 0.5ral | 2 | IM/Quad (Electro) |
| | | | **MF59C** | Protein TBD | 0.05mg | 0.5ml | | 2 IM/Glut |
| 10 | 37-40 | 1, 2 | - | pCMV 140 dV2 **SF162** DNA | 1.0mg | | | |
| | | | | pCMV 140 dV2 **TV1** DNA | 1.0mg | 0.5ml | 2 | IM/Quad (Electro) |
| | | 3 | - | pCMV 140 dV2 **SF162** DNA | 1.0mg | | | |
| | | | | pCMV 140 dV2 **TV1** DNA | 1.0mg | 0.5ml | 2 | IM/Quad (Electro) |
| | | | **MF59C** | Protein TBD | 0.05mg | 0.5ml | 2 | IM/Glut |
| | | | - | pCMV 140 dV2 **SF162** | 1.0mg | | | |
| | | | | DNA pCMV 140 dV2 **TV1** DNA | 1.0mg | 0.5ml | 2 | IM/Quad (Electro) |
| 11 | 41-44 | 1, 2 | - | pCMV 140 dV2 **SF162** DNA | 1.0mg | | | |
| | | | | pCMV 140 dV2 **TV1** DNA | 1.0mg | 0.5ml | 2 | IM/Quad (Electro) |
| | | 3 | **MF59C** | Protein TBD | 0.05mg | 0.5ml | 2 | IM/Glut |

The MF59C adjuvant is a microfluidized emulsion containing 5% squalene, 0.5% tween 80, 0.5% span 85, in 10mM citrate pH 6, stored in 10mL aliquots at 4°C.

Immunogens are prepared as described in the following table (Table E) for administration to animals in the various groups. Concentrations may vary from those described in the table, for example depending on the sequences and/or proteins being used.

**Table E**

| Group | Preparation |
|---|---|
| **1-9** | **Immunization 1-3: pCMV and pSIN based plasmid DNA in Saline + Electroporation** Subtype B and C plasmids will be provided frozen at a concentration of 1.0mg/ml in sterile 0.9% saline. Store at -80°C until use. Thaw DNA at room temperature; the material should be clear or slightly opaque, with no particulate matter. Animals will be shaved prior to immunization, under sedation of 1x dose IP (by animal weight) of Ketamine-Xylazine (80mg/ml - 4mg/ml). Immunize each rabbit with 0.5ml DNA mixture per side (IM/Quadriceps), 1.0ml per animal. Follow the DNA injection with Electroporation using a 6-needle circular array with 1cm diameter, 1cm needle length. Electroporation pulses were given at 20V/mm, 50ms pulse length, 1 pulse/s. |
| | |
| | **Immunization 3: Protein Immunization** Proteins will be provided at 0.1mg/ in citrate buffer. Store at -80°C until use. Thaw at room temperature; material should be clear with no particulate matter. Add equal volume of MF59C adjuvant to thawed protein and mix well by inverting the tube. Immunize each rabbit with 0.5ml adjuvanted protein per side, IM/Glut for a total of 1.0ml per animal. Use material within 1 hour of the addition of adjuvant. |
| | |
| | **Immunization 1-3: Combined subtype B and C plasmid DNA in Saline** The immunogen will be provided at 2.0mg/ml total DNA (1mg/ml of each plasmid) in sterile 0.9% saline. Store at -80°C until use. Thaw DNA at room temperature; the material should be clear or slightly opaque, with no particulate matter. Animals will be shaved prior to immunization, under sedation of 1x dose IP (by animal weight) of Ketamine-Xylazine (80mg/ml - 4mg/ml). Immunize each rabbit with 0.5ml DNA mixture per side (IM/Quadriceps), 1.0ml per animal. Follow the DNA injection with Electroporation using a 6-needle circular array with 1cm diameter, 1cm needle length. Electroporation pulses were given at 20V/mm, 50ms pulse length, 1 pulse/s. |
| | |
| **10-11** | **Immunization 3: Protein Immunization** Proteins will be provided at 0.1mg/ml in citrate buffer. Store at -80°C until use. Thaw at room temperature; material should be clear with no particulate matter. Add equal volume of MF59C adjuvant to thawed protein and mix well by inverting the tube. Immunize each rabbit with 0.5ml adjuvanted protein per side, IM/Glut for a total of 1.0ml per animal. Use material within 1 hour of the addition of adjuvant. |

The immunization (Table F) and bleeding (Table G) schedules are as follows:

**Table F**

| **Imm'n:** | ***1*** | ***2*** | ***3*** | ***3*** |
|---|---|---|---|---|
| **Weeks:** | 0 | 4 | 16 | 16 |
| **Group** | | | | |
| **1** | pCMV160TV1 DNA | pCMV160 TV1 DNA | pCMV 160 TV1 DNA | Protein + MF59C |
| **2** | pCMV 160 dV2 TV1 DNA | pCMV 160 dV2 TV1 DNA | pCMV 160 dV2 TV1 DNA | Protein + MF59C |
| **3** | pCMV 160 dV1/V2 TV1 DNA | pCMV 160 dV1/N2 TV1 DNA | pCMV 160 dV1/V2 TV1 DNA | Protein + MF59C |
| **4** | pCMV 140 TV1 DNA | pCMV 140 TV1 DNA pCMV 140 TV1 DNA | | Prptein + MF59C |
| **5** | pCMV 140 dV2 TV1 DNA | pCMV 140 dV2 TV1 DNA | pCMV 140 dV2 TY1 DNA | Protein + Mf59C |
| **6** | pCMV 140 dV1/N2 TV1 DNA | pCMV 140 dV1/V2 TV1 DNA | pCMV 140 dV1/V2 TV1 DNA | Protein + MF59C |
| **7** | pSIN 140 dV2 SF162 DNA | pSIN 140 dV2 SF162 DNA | pSIN 140 dV2 SFI62 DNA | Protein + MF59C |
| **8** | pCMV 140 dV2 SF162 DNA | pCMV 140 dV2 SF162 DNA | pCMV 140 dV2 SF162 DNA | Protein + MF59C |
| **9** | pCMV 140 Q154 SF162 DNA | pCMV 140 Q154 SF162 DNA | PCMV 140 Q1S4 SF162 DNA | Protein + MF59C |
| **10** | pCMV 140 dV2 SF162 DNA + | pCMV 140 dV2 SF162 DNA + pCMV | 140 dV2 SF162 DNA + | Protein + MF59C |
| **11** | pCMV 140 dV2 TV1 DNA pCMV 140 dV2 SF162 DNA + | pCMV 140 dV2 TV1 DNA pCMV 140 dV2 SF162 DNA+ | pCMV 140 dV2 TV1 DNA pCMV 140 dV2 SF162 DNA + | Protein + MF59C |
| | pCMV 140 dV1/V2 TV1 DNA | pCMV 140 dV1/V2 TV1 DNA | pCMV 140 dV1/V2 TV1 DNA | |

**Table G**

| **Bleed:** | ***0*** | ***1*** | ***2*** | ***3*** | ***4*** | ***5*** | ***6*** | ***7*** | ***8*** | ***9*** | ***10*** |
|---|---|---|---|---|---|---|---|---|---|---|---|
| **Week:** | -3 | 4 | 6 | 8 | 12 | 16 | 18 | 20 | 24 | 28 | TBD |
| **Sample:** | Clotted Bld. for Serum | Clotted Bld. for Serum | Clotted Bld. for Serum | Clotted Bld. for Serum | Clotted Bld. for Serum | Clotted Bld. for Serum | Clotted Bld. for Serum | Clotted Bld. for Serum | Clotted Bld. for Serum | Clotted Bld. for Serum | Clotted Bld. for Serum |
| **Volume:** | 20cc each | 20cc each | 20cc each | 20cc each | 20cc each | 20cc each | 20cc each | 20cc each | 20cc each | 20cc each | 20cc each |
| **Method:** | AA/MEV | AA/MEV | AA/MEV | AA/MEV | AA/MEV | AA/MEV | AA/MEV | AA/MEV | AA/MEV | AA/MEV | CP |

### Example 10

### Mice Immunization Studies with Gag and Pol Constructs

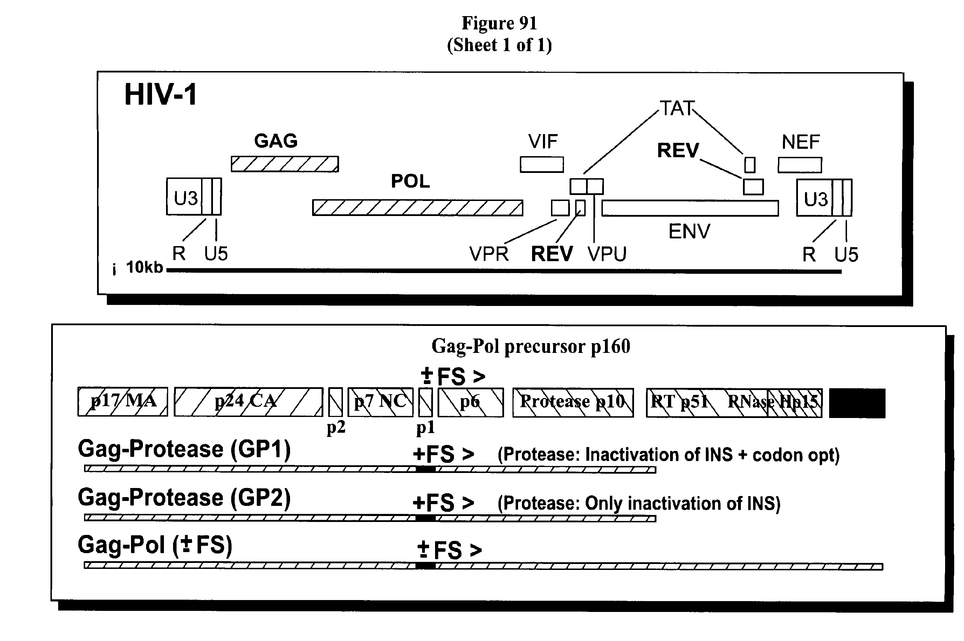

Cellular and Humoral immune responses were evaluated in mice (essentially as described in Example 4) for the following constructs: Gag, GagProtease(+FS) (GP1, protease codon optimized and inactivation of INS; GP2, protease only inactivation of INS), GagPolΔintegrase with frameshift (gagFSpol), and GagPolΔintegrase in-frame (GagPol) (see Figure 91). Versions of GagPolΔintegrase in-frame were also designed with attenuated (GagPolAtt) or non-functional Protease (GagPolIna).

In vitro expression data showed comparable expression of p55Gag and p66RT using Gag alone, GagProtease(+FS), GagFSpol and GagPolIna. Constructs with fully functional or attenuated protease (GagPol or GagPolAtt) were less efficient in expression of p55Gag and p66RT, possibly due to cytotoxic effects of protease.

DNA immunization of mice using Gag vs. GP1 and GP2 in pCMV vectors was performed intramuscularly in the tibialis anterior. Mice were immunized at the start of the study (0 week) and 4 weeks later. Bleeds were performed at 0, 4, and 6 weeks. DNA doses used were as follows: 20 µg, 2 µg, 0.2 µg, and 0.02 µg.

DNA immunization of mice using Gag vs. gagFSpol in pCMV vectors was performed intramuscularly in the tibialis anterior. Mice were immunized at the start of the study (0 week) and challenged 4 weeks later with recombinant vaccinia virus encoding Gag (rVVgag). Bleeds were performed at 0 and 4 weeks. DNA doses used were as follows: 20 µg, 2 µg, 0.2 µg, and 0.02 µg.

DNA immunization of mice using Gag vs. gagFSpol and gagpol in pCMV vectors was performed intramuscularly in the tibialis anterior. Mice were immunized at the start of the study (0 week) and challenged 4 weeks later with recombinant vaccinia virus encoding Gag (rVVgag). Bleeds were performed at 0 and 4 weeks. DNA doses used were as follows: 2 µg, 0.2 µg, 0.02 µg, and 0.002 µg.

Cellular immune responses against Gag were comparable for all tested variants, for example, Gag, GagProtease, gagFSpol and GagPolIna all had comparable potencies.

Humoral immune responses to Gag were also comparable with the exception of GP2 and especially GP1. Humoral immune responses were weaker in constructs comprising functional or attenuated proteases which may be due to less efficient secretion of p55Gag caused by overactive protease.

In vitro and in vivo experiments, performed in support of the present invention, suggest that the expression and immunogenicity of Gag was comparable with all constructs. Exceptions were GagPol in-frame with fully functional or attenuated protease. This may be the result of cytotoxic effects of protease. The immune response in mice correlated with relative levels of expression in vitro.

### Example 11

### Protein Expression, Immunogenicity, and Generation of Neutralizing Antibodies Using Type C Derived Envelope Polypeptides

Envelope (Env) vaccines derived from the subtype C primary isolate, TV1, recovered from a South African individual, were tested in rabbits as follows. Gene cassettes were designed to express the gp120 (surface antigen), gp140 (surface antigen plus ectodomain of transmembrane protein, gp41), and full-length (gp120 plus gp41) gp160 forms of the HIV-1 envelope polyprotein with and without deletions of the variable loop regions, V2 and V1V2. All of the genes were sequence-modified to enhance expression of the encoded Env glycoproteins in a Rev-independent fashion and they were subsequently cloned into pCMV-based plasmid vectors for DNA vaccine and protein production applications as described above. The sequences were codon optimized as described herein. Briefly, all the modified envelope genes were cloned into the Chiron pCMVlink plasmid vector, preferably into EcoRI/XhoI sites.

### A. Protein Expression

Full-length (gp160), truncated gp140 (Env ectodomain only) and gp120 native versions of the TV1 Env antigen were produced from the expression cassettes described herein. The gp140 encoding sequences were transiently transfected into 293T cells. The expression levels of the gene products were evaluated by an in-house antigen capture ELISA. Envelope genes constructed from the native sequences of TV001c8.2, TV001c8.5 and TV002c12.1 expressed the correct proteins in vitro, with gp140TV001c8.2 exhibiting the highest level of expression. In addition, the Env protein expressed from the TV1-derived clone 8.2 was found to bind the CD4 receptor protein indicating that this feature of the expressed protein is maintained in a functional conformation. The receptor binding properties/functionality of the expressed TV1 gp160 protein result was also confirmed by a cell-fusion assay.

Total expression increased approximately 10-fold for synthetic gp140 constructs compared with the native gp140 gene cassettes. Both the modified gp120 and gap140 variants secreted high amounts of protein in the supernatant. In addition, the V2 and V1V2 deleted forms of gp140 expressed approximately 2-fold more protein than the intact gp140. Overall, the expression levels of synthetic gp140 gene variants increased 10 to 26-fold compared with the gp140 gene with native sequences.

In sum, each synthetic construct tested showed more than 10-fold increased levels of expression relative to those using the native coding sequences. Moreover, all expressed proteins were of the expected molecular weights and were shown to bind CD4. Stable CHO cell lines were derived and small-scale protein purification methods were used to produce small quantities of each of the undeleted and V-deleted oligomeric forms (o-gp140) of these proteins for vaccine studies.

### B. Neutralization properties of TV001 and TV002 viral isolates

The transient expression experiment showed that the envelope genes derived from the TV001 and TV002 virus isolates expressed the desired protein products. Relative neutralization sensitivities of these two viral strains using sera from 18 infected South African individuals (subtypes B and C) were as follows. At a 1:10 serum dilution, the TV2 strain was neutralized by 18 of 18 sera; at 1:50, 16 of 18; at 1:250, 15/18. In comparison, the TV1 isolate was neutralized by 15 of 18 at 1:10; only 6 of 18 at 1:50; and none of the specimens at 1:250. In addition, the TV001 patient serum showed neutralization activity against the TV002 isolate at all dilutions tested. In contrast, the TV002 showed neutralization of TV001 only at the 1:10 serum dilution. These results suggest that TV001 isolate is capable of inducing a broader and more potent neutralizing antibody response in its infected host than TV002.

### C. Immunogenicity of the modified TV1 Eny DNA and protein antigens in rabbit studies

TV1 Env DNA (comprising the synthetic expression cassettes) and protein vaccines were administrated as shown in the following Table H.

**Table H**

| **Groups** | **Plasmid DNA (0, 4, and 20 wks)** | **Protein boost (20 wks)** |
|---|---|---|
| 1 | pCMVgp160.TV1 | **o-gp140.TV1** |
| 2 | pCMVgp160dV2.TV1 | **o-gp140dV2.TV1** |
| 3 | pCMVgp160dV1V2.TV1 | **o-gp140dV1V2.TV1** |
| 4 | pCMVgp140.TV1 | **o-gp140.TV1** |
| 5 | pCMVgp140dV2.TV1 | **o-gp140dV2.TV1** |
| 6 | pCMVgp140dV1V2.TV1 | **o-gp140dV1V2.TV1** |
| 7 | pCMVgp140dV2.SF162 | **o-gp140dV2.SF162** |

Seven groups of 4 rabbits per group were immunized with the designated plasmid DNA and oligomeric Env protein antigens. Three doses of DNA, 1mg of DNA per animal per immunization, were administrated intramuscularly by needle injection followed by electroporation on weeks 0, 4, and 20 weeks. A single dose of 100 ug of Env protein in MF59 adjuvant also was given intramuscularly in a separate site at 20 weeks.

The DNA immunization used subtype C sequence-modified genes (TV1) -- gp160, gp160dV2, gp160dV1V2, gp140, gp140dV2 and gp140dV1V2 -- as well as a subtype B SF162 sequence modified gp140dV2. DNA immunizations were performed at 0, 4, and 20 weeks by needle injection by the intramuscular route using electroporation to facilitate transfection of the muscle cells and of resident antigen presenting cells.

A single Env protein booster (in MF59 adjuvant) was given at 20 weeks by intramuscular injection at a separate site. Antibody titers were evaluated by ELISA following each successive immunization. Serum specimens were collected at 0, 4, 6, 8, 12, 22, and 24 weeks. Serum antibody titers were measured on ELISA. 96-well plates were coated with a protein in a concentration of 1ug/ml. Serum samples were diluted serially 3-fold. Goat anti-rabbit peroxidase conjugate (1:20,000) was used for detection. TMB was used as the substrate, and the antibody titers were read at 0.6 OD at 450nm.

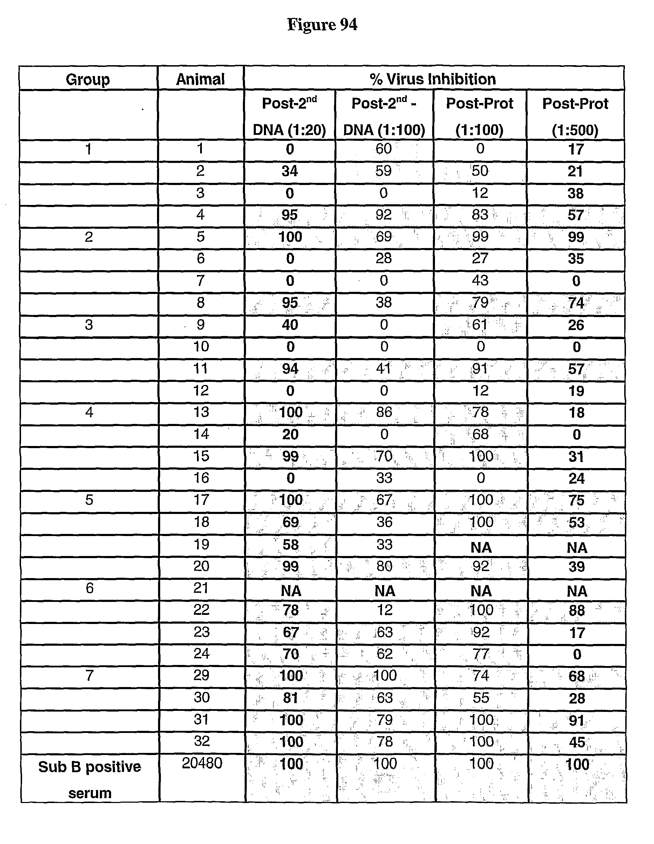

Neutralizing antibody responses against PBMC-grown R5 HIV-1 strains were monitored in the sera collected from the immunized rabbits using two different assays in two different laboratories, the 5.25 reporter cell-line based assay at Chiron and the PBMC-based assay of David Montefiori at Duke University. Results are shown in Figures 94, 95, and 96. The Chiron assay was conducted essentially as follows. Neutralizing antibody responses against the PBMC-grown subtype C TV001 and TV002 strains were measured using an in-house reporter cell line assay that uses the 5.25 cell line. This cell has CD4, CCR5, CXCR4 and BONZO receptor/co-receptors on its cell membrane. The parental CEM cell line was derived from a 4-year-old Caucasian female with acute lymphoblastic leukemia, which was fused with the human B cell line 721.174, creating CEMx174. LTR-GFP was transfected into the cells after the CCR5 gene (about 1.1 kb) was cloned into the BamH-I (5') and Sal-I (3') of the pBABE puro retroviral vector, and subsequently introduced into the CEMx174. The green fluorescence protein (GFP) of the cells was detected by flow cytometer (FACScan). For the virus neutralization assay, 50 ul of titrated virus and 50 ul of diluted immune or pre-immune serum were incubated at room temperature for one hour. This mixture was added into wells with 10⁴/ml cells plated in a 24 well plate, and incubated at 37°C for 5 to 7 days. The cells were then fixed with 2% of formaldehyde after washing with PBS. Fifteen thousand events (cells) were collected for each sample on a Becton Dickinson FACScan using Cellquest software. The data presented were the mean of the triplicate wells. The percent neutralization was calculated compared to the virus control using the following equation: % virus Inhibition = (virus control-experimental)/(virus control -cell control) x 100. Any virus inhibition observed in the pre-bleed has been subtracted for each individual animal. Values >50% are considered positive and are highlighted in gray.

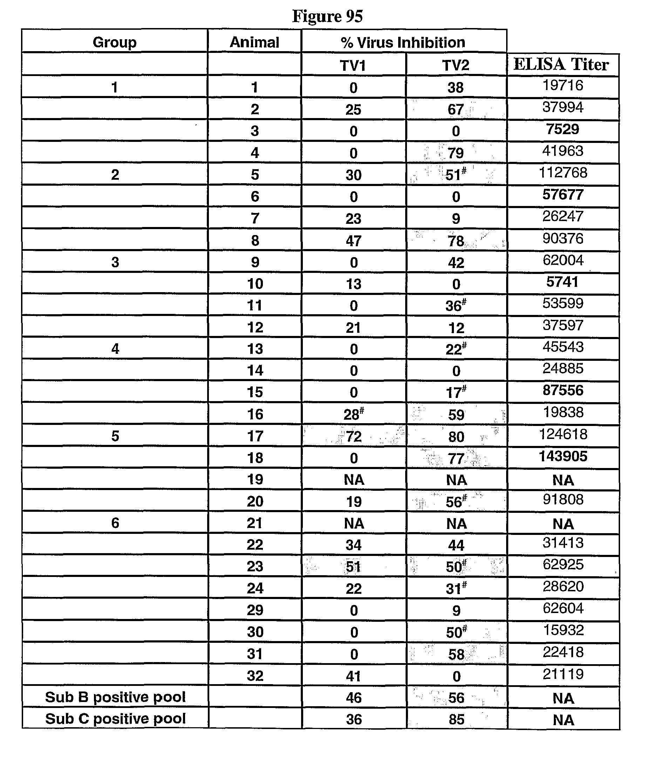

In Figure 95, the "#" indicates that animals had high levels of virus inhibition in pre-bleed serum (>20% virus inhibition) that impacted the magnitude of the observed inhibition and in some cases, our ability to score the serum as a positive or negative for the presence of significant neutralizing antibody activity (< 50% inhibition).

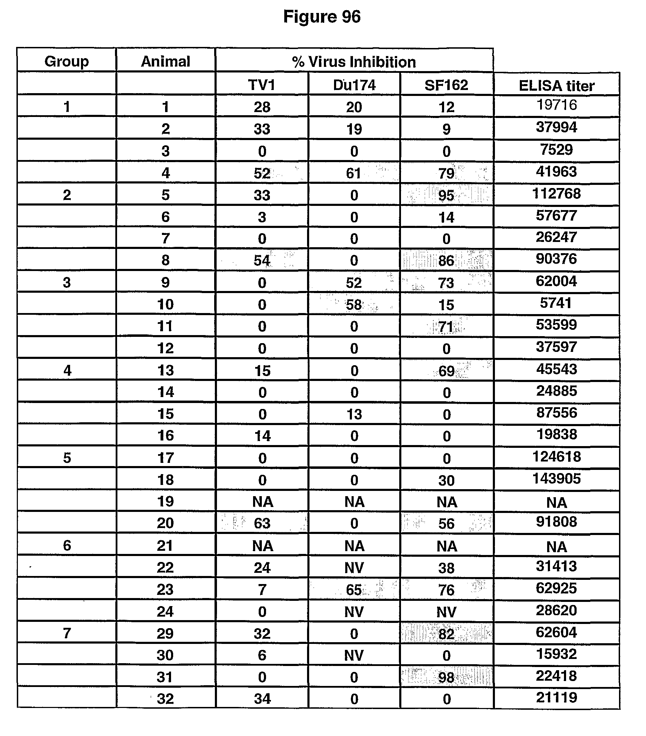

For the data presented in Figure 96, serum samples were collected after a single protein boost (post-third) were screened in triplicate at a 1:8 dilution with virus (1:24 after addition of cells). Values shown are the % reduction in p24 synthesis relative to that in the corresponding pre-bleed control samples. Zero values indicate no or negative values were measured. NV, not valid due to virus inhibition in pre-immune serum. Neutralization was considered positive when p24 was reduced by at least 80%; these samples are highlighted in dark gray. Sample with lighter gray shading showed at least a 50% reduction in p24 synthesis.

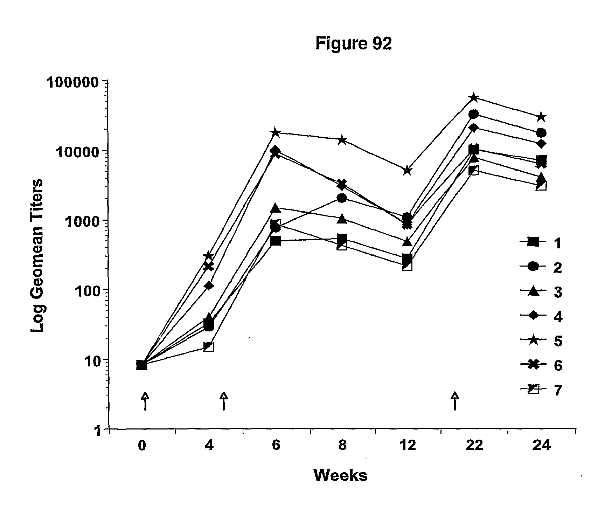

Figure 92 shows the ELISA data when plates were coated with the monomeric gp120.TV1 protein. This protein is homologous to the subtype C genes used for the immunization. All immunization groups produced high antibody titers after the second DNA immunization. The groups immunized with gp140 forms of DNA have relatively higher geometric mean antibody titers as compared to the groups using gp160 forms after both first and second DNA immunizations. Both the gp140.TV1 and gp140dV1V2.TV1 genes produced high antibody titers at about 10⁴ at two weeks post second DNA; the gp140dV2.TV1 plasmid yielded the highest titers of antibodies (>10⁴) at this time point and all others.. The binding antibody titers to the gp120.TV1 protein were higher for the group immunized with the homologous gp140dV2.TV1 genes than that with the heterologous gp140dV2.SF162 gene which showed titers of about 10³. All the groups, showed some decline in antibody titers by 8 weeks post the second DNA immunization. Following the DNA plus protein booster at 20 weeks, all groups reached titers above that previously observed after the second DNA immunization (0. 5 -1.0 log increases were observed). After the protein boost, all animals receiving the o-gp140dV2.TV1 protein whether primed by the gp140dV2.TV1 or gp160dV2.TV1 DNA, showed the highest Ab titers.

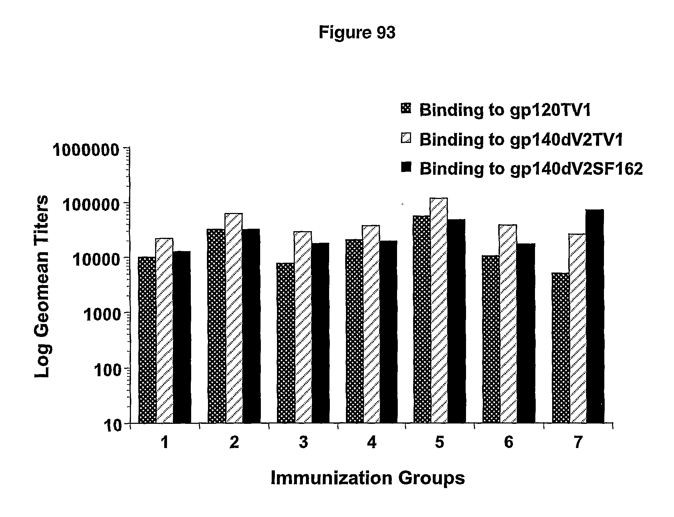

Binding antibody titers were also measured using ELISA plates coated with either oligomeric subtype C o-gp140dV2.TV1 or subtype B o-gp140dV2.SF162 proteins (Figure 93). For all the TV1 Env immunized groups, the antibody titers measured using the oligomeric protein, o-gp140dV2.TV1 were higher than those measured using the monomeric (non-V2-deleted) protein, gp120.TV1. In fact, for these groups, the titers observed with the heterologous subtype B o-gp140dV2.SF162 protein were comparable to or greater than those measured with the subtype C TV1 gp120. Nevertheless, all groups immunized with subtype C immunogens showed higher titers binding to the subtype C o-gp140dV2.TV1 protein than to the subtype B protein gp140dV2.SF162. Conversely, the group immunized with the gp140dV2.SF162 immunogen showed higher antibody titers with the oligomeric subtype B protein relative its subtype C counterpart. Overall, all three assays demonstrated that high antibody cross-reactive antibodies were generated by the subtype CTV1-based DNA and protein immunogens.

The results indicate that the subtype C TV1-derived Env DNA and protein antigens are immunogenic inducing high titers of antibodies in immunized rabbits and substantial evidence of neutralizing antibodies against both subtype B and subtype C R5 virus strains. In particular, the gp140dV2.TV1 antigens have induced consistent neutralizing responses against the subtype B SF162EnvDV2 and subtype C TV2 strains. Thus, TV1-based Env DNA and protein-based antigens are immunogenic and induce high titer antibody responses reactive with both subtype C and subtype B HIV-1 Env antigens. Neutralizing antibody responses against the neutralization sensitive subtype B R5 HIV-1_{SF162DV2} strain were observed in some groups after only two DNA immunizations. Following a single booster immunization with Env protein, the majority of rabbits in groups that received V2-deleted forms of the TV1 Env showed neutralization activity against the closely related subtype C TV2 primary strain.

### Example 12

### Immunological Responses in Rhesus Macaques

Cellular and humoral immune responses were evaluated in three groups of rhesus macaques (each group was made up of four animals) in an immunization study structured as shown in Table I. The route of administration for the immunizing composition was electroporation in each case. Antibody titers are shown in Table I for two weeks post-second immunization.

**Table I**

| Group | Formulation of Immunizing Composition * | Animal # | Titer |
|---|---|---|---|
| 1 | pCMVgag (3.5 mg) + pCMVenv (2.0 mg) | A | 3,325 |
| | | B | 4,000 |
| | | C (previously immunized with HCV core ISCOM S, rVVC core E1) | 1,838 |
| | | D (previously immunized with HCV core ISCOMS, rVVC core E1) | 1,850 |
| 2 | pCMVgag (3.5 mg) + pCMVpol (4.2 mg) | A (previously immunized with HCV core ISCOMS, rVVC core E1, p55gag_{LAI}(VLP)) | 525 |
| | | B | 5,313 |
| | | C | 6,450 |
| | | D | 5,713 |
| 3 | pCMVgag-pol (5.0 mg) | A (previously immunized with HCV core ISCOMS, rVVC core E1, pCMVgagSF2) | 0 |
| | | B (previously immunized with rVVC/E1, pCMV Epo-Epi, HIV/HCV-VLP, pCMVgagSF2, pUCgp120 SF2) | 1,063 |
| | | C | 513 |
| | | D (previously immunized with rVVC/E1, HIV/HCV-VLP) | 713 |

| | | | |
|---|---|---|---|
| * pCMVgag = pCMVKm2.GagMod Type C Botswana pCMVenv = pCMVLink.gp140env.dV2.TV1 (Type C) pCMVpol = pCMVKm2.p2Pol.mut.Ina Type C Botswana pCMVgag-pol = pCMVKm2.gagCpol.mut.Ina Type C Botswana | | | |

Pre-immune sera were obtained at week 0 before the first immunization. The first immunization was given at week 0. The second immunization was given at week 4. The first bleed was performed at 2 weeks post-second immunization (i.e., at week 6). A third immunization will be given at week 8 and a fourth at week 16. Animals 2A, 3A, 3B and 3D had been vaccinated previously (approximately 4 years or more) with gag plasmid DNA or gag VLP (subtype B).

Bulk CTL, ⁵¹Cr-release assays, and flow cell cytometry methods were used to obtain the data in Tables J and K. Reagents used for detecting gag- and pol-specific T-cells were (i) synthetic, overlapping peptides spanning "gagCpol" antigen (n=377), typically the peptides were pools of 15-mers with overlap by 11, the pools were as follows, pool 1, n=1-82, pool 2, n=83-164, pool 3, n=165-271, pool 4, n=272,-377, accordingly pools 1 and 2 are "gag"-specific, and pools 3 and 4 are "pol"-specific, and (ii) recombinant vaccinia virus (rVV), for example, rVVgag965, rVVp2Pol975 (contains p2p7gag975), and VV_{wr}parent.

Gag-specific IFNγ + CD8 + T-cells, Gag-specific IFNγ + CD4 + T-cells, Pol-specific IFNγ + CD8 + T-cells, and Pol-specific IFNγ + CD4 + T-cells in blood were determined for each animal described in Table I above, post second immunization. The results are presented in Tables J and K. It is possible that some of the pol-specific activity shown in Table K was directed against p2p7gag.

**Table J**

| Gag Assay Results | | | | | | | | |
|---|---|---|---|---|---|---|---|---|
| Group/Animal | Immunizing Composition | Gag Specific CD4+ Responses | | | | Gag Specific CD8+ Responses | | |
| | | LPA(SI) | | | Flow | CTL | | Flow |
| | | p55 | Pool 1 | Pool 2 | IFNg+ | Pool 1 | Pool 2 | IFNg+ |
| 1A | pCMVgag | 3.3 | 5.9 | 3.8 | 496 | minus | minus | 225 |
| | pCMVenv | | | | | | | |
| 1B | pCMVgag | 11.8 | 4.4 | 1.5 | 786 | minus | minus | 160 |
| | pCMVenv | | | | | | | |
| 1C | pCMVgag | 5.7 | 1.1 | 2.4 | 361 | plus | plus | 715 |
| | pCMVenv | | | | | | | |
| 1D | pCMVgag | 6.5 | 3.1 | 1.6 | 500 | plus | ? | 596 |
| | pCMVenv | | | | | | | |
| 2A | pCMVgag | 4.8 | 4.8 | 1.6 | 405 | plus | minus | 1136 |
| | pCMVpol | | | | | | | |
| 2B | pCMVgag | 12.5 | 6.8 | 3.3 | 1288 | plus | minus | 2644 |
| | pCMVpol | | | | | | | |
| 2C | pCMVgag | 6.0 | 3.8 | 2.1 | 776 | minus | minus | 0 |
| | pCMVpol | | | | | | | |
| 2D | pCMVgag | 18.9 | 13.5 | 5.4 | 1351 | minus | minus | 145 |
| | pCMVpol | | | | | | | |
| 3A | pCMV gagpol | 12.2 | 7.0 | 1.5 | 560 | plus | plus | 3595 |
| 3B | pCMV gagpol | 2.7 | 5.6 | 1.3 | 508 | plus | ? | 3256 |
| 3C | pCMV gagpol | 11.6 | 5.0 | 1.2 | 289 | minus | ? | 617 |
| 3D | pCMV gagpol | 1.5 | 1.2 | 1.4 | 120 | minus | minus | 277 |

| | | | | | | | | |
|---|---|---|---|---|---|---|---|---|
| ? = might be positive on rVVp2Pol. | | | | | | | | |

**Table K**

| Pol Assay Results | | | | | | | |
|---|---|---|---|---|---|---|---|
| Group / Animal | Immunizing Composition | Pol Specific CD4+ Response | | | Pol Specific CD8+ Responses | | |
| | | LPA(SI) | | Flow | CTL | | Flow |
| | | Pool 3 | Pool 4 | IFNg+ | Pool 3 | Pool 4 | IFNg+ |
| 1A | pCMVgag | 1 | 1.2 | 0 | minus | minus | 0 |
| | pCMVenv | | | | | | |
| 1B | pCMVgag | 1 | 1 | 0 | minus | minus | 0 |
| | pCMVenv | | | | | | |
| 1C | pCMVgag | 1 | 1.1 | 0 | minus | minus | 0 |
| | pCMVenv | | | | | | |
| 1D | pCMVgag | 1.2 | 1.3 | 0 | minus | minus | 262 |
| | pCMVenv | | | | | | |
| 2A | pCMVgag | 1.1 | 0.9 | 92 | minus | minus | 459 |
| | pCMVpol | | | | | | |
| 2B | pCMVgag | 2.5 | 1.8 | 107 | minus | minus | 838 |
| | pCMVpol | | | | | | |
| 2C | pCMVgag | 1.2 | 1.1 | 52 | plus | minus | 580 |
| | pCMVpol | | | | | | |
| 2D | pCMVgag | 2.5 | 2.7 | 113 | plus | plus | 5084 |
| | pCMVpol | | | | | | |
| 3A | pCMV gagpol | 2.7 | 2.4 | 498 | minus | minus | 3631 |
| 3B | pCMV gagpol | 1.1 | 1 | 299 | minus | minus | 1346 |
| 3C | pCMV gagpol | 2.1 | 1.4 | 369 | minus | minus | 399 |
| 3D | pCMV gagpol | 1.3 | 1.8 | 75 | minus | minus | 510 |

These results support that the constructs of the present invention are capable of generating specific cellular and humoral responses against the selected HIV-polypeptide antigens.

Although preferred embodiments of the subject invention have been described in some detail, it is understood that obvious variations can be made without departing from the spirit and the scope of the invention as defined by the appended claims.

### Embodiments of the invention:

1. A synthetic polynucleotide encoding two or more immunogenic HIV polypeptides, wherein at least two of said polypeptides are derived from different HIV subtypes.
2. The synthetic polynucleotide of embodiment 1, wherein the HIV subtypes are subtypes B and C.
3. The synthetic polynucleotide of embodiment 1, wherein said HIV polypeptides are selected from the group consisting of *Gag, Env, Pol, Tat, Rev, Nef, Vpr, Vpu, Vif* and combinations thereof.
4. The synthetic polynucleotide of embodiment 1, wherein the polynucleotide encodes *Tat, Rev* and *Nef.*
5. The synthetic polynucleotide of embodiment 1, wherein the polynucleotide encodes *Vif, Vpr* and *Vpu.*
6. The synthetic polynucleotide of embodiment 1, wherein one or more of said HIV polypeptides comprises one or more mutations.
7. The synthetic polynucleotide of embodiment 6, wherein the HIV polypeptides comprise *Pol* and the mutations are selected from the group consisting of mutations that reduce or eliminate protease function, mutations that delete the catalytic center of primer grip region of reverse transcriptase, mutations that inactive the catalytic center of DNA binding domain of integrase.
8. The synthetic polynucleotide of embodiment 6, wherein the HIV polypeptides comprise *Env* and the mutations comprise mutations in the cleavage site or mutations in the glycosylation site.
9. The synthetic polynucleotide of embodiment 6, wherein the HIV polypeptides comprise *Tat* and the mutations comprise mutations in the transactivation domain.
10. The synthetic polynucleotide of embodiment 6, wherein the HIV polypeptides comprise Rev and the mutations comprise mutations in the RNA binding-nuclear localization region or mutations in the activation domain.
11. The synthetic polynucleotide of embodiment 6, wherein the HIV polypeptides comprise *Nef and* the mutations are selected from the group consisting of mutations of myristoylation signal, mutations in oligomerization, mutations affecting infectivity and mutations affecting CD4 down regulation.
12. The synthetic polynucleotide of embodiment 6, wherein the HIV polypeptides comprise vif, vpr or vpu.
13. The synthetic polynucleotide of embodiment 1, further comprising a sequence encoding an additional antigenic polypeptide.
14. An expression cassette comprising the synthetic polynucleotide of any of embodiments 1-13.
15. A recombinant expression system for use in a selected host cell, comprising, an expression cassette of embodiment 14, and wherein said polynucleotide sequence is operably linked to control elements compatible with expression in the selected host cell.
16. The recombinant expression system of embodiment 15, wherein said control elements are selected from the group consisting of a transcription promoter, a transcription enhancer element, a transcription termination signal, polyadenylation sequences, sequences for optimization of initiation of translation, and translation termination sequences.
17. The recombinant expression system of embodiment 16, wherein said transcription promoter is selected from the group consisting of CMV, CMV+intron A, SV40, RSV, HIV-Ltr, MMLV-ltr, and metallothionein.
18. A cell comprising an expression cassette of embodiment 14, and wherein said polynucleotide sequence is operably linked to control elements compatible with expression in the selected cell.
19. The cell of embodiment 18, wherein the cell is a mammalian cell.
20. The cell of embodiment 19, wherein the cell is selected from the group consisting of BHK, VERO, HT1080, 293, RD, COS-7 and CHO cells.
21. The cell of embodiment 20, wherein the cell is a CHO cell.
22. The cell of embodiment 18, wherein the cell is an insect cell.
23. The cell of embodiment 22, wherein the cell is either *Trichoplusia ni* (Tn5) or Sf9 insect cells.
24. The cell of embodiment 18, wherein the cell is a bacterial cell.
25. The cell of embodiment 18, wherein the cell is a yeast cell.
26. The cell of embodiment 18, wherein the cell is a plant cell.
27. The cell of embodiment 18, wherein the cell is an antigen presenting cell.
28. The cell of embodiment 27, wherein the antigen presenting cell is a lymphoid cell selected from the group consisting of macrophage, monocytes, dendritic cells, B-cells, T-cells, stem cells and progenitor cells thereof.
29. The cell of embodiment 18, wherein the cell is a primary cell.
30. The cell of embodiment 18, wherein the cell is an immortalized cell.
31. The cell of embodiment 18, wherein the cell is a tumor-derived cell.
32. A method for producing a polypeptide including two or more HIV polypeptides from different subtypes, said method comprising,
   incubating the cells of embodiment 18, under conditions for producing said polypeptide.
33. A gene delivery vector for use in a mammalian subject, comprising
   a suitable gene delivery vector for use in said subject, wherein the vector comprises an expression cassette of embodiment 14, and wherein said polynucleotide sequence is operably linked to control elements compatible with expression in the subj ect.
34. A method of DNA immunization of a subject, comprising,
   introducing a gene delivery vector of embodiment 33 into said subject under conditions that are compatible with expression of said expression cassette in said subject.
35. The method of embodiment 34, wherein said gene delivery vector is a nonviral vector.
36. The method of embodiment 34, wherein said vector is delivered using a particulate carrier.
37. The method of embodiment 36, wherein said vector is coated on a gold or tungsten particle and said coated particle is delivered to said subject using a gene gun.
38. The method of embodiment 34, wherein said vector is encapsulated in a liposome preparation.
39. The method of embodiment 34, wherein said vector is a viral vector.
40. The method of embodiment 34, wherein said viral vector is a retroviral vector.
41. The method of embodiment 40, wherein said viral vector is a lentiviral vector.
42. The method of embodiment 34, wherein said subject is a mammal.
43. The method of embodiment 69, wherein said mammal is a human.
44. A method of generating an immune response in a subject, comprising
   transfecting cells of said subject a gene delivery vector of embodiment 33, under conditions that permit the expression of said polynucleotide and production of said polypeptide, thereby eliciting an immunological response to said polypeptide.
45. The method of embodiment 44, wherein said vector is a nonviral vector.
46. The method of embodiment 44, wherein said vector is delivered using a particulate carrier.
47. The method of embodiment 44, wherein said vector is coated on a gold or tungsten particle and said coated particle is delivered to said vertebrate cell using a gene gun.
48. The method of embodiment 44, wherein said vector is encapsulated in a liposome preparation.
49. The method of embodiment 44, wherein said vector is a viral vector.
50. The method of embodiment 49, wherein said viral vector is a retroviral vector.
51. The method of embodiment 49, wherein said viral vector is a lentiviral vector.
52. The method of embodiment 44, wherein said subject is a mammal.
53. The method of embodiment 44, wherein said mammal is a human.
54. The method of embodiment 44, wherein said transfecting is done *ex vivo* and said transfected cells are reintroduced into said subject.
55. The method of embodiment 44, wherein said transfecting is *done in vivo* in said subject.
56. The method of embodiment 44, where said immune response is a humoral immune response.
57. The method of embodiment 44, where said immune response is a cellular immune response.
58. The method of embodiment 44, wherein the gene delivery vector is administered intramuscularly, intramucosally, intranasally, subcutaneously, intradermally, transdermally, intravaginally, intrarectally, orally or intravenously.

## Claims

1. A first molecule, wherein said first molecule is one or more synthetic polynucleotides encoding an immunogenic HIV polypeptide, and a second molecule, wherein said second molecule is one or more immunogenic HIV polypeptides, for use in a method of generating an immune response, wherein the immunogenic HIV polypeptides are selected from at least two HIV subtypes.

2. The first and second molecules of claim 1, wherein said method involves co-administration of the first and second molecules in a prime-boost method where one or more molecules are delivered in a priming step and subsequently one or more molecules are delivered in a boosting step.

3. The first and second molecules of claim 1 or claim 2, wherein said method involves the delivery of one or more synthetic polynucleotides encoding an immunogenic HIV polypeptide followed by the delivery of one or more immunogenic HIV polypeptides.

4. The first and second molecules of any preceding claim, wherein said method involves multiple primes with one or more synthetic polynucleotides encoding an immunogenic HIV polypeptide followed by multiple boosts with one or more immunogenic HIV polypeptides.

5. The first and second molecules of any proceeding claim, wherein the first molecule encodes the same polypeptide as the immunogenic HIV polypeptide of the second molecule.

6. The first and second molecules of any one of claims 1-4, wherein the first molecule encodes a different polypeptide than the immunogenic HIV polypeptide of the second molecule.

7. The first and second molecules of any proceeding claim, wherein the immunogenic HIV polypeptide is selected from the group consisting of Gag, Env, Pol, tat, rev, nef, vif, vpr, vpu and/or regions within Gag, Env, or Pol.

8. The first and second molecules of claim 7, wherein the synthetic polynucleotide encodes Env or a region within Env and the immunogenic polypeptides is Env or a region within Env.

9. The first and second molecules of claim 7, wherein the synthetic polynucleotide encodes Gag and Pol, and the immunogenic polypeptides is Env or a region within Env.

10. The first and second molecules of any one of claims 7, 8 or 9, wherein the region within Env is selected from the group consisting of gp120, o-gp140, gap140, and gp160.


SEQUENCE LISTING

<110> NOVARTIS VACCINES AND DIAGNOSTICS, INC
      ZUR MEGEDE, Jan
      BARNETT, Susan
      LIAN, Ying

<120> POLYNUCLEOTIDES ENCODING ANTIGENIC HIV TYPE B AND/OR
      TYPE C POLYPEPTIDES, POLYPEPTIDES AND USES THEREOF

<130> 2302-18702.40 / 18702.003

<140> PCT/US02/21421
<141> 2002-07-05

<160> 93

<170> PatentIn Ver. 2.0

<210> 1
<211> 9781
<212> DNA
<213> Artificial Sequence

<220>
<223> Description of Artificial Sequence: 8_5_TV1_C.ZA

<400> 1
tggaagggtt aatttactcc aagaaaaggc aagaaatcct tgatttgtgg gtctatcaca 60
cacaaggctt cttccctgat tggcaaaact acacaccggg gccaggggtc agatatccac 120
tgacctttgg atggtgctac aagctagtgc cagttgaccc aggggaggtg gaagaggcca 180
acggaggaga agacaactgt ttgctacacc ctatgagcca acatggagca gaggatgaag 240
atagagaagt attaaagtgg aagtttgaca gcctcctagc acgcagacac atggcccgcg 300
agctacatcc ggagtattac aaagactgct gacacagaag ggactttccg cctgggactt 360
tccactgggg cgttccggga ggtgtggtct gggcgggact tgggagtggt caaccctcag 420
atgctgcata taagcagctg cttttcgcct gtactgggtc tctctcggta gaccagatct 480
gagcctggga gccctctggc tatctaggga acccactgct taagcctcaa taaagcttgc 540
cttgagtgct ttaagtagtg tgtgcccatc tgttgtgtga ctctggtaac tagagatccc 600
tcagaccctt tgtggtagtg tggaaaatct ctagcagtgg cgcccgaaca gggaccagaa 660
agtgaaagtg agaccagagg agatctctcg acgcaggact cggcttgctg aagtgcacac 720
ggcaagaggc gagaggggcg gctggtgagt acgccaattt tacttgacta gcggaggcta 780
gaaggagaga gatgggtgcg agagcgtcaa tattaagcgg cggaaaatta gataaatggg 840
aaagaattag gttaaggcca gggggaaaga aacattatat gttaaaacat ctagtatggg 900
caagcaggga gctggaaaga tttgcactta accctggcct gttagaaaca tcagaaggct 960
gtaaacaaat aataaaacag ctacaaccag ctcttcagac aggaacagag gaacttagat 1020
cattattcaa cacagtagca actctctatt gtgtacataa agggatagag gtacgagaca 1080
ccaaggaagc cttagacaag atagaggaag aacaaaacaa atgtcagcaa aaagcacaac 1140
aggcaaaagc agctgacgaa aaggtcagtc aaaattatcc tatagtacag aatgcccaag 1200
ggcaaatggt acaccaagct atatcaccta gaacattgaa tgcatggata aaagtaatag 1260
aggaaaaggc tttcaatcca gaggaaatac ccatgtttac agcattatca gaaggagcca 1320
ccccacaaga tttaaacaca atgttaaata cagtgggggg acatcaagca gccatgcaaa 1380
tgttaaaaga taccatcaat gaggaggctg cagaatggga taggacacat ccagtacatg 1440
cagggcctgt tgcaccaggc cagatgagag aaccaagggg aagtgacata gcaggaacta 1500
ctagtaccct tcaggaacaa atagcatgga tgacaagtaa tccacctatt ccagtagaag 1560

acatctataa aagatggata attctggggt taaataaaat agtaagaatg tatagccctg 1620
ttagcatttt ggacataaaa caagggccaa aagaaccctt tagagactat gtagaccggt 1680
tctttaaaac cttaagagct gaacaagcta cacaagatgt aaagaattgg atgacagaca 1740
ccttgttggt ccaaaatgcg aacccagatt gtaagaccat tttaagagca ttaggaccag 1800
gggcctcatt agaagaaatg atgacagcat gtcagggagt gggaggacct agccataaag 1860
caagagtgtt ggctgaggca atgagccaag caaacagtaa catactagtg cagagaagca 1920
attttaaagg ctctaacaga attattaaat gtttcaactg tggcaaagta gggcacatag 1980
ccagaaattg cagggcccct aggaaaaagg gctgttggaa atgtggacag gaaggacacc 2040
aaatgaaaga ctgtactgag aggcaggcta attttttagg gaaaatttgg ccttcccaca 2100
aggggaggcc agggaatttc ctccagaaca gaccagagcc aacagcccca ccagcagaac 2160
caacagcccc accagcagag agcttcaggt tcgaggagac aacccccgtg ccgaggaagg 2220
agaaagagag ggaaccttta acttccctca aatcactctt tggcagcgac cccttgtctc 2280
aataaaagta gagggccaga taaaggaggc tctcttagac acaggagcag atgatacagt 2340
attagaagaa atagatttgc cagggaaatg gaaaccaaaa atgatagggg gaattggagg 2400
ttttatcaaa gtaagacagt atgatcaaat acttatagaa atttgtggaa aaaaggctat 2460
aggtacagta ttagtagggc ctacaccagt caacataatt ggaagaaatc tgttaactca 2520
gcttggatgc acactaaatt ttccaattag tcctattgaa actgtaccag taaaattaaa 2580
accaggaatg gatggcccaa aggtcaaaca atggccattg acagaagaaa aaataaaagc 2640
attaacagca atttgtgagg aaatggagaa ggaaggaaaa attacaaaaa ttgggcctga 2700
taatccatat aacactccag tatttgccat aaaaaagaag gacagtacta agtggagaaa 2760
attagtagat ttcagggaac tcaataaaag aactcaagac ttttgggaag ttcaattagg 2820
aataccacac ccagcaggat taaaaaagaa aaaatcagtg acagtgctag atgtggggga 2880
tgcatatttt tcagttcctt tagatgaaag cttcaggaaa tatactgcat tcaccatacc 2940
tagtataaac aatgaaacac cagggattag atatcaatat aatgtgctgc cacagggatg 3000
gaaaggatca ccagcaatat tccagagtag catgacaaaa atcttagagc ccttcagagc 3060
aaaaaatcca gacatagtta tctatcaata tatggatgac ttgtatgtag gatctgactt 3120
agaaataggg caacatagag caaaaataga agagttaagg gaacatttat tgaaatgggg 3180
atttacaaca ccagacaaga aacatcaaaa agaaccccca tttctttgga tggggtatga 3240
actccatcct gacaaatgga cagtacaacc tatactgctg ccagaaaagg atagttggac 3300
tgtcaatgat atacagaagt tagtgggaaa attaaactgg gcaagtcaga tttacccagg 3360
gattaaagta aggcaactct gtaaactcct caggggggcc aaagcactaa cagacatagt 3420
accactaact gaagaagcag aattagaatt ggcagagaac agggaaattt taagagaacc 3480
agtacatgga gtatattatg atccatcaaa agacttgata gctgaaatac agaaacaggg 3540
gcatgaacaa tggacatatc aaatttatca agaaccattt aaaaatctga aaacaggaaa 3600
gtatgcaaaa atgaggacta cccacactaa tgatgtaaaa cagttaacag aggcagtgca 3660
aaaaatagcc atggaaagca tagtaatatg gggaaagact cctaaattta gactacccat 3720
ccaaaaagaa acatgggaga catggtggac agactattgg caagccacct ggatccctga 3780
gtgggagttt gttaataccc ctcccctagt aaaattatgg taccaactag aaaaagatcc 3840
catagcagga gtagaaactt tctatgtaga tggagcaact aatagggaag ctaaaatagg 3900
aaaagcaggg tatgttactg acagaggaag gcagaaaatt gttactctaa ctaacacaac 3960
aaatcagaag actgagttac aagcaattca gctagctctg caggattcag gatcagaagt 4020
aaacatagta acagactcac agtatgcatt aggaatcatt caagcacaac cagataagag 4080
tgactcagag atatttaacc aaataataga acagttaata aacaaggaaa gaatctacct 4140
gtcatgggta ccagcacata aaggaattgg gggaaatgaa caagtagata aattagtaag 4200
taagggaatt aggaaagtgt tgtttctaga tggaatagat aaagctcaag aagagcatga 4260
aaggtaccac agcaattgga gagcaatggc taatgagttt aatctgccac ccatagtagc 4320
aaaagaaata gtagctagct gtgataaatg tcagctaaaa ggggaagcca tacatggaca 4380
agtcgactgt agtccaggga tatggcaatt agattgtacc catttagagg gaaaaatcat 4440
cctggtagca gtccatgtag ctagtggcta catggaagca gaggttatcc cagcagaaac 4500
aggacaagaa acagcatatt ttatattaaa attagcagga agatggccag tcaaagtaat 4560
acatacagac aatggcagta attttaccag tactgcagtt aaggcagcct gttggtgggc 4620
aggtatccaa caggaatttg gaattcccta caatccccaa agtcagggag tggtagaatc 4680
catgaataaa gaattaaaga aaataatagg acaagtaaga gatcaagctg agcaccttaa 4740
gacagcagta caaatggcag tattcattca caattttaaa agaaaagggg gaattggggg 4800

gtacagtgca ggggaaagaa taatagacat aatagcaaca gacatacaaa ctaaagaatt 4860
acaaaaacaa attataagaa ttcaaaattt tcgggtttat tacagagaca gcagagaccc 4920
tatttggaaa ggaccagccg aactactctg gaaaggtgaa ggggtagtag taatagaaga 4980
taaaggtgac ataaaggtag taccaaggag gaaagcaaaa atcattagag attatggaaa 5040
acagatggca ggtgctgatt gtgtggcagg tggacaggat gaagattaga gcatggaata 5100
gtttagtaaa gcaccatatg tatatatcaa ggagagctag tggatgggtc tacagacatc 5160
attttgaaag cagacatcca aaagtaagtt cagaagtaca tatcccatta ggggatgcta 5220
gattagtaat aaaaacatat tggggtttgc agacaggaga aagagattgg catttgggtc 5280
atggagtctc catagaatgg agactgagag aatacagcac acaagtagac cctgacctgg 5340
cagaccagct aattcacatg cattattttg attgttttac agaatctgcc ataagacaag 5400
ccatattagg acacatagtt tttcctaggt gtgactatca agcaggacat aagaaggtag 5460
gatctctgca atacttggca ctgacagcat tgataaaacc aaaaaagaga aagccacctc 5520
tgcctagtgt tagaaaatta gtagaggata gatggaacga cccccagaag accaggggcc 5580
gcagagggaa ccatacaatg aatggacact agagattcta gaagaactca agcaggaagc 5640
tgtcagacac tttcctagac catggctcca tagcttagga caatatatct atgaaaccta 5700
tggggatact tggacgggag ttgaagctat aataagagta ctgcaacaac tactgttcat 5760
tcatttcaga attggatgcc aacatagcag aataggcatc ttgcgacaga gaagagcaag 5820
aaatggagcc agtagatcct aaactaaagc cctggaacca tccaggaagc caacctaaaa 5880
cagcttgtaa taattgcttt tgcaaacact gtagctatca ttgtctagtt tgctttcaga 5940
caaaaggttt aggcatttcc tatggcagga agaagcggag acagcgacga agcgctcctc 6000
caagtggtga agatcatcaa aatcctctat caaagcagta agtacacata gtagatgtaa 6060
tggtaagttt aagtttattt aaaggagtag attatagatt aggagtagga gcattgatag 6120
tagcactaat catagcaata atagtgtgga ccatagcata tatagaatat aggaaattgg 6180
taagacaaaa gaaaatagac tggttaatta aaagaattag ggaaagagca gaagacagtg 6240
gcaatgagag tgatggggac acagaagaat tgtcaacaat ggtggatatg ggcatcttag 6300
ggcttctgga tgctaatgat ttgtaacacg gaggacttgt gggtcacagt ctactatggg 6360
gtacctgtgt ggagagaagc aaaaactact ctattctgtg catcagatgc taaagcatat 6420
gagacagaag tgcataatgt ctgggctaca catgcttgtg tacccacaga ccccaaccca 6480
caagaaatag ttttgggaaa tgtaacagaa aattttaata tgtggaaaaa taacatggca 6540
gatcagatgc atgaggatat aatcagttta tgggatcaaa gcctaaagcc atgtgtaaag 6600
ttgaccccac tctgtgtcac tttaaactgt acagatacaa atgttacagg taatagaact 6660
gttacaggta atacaaatga taccaatatt gcaaatgcta catataagta tgaagaaatg 6720
aaaaattgct ctttcaatgc aaccacagaa ttaagagata agaaacataa agagtatgca 6780
ctcttttata aacttgatat agtaccactt aatgaaaata gtaacaactt tacatataga 6840
ttaataaatt gcaatacctc aaccataaca caagcctgtc caaaggtctc ttttgacccg 6900
attcctatac attactgtgc tccagctgat tatgcgattc taaagtgtaa taataagaca 6960
ttcaatggga caggaccatg ttataatgtc agcacagtac aatgtacaca tggaattaag 7020
ccagtggtat caactcaact actgttaaat ggtagtctag cagaagaagg gataataatt 7080
agatctgaaa atttgacaga gaataccaaa acaataatag tacatcttaa tgaatctgta 7140
gagattaatt gtacaaggcc caacaataat acaaggaaaa gtgtaaggat aggaccagga 7200
caagcattct atgcaacaaa tgacgtaata ggaaacataa gacaagcaca ttgtaacatt 7260
agtacagata gatggaataa aactttacaa caggtaatga aaaaattagg agagcatttc 7320
cctaataaaa caataaaatt tgaaccacat gcaggagggg atctagaaat tacaatgcat 7380
agctttaatt gtagaggaga atttttctat tgcaatacat caaacctgtt taatagtaca 7440
tactacccta agaatggtac atacaaatac aatggtaatt caagcttacc catcacactc 7500
caatgcaaaa taaaacaaat tgtacgcatg tggcaagggg taggacaagc aatgtatgcc 7560
cctcccattg caggaaacat aacatgtaga tcaaacatca caggaatact attgacacgt 7620
gatgggggat ttaacaacac aaacaacgac acagaggaga cattcagacc tggaggagga 7680
gatatgaggg ataactggag aagtgaatta tataaatata aagtggtaga aattaagcca 7740
ttgggaatag cacccactaa ggcaaaaaga agagtggtgc agagaaaaaa aagagcagtg 7800
ggaataggag ctgtgttcct tgggttcttg ggagcagcag gaagcactat gggcgcagcg 7860
tcaataacgc tgacggtaca ggccagacaa ctgttgtctg gtatagtgca acagcaaagc 7920
aatttgctga aggctataga ggcgcaacag catatgttgc aactcacagt ctggggcatt 7980
aagcagctcc aggcgagagt cctggctata gaaagatacc taaaggatca acagctccta 8040

gggatttggg gctgctctgg aagactcatc tgcaccactg ctgtgccttg gaactccagt 8100
tggagtaata aatctgaagc agatatttgg gataacatga cttggatgca gtgggataga 8160
gaaattaata attacacaga aacaatattc aggttgcttg aagactcgca aaaccagcag 8220
gaaaagaatg aaaaagattt attagaattg gacaagtgga ataatctgtg gaattggttt 8280
gacatatcaa actggctgtg gtatataaaa atattcataa tgatagtagg aggcttgata 8340
ggtttaagaa taatttttgc tgtgctctct atagtgaata gagttaggca gggatactca 8400
cctttgtcat ttcagaccct taccccaagc ccgaggggac tcgacaggct cggaggaatc 8460
gaagaagaag gtggagagca agacagagac agatccatac gattggtgag cggattcttg 8520
tcgcttgcct gggacgatct gcggagcctg tgcctcttca gctaccaccg cttgagagac 8580
ttcatattaa ttgcagtgag ggcagtggaa cttctgggac acagcagtct caggggacta 8640
cagagggggt gggagatcct taagtatctg ggaagtcttg tgcagtattg gggtctagag 8700
ctaaaaaaga gtgctattag tccgcttgat accatagcaa tagcagtagc tgaaggaaca 8760
gataggatta tagaattggt acaaagaatt tgtagagcta tcctcaacat acctaggaga 8820
ataagacagg gctttgaagc agctttgcta taaaatggga ggcaagtggt caaaacgcag 8880
catagttgga tggcctgcag taagagaaag aatgagaaga actgagccag cagcagaggg 8940
agtaggagca gcgtctcaag acttagatag acatggggca cttacaagca gcaacacacc 9000
tgctactaat gaagcttgtg cctggctgca agcacaagag gaggacggag atgtaggctt 9060
tccagtcaga cctcaggtac ctttaagacc aatgacttat aagagtgcag tagatctcag 9120
cttcttttta aaagaaaagg ggggactgga agggttaatt tactctagga aaaggcaaga 9180
aatccttgat ttgtgggtct ataacacaca aggcttcttc cctgattggc aaaactacac 9240
atcggggcca ggggtccgat tcccactgac ctttggatgg tgcttcaagc tagtaccagt 9300
tgacccaagg gaggtgaaag aggccaatga aggagaagac aactgtttgc tacaccctat 9360
gagccaacat ggagcagagg atgaagatag agaagtatta aagtggaagt ttgacagcct 9420
tctagcacac agacacatgg cccgcgagct acatccggag tattacaaag actgctgaca 9480
cagaagggac tttccgcctg ggactttcca ctggggcgtt ccgggaggtg tggtctgggc 9540
gggacttggg agtggtcacc ctcagatgct gcatataagc agctgctttt cgcttgtact 9600
gggtctctct cggtagacca gatctgagcc tgggagctct ctggctatct agggaaccca 9660
ctgcttaggc ctcaataaag cttgccttga gtgctctaag tagtgtgtgc ccatctgttg 9720
tgtgactctg gtaactagag atccctcaga ccctttgtgg tagtgtggaa aatctctagc 9780
a 9781

<210> 2
<211> 842
<212> PRT
<213> Artificial Sequence

<220>
<223> Description of Artificial Sequence: SF162

<400> 2
Met Asp Ala Met Lys Arg Gly Leu Cys Cys Val Leu Leu Leu Cys Gly
1               5                   10                  15

Ala Val Phe Val Ser Pro Ser Ala Val Glu Lys Leu Trp Val Thr Val
            20                  25                  30

Tyr Tyr Gly Val Pro Val Trp Lys Glu Ala Thr Thr Thr Leu Phe Cys
        35                  40                  45

Ala Ser Asp Ala Lys Ala Tyr Asp Thr Glu Val His Asn Val Trp Ala
    50                  55                  60

Thr His Ala Cys Val Pro Thr Asp Pro Asn Pro Gln Glu Ile Val Leu
65                  70                  75                  80

Glu Asn Val Thr Glu Asn Phe Asn Met Trp Lys Asn Asn Met Val Glu
                85                  90                  95

Gln Met His Glu Asp Ile Ile Ser Leu Trp Asp Gln Ser Leu Lys Pro
            100                 105                 110

Cys Val Lys Leu Thr Pro Leu Cys Val Thr Leu His Cys Thr Asn Leu
        115                 120                 125

Lys Asn Ala Thr Asn Thr Lys Ser Ser Asn Trp Lys Glu Met Asp Arg
    130                 135                 140

Gly Glu Ile Lys Asn Cys Ser Phe Lys Val Thr Thr Ser Ile Arg Asn
145                 150                 155                 160

Lys Met Gln Lys Glu Tyr Ala Leu Phe Tyr Lys Leu Asp Val Val Pro
                165                 170                 175

Ile Asp Asn Asp Asn Thr Ser Tyr Lys Leu Ile Asn Cys Asn Thr Ser
            180                 185                 190
Val Ile Thr Gln Ala Cys Pro Lys Val Ser Phe Glu Pro Ile Pro Ile
        195                 200                 205

His Tyr Cys Ala Pro Ala Gly Phe Ala Ile Leu Lys Cys Asn Asp Lys
    210                 215                 220

Lys Phe Asn Gly Ser Gly Pro Cys Thr Asn Val Ser Thr Val Gln Cys
225                 230                 235                 240

Thr His Gly Ile Arg Pro Val Val Ser Thr Gln Leu Leu Leu Asn Gly
                245                 250                 255

Ser Leu Ala Glu Glu Gly Val Val Ile Arg Ser Glu Asn Phe Thr Asp
            260                 265                 270

Asn Ala Lys Thr Ile Ile Val Gln Leu Lys Glu Ser Val Glu Ile Asn
        275                 280                 285

Cys Thr Arg Pro Asn Asn Asn Thr Arg Lys Ser Ile Thr Ile Gly Pro
    290                 295                 300

Gly Arg Ala Phe Tyr Ala Thr Gly Asp Ile Ile Gly Asp Ile Arg Gln
305                 310                 315                 320

Ala His Cys Asn Ile Ser Gly Glu Lys Trp Asn Asn Thr Leu Lys Gln
                325                 330                 335

Ile Val Thr Lys Leu Gln Ala Gln Phe Gly Asn Lys Thr Ile Val Phe
            340                 345                 350

Lys Gln Ser Ser Gly Gly Asp Pro Glu Ile Val Met His Ser Phe Asn
        355                 360                 365

Cys Gly Gly Glu Phe Phe Tyr Cys Asn Ser Thr Gln Leu Phe Asn Ser
    370                 375                 380

Thr Trp Asn Asn Thr Ile Gly Pro Asn Asn Thr Asn Gly Thr Ile Thr
385                 390                 395                 400

Leu Pro Cys Arg Ile Lys Gln Ile Ile Asn Arg Trp Gln Glu Val Gly
                405                 410                 415

Lys Ala Met Tyr Ala Pro Pro Ile Arg Gly Gln Ile Arg Cys Ser Ser
            420                 425                 430

Asn Ile Thr Gly Leu Leu Leu Thr Arg Asp Gly Gly Lys Glu Ile Ser
        435                 440                 445

Asn Thr Thr Glu Ile Phe Arg Pro Gly Gly Gly Asp Met Arg Asp Asn
    450                 455                 460

Trp Arg Ser Glu Leu Tyr Lys Tyr Lys Val Val Lys Ile Glu Pro Leu
465                 470                 475                 480

Gly Val Ala Pro Thr Lys Ala Lys Arg Arg Val Val Gln Arg Glu Lys
                485                 490                 495

Arg Ala Val Thr Leu Gly Ala Met Phe Leu Gly Phe Leu Gly Ala Ala
            500                 505                 510
Gly Ser Thr Met Gly Ala Arg Ser Leu Thr Leu Thr Val Gln Ala Arg
        515                 520                 525

Gln Leu Leu Ser Gly Ile Val Gln Gln Gln Asn Asn Leu Leu Arg Ala
    530                 535                 540

Ile Glu Ala Gln Gln His Leu Leu Gln Leu Thr Val Trp Gly Ile Lys
545                 550                 555                 560

Gln Leu Gln Ala Arg Val Leu Ala Val Glu Arg Tyr Leu Lys Asp Gln
                565                 570                 575

Gln Leu Leu Gly Ile Trp Gly Cys Ser Gly Lys Leu Ile Cys Thr Thr
            580                 585                 590

Ala Val Pro Trp Asn Ala Ser Trp Ser Asn Lys Ser Leu Asp Gln Ile
        595                 600                 605

Trp Asn Asn Met Thr Trp Met Glu Trp Glu Arg Glu Ile Asp Asn Tyr
    610                 615                 620

Thr Asn Leu Ile Tyr Thr Leu Ile Glu Glu Ser Gln Asn Gln Gln Glu
625                 630                 635                 640

Lys Asn Glu Gln Glu Leu Leu Glu Leu Asp Lys Trp Ala Ser Leu Trp
                645                 650                 655

Asn Trp Phe Asp Ile Ser Lys Trp Leu Trp Tyr Ile Lys Ile Phe Ile

660                 665                 670

Met Ile Val Gly Gly Leu Val Gly Leu Arg Ile Val Phe Thr Val Leu
        675                 680                 685

Ser Ile Val Asn Arg Val Arg Gln Gly Tyr Ser Pro Leu Ser Phe Gln
    690                 695                 700

Thr Arg Phe Pro Ala Pro Arg Gly Pro Asp Arg Pro Glu Gly Ile Glu
705                 710                 715                 720

Glu Glu Gly Gly Glu Arg Asp Arg Asp Arg Ser Ser Pro Leu Val His
                725                 730                 735

Gly Leu Leu Ala Leu Ile Trp Asp Asp Leu Arg Ser Leu Cys Leu Phe
            740                 745                 750

Ser Tyr His Arg Leu Arg Asp Leu Ile Leu Ile Ala Ala Arg Ile Val
        755                 760                 765

Glu Leu Leu Gly Arg Arg Gly Trp Glu Ala Leu Lys Tyr Trp Gly Asn
    770                 775                 780

Leu Leu Gln Tyr Trp Ile Gln Glu Leu Lys Asn Ser Ala Val Ser Leu
785                 790                 795                 800

Phe Asp Ala Ile Ala Ile Ala Val Ala Glu Gly Thr Asp Arg Ile Ile
                805                 810                 815

Glu Val Ala Gln Arg Ile Gly Arg Ala Phe Leu His Ile Pro Arg Arg
            820                 825                 830
Ile Arg Gln Gly Phe Glu Arg Ala Leu Leu
        835                 840


<210> 3
<211> 867
<212> PRT
<213> Artificial Sequence

<220>
<223> Description of Artificial Sequence: TV1.8_2

<400> 3
Met Arg Val Met Gly Thr Gln Lys Asn Cys Gln Gln Trp Trp Ile Trp
  1               5                  10                  15

Gly Ile Leu Gly Phe Trp Met Leu Met Ile Cys Asn Thr Glu Asp Leu
            20                  25                  30

Trp Val Thr Val Tyr Tyr Gly Val Pro Val Trp Arg Asp Ala Lys Thr
        35                  40                  45

Thr Leu Phe Cys Ala Ser Asp Ala Lys Ala Tyr Glu Thr Glu Val His

50                  55                  60

Asn Val Trp Ala Thr His Ala Cys Val Pro Thr Asp Pro Asn Pro Gln
65                  70                  75                  80

Glu Ile Val Leu Gly Asn Val Thr Glu Asn Phe Asn Met Trp Lys Asn
                85                  90                  95

Asp Met Ala Asp Gln Met His Glu Asp Val Ile Ser Leu Trp Asp Gln
            100                 105                 110

Ser Leu Lys Pro Cys Val Lys Leu Thr Pro Leu Cys Val Thr Leu Asn
        115                 120                 125

Cys Thr Asp Thr Asn Val Thr Gly Asn Arg Thr Val Thr Gly Asn Ser
    130                 135                 140

Thr Asn Asn Thr Asn Gly Thr Gly Ile Tyr Asn Ile Glu Glu Met Lys
145                 150                 155                 160

Asn Cys Ser Phe Asn Ala Thr Thr Glu Leu Arg Asp Lys Lys His Lys
                165                 170                 175

Glu Tyr Ala Leu Phe Tyr Arg Leu Asp Ile Val Pro Leu Asn Glu Asn
            180                 185                 190

Ser Asp Asn Phe Thr Tyr Arg Leu Ile Asn Cys Asn Thr Ser Thr Ile
        195                 200                 205

Thr Gln Ala Cys Pro Lys Val Ser Phe Asp Pro Ile Pro Ile His Tyr
    210                 215                 220

Cys Ala Pro Ala Gly Tyr Ala Ile Leu Lys Cys Asn Asn Lys Thr Phe
225                 230                 235                 240

Asn Gly Thr Gly Pro Cys Tyr Asn Val Ser Thr Val Gln Cys Thr His
                245                 250                 255

Gly Ile Lys Pro Val Val Ser Thr Gln Leu Leu Leu Asn Gly Ser Leu
            260                 265                 270

Ala Glu Glu Gly Ile Ile Ile Arg Ser Glu Asn Leu Thr Glu Asn Thr
        275                 280                 285

Lys Thr Ile Ile Val His Leu Asn Glu Ser Val Glu Ile Asn Cys Thr
    290                 295                 300

Arg Pro Asn Asn Asn Thr Arg Lys Ser Val Arg Ile Gly Pro Gly Gln
305                 310                 315                 320

Ala Phe Tyr Ala Thr Asn Asp Val Ile Gly Asn Ile Arg Gln Ala His
                325                 330                 335

Cys Asn Ile Ser Thr Asp Arg Trp Asn Lys Thr Leu Gln Gln Val Met

340                 345                 350

Lys Lys Leu Gly Glu His Phe Pro Asn Lys Thr Ile Gln Phe Lys Pro
        355                 360                 365

His Ala Gly Gly Asp Leu Glu Ile Thr Met His Ser Phe Asn Cys Arg
    370                 375                 380

Gly Glu Phe Phe Tyr Cys Asn Thr Ser Asn Leu Phe Asn Ser Thr Tyr
385                 390                 395                 400

His Ser Asn Asn Gly Thr Tyr Lys Tyr Asn Gly Asn Ser Ser Ser Pro
                405                 410                 415

Ile Thr Leu Gln Cys Lys Ile Lys Gln Ile Val Arg Met Trp Gln Gly
            420                 425                 430

Val Gly Gln Ala Thr Tyr Ala Pro Pro Ile Ala Gly Asn Ile Thr Cys
        435                 440                 445

Arg Ser Asn Ile Thr Gly Ile Leu Leu Thr Arg Asp Gly Gly Phe Asn
    450                 455                 460

Thr Thr Asn Asn Thr Glu Thr Phe Arg Pro Gly Gly Gly Asp Met Arg
465                 470                 475                 480

Asp Asn Trp Arg Ser Glu Leu Tyr Lys Tyr Lys Val Val Glu Ile Lys
                485                 490                 495

Pro Leu Gly Ile Ala Pro Thr Lys Ala Lys Arg Arg Val Val Gln Arg
            500                 505                 510

Glu Lys Arg Ala Val Gly Ile Gly Ala Val Phe Leu Gly Phe Leu Gly
        515                 520                 525

Ala Ala Gly Ser Thr Met Gly Ala Ala Ser Ile Thr Leu Thr Val Gln
    530                 535                 540

Ala Arg Gln Leu Leu Ser Gly Ile Val Gln Gln Gln Ser Asn Leu Leu
545                 550                 555                 560

Lys Ala Ile Glu Ala Gln Gln His Met Leu Gln Leu Thr Val Trp Gly
                565                 570                 575

Ile Lys Gln Leu Gln Ala Arg Val Leu Ala Ile Glu Arg Tyr Leu Lys
            580                 585                 590

Asp Gln Gln Leu Leu Gly Ile Trp Gly Cys Ser Gly Arg Leu Ile Cys
        595                 600                 605

Thr Thr Ala Val Pro Trp Asn Ser Ser Trp Ser Asn Lys Ser Glu Lys
    610                 615                 620

Asp Ile Trp Asp Asn Met Thr Trp Met Gln Trp Asp Arg Glu Ile Ser

625                 630                 635                 640

Asn Tyr Thr Gly Leu Ile Tyr Asn Leu Leu Glu Asp Ser Gln Asn Gln
                645                 650                 655

Gln Glu Lys Asn Glu Lys Asp Leu Leu Glu Leu Asp Lys Trp Asn Asn
            660                 665                 670

Leu Trp Asn Trp Phe Asp Ile Ser Asn Trp Pro Trp Tyr Ile Lys Ile
        675                 680                 685

Phe Ile Met Ile Val Gly Gly Leu Ile Gly Leu Arg Ile Ile Phe Ala
    690                 695                 700

Val Leu Ser Ile Val Asn Arg Val Arg Gln Gly Tyr Ser Pro Leu Ser
705                 710                 715                 720

Phe Gln Thr Leu Thr Pro Ser Pro Arg Gly Leu Asp Arg Leu Gly Gly
                725                 730                 735

Ile Glu Glu Glu Gly Gly Glu Gln Asp Arg Asp Arg Ser Ile Arg Leu
            740                 745                 750

Val Ser Gly Phe Leu Ser Leu Ala Trp Asp Asp Leu Arg Asn Leu Cys
        755                 760                 765

Leu Phe Ser Tyr His Arg Leu Arg Asp Phe Ile Leu Ile Ala Val Arg
    770                 775                 780

Ala Val Glu Leu Leu Gly His Ser Ser Leu Arg Gly Leu Gln Arg Gly
785                 790                 795                 800

Trp Glu Ile Leu Lys Tyr Leu Gly Ser Leu Val Gln Tyr Trp Gly Leu
                805                 810                 815

Glu Leu Lys Lys Ser Ala Ile Ser Leu Leu Asp Thr Ile Ala Ile Thr
            820                 825                 830

Val Ala Glu Gly Thr Asp Arg Ile Ile Glu Leu Val Gln Arg Ile Cys
        835                 840                 845

Arg Ala Ile Leu Asn Ile Pro Arg Arg Ile Arg Gln Gly Phe Glu Ala
    850                 855                 860

Ala Leu Leu
865

<210> 4
<211> 869
<212> PRT
<213> Artificial Sequence

<220>
<223> Description of Artificial Sequence: TV1.8_5

<400> 4
Met Arg Val Met Gly Thr Gln Lys Asn Cys Gln Gln Trp Trp Ile Trp
1               5                   10                  15

Gly Ile Leu Gly Phe Trp Met Leu Met Ile Cys Asn Thr Glu Asp Leu
            20                  25                  30

Trp Val Thr Val Tyr Tyr Gly Val Pro Val Trp Arg Glu Ala Lys Thr
        35                  40                  45

Thr Leu Phe Cys Ala Ser Asp Ala Lys Ala Tyr Glu Thr Glu Val His
    50                  55                  60

Asn Val Trp Ala Thr His Ala Cys Val Pro Thr Asp Pro Asn Pro Gln
65                  70                  75                  80

Glu Ile Val Leu Gly Asn Val Thr Glu Asn Phe Asn Met Trp Lys Asn
                85                  90                  95

Asn Met Ala Asp Gln Met His Glu Asp Ile Ile Ser Leu Trp Asp Gln
            100                 105                 110

Ser Leu Lys Pro Cys Val Lys Leu Thr Pro Leu Cys Val Thr Leu Asn
        115                 120                 125

Cys Thr Asp Thr Asn Val Thr Gly Asn Arg Thr Val Thr Gly Asn Thr
    130                 135                 140

Asn Asp Thr Asn Ile Ala Asn Ala Thr Tyr Lys Tyr Glu Glu Met Lys
145                 150                 155                 160

Asn Cys Ser Phe Asn Ala Thr Thr Glu Leu Arg Asp Lys Lys His Lys
                165                 170                 175

Glu Tyr Ala Leu Phe Tyr Lys Leu Asp Ile Val Pro Leu Asn Glu Asn
            180                 185                 190

Ser Asn Asn Phe Thr Tyr Arg Leu Ile Asn Cys Asn Thr Ser Thr Ile
        195                 200                 205

Thr Gln Ala Cys Pro Lys Val Ser Phe Asp Pro Ile Pro Ile His Tyr
    210                 215                 220

Cys Ala Pro Ala Asp Tyr Ala Ile Leu Lys Cys Asn Asn Lys Thr Phe
225                 230                 235                 240

Asn Gly Thr Gly Pro Cys Tyr Asn Val Ser Thr Val Gln Cys Thr His
                245                 250                 255

Gly Ile Lys Pro Val Val Ser Thr Gln Leu Leu Leu Asn Gly Ser Leu
            260                 265                 270

Ala Glu Glu Gly Ile Ile Ile Arg Ser Glu Asn Leu Thr Glu Asn Thr

275                 280                 285

Lys Thr Ile Ile Val His Leu Asn Glu Ser Val Glu Ile Asn Cys Thr
    290                 295                 300

Arg Pro Asn Asn Asn Thr Arg Lys Ser Val Arg Ile Gly Pro Gly Gln
305                 310                 315                 320

Ala Phe Tyr Ala Thr Asn Asp Val Ile Gly Asn Ile Arg Gln Ala His
                325                 330                 335

Cys Asn Ile Ser Thr Asp Arg Trp Asn Lys Thr Leu Gln Gln Val Met
            340                 345                 350

Lys Lys Leu Gly Glu His Phe Pro Asn Lys Thr Ile Lys Phe Glu Pro
        355                 360                 365

His Ala Gly Gly Asp Leu Glu Ile Thr Met His Ser Phe Asn Cys Arg
    370                 375                 380

Gly Glu Phe Phe Tyr Cys Asn Thr Ser Asn Leu Phe Asn Ser Thr Tyr
385                 390                 395                 400

Tyr Pro Lys Asn Gly Thr Tyr Lys Tyr Asn Gly Asn Ser Ser Leu Pro
                405                 410                 415

Ile Thr Leu Gln Cys Lys Ile Lys Gln Ile Val Arg Met Trp Gln Gly
            420                 425                 430

Val Gly Gln Ala Met Tyr Ala Pro Pro Ile Ala Gly Asn Ile Thr Cys
        435                 440                 445

Arg Ser Asn Ile Thr Gly Ile Leu Leu Thr Arg Asp Gly Gly Phe Asn
    450                 455                 460

Asn Thr Asn Asn Asp Thr Glu Glu Thr Phe Arg Pro Gly Gly Gly Asp
465                 470                 475                 480

Met Arg Asp Asn Trp Arg Ser Glu Leu Tyr Lys Tyr Lys Val Val Glu
                485                 490                 495

Ile Lys Pro Leu Gly Ile Ala Pro Thr Lys Ala Lys Arg Arg Val Val
            500                 505                 510

Gln Arg Lys Lys Arg Ala Val Gly Ile Gly Ala Val Phe Leu Gly Phe
        515                 520                 525

Leu Gly Ala Ala Gly Ser Thr Met Gly Ala Ala Ser Ile Thr Leu Thr
    530                 535                 540

Val Gln Ala Arg Gln Leu Leu Ser Gly Ile Val Gln Gln Gln Ser Asn
545                 550                 555                 560

Leu Leu Lys Ala Ile Glu Ala Gln Gln His Met Leu Gln Leu Thr Val

565                 570                 575

Trp Gly Ile Lys Gln Leu Gln Ala Arg Val Leu Ala Ile Glu Arg Tyr
            580                 585                 590

Leu Lys Asp Gln Gln Leu Leu Gly Ile Trp Gly Cys Ser Gly Arg Leu
        595                 600                 605

Ile Cys Thr Thr Ala Val Pro Trp Asn Ser Ser Trp Ser Asn Lys Ser
    610                 615                 620

Glu Ala Asp Ile Trp Asp Asn Met Thr Trp Met Gln Trp Asp Arg Glu
625                 630                 635                 640

Ile Asn Asn Tyr Thr Glu Thr Ile Phe Arg Leu Leu Glu Asp Ser Gln
                645                 650                 655

Asn Gln Gln Glu Lys Asn Glu Lys Asp Leu Leu Glu Leu Asp Lys Trp
            660                 665                 670

Asn Asn Leu Trp Asn Trp Phe Asp Ile Ser Asn Trp Leu Trp Tyr Ile
        675                 680                 685

Lys Ile Phe Ile Met Ile Val Gly Gly Leu Ile Gly Leu Arg Ile Ile
    690                 695                 700

Phe Ala Val Leu Ser Ile Val Asn Arg Val Arg Gln Gly Tyr Ser Pro
705                 710                 715                 720

Leu Ser Phe Gln Thr Leu Thr Pro Ser Pro Arg Gly Leu Asp Arg Leu
                725                 730                 735

Gly Gly Ile Glu Glu Glu Gly Gly Glu Gln Asp Arg Asp Arg Ser Ile
            740                 745                 750

Arg Leu Val Ser Gly Phe Leu Ser Leu Ala Trp Asp Asp Leu Arg Ser
        755                 760                 765

Leu Cys Leu Phe Ser Tyr His Arg Leu Arg Asp Phe Ile Leu Ile Ala
    770                 775                 780

Val Arg Ala Val Glu Leu Leu Gly His Ser Ser Leu Arg Gly Leu Gln
785                 790                 795                 800

Arg Gly Trp Glu Ile Leu Lys Tyr Leu Gly Ser Leu Val Gln Tyr Trp
                805                 810                 815

Gly Leu Glu Leu Lys Lys Ser Ala Ile Ser Pro Leu Asp Thr Ile Ala
            820                 825                 830

Ile Ala Val Ala Glu Gly Thr Asp Arg Ile Ile Glu Leu Val Gln Arg
        835                 840                 845

Ile Cys Arg Ala Ile Leu Asn Ile Pro Arg Arg Ile Arg Gln Gly Phe

850                 855                 860

Glu Ala Ala Leu Leu
865



<210> 5
<211> 854
<212> PRT
<213> Artificial Sequence

<220>
<223> Description of Artificial Sequence: TV2.12-5/1

<400> 5
Met Arg Ala Arg Gly Ile Leu Lys Asn Tyr Arg His Trp Trp Ile Trp
  1               5                  10                  15

Gly Ile Leu Gly Phe Trp Met Leu Met Met Cys Asn Val Lys Gly Leu
             20                  25                  30

Trp Val Thr Val Tyr Tyr Gly Val Pro Val Gly Arg Glu Ala Lys Thr
         35                  40                  45

Thr Leu Phe Cys Ala Ser Asp Ala Lys Ala Tyr Glu Lys Glu Val His
     50                  55                  60

Asn Val Trp Ala Thr His Ala Cys Val Pro Thr Asp Pro Asn Pro Gln
 65                  70                  75                  80

Glu Val Ile Leu Gly Asn Val Thr Glu Asn Phe Asn Met Trp Lys Asn
                 85                  90                  95

Asp Met Val Asp Gln Met Gln Glu Asp Ile Ile Ser Leu Trp Asp Gln
            100                 105                 110

Ser Leu Lys Pro Cys Val Lys Leu Thr Pro Leu Cys Val Thr Leu Asn
        115                 120                 125

Cys Thr Asn Ala Thr Val Asn Tyr Asn Asn Thr Ser Lys Asp Met Lys
    130                 135                 140

Asn Cys Ser Phe Tyr Val Thr Thr Glu Leu Arg Asp Lys Lys Lys Lys
145                 150                 155                 160

Glu Asn Ala Leu Phe Tyr Arg Leu Asp Ile Val Pro Leu Asn Asn Arg
                165                 170                 175

Lys Asn Gly Asn Ile Asn Asn Tyr Arg Leu Ile Asn Cys Asn Thr Ser
            180                 185                 190

Ala Ile Thr Gln Ala Cys Pro Lys Val Ser Phe Asp Pro Ile Pro Ile
        195                 200                 205

His Tyr Cys Ala Pro Ala Gly Tyr Ala Pro Leu Lys Cys Asn Asn Lys
    210                 215                 220

Lys Phe Asn Gly Ile Gly Pro Cys Asp Asn Val Ser Thr Val Gln Cys
225                 230                 235                 240

Thr His Gly Ile Lys Pro Val Val Ser Thr Gln Leu Leu Leu Asn Gly
                245                 250                 255

Ser Leu Ala Glu Glu Glu Ile Ile Ile Arg Ser Glu Asn Leu Thr Asn
            260                 265                 270

Asn Val Lys Thr Ile Ile Val His Leu Asn Glu Ser Ile Glu Ile Lys
        275                 280                 285

Cys Thr Arg Pro Gly Asn Asn Thr Arg Lys Ser Val Arg Ile Gly Pro
    290                 295                 300

Gly Gln Ala Phe Tyr Ala Thr Gly Asp Ile Ile Gly Asp Ile Arg Gln
305                 310                 315                 320

Ala His Cys Asn Ile Ser Lys Asn Glu Trp Asn Thr Thr Leu Gln Arg
                325                 330                 335

Val Ser Gln Lys Leu Gln Glu Leu Phe Pro Asn Ser Thr Gly Ile Lys
            340                 345                 350

Phe Ala Pro His Ser Gly Gly Asp Leu Glu Ile Thr Thr His Ser Phe
        355                 360                 365

Asn Cys Gly Gly Glu Phe Phe Tyr Cys Asn Thr Thr Asp Leu Phe Asn
    370                 375                 380

Ser Thr Tyr Ser Asn Gly Thr Cys Thr Asn Gly Thr Cys Met Ser Asn
385                 390                 395                 400

Asn Thr Glu Arg Ile Thr Leu Gln Cys Arg Ile Lys Gln Ile Ile Asn
                405                 410                 415

Met Trp Gln Glu Val Gly Arg Ala Met Tyr Ala Pro Pro Ile Ala Gly
            420                 425                 430

Asn Ile Thr Cys Arg Ser Asn Ile Thr Gly Leu Leu Leu Thr Arg Asp
        435                 440                 445

Gly Gly Asp Asn Asn Thr Glu Thr Glu Thr Phe Arg Pro Gly Gly Gly
    450                 455                 460

Asp Met Arg Asp Asn Trp Arg Ser Glu Leu Tyr Lys Tyr Lys Val Val
465                 470                 475                 480

Glu Ile Lys Pro Leu Gly Val Ala Pro Thr Ala Ala Lys Arg Arg Val
                485                 490                 495

Val Glu Arg Glu Lys Arg Ala Val Gly Ile Gly Ala Val Phe Leu Gly
            500                 505                 510

Phe Leu Gly Ala Ala Gly Ser Thr Met Gly Ala Ala Ser Ile Thr Leu
        515                 520                 525

Thr Val Gln Ala Arg Gln Leu Leu Ser Gly Ile Val Gln Gln Gln Ser
    530                 535                 540

Asn Leu Leu Arg Ala Ile Glu Ala Gln Gln His Met Leu Gln Leu Thr
545                 550                 555                 560

Val Trp Gly Ile Lys Gln Leu Gln Ala Arg Val Leu Ala Ile Glu Arg
                565                 570                 575

Tyr Leu Gln Asp Gln Gln Leu Leu Gly Leu Trp Gly Cys Ser Gly Lys
            580                 585                 590
Leu Ile Cys Thr Thr Asn Val Leu Trp Asn Ser Ser Trp Ser Asn Lys
        595                 600                 605

Thr Gln Ser Asp Ile Trp Asp Asn Met Thr Trp Met Gln Trp Asp Arg
    610                 615                 620

Glu Ile Ser Asn Tyr Thr Asn Thr Ile Tyr Arg Leu Leu Glu Asp Ser
625                 630                 635                 640

Gln Ser Gln Gln Glu Arg Asn Glu Lys Asp Leu Leu Ala Leu Asp Arg
                645                 650                 655

Trp Asn Asn Leu Trp Asn Trp Phe Ser Ile Thr Asn Trp Leu Trp Tyr
            660                 665                 670

Ile Lys Ile Phe Ile Met Ile Val Gly Gly Leu Ile Gly Leu Arg Ile
        675                 680                 685

Ile Phe Ala Val Leu Ser Leu Val Asn Arg Val Arg Gln Gly Tyr Ser
    690                 695                 700

Pro Leu Ser Leu Gln Thr Leu Ile Pro Asn Pro Arg Gly Pro Asp Arg
705                 710                 715                 720

Leu Gly Gly Ile Glu Glu Glu Gly Gly Glu Gln Asp Ser Ser Arg Ser
                725                 730                 735

Ile Arg Leu Val Ser Gly Phe Leu Thr Leu Ala Trp Asp Asp Leu Arg
            740                 745                 750

Ser Leu Cys Leu Phe Cys Tyr His Arg Leu Arg Asp Phe Ile Leu Ile
        755                 760                 765

Val Val Arg Ala Val Glu Leu Leu Gly His Ser Ser Leu Arg Gly Leu
    770                 775                 780

Gln Arg Gly Trp Gly Thr Leu Lys Tyr Leu Gly Ser Leu Val Gln Tyr

785                 790                 795                 800

Trp Gly Leu Glu Leu Lys Lys Ser Ala Ile Asn Leu Leu Asp Thr Ile
                805                 810                 815

Ala Ile Ala Val Ala Glu Gly Thr Asp Arg Ile Leu Glu Phe Ile Gln
            820                 825                 830

Asn Leu Cys Arg Gly Ile Arg Asn Val Pro Arg Arg Ile Arg Gln Gly
        835                 840                 845

Phe Glu Ala Ala Leu Gln
    850


<210> 6
<211> 860
<212> PRT
<213> Artificial Sequence

<220>
<223> Description of Artificial Sequence: consensus
      sequence

<400> 6
Met Arg Val Met Gly Thr Gln Lys Asn Cys Gln Gln Trp Trp Ile Trp
  1               5                  10                  15

Gly Ile Leu Gly Phe Trp Met Leu Met Ile Cys Asn Val Glu Asp Leu
             20                  25                  30

Trp Val Thr Val Tyr Tyr Gly Val Pro Val Trp Arg Glu Ala Lys Thr
         35                  40                  45

Thr Leu Phe Cys Ala Ser Asp Ala Lys Ala Tyr Glu Thr Glu Val His
     50                  55                  60

Asn Val Trp Ala Thr His Ala Cys Val Pro Thr Asp Pro Asn Pro Gln
 65                  70                  75                  80

Glu Ile Val Leu Gly Asn Val Thr Glu Asn Phe Asn Met Trp Lys Asn
                 85                  90                  95

Asn Met Val Asp Gln Met His Glu Asp Ile Ile Ser Leu Trp Asp Gln
            100                 105                 110

Ser Leu Lys Pro Cys Val Lys Leu Thr Pro Leu Cys Val Thr Leu Asn
        115                 120                 125

Cys Thr Asn Thr Asn Val Thr Gly Asn Arg Thr Val Thr Gly Asn Ser
    130                 135                 140

Asn Ser Asn Ala Ala Tyr Glu Glu Met Lys Asn Cys Ser Phe Asn Val
145                 150                 155                 160

Thr Thr Glu Leu Arg Asp Lys Lys His Lys Glu Tyr Ala Leu Phe Tyr
                165                 170                 175

Lys Leu Asp Ile Val Pro Leu Asn Asn Glu Asn Ser Asn Asn Phe Thr
            180                 185                 190

Tyr Arg Leu Ile Asn Cys Asn Thr Ser Thr Ile Thr Gln Ala Cys Pro
        195                 200                 205

Lys Val Ser Phe Asp Pro Ile Pro Ile His Tyr Cys Ala Pro Ala Gly
    210                 215                 220

Tyr Ala Ile Leu Lys Cys Asn Asn Lys Thr Phe Asn Gly Thr Gly Pro
225                 230                 235                 240

Cys Tyr Asn Val Ser Thr Val Gln Cys Thr His Gly Ile Lys Pro Val
                245                 250                 255

Val Ser Thr Gln Leu Leu Leu Asn Gly Ser Leu Ala Glu Glu Gly Ile
            260                 265                 270

Ile Ile Arg Ser Glu Asn Leu Thr Glu Asn Thr Lys Thr Ile Ile Val
        275                 280                 285

His Leu Asn Glu Ser Val Glu Ile Asn Cys Thr Arg Pro Asn Asn Asn
    290                 295                 300
Thr Arg Lys Ser Val Arg Ile Gly Pro Gly Gln Ala Phe Tyr Ala Thr
305                 310                 315                 320

Asn Asp Ile Ile Gly Asn Ile Arg Gln Ala His Cys Asn Ile Ser Thr
                325                 330                 335

Asp Arg Trp Asn Lys Thr Leu Gln Gln Val Met Lys Lys Leu Gln Glu
            340                 345                 350

His Phe Pro Asn Lys Thr Ile Lys Phe Lys Pro His Ala Gly Gly Asp
        355                 360                 365

Leu Glu Ile Thr Met His Ser Phe Asn Cys Arg Gly Glu Phe Phe Tyr
    370                 375                 380

Cys Asn Thr Ser Asn Leu Phe Asn Ser Thr Tyr His Asn Asn Gly Thr
385                 390                 395                 400

Tyr Lys Tyr Asn Gly Asn Ser Ser Pro Ile Thr Leu Gln Cys Lys Ile
                405                 410                 415

Lys Gln Ile Ile Arg Met Trp Gln Gly Val Gly Gln Ala Met Tyr Ala
            420                 425                 430

Pro Pro Ile Ala Gly Asn Ile Thr Cys Arg Ser Asn Ile Thr Gly Ile
        435                 440                 445

Leu Leu Thr Arg Asp Gly Gly Phe Asn Asn Thr Asn Thr Thr Glu Thr
    450                 455                 460

Phe Arg Pro Gly Gly Gly Asp Met Arg Asp Asn Trp Arg Ser Glu Leu
465                 470                 475                 480

Tyr Lys Tyr Lys Val Val Glu Ile Lys Pro Leu Gly Ile Ala Pro Thr
                485                 490                 495

Lys Ala Lys Arg Arg Val Val Gln Arg Glu Lys Arg Ala Val Gly Ile
            500                 505                 510

Gly Ala Val Phe Leu Gly Phe Leu Gly Ala Ala Gly Ser Thr Met Gly
        515                 520                 525

Ala Ala Ser Ile Thr Leu Thr Val Gln Ala Arg Gln Leu Leu Ser Gly
    530                 535                 540

Ile Val Gln Gln Gln Ser Asn Leu Leu Lys Ala Ile Glu Ala Gln Gln
545                 550                 555                 560

His Met Leu Gln Leu Thr Val Trp Gly Ile Lys Gln Leu Gln Ala Arg
                565                 570                 575

Val Leu Ala Ile Glu Arg Tyr Leu Lys Asp Gln Gln Leu Leu Gly Ile
            580                 585                 590

Trp Gly Cys Ser Gly Lys Leu Ile Cys Thr Thr Ala Val Pro Trp Asn
        595                 600                 605

Ser Ser Trp Ser Asn Lys Ser Glu Ala Asp Ile Trp Asp Asn Met Thr
    610                 615                 620
Trp Met Gln Trp Asp Arg Glu Ile Ser Asn Tyr Thr Asn Thr Ile Tyr
625                 630                 635                 640

Arg Leu Leu Glu Asp Ser Gln Asn Gln Gln Glu Lys Asn Glu Lys Asp
                645                 650                 655

Leu Leu Glu Leu Asp Lys Trp Asn Asn Leu Trp Asn Trp Phe Asp Ile
            660                 665                 670

Ser Asn Trp Leu Trp Tyr Ile Lys Ile Phe Ile Met Ile Val Gly Gly
        675                 680                 685

Leu Ile Gly Leu Arg Ile Ile Phe Ala Val Leu Ser Ile Val Asn Arg
    690                 695                 700

Val Arg Gln Gly Tyr Ser Pro Leu Ser Phe Gln Thr Leu Thr Pro Ser
705                 710                 715                 720

Pro Arg Gly Pro Asp Arg Leu Gly Gly Ile Glu Glu Glu Gly Gly Glu
                725                 730                 735

Gln Asp Arg Asp Arg Ser Ile Arg Leu Val Ser Gly Phe Leu Ser Leu

740                 745                 750

Ala Trp Asp Asp Leu Arg Ser Leu Cys Leu Phe Ser Tyr His Arg Leu
        755                 760                 765

Arg Asp Phe Ile Leu Ile Ala Val Arg Ala Val Glu Leu Leu Gly His
    770                 775                 780

Ser Ser Leu Arg Gly Leu Gln Arg Gly Trp Glu Ile Leu Lys Tyr Leu
785                 790                 795                 800

Gly Ser Leu Val Gln Tyr Trp Gly Leu Glu Leu Lys Lys Ser Ala Ile
                805                 810                 815

Ser Leu Leu Asp Thr Ile Ala Ile Ala Val Ala Glu Gly Thr Asp Arg
            820                 825                 830

Ile Ile Glu Leu Val Gln Arg Ile Cys Arg Ala Ile Leu Asn Ile Pro
        835                 840                 845

Arg Arg Ile Arg Gln Gly Phe Glu Ala Ala Leu Leu
    850                 855                 860

<210> 7
<211> 4
<212> PRT
<213> Artificial Sequence

<220>
<223> Description of Artificial Sequence: catalytic center

<400> 7
Tyr Met Asp Asp
  1

<210> 8
<211> 4
<212> PRT
<213> Artificial Sequence

<220>
<223> Description of Artificial Sequence: primer grip region

<400> 8
Trp Met Gly Tyr
  1

<210> 9
<211> 5274
<212> DNA
<213> Artificial Sequence

<220>
<223> Description of Artificial Sequence:
      gagCpolInaTatRevNef.opt_B

<400> 9
gtcgacgcca ccatgggcgc ccgcgccagc gtgctgagcg gcggcgagct ggacaagtgg 60
gagaagatcc gcctgcgccc cggcggcaag aagaagtaca agctgaagca catcgtgtgg 120
gccagccgcg agctggagcg cttcgccgtg aaccccggcc tgctggagac cagcgagggc 180
tgccgccaga tcctgggcca gctgcagccc agcctgcaga ccggcagcga ggagctgcgc 240
agcctgtaca acaccgtggc caccctgtac tgcgtgcacc agcgcatcga cgtcaaggac 300
accaaggagg ccctggagaa gatcgaggag gagcagaaca agtccaagaa gaaggcccag 360
caggccgccg ccgccgccgg caccggcaac agcagccagg tgagccagaa ctaccccatc 420
gtgcagaacc tgcagggcca gatggtgcac caggccatca gcccccgcac cctgaacgcc 480
tgggtgaagg tggtggagga gaaggccttc agccccgagg tgatccccat gttcagcgcc 540
ctgagcgagg gcgccacccc ccaggacctg aacacgatgt tgaacaccgt gggcggccac 600
caggccgcca tgcagatgct gaaggagacc atcaacgagg aggccgccga gtgggaccgc 660
gtgcaccccg tgcacgccgg ccccatcgcc cccggccaga tgcgcgagcc ccgcggcagc 720
gacatcgccg gcaccaccag caccctgcag gagcagatcg gctggatgac caacaacccc 780
cccatccccg tgggcgagat ctacaagcgg tggatcatcc tgggcctgaa caagatcgtg 840
cggatgtaca gccccaccag catcctggac atccgccagg gccccaagga gcccttccgc 900
gactacgtgg accgcttcta caagaccctg cgcgctgagc aggccagcca ggacgtgaag 960
aactggatga ccgagaccct gctggtgcag aacgccaacc ccgactgcaa gaccatcctg 1020
aaggctctcg gccccgcggc caccctggag gagatgatga ccgcctgcca gggcgtgggc 1080
ggccccggcc acaaggcccg cgtgctggcc gaggcgatga gccaggtgac gaacccggcg 1140
accatcatga tgcagcgcgg caacttccgc aaccagcgga agaccgtcaa gtgcttcaac 1200
tgcggcaagg agggccacac cgccaggaac tgccgcgccc cccgcaagaa gggctgctgg 1260
cgctgcggcc gcgagggcca ccagatgaag gactgcaccg agcgccaggc caacttcctg 1320
ggcaagatct ggcccagcta caagggccgc cccggcaact tcctgcagag ccgccccgag 1380
cccaccgccc cccccgagga gagcttccgc ttcggcgagg agaagaccac ccccagccag 1440
aagcaggagc ccatcgacaa ggagctgtac cccctgacca gcctgcgcag cctgttcggc 1500
aacgacccca gcagccagaa agaattcaag gcccgcgtgc tggccgaggc gatgagccag 1560
gtgacgaacc cggcgaccat catgatgcag cgcggcaact tccgcaacca gcggaagacc 1620
gtcaagtgct tcaactgcgg caaggagggc cacaccgcca ggaactgccg cgccccccgc 1680
aagaagggct gctggcgctg cggccgcgaa ggacaccaaa tgaaagattg cactgagaga 1740
caggctaatt tcttccgcga ggacctggcc ttcctgcagg gcaaggcccg cgagttcagc 1800
agcgagcaga cccgcgccaa cagccccacc cgccgcgagc tgcaggtgtg gggcggcgag 1860
aacaacagcc tgagcgaggc cggcgccgac cgccagggca ccgtgagctt caacttcccc 1920
cagatcaccc tgtggcagcg ccccctggtg accatcagga tcggcggcca gctcaaggag 1980
gcgctgctcg ccaccggcgc cgacgacacc gtgctggagg agatgaacct gcccggcaag 2040
tggaagccca agatgatcgg cgggatcggg ggcttcatca aggtgcggca gtacgaccag 2100
atccccgtgg agatctgcgg ccacaaggcc atcggcaccg tgctggtggg ccccaccccc 2160
gtgaacatca tcggccgcaa cctgctgacc cagatcggct gcaccctgaa cttccccatc 2220
agccccatcg agacggtgcc cgtgaagctg aagccgggga tggacggccc caaggtcaag 2280
cagtggcccc tgaccgagga gaagatcaag gccctggtgg agatctgcac cgagatggag 2340
aaggagggca agatcagcaa gatcggcccc gagaacccct acaacacccc cgtgttcgcc 2400
atcaagaaga aggacagcac caagtggcgc aagctggtgg acttccgcga gctgaacaag 2460
cgcacccagg acttctggga ggtgcagctg ggcatccccc accccgccgg cctgaagaag 2520
aagaagagcg tgaccgtgct ggacgtgggc gacgcctact tcagcgtgcc cctggacaag 2580
gacttccgca agtacaccgc cttcaccatc cccagcatca acaacgagac ccccggcatc 2640
cgctaccagt acaacgtgct gccccagggc tggaagggca gccccgccat cttccagagc 2700
agcatgacca agatcctgga gcccttccgc aagcagaacc ccgacatcgt gatctaccag 2760
gcccccctgt acgtgggcag cgacctggag atcggccagc accgcaccaa gatcgaggag 2820
ctgcgccagc acctgctgcg ctggggcttc accacccccg acaagaagca ccagaaggag 2880

ccccccttcc tgcccatcga gctgcacccc gacaagtgga ccgtgcagcc catcatgctg 2940
cccgagaagg acagctggac cgtgaacgac atccagaagc tggtgggcaa gctgaactgg 3000
gccagccaga tctacgccgg catcaaggtg aagcagctgt gcaagctgct gcgcggcacc 3060
aaggccctga ccgaggtgat ccccctgacc gaggaggccg agctggagct ggccgagaac 3120
cgcgagatcc tgaaggagcc cgtgcacgag gtgtactacg accccagcaa ggacctggtg 3180
gccgagatcc agaagcaggg ccagggccag tggacctacc agatctacca ggagcccttc 3240
aagaacctga agaccggcaa gtacgcccgc atgcgcggcg cccacaccaa cgacgtgaag 3300
cagctgaccg aggccgtgca gaaggtgagc accgagagca tcgtgatctg gggcaagatc 3360
cccaagttca agctgcccat ccagaaggag acctgggagg cctggtggat ggagtactgg 3420
caggccacct ggatccccga gtgggagttc gtgaacaccc cccccctggt gaagctgtgg 3480
taccagctgg agaaggagcc catcgtgggc gccgagacct tctacgtgga cggcgccgcc 3540
aaccgcgaga ccaagctggg caaggccggc tacgtgaccg accggggccg gcagaaggtg 3600
gtgagcatcg ccgacaccac caaccagaag accgagctgc aggccatcca cctggccctg 3660
caggacagcg gcctggaggt gaacatcgtg accgacagcc agtacgccct gggcatcatc 3720
caggcccagc ccgacaagag cgagagcgag ctggtgagcc agatcatcga gcagctgatc 3780
aagaaggaga aggtgtacct ggcctgggtg cccgcccaca agggcatcgg cggcaacgag 3840
caggtggaca agctggtgag cgccggcatc cgcaaggtgc tgttcctgaa cggcatcgat 3900
ggcggcatcg tgatctacca gtacatggac gacctgtacg tgggcagcgg cggccctagg 3960
gagcccgtgg acccccgcct ggagccctgg aagcaccccg gcagccagcc caagaccgcc 4020
ggcaccaact gctactgcaa gaagtgctgc ttccactgcc aggtgagctt catcaccaag 4080
ggcctgggca tcagctacgg ccgcaagaag cgccgccagc gccgccgcgc cccccccgac 4140
agcgaggtgc accaggtgag cctgcccaag cagcccgcca gccagcccca gggcgacccc 4200
accggcccca aggagagcaa gaagaaggtg gagcgcgaga ccgagaccga ccccgtgcac 4260
cccggggccg gccgcagcgg cgacagcgac gaggagctgc tgcagaccgt gcgcttcatc 4320
aagttcctgt accagagcaa ccccctgccc agccccaagg gcacccgcca ggccgacctg 4380
aaccgccgcc gccgctggcg cgagcgccag cgccagatcc agagcatcag cgcctggatc 4440
atcagcaccc acctgggccg cagcaccgag cccgtgcccc tgcagctgcc ccccgacctg 4500
cgcctgaacc tggactgcag cgaggactgc ggcaccagcg gcacccaggg cgtgggcagc 4560
ccccaggtgc tgggcgagag ccccgccgtg ctggacagcg gcaccaagga gctcgaggcc 4620
ggcaagtgga gcaagcgcat gagcggctgg agcgccgtgc gcgagcgcat gaagcgcgcc 4680
gagcccgccg agcccgccgc cgacggcgtg ggcgccgtga gccgcgacct ggagaagcac 4740
ggcgccatca ccagcagcaa caccgccgcc aacaacgccg actgcgcctg gctggaggcc 4800
caggaggacg aggacgtggg cttccccgtg cgcccccagg tgcccctgcg ccccatgacc 4860
tacaaggccg ccctggacct gagccacttc ctgaaggaga agggcggcct ggagggcctg 4920
atctacagcc agaagcgcca ggacatcctg gacctgtgga tccaccacac ccagggctac 4980
ttccccggct ggcagaacta cacccccggc cccggcatcc gctaccccct gaccttcggc 5040
tggtgcttca agctggtgcc cgtggacccc gactacgtgg aggaggccaa cgccggcgag 5100
aacaacagcc tgctgcaccc catgagccag cacggcatgg acgaccccga gaaggaggtg 5160
ctggtgtggc gcttcgacag ccgcctggcc ttccaccaca tggcccgcga gctgcacccc 5220
gagtactaca aggactgcga ttaaaagctt cccggggcta gcaccggttc taga 5274

<210> 10
<211> 4773
<212> DNA
<213> Artificial Sequence

<220>
<223> Description of Artificial Sequence:
      GagProtInaRTmutTatRevNef.opt_B

<400> 10
gccaccatgg gcgcccgcgc cagcgtgctg agcggcggcg agctggacaa gtgggagaag 60
atccgcctgc gccccggcgg caagaagaag tacaagctga agcacatcgt gtgggccagc 120
cgcgagctgg agcgcttcgc cgtgaacccc ggcctgctgg agaccagcga gggctgccgc 180

cagatcctgg gccagctgca gcccagcctg cagaccggca gcgaggagct gcgcagcctg 240
tacaacaccg tggccaccct gtactgcgtg caccagcgca tcgacgtcaa ggacaccaag 300
gaggccctgg agaagatcga ggaggagcag aacaagtcca agaagaaggc ccagcaggcc 360
gccgccgccg ccggcaccgg caacagcagc caggtgagcc agaactaccc catcgtgcag 420
aacctgcagg gccagatggt gcaccaggcc atcagccccc gcaccctgaa cgcctgggtg 480
aaggtggtgg aggagaaggc cttcagcccc gaggtgatcc ccatgttcag cgccctgagc 540
gagggcgcca ccccccagga cctgaacacg atgttgaaca ccgtgggcgg ccaccaggcc 600
gccatgcaga tgctgaagga gaccatcaac gaggaggccg ccgagtggga ccgcgtgcac 660
cccgtgcacg ccggccccat cgcccccggc cagatgcgcg agccccgcgg cagcgacatc 720
gccggcacca ccagcaccct gcaggagcag atcggctgga tgaccaacaa cccccccatc 780
cccgtgggcg agatctacaa gcggtggatc atcctgggcc tgaacaagat cgtgcggatg 840
tacagcccca ccagcatcct ggacatccgc cagggcccca aggagccctt ccgcgactac 900
gtggaccgct tctacaagac cctgcgcgct gagcaggcca gccaggacgt gaagaactgg 960
atgaccgaga ccctgctggt gcagaacgcc aaccccgact gcaagaccat cctgaaggct 1020
ctcggccccg cggccaccct ggaggagatg atgaccgcct gccagggcgt gggcggcccc 1080
ggccacaagg cccgcgtgct ggccgaggcg atgagccagg tgacgaaccc ggcgaccatc 1140
atgatgcagc gcggcaactt ccgcaaccag cggaagaccg tcaagtgctt caactgcggc 1200
aaggagggcc acaccgccag gaactgccgc gccccccgca agaagggctg ctggcgctgc 1260
ggccgcgagg gccaccagat gaaggactgc accgagcgcc aggccaactt cctgggcaag 1320
atctggccca gctacaaggg ccgccccggc aacttcctgc agagccgccc cgagcccacc 1380
gccccccccg aggagagctt ccgcttcggc gaggagaaga ccacccccag ccagaagcag 1440
gagcccatcg acaaggagct gtaccccctg accagcctgc gcagcctgtt cggcaacgac 1500
cccagcagcc agaaagaatt cccccagatc accctgtggc agcgccccct ggtgaccatc 1560
aggatcggcg gccagctcaa ggaggcgctg ctcgccaccg gcgccgacga caccgtgctg 1620
gaggagatga acctgcccgg caagtggaag cccaagatga tcggcgggat cgggggcttc 1680
atcaaggtgc ggcagtacga ccagatcccc gtggagatct gcggccacaa ggccatcggc 1740
accgtgctgg tgggccccac ccccgtgaac atcatcggcc gcaacctgct gacccagatc 1800
ggctgcaccc tgaacttccc catcagcccc atcgagacgg tgcccgtgaa gctgaagccg 1860
gggatggacg gccccaaggt caagcagtgg cccctgaccg aggagaagat caaggccctg 1920
gtggagatct gcaccgagat ggagaaggag ggcaagatca gcaagatcgg ccccgagaac 1980
ccctacaaca cccccgtgtt cgccatcaag aagaaggaca gcaccaagtg gcgcaagctg 2040
gtggacttcc gcgagctgaa caagcgcacc caggacttct gggaggtgca gctgggcatc 2100
ccccaccccg ccggcctgaa gaagaagaag agcgtgaccg tgctggacgt gggcgacgcc 2160
tacttcagcg tgcccctgga caaggacttc cgcaagtaca ccgccttcac catccccagc 2220
atcaacaacg agacccccgg catccgctac cagtacaacg tgctgcccca gggctggaag 2280
ggcagccccg ccatcttcca gagcagcatg accaagatcc tggagccctt ccgcaagcag 2340
aaccccgaca tcgtgatcta ccaggccccc ctgtacgtgg gcagcgacct ggagatcggc 2400
cagcaccgca ccaagatcga ggagctgcgc cagcacctgc tgcgctgggg cttcaccacc 2460
cccgacaaga agcaccagaa ggagcccccc ttcctgccca tcgagctgca ccccgacaag 2520
tggaccgtgc agcccatcat gctgcccgag aaggacagct ggaccgtgaa cgacatccag 2580
aagctggtgg gcaagctgaa ctgggccagc cagatctacg ccggcatcaa ggtgaagcag 2640
ctgtgcaagc tgctgcgcgg caccaaggcc ctgaccgagg tgatccccct gaccgaggag 2700
gccgagctgg agctggccga gaaccgcgag atcctgaagg agcccgtgca cgaggtgtac 2760
tacgacccca gcaaggacct ggtggccgag atccagaagc agggccaggg ccagtggacc 2820
taccagatct accaggagcc cttcaagaac ctgaagaccg gcaagtacgc ccgcatgcgc 2880
ggcgcccaca ccaacgacgt gaagcagctg accgaggccg tgcagaaggt gagcaccgag 2940
agcatcgtga tctggggcaa gatccccaag ttcaagctgc ccatccagaa ggagacctgg 3000
gaggcctggt ggatggagta ctggcaggcc acctggatcc ccgagtggga gttcgtgaac 3060
accccccccc tggtgaagct gtggtaccag ctggagaagg agcccatcgt gggcgccgag 3120
accttctacg tggacggcgc cgccaaccgc gagaccaagc tgggcaaggc cggctacgtg 3180
accgaccggg gccggcagaa ggtggtgagc atcgccgaca ccaccaacca gaagaccgag 3240
ctgcaggcca tccacctggc cctgcaggac agcggcctgg aggtgaacat cgtgaccgac 3300
agccagtacg ccctgggcat catccaggcc cagcccgaca agagcgagag cgagctggtg 3360
agccagatca tcgagcagct gatcaagaag gagaaggtgt acctggcctg ggtgcccgcc 3420

cacaagggca tcggcggcaa cgagcaggtg gacaagctgg tgagcgccgg catccgcaag 3480
gtgctcaagc ttgagcccgt ggacccccgc ctggagccct ggaagcaccc cggcagccag 3540
cccaagaccg ccggcaccaa ctgctactgc aagaagtgct gcttccactg ccaggtgagc 3600
ttcatcacca agggcctggg catcagctac ggccgcaaga agcgccgcca gcgccgccgc 3660
gccccccccg acagcgaggt gcaccaggtg agcctgccca agcagcccgc cagccagccc 3720
cagggcgacc ccaccggccc caaggagagc aagaagaagg tggagcgcga gaccgagacc 3780
gaccccgtgc accccggggc cggccgcagc ggcgacagcg acgaggagct gctgcagacc 3840
gtgcgcttca tcaagttcct gtaccagagc aaccccctgc ccagccccaa gggcacccgc 3900
caggccgacc tgaaccgccg ccgccgctgg cgcgagcgcc agcgccagat ccagagcatc 3960
agcgcctgga tcatcagcac ccacctgggc cgcagcaccg agcccgtgcc cctgcagctg 4020
ccccccgacc tgcgcctgaa cctggactgc agcgaggact gcggcaccag cggcacccag 4080
ggcgtgggca gcccccaggt gctgggcgag agccccgccg tgctggacag cggcaccaag 4140
gagctcgagg ccggcaagtg gagcaagcgc atgagcggct ggagcgccgt gcgcgagcgc 4200
atgaagcgcg ccgagcccgc cgagcccgcc gccgacggcg tgggcgccgt gagccgcgac 4260
ctggagaagc acggcgccat caccagcagc aacaccgccg ccaacaacgc cgactgcgcc 4320
tggctggagg cccaggagga cgaggacgtg ggcttccccg tgcgccccca ggtgcccctg 4380
cgccccatga cctacaaggc cgccctggac ctgagccact tcctgaagga gaagggcggc 4440
ctggagggcc tgatctacag ccagaagcgc caggacatcc tggacctgtg gatccaccac 4500
acccagggct acttccccgg ctggcagaac tacacccccg gccccggcat ccgctacccc 4560
ctgaccttcg gctggtgctt caagctggtg cccgtggacc ccgactacgt ggaggaggcc 4620
aacgccggcg agaacaacag cctgctgcac cccatgagcc agcacggcat ggacgacccc 4680
gagaaggagg tgctggtgtg gcgcttcgac agccgcctgg ccttccacca catggcccgc 4740
gagctgcacc ccgagtacta caaggactgc taa 4773

<210> 11
<211> 2799
<212> DNA
<213> Artificial Sequence

<220>
<223> Description of Artificial Sequence:
      GagTatRevNef.opt_B

<400> 11
gccaccatgg gcgcccgcgc cagcgtgctg agcggcggcg agctggacaa gtgggagaag 60
atccgcctgc gccccggcgg caagaagaag tacaagctga agcacatcgt gtgggccagc 120
cgcgagctgg agcgcttcgc cgtgaacccc ggcctgctgg agaccagcga gggctgccgc 180
cagatcctgg gccagctgca gcccagcctg cagaccggca gcgaggagct gcgcagcctg 240
tacaacaccg tggccaccct gtactgcgtg caccagcgca tcgacgtcaa ggacaccaag 300
gaggccctgg agaagatcga ggaggagcag aacaagtcca agaagaaggc ccagcaggcc 360
gccgccgccg ccggcaccgg caacagcagc caggtgagcc agaactaccc catcgtgcag 420
aacctgcagg gccagatggt gcaccaggcc atcagccccc gcaccctgaa cgcctgggtg 480
aaggtggtgg aggagaaggc cttcagcccc gaggtgatcc ccatgttcag cgccctgagc 540
gagggcgcca ccccccagga cctgaacacg atgttgaaca ccgtgggcgg ccaccaggcc 600
gccatgcaga tgctgaagga gaccatcaac gaggaggccg ccgagtggga ccgcgtgcac 660
cccgtgcacg ccggccccat cgcccccggc cagatgcgcg agccccgcgg cagcgacatc 720
gccggcacca ccagcaccct gcaggagcag atcggctgga tgaccaacaa cccccccatc 780
cccgtgggcg agatctacaa gcggtggatc atcctgggcc tgaacaagat cgtgcggatg 840
tacagcccca ccagcatcct ggacatccgc cagggcccca aggagccctt ccgcgactac 900
gtggaccgct tctacaagac cctgcgcgct gagcaggcca gccaggacgt gaagaactgg 960
atgaccgaga ccctgctggt gcagaacgcc aaccccgact gcaagaccat cctgaaggct 1020
ctcggccccg cggccaccct ggaggagatg atgaccgcct gccagggcgt gggcggcccc 1080
ggccacaagg cccgcgtgct ggccgaggcg atgagccagg tgacgaaccc ggcgaccatc 1140
atgatgcagc gcggcaactt ccgcaaccag cggaagaccg tcaagtgctt caactgcggc 1200

aaggagggcc acaccgccag gaactgccgc gccccccgca agaagggctg ctggcgctgc 1260
ggccgcgagg gccaccagat gaaggactgc accgagcgcc aggccaactt cctgggcaag 1320
atctggccca gctacaaggg ccgccccggc aacttcctgc agagccgccc cgagcccacc 1380
gccccccccg aggagagctt ccgcttcggc gaggagaaga ccacccccag ccagaagcag 1440
gagcccatcg acaaggagct gtaccccctg accagcctgc gcagcctgtt cggcaacgac 1500
cccagcagcc aggaattcga gcccgtggac ccccgcctgg agccctggaa gcaccccggc 1560
agccagccca agaccgccgg caccaactgc tactgcaaga agtgctgctt ccactgccag 1620
gtgagcttca tcaccaaggg cctgggcatc agctacggcc gcaagaagcg ccgccagcgc 1680
cgccgcgccc cccccgacag cgaggtgcac caggtgagcc tgcccaagca gcccgccagc 1740
cagccccagg gcgaccccac cggccccaag gagagcaaga agaaggtgga gcgcgagacc 1800
gagaccgacc ccgtgcaccc cggggccggc cgcagcggcg acagcgacga ggagctgctg 1860
cagaccgtgc gcttcatcaa gttcctgtac cagagcaacc ccctgcccag ccccaagggc 1920
acccgccagg ccgacctgaa ccgccgccgc cgctggcgcg agcgccagcg ccagatccag 1980
agcatcagcg cctggatcat cagcacccac ctgggccgca gcaccgagcc cgtgcccctg 2040
cagctgcccc ccgacctgcg cctgaacctg gactgcagcg aggactgcgg caccagcggc 2100
acccagggcg tgggcagccc ccaggtgctg ggcgagagcc ccgccgtgct ggacagcggc 2160
accaaggagc tcgaggccgg caagtggagc aagcgcatga gcggctggag cgccgtgcgc 2220
gagcgcatga agcgcgccga gcccgccgag cccgccgccg acggcgtggg cgccgtgagc 2280
cgcgacctgg agaagcacgg cgccatcacc agcagcaaca ccgccgccaa caacgccgac 2340
tgcgcctggc tggaggccca ggaggacgag gacgtgggct tccccgtgcg cccccaggtg 2400
cccctgcgcc ccatgaccta caaggccgcc ctggacctga gccacttcct gaaggagaag 2460
ggcggcctgg agggcctgat ctacagccag aagcgccagg acatcctgga cctgtggatc 2520
caccacaccc agggctactt ccccggctgg cagaactaca cccccggccc cggcatccgc 2580
taccccctga ccttcggctg gtgcttcaag ctggtgcccg tggaccccga ctacgtggag 2640
gaggccaacg ccggcgagaa caacagcctg ctgcacccca tgagccagca cggcatggac 2700
gaccccgaga aggaggtgct ggtgtggcgc ttcgacagcc gcctggcctt ccaccacatg 2760
gcccgcgagc tgcaccccga gtactacaag gactgctaa 2799

<210> 12
<211> 5145
<212> DNA
<213> Artificial Sequence

<220>
<223> Description of Artificial Sequence:
      GagComplPolmutInaTatRevNef_C

<400> 12
gccaccatgg gcgcccgcgc cagcatcctg cgcggcggca agctggacgc ctgggagcgc 60
atccgcctgc gccccggcgg caagaagtgc tacatgatga agcacctggt gtgggccagc 120
cgcgagctgg agaagttcgc cctgaacccc ggcctgctgg agaccagcga gggctgcaag 180
cagatcatcc gccagctgca ccccgccctg cagaccggca gcgaggagct gaagagcctg 240
ttcaacaccg tggccaccct gtactgcgtg cacgagaaga tcgaggtccg cgacaccaag 300
gaggccctgg acaagatcga ggaggagcag aacaagtgcc agcagaagat ccagcaggcc 360
gaggccgccg acaagggcaa ggtgagccag aactacccca tcgtgcagaa cctgcagggc 420
cagatggtgc accaggccat cagcccccgc accctgaacg cctgggtgaa ggtgatcgag 480
gagaaggcct tcagccccga ggtgatcccc atgttcaccg ccctgagcga gggcgccacc 540
ccccaggacc tgaacacgat gttgaacacc gtgggcggcc accaggccgc catgcagatg 600
ctgaaggaca ccatcaacga ggaggccgcc gagtgggacc gcgtgcaccc cgtgcacgcc 660
ggccccatcg cccccggcca gatgcgcgag ccccgcggca gcgacatcgc cggcaccacc 720
agcaccctgc aggagcagat cgcctggatg accagcaacc cccccatccc cgtgggcgac 780
atctacaagc ggtggatcat cctgggcctg aacaagatcg tgcggatgta cagccccgtg 840
agcatcctgg acatcaagca gggccccaag gagcccttcc gcgactacgt ggaccgcttc 900
ttcaagaccc tgcgcgccga gcagagcacc caggaggtga agaactggat gaccgacacc 960

ctgctggtgc agaacgccaa ccccgactgc aagaccatcc tgcgcgctct cggccccggc 1020
gccagcctgg aggagatgat gaccgcctgc cagggcgtgg gcggccccag ccacaaggcc 1080
cgcgtgctgg ccgaggcgat gagccaggcc aacaccagcg tgatgatgca gaagagcaac 1140
ttcaagggcc cccggcgcat cgtcaagtgc ttcaactgcg gcaaggaggg ccacatcgcc 1200
cgcaactgcc gcgccccccg caagaagggc tgctggaagt gcggcaagga gggccaccag 1260
atgaaggact gcaccgagcg ccaggccaac ttcctgggca agatctggcc cagccacaag 1320
ggccgccccg gcaacttcct gcagagccgc cccgagccca ccgccccccc cgccgagagc 1380
ttccgcttcg aggagaccac ccccggccag aagcaggaga gcaaggaccg cgagaccctg 1440
accagcctga agagcctgtt cggcaacgac cccctgagcc aagaattcgc cgaggccatg 1500
agccaggcca ccagcgccaa catcctgatg cagcgcagca acttcaaggg ccccaagcgc 1560
atcatcaagt gcttcaactg cggcaaggag ggccacatcg cccgcaactg ccgcgccccc 1620
cgcaagaagg gctgctggaa gtgcggcaag gagggccacc agatgaagga ctgcaccgag 1680
cgccaggcca acttcttccg cgaggacctg gccttccccc agggcaaggc ccgcgagttc 1740
cccagcgagc agaaccgcgc caacagcccc accagccgcg agctgcaggt gcgcggcgac 1800
aacccccgca gcgaggccgg cgccgagcgc cagggcaccc tgaacttccc ccagatcacc 1860
ctgtggcagc gccccctggt gagcatcaag gtgggcggcc agatcaagga ggccctgctg 1920
gccaccggcg ccgacgacac cgtgctggag gagatgagcc tgcccggcaa gtggaagccc 1980
aagatgatcg gcggcatcgg cggcttcatc aaggtgcgcc agtacgacca gatcctgatc 2040
gagatctgcg gcaagaaggc catcggcacc gtgctgatcg gccccacccc cgtgaacatc 2100
atcggccgca acatgctgac ccagctgggc tgcaccctga acttccccat cagccccatc 2160
gagaccgtgc ccgtgaagct gaagcccggc atggacggcc ccaaggtgaa gcagtggccc 2220
ctgaccgagg agaagatcaa ggccctgacc gccatctgcg aggagatgga gaaggagggc 2280
aagatcacca agatcggccc cgagaacccc tacaacaccc ccgtgttcgc catcaagaag 2340
aaggacagca ccaagtggcg caagctggtg gacttccgcg agctgaacaa gcgcacccag 2400
gacttctggg aggtgcagct gggcatcccc caccccgccg gcctgaagaa gaagaagagc 2460
gtgaccgtgc tggacgtggg cgacgcctac ttcagcgtgc ccctggacga ggacttccgc 2520
aagtacaccg ccttcaccat ccccagcatc aacaacgaga cccccggcat ccgctaccag 2580
tacaacgtgc tgccccaggg ctggaagggc agccccagca tcttccagag cagcatgacc 2640
aagatcctgg agcccttccg cgcccgcaac cccgagatcg tgatctacca ggcccccctg 2700
tacgtgggca gcgacctgga gatcggccag caccgcgcca agatcgagga gctgcgcaag 2760
cacctgctgc gctggggctt caccaccccc gacaagaagc accagaagga gccccccttc 2820
ctgcccatcg agctgcaccc cgacaagtgg accgtgcagc ccatcgagct gcccgagaag 2880
gagagctgga ccgtgaacga catccagaag ctggtgggca agctgaactg ggccagccag 2940
atctaccccg gcatcaaggt gcgccagctg tgcaagctgc tgcgcggcgc caaggccctg 3000
accgacatcg tgcccctgac cgaggaggcc gagctggagc tggccgagaa ccgcgagatc 3060
ctgcgcgagc ccgtgcacgg cgtgtactac gaccccagca aggacctggt ggccgagatc 3120
cagaagcagg gccacgacca gtggacctac cagatctacc aggagccctt caagaacctg 3180
aagaccggca agtacgccaa gatgcgcacc gcccacacca acgacgtgaa gcagctgacc 3240
gaggccgtgc agaagatcgc catggagagc atcgtgatct ggggcaagac ccccaagttc 3300
cgcctgccca tccagaagga gacctgggag acctggtgga ccgactactg gcaggccacc 3360
tggatccccg agtgggagtt cgtgaacacc ccccccctgg tgaagctgtg gtaccagctg 3420
gagaaggagc ccatcatcgg cgccgagacc ttctacgtgg acggcgccgc caaccgcgag 3480
accaagatcg gcaaggccgg ctacgtgacc gaccggggcc ggcagaagat cgtgagcctg 3540
accgagacca ccaaccagaa gaccgagctg caggccatcc agctggccct gcaggacagc 3600
ggcagcgagg tgaacatcgt gaccgacagc cagtacgccc tgggcatcat ccaggcccag 3660
cccgacaaga gcgagagcga gctggtgaac cagatcatcg agcagctgat caagaaggag 3720
aaggtgtacc tgagctgggt gcccgcccac aagggcatcg gcggcaacga gcagatcgac 3780
aagctggtga gcaagggcat ccgcaaggtg ctgttcctgg acggcatcga tggcggcatc 3840
gtgatctacc agtacatgga cgacctgtac gtgggcagcg gcggccctag ggagcccgtg 3900
gaccccaacc tggagccctg gaaccacccc ggcagccagc ccaagaccgc cggcaacaag 3960
tgctactgca agcactgcag ctaccactgc ctggtgagct tccagaccaa gggcctgggc 4020
atcagctacg gccgcaagaa gcgccgccag cgccgcagcg ccccccccag cagcgaggac 4080
caccagaacc ccatcagcaa gcagcccctg ccccagaccc gcggcgaccc caccggcagc 4140
gaggagagca agaagaaggt ggagagcaag accgagaccg accccttcga ccccggggcc 4200

ggccgcagcg gcgacagcga cgaggccctg ctgcaggccg tgcgcatcat caagatcctg 4260
taccagagca acccctaccc caagcccgag ggcacccgcc aggccgacct gaaccgccgc 4320
cgccgctggc gcgcccgcca gcgccagatc cacagcatca gcgagcgcat cctgagcacc 4380
tgcctgggcc gccccgccga gcccgtgccc ttccagctgc cccccgacct gcgcctgcac 4440
atcgactgca gcgagagcag cggcaccagc ggcacccagc agagccaggg caccaccgag 4500
ggcgtgggca gccccctcga ggccggcaag tggagcaaga gcagcatcgt gggctggccc 4560
gccgtgcgcg agcgcatccg ccgcaccgag cccgccgccg agggcgtggg cgccgccagc 4620
caggacctgg acaagcacgg cgccctgacc agcagcaaca ccgccgccaa caacgccgac 4680
tgcgcctggc tggaggccca ggaggaggag gaggaggtgg gcttccccgt gcgcccccag 4740
gtgcccctgc gccccatgac ctacaaggcc gccttcgacc tgagcttctt cctgaaggag 4800
aagggcggcc tggagggcct gatctacagc aagaagcgcc aggagatcct ggacctgtgg 4860
gtgtaccaca cccagggctt cttccccggc tggcagaact acacccccgg ccccggcgtg 4920
cgctaccccc tgaccttcgg ctggtgcttc aagctggtgc ccgtggaccc ccgcgaggtg 4980
gaggaggcca acaagggcga gaacaactgc ctgctgcacc ccatgagcca gcacggcatg 5040
gaggacgagg accgcgaggt gctgaagtgg aagttcgaca gcagcctggc ccgccgccac 5100
atggcccgcg agctgcaccc cgagtactac aaggactgcg cctaa 5145

<210> 13
<211> 4716
<212> DNA
<213> Artificial Sequence

<220>
<223> Description of Artificial Sequence:
      GagProtInaRTmutTatRevNef_C

<400> 13
gccaccatgg gcgcccgcgc cagcatcctg cgcggcggca agctggacgc ctgggagcgc 60
atccgcctgc gccccggcgg caagaagtgc tacatgatga agcacctggt gtgggccagc 120
cgcgagctgg agaagttcgc cctgaacccc ggcctgctgg agaccagcga gggctgcaag 180
cagatcatcc gccagctgca ccccgccctg cagaccggca gcgaggagct gaagagcctg 240
ttcaacaccg tggccaccct gtactgcgtg cacgagaaga tcgaggtccg cgacaccaag 300
gaggccctgg acaagatcga ggaggagcag aacaagtgcc agcagaagat ccagcaggcc 360
gaggccgccg acaagggcaa ggtgagccag aactacccca tcgtgcagaa cctgcagggc 420
cagatggtgc accaggccat cagcccccgc accctgaacg cctgggtgaa ggtgatcgag 480
gagaaggcct tcagccccga ggtgatcccc atgttcaccg ccctgagcga gggcgccacc 540
ccccaggacc tgaacacgat gttgaacacc gtgggcggcc accaggccgc catgcagatg 600
ctgaaggaca ccatcaacga ggaggccgcc gagtgggacc gcgtgcaccc cgtgcacgcc 660
ggccccatcg cccccggcca gatgcgcgag ccccgcggca gcgacatcgc cggcaccacc 720
agcaccctgc aggagcagat cgcctggatg accagcaacc cccccatccc cgtgggcgac 780
atctacaagc ggtggatcat cctgggcctg aacaagatcg tgcggatgta cagccccgtg 840
agcatcctgg acatcaagca gggccccaag gagcccttcc gcgactacgt ggaccgcttc 900
ttcaagaccc tgcgcgccga gcagagcacc caggaggtga agaactggat gaccgacacc 960
ctgctggtgc agaacgccaa ccccgactgc aagaccatcc tgcgcgctct cggccccggc 1020
gccagcctgg aggagatgat gaccgcctgc cagggcgtgg gcggccccag ccacaaggcc 1080
cgcgtgctgg ccgaggcgat gagccaggcc aacaccagcg tgatgatgca gaagagcaac 1140
ttcaagggcc cccggcgcat cgtcaagtgc ttcaactgcg gcaaggaggg ccacatcgcc 1200
cgcaactgcc gcgccccccg caagaagggc tgctggaagt gcggcaagga gggccaccag 1260
atgaaggact gcaccgagcg ccaggccaac ttcctgggca agatctggcc cagccacaag 1320
ggccgccccg gcaacttcct gcagagccgc cccgagccca ccgccccccc cgccgagagc 1380
ttccgcttcg aggagaccac ccccggccag aagcaggaga gcaaggaccg cgagaccctg 1440
accagcctga agagcctgtt cggcaacgac cccctgagcc agaaagaatt cccccagatc 1500
accctgtggc agcgccccct ggtgagcatc aaggtgggcg gccagatcaa ggaggccctg 1560
ctggccaccg gcgccgacga caccgtgctg gaggagatga gcctgcccgg caagtggaag 1620

cccaagatga tcggcggcat cggcggcttc atcaaggtgc gccagtacga ccagatcctg 1680
atcgagatct gcggcaagaa ggccatcggc accgtgctga tcggccccac ccccgtgaac 1740
atcatcggcc gcaacatgct gacccagctg ggctgcaccc tgaacttccc catcagcccc 1800
atcgagaccg tgcccgtgaa gctgaagccc ggcatggacg gccccaaggt gaagcagtgg 1860
cccctgaccg aggagaagat caaggccctg accgccatct gcgaggagat ggagaaggag 1920
ggcaagatca ccaagatcgg ccccgagaac ccctacaaca cccccgtgtt cgccatcaag 1980
aagaaggaca gcaccaagtg gcgcaagctg gtggacttcc gcgagctgaa caagcgcacc 2040
caggacttct gggaggtgca gctgggcatc ccccaccccg ccggcctgaa gaagaagaag 2100
agcgtgaccg tgctggacgt gggcgacgcc tacttcagcg tgcccctgga cgaggacttc 2160
cgcaagtaca ccgccttcac catccccagc atcaacaacg agacccccgg catccgctac 2220
cagtacaacg tgctgcccca gggctggaag ggcagcccca gcatcttcca gagcagcatg 2280
accaagatcc tggagccctt ccgcgcccgc aaccccgaga tcgtgatcta ccaggccccc 2340
ctgtacgtgg gcagcgacct ggagatcggc cagcaccgcg ccaagatcga ggagctgcgc 2400
aagcacctgc tgcgctgggg cttcaccacc cccgacaaga agcaccagaa ggagcccccc 2460
ttcctgccca tcgagctgca ccccgacaag tggaccgtgc agcccatcga gctgcccgag 2520
aaggagagct ggaccgtgaa cgacatccag aagctggtgg gcaagctgaa ctgggccagc 2580
cagatctacc ccggcatcaa ggtgcgccag ctgtgcaagc tgctgcgcgg cgccaaggcc 2640
ctgaccgaca tcgtgcccct gaccgaggag gccgagctgg agctggccga gaaccgcgag 2700
atcctgcgcg agcccgtgca cggcgtgtac tacgacccca gcaaggacct ggtggccgag 2760
atccagaagc agggccacga ccagtggacc taccagatct accaggagcc cttcaagaac 2820
ctgaagaccg gcaagtacgc caagatgcgc accgcccaca ccaacgacgt gaagcagctg 2880
accgaggccg tgcagaagat cgccatggag agcatcgtga tctggggcaa gacccccaag 2940
ttccgcctgc ccatccagaa ggagacctgg gagacctggt ggaccgacta ctggcaggcc 3000
acctggatcc ccgagtggga gttcgtgaac accccccccc tggtgaagct gtggtaccag 3060
ctggagaagg agcccatcat cggcgccgag accttctacg tggacggcgc cgccaaccgc 3120
gagaccaaga tcggcaaggc cggctacgtg accgaccggg gccggcagaa gatcgtgagc 3180
ctgaccgaga ccaccaacca gaagaccgag ctgcaggcca tccagctggc cctgcaggac 3240
agcggcagcg aggtgaacat cgtgaccgac agccagtacg ccctgggcat catccaggcc 3300
cagcccgaca agagcgagag cgagctggtg aaccagatca tcgagcagct gatcaagaag 3360
gagaaggtgt acctgagctg ggtgcccgcc cacaagggca tcggcggcaa cgagcagatc 3420
gacaagctgg tgagcaaggg catccgcaag gtgctcaagc ttgagcccgt ggaccccaac 3480
ctggagccct ggaaccaccc cggcagccag cccaagaccg ccggcaacaa gtgctactgc 3540
aagcactgca gctaccactg cctggtgagc ttccagacca agggcctggg catcagctac 3600
ggccgcaaga agcgccgcca gcgccgcagc gcccccccca gcagcgagga ccaccagaac 3660
cccatcagca agcagcccct gccccagacc cgcggcgacc ccaccggcag cgaggagagc 3720
aagaagaagg tggagagcaa gaccgagacc gaccccttcg accccggggc cggccgcagc 3780
ggcgacagcg acgaggccct gctgcaggcc gtgcgcatca tcaagatcct gtaccagagc 3840
aacccctacc ccaagcccga gggcacccgc caggccgacc tgaaccgccg ccgccgctgg 3900
cgcgcccgcc agcgccagat ccacagcatc agcgagcgca tcctgagcac ctgcctgggc 3960
cgccccgccg agcccgtgcc cttccagctg ccccccgacc tgcgcctgca catcgactgc 4020
agcgagagca gcggcaccag cggcacccag cagagccagg gcaccaccga gggcgtgggc 4080
agccccctcg aggccggcaa gtggagcaag agcagcatcg tgggctggcc cgccgtgcgc 4140
gagcgcatcc gccgcaccga gcccgccgcc gagggcgtgg gcgccgccag ccaggacctg 4200
gacaagcacg gcgccctgac cagcagcaac accgccgcca acaacgccga ctgcgcctgg 4260
ctggaggccc aggaggagga ggaggaggtg ggcttccccg tgcgccccca ggtgcccctg 4320
cgccccatga cctacaaggc cgccttcgac ctgagcttct tcctgaagga gaagggcggc 4380
ctggagggcc tgatctacag caagaagcgc caggagatcc tggacctgtg ggtgtaccac 4440
acccagggct tcttccccgg ctggcagaac tacacccccg gccccggcgt gcgctacccc 4500
ctgaccttcg gctggtgctt caagctggtg cccgtggacc cccgcgaggt ggaggaggcc 4560
aacaagggcg agaacaactg cctgctgcac cccatgagcc agcacggcat ggaggacgag 4620
gaccgcgagg tgctgaagtg gaagttcgac agcagcctgg cccgccgcca catggcccgc 4680
gagctgcacc ccgagtacta caaggactgc gcctaa 4716

<210> 14

<211> 4419
<212> DNA
<213> Artificial Sequence

<220>
<223> Description of Artificial Sequence:
      GagRTmutTatRevNef_C

<400> 14
gccaccatgg gcgcccgcgc cagcatcctg cgcggcggca agctggacgc ctgggagcgc 60
atccgcctgc gccccggcgg caagaagtgc tacatgatga agcacctggt gtgggccagc 120
cgcgagctgg agaagttcgc cctgaacccc ggcctgctgg agaccagcga gggctgcaag 180
cagatcatcc gccagctgca ccccgccctg cagaccggca gcgaggagct gaagagcctg 240
ttcaacaccg tggccaccct gtactgcgtg cacgagaaga tcgaggtccg cgacaccaag 300
gaggccctgg acaagatcga ggaggagcag aacaagtgcc agcagaagat ccagcaggcc 360
gaggccgccg acaagggcaa ggtgagccag aactacccca tcgtgcagaa cctgcagggc 420
cagatggtgc accaggccat cagcccccgc accctgaacg cctgggtgaa ggtgatcgag 480
gagaaggcct tcagccccga ggtgatcccc atgttcaccg ccctgagcga gggcgccacc 540
ccccaggacc tgaacacgat gttgaacacc gtgggcggcc accaggccgc catgcagatg 600
ctgaaggaca ccatcaacga ggaggccgcc gagtgggacc gcgtgcaccc cgtgcacgcc 660
ggccccatcg cccccggcca gatgcgcgag ccccgcggca gcgacatcgc cggcaccacc 720
agcaccctgc aggagcagat cgcctggatg accagcaacc cccccatccc cgtgggcgac 780
atctacaagc ggtggatcat cctgggcctg aacaagatcg tgcggatgta cagccccgtg 840
agcatcctgg acatcaagca gggccccaag gagcccttcc gcgactacgt ggaccgcttc 900
ttcaagaccc tgcgcgccga gcagagcacc caggaggtga agaactggat gaccgacacc 960
ctgctggtgc agaacgccaa ccccgactgc aagaccatcc tgcgcgctct cggccccggc 1020
gccagcctgg aggagatgat gaccgcctgc cagggcgtgg gcggccccag ccacaaggcc 1080
cgcgtgctgg ccgaggcgat gagccaggcc aacaccagcg tgatgatgca gaagagcaac 1140
ttcaagggcc cccggcgcat cgtcaagtgc ttcaactgcg gcaaggaggg ccacatcgcc 1200
cgcaactgcc gcgccccccg caagaagggc tgctggaagt gcggcaagga gggccaccag 1260
atgaaggact gcaccgagcg ccaggccaac ttcctgggca agatctggcc cagccacaag 1320
ggccgccccg gcaacttcct gcagagccgc cccgagccca ccgccccccc cgccgagagc 1380
ttccgcttcg aggagaccac ccccggccag aagcaggaga gcaaggaccg cgagaccctg 1440
accagcctga agagcctgtt cggcaacgac cccctgagcc agaaagaatt ccccatcagc 1500
cccatcgaga ccgtgcccgt gaagctgaag cccggcatgg acggccccaa ggtgaagcag 1560
tggcccctga ccgaggagaa gatcaaggcc ctgaccgcca tctgcgagga gatggagaag 1620
gagggcaaga tcaccaagat cggccccgag aacccctaca acacccccgt gttcgccatc 1680
aagaagaagg acagcaccaa gtggcgcaag ctggtggact tccgcgagct gaacaagcgc 1740
acccaggact tctgggaggt gcagctgggc atcccccacc ccgccggcct gaagaagaag 1800
aagagcgtga ccgtgctgga cgtgggcgac gcctacttca gcgtgcccct ggacgaggac 1860
ttccgcaagt acaccgcctt caccatcccc agcatcaaca acgagacccc cggcatccgc 1920
taccagtaca acgtgctgcc ccagggctgg aagggcagcc ccagcatctt ccagagcagc 1980
atgaccaaga tcctggagcc cttccgcgcc cgcaaccccg agatcgtgat ctaccaggcc 2040
cccctgtacg tgggcagcga cctggagatc ggccagcacc gcgccaagat cgaggagctg 2100
cgcaagcacc tgctgcgctg gggcttcacc acccccgaca agaagcacca gaaggagccc 2160
cccttcctgc ccatcgagct gcaccccgac aagtggaccg tgcagcccat cgagctgccc 2220
gagaaggaga gctggaccgt gaacgacatc cagaagctgg tgggcaagct gaactgggcc 2280
agccagatct accccggcat caaggtgcgc cagctgtgca agctgctgcg cggcgccaag 2340
gccctgaccg acatcgtgcc cctgaccgag gaggccgagc tggagctggc cgagaaccgc 2400
gagatcctgc gcgagcccgt gcacggcgtg tactacgacc ccagcaagga cctggtggcc 2460
gagatccaga agcagggcca cgaccagtgg acctaccaga tctaccagga gcccttcaag 2520
aacctgaaga ccggcaagta cgccaagatg cgcaccgccc acaccaacga cgtgaagcag 2580
ctgaccgagg ccgtgcagaa gatcgccatg gagagcatcg tgatctgggg caagaccccc 2640
aagttccgcc tgcccatcca gaaggagacc tgggagacct ggtggaccga ctactggcag 2700

gccacctgga tccccgagtg ggagttcgtg aacacccccc ccctggtgaa gctgtggtac 2760
cagctggaga aggagcccat catcggcgcc gagaccttct acgtggacgg cgccgccaac 2820
cgcgagacca agatcggcaa ggccggctac gtgaccgacc ggggccggca gaagatcgtg 2880
agcctgaccg agaccaccaa ccagaagacc gagctgcagg ccatccagct ggccctgcag 2940
gacagcggca gcgaggtgaa catcgtgacc gacagccagt acgccctggg catcatccag 3000
gcccagcccg acaagagcga gagcgagctg gtgaaccaga tcatcgagca gctgatcaag 3060
aaggagaagg tgtacctgag ctgggtgccc gcccacaagg gcatcggcgg caacgagcag 3120
atcgacaagc tggtgagcaa gggcatccgc aaggtgctca agcttgagcc cgtggacccc 3180
aacctggagc cctggaacca ccccggcagc cagcccaaga ccgccggcaa caagtgctac 3240
tgcaagcact gcagctacca ctgcctggtg agcttccaga ccaagggcct gggcatcagc 3300
tacggccgca agaagcgccg ccagcgccgc agcgcccccc ccagcagcga ggaccaccag 3360
aaccccatca gcaagcagcc cctgccccag acccgcggcg accccaccgg cagcgaggag 3420
agcaagaaga aggtggagag caagaccgag accgacccct tcgaccccgg ggccggccgc 3480
agcggcgaca gcgacgaggc cctgctgcag gccgtgcgca tcatcaagat cctgtaccag 3540
agcaacccct accccaagcc cgagggcacc cgccaggccg acctgaaccg ccgccgccgc 3600
tggcgcgccc gccagcgcca gatccacagc atcagcgagc gcatcctgag cacctgcctg 3660
ggccgccccg ccgagcccgt gcccttccag ctgccccccg acctgcgcct gcacatcgac 3720
tgcagcgaga gcagcggcac cagcggcacc cagcagagcc agggcaccac cgagggcgtg 3780
ggcagccccc tcgaggccgg caagtggagc aagagcagca tcgtgggctg gcccgccgtg 3840
cgcgagcgca tccgccgcac cgagcccgcc gccgagggcg tgggcgccgc cagccaggac 3900
ctggacaagc acggcgccct gaccagcagc aacaccgccg ccaacaacgc cgactgcgcc 3960
tggctggagg cccaggagga ggaggaggag gtgggcttcc ccgtgcgccc ccaggtgccc 4020
ctgcgcccca tgacctacaa ggccgccttc gacctgagct tcttcctgaa ggagaagggc 4080
ggcctggagg gcctgatcta cagcaagaag cgccaggaga tcctggacct gtgggtgtac 4140
cacacccagg gcttcttccc cggctggcag aactacaccc ccggccccgg cgtgcgctac 4200
cccctgacct tcggctggtg cttcaagctg gtgcccgtgg acccccgcga ggtggaggag 4260
gccaacaagg gcgagaacaa ctgcctgctg caccccatga gccagcacgg catggaggac 4320
gaggaccgcg aggtgctgaa gtggaagttc gacagcagcc tggcccgccg ccacatggcc 4380
cgcgagctgc accccgagta ctacaaggac tgcgcctaa 4419

<210> 15
<211> 2742
<212> DNA
<213> Artificial Sequence

<220>
<223> Description of Artificial Sequence: GagTatRevNef_C

<400> 15
gccaccatgg gcgcccgcgc cagcatcctg cgcggcggca agctggacgc ctgggagcgc 60
atccgcctgc gccccggcgg caagaagtgc tacatgatga agcacctggt gtgggccagc 120
cgcgagctgg agaagttcgc cctgaacccc ggcctgctgg agaccagcga gggctgcaag 180
cagatcatcc gccagctgca ccccgccctg cagaccggca gcgaggagct gaagagcctg 240
ttcaacaccg tggccaccct gtactgcgtg cacgagaaga tcgaggtccg cgacaccaag 300
gaggccctgg acaagatcga ggaggagcag aacaagtgcc agcagaagat ccagcaggcc 360
gaggccgccg acaagggcaa ggtgagccag aactacccca tcgtgcagaa cctgcagggc 420
cagatggtgc accaggccat cagcccccgc accctgaacg cctgggtgaa ggtgatcgag 480
gagaaggcct tcagccccga ggtgatcccc atgttcaccg ccctgagcga gggcgccacc 540
ccccaggacc tgaacacgat gttgaacacc gtgggcggcc accaggccgc catgcagatg 600
ctgaaggaca ccatcaacga ggaggccgcc gagtgggacc gcgtgcaccc cgtgcacgcc 660
ggccccatcg cccccggcca gatgcgcgag ccccgcggca gcgacatcgc cggcaccacc 720
agcaccctgc aggagcagat cgcctggatg accagcaacc cccccatccc cgtgggcgac 780
atctacaagc ggtggatcat cctgggcctg aacaagatcg tgcggatgta cagccccgtg 840
agcatcctgg acatcaagca gggccccaag gagcccttcc gcgactacgt ggaccgcttc 900

ttcaagaccc tgcgcgccga gcagagcacc caggaggtga agaactggat gaccgacacc 960
ctgctggtgc agaacgccaa ccccgactgc aagaccatcc tgcgcgctct cggccccggc 1020
gccagcctgg aggagatgat gaccgcctgc cagggcgtgg gcggccccag ccacaaggcc 1080
cgcgtgctgg ccgaggcgat gagccaggcc aacaccagcg tgatgatgca gaagagcaac 1140
ttcaagggcc cccggcgcat cgtcaagtgc ttcaactgcg gcaaggaggg ccacatcgcc 1200
cgcaactgcc gcgccccccg caagaagggc tgctggaagt gcggcaagga gggccaccag 1260
atgaaggact gcaccgagcg ccaggccaac ttcctgggca agatctggcc cagccacaag 1320
ggccgccccg gcaacttcct gcagagccgc cccgagccca ccgccccccc cgccgagagc 1380
ttccgcttcg aggagaccac ccccggccag aagcaggaga gcaaggaccg cgagaccctg 1440
accagcctga agagcctgtt cggcaacgac cccctgagcc aagaattcga gcccgtggac 1500
cccaacctgg agccctggaa ccaccccggc agccagccca agaccgccgg caacaagtgc 1560
tactgcaagc actgcagcta ccactgcctg gtgagcttcc agaccaaggg cctgggcatc 1620
agctacggcc gcaagaagcg ccgccagcgc cgcagcgccc cccccagcag cgaggaccac 1680
cagaacccca tcagcaagca gcccctgccc cagacccgcg gcgaccccac cggcagcgag 1740
gagagcaaga agaaggtgga gagcaagacc gagaccgacc ccttcgaccc cggggccggc 1800
cgcagcggcg acagcgacga ggccctgctg caggccgtgc gcatcatcaa gatcctgtac 1860
cagagcaacc cctaccccaa gcccgagggc acccgccagg ccgacctgaa ccgccgccgc 1920
cgctggcgcg cccgccagcg ccagatccac agcatcagcg agcgcatcct gagcacctgc 1980
ctgggccgcc ccgccgagcc cgtgcccttc cagctgcccc ccgacctgcg cctgcacatc 2040
gactgcagcg agagcagcgg caccagcggc acccagcaga gccagggcac caccgagggc 2100
gtgggcagcc ccctcgaggc cggcaagtgg agcaagagca gcatcgtggg ctggcccgcc 2160
gtgcgcgagc gcatccgccg caccgagccc gccgccgagg gcgtgggcgc cgccagccag 2220
gacctggaca agcacggcgc cctgaccagc agcaacaccg ccgccaacaa cgccgactgc 2280
gcctggctgg aggcccagga ggaggaggag gaggtgggct tccccgtgcg cccccaggtg 2340
cccctgcgcc ccatgaccta caaggccgcc ttcgacctga gcttcttcct gaaggagaag 2400
ggcggcctgg agggcctgat ctacagcaag aagcgccagg agatcctgga cctgtgggtg 2460
taccacaccc agggcttctt ccccggctgg cagaactaca cccccggccc cggcgtgcgc 2520
taccccctga ccttcggctg gtgcttcaag ctggtgcccg tggacccccg cgaggtggag 2580
gaggccaaca agggcgagaa caactgcctg ctgcacccca tgagccagca cggcatggag 2640
gacgaggacc gcgaggtgct gaagtggaag ttcgacagca gcctggcccg ccgccacatg 2700
gcccgcgagc tgcaccccga gtactacaag gactgcgcct aa 2742

<210> 16
<211> 861
<212> DNA
<213> Artificial Sequence

<220>
<223> Description of Artificial Sequence:
      int.opt.mut.SF2

<400> 16
ttcctgaacg gcatcgacaa ggcccaggag gagcacgaga agtaccacag caactggcgc 60
gccatggcca gcgacttcaa cctgcccccc gtggtggcca aggagatcgt ggccagcgcc 120
gacaagtgcc agctgaaggg cgaggccatg cacggccagg tggactgcag ccccggcatc 180
tggcagctgg cctgcaccca cctggagggc aagatcatcc tggtggccgt gcacgtggcc 240
agcggctaca tcgaggccga ggtgatcccc gccgagaccg gccaggagac cgcctacttc 300
ctgctgaagc tggccggccg ctggcccgtg aagaccatcc acaccgccaa cggcagcaac 360
ttcaccagca ccaccgtgaa ggccgcctgc tggtgggccg gcatcaagca ggagttcggc 420
atcccctaca acccccagag ccagggcgtg gtggcgagca tgaacaacga gctgaagaag 480
atcatcggcc aggtgcgcga ccaggccgag cacctgaaga ccgccgtgca gatggccgtg 540
ttcatccaca acttcaagcg caagggcggc atcggcggct acagcgccgg cgagcgcatc 600
gtggacatca tcgccaccga catccagacc aaggagctgc agaagcagat caccaagatc 660
cagaacttcc gcgtgtacta ccgcgacaac aaggaccccc tgaagggccc cgccaagctg 720

ctgtggaagg gcgagggcgc cgtggtgatc caggacaaca gcgacatcaa ggtggtgccc 780
cgccgcaagg ccaagatcat ccgcgactac ggcaagcaga tggccggcga cgactgcgtg 840
gccagccgcc aggacgagga c 861

<210> 17
<211> 864
<212> DNA
<213> Artificial Sequence

<220>
<223> Description of Artificial Sequence: int.opt.SF2

<400> 17
ttcctgaacg gcatcgacaa ggcccaggag gagcacgaga agtaccacag caactggcgc 60
gccatggcca gcgacttcaa cctgcccccc gtggtggcca aggagatcgt ggccagctgc 120
gacaagtgcc agctgaaggg cgaggccatg cacggccagg tggactgcag ccccggcatc 180
tggcagctgg actgcaccca cctggagggc aagatcatcc tggtggccgt gcacgtggcc 240
agcggctaca tcgaggccga ggtgatcccc gccgagaccg gccaggagac cgcctacttc 300
ctgctgaagc tggccggccg ctggcccgtg aagaccatcc acaccgacaa cggcagcaac 360
ttcaccagca ccaccgtgaa ggccgcctgc tggtgggccg gcatcaagca ggagttcggc 420
atcccctaca acccccagag ccagggcgtg gtggagagca tgaacaacga gctgaagaag 480
atcatcggcc aggtgcgcga ccaggccgag cacctgaaga ccgccgtgca gatggccgtg 540
ttcatccaca acttcaagcg caagggcggc atcggcggct acagcgccgg cgagcgcatc 600
gtggacatca tcgccaccga catccagacc aaggagctgc agaagcagat caccaagatc 660
cagaacttcc gcgtgtacta ccgcgacaac aaggaccccc tgtggaaggg ccccgccaag 720
ctgctgtgga agggcgaggg cgccgtggtg atccaggaca acagcgacat caaggtggtg 780
ccccgccgca aggccaagat catccgcgac tacggcaagc agatggccgg cgacgactgc 840
gtggccagcc gccaggacga ggac 864

<210> 18
<211> 861
<212> DNA
<213> Artificial Sequence

<220>
<223> Description of Artificial Sequence: int.opt.mut_C

<400> 18
ttcctggacg gcatcgacaa ggcccaggag gagcacgagc gctaccacag caactggcgc 60
gccatggcca acgagttcaa cctgcccccc atcgtggcca aggagatcgt ggccagcgcc 120
gacaagtgcc agctgaaggg cgaggccatc cacggccagg tggactgcag ccccggcatc 180
tggcagctgg cctgcaccca cctggagggc aagatcatcc tggtggccgt gcacgtggcc 240
agcggctaca tggaggccga ggtgatcccc gccgagaccg gccaggagac cgcctacttc 300
atcctgaagc tggccggccg ctggcccgtg aaggtgatcc acaccgccaa cggcagcaac 360
ttcaccagca ccgccgtgaa ggccgcctgc tggtgggccg gcatccagca ggagttcggc 420
atcccctaca acccccagag ccagggcgtg gtggcgagca tgaacaagga gctgaagaag 480
atcatcggcc aggtgcgcga ccaggccgag cacctgaaga ccgccgtgca gatggccgtg 540
ttcatccaca acttcaagcg caagggcggc atcggcggct acagcgccgg cgagcgcatc 600
atcgacatca tcgccaccga catccagacc aaggagctgc agaagcagat catccgcatc 660
cagaacttcc gcgtgtacta ccgcgacagc cgcgacccca tcaagggccc cgccgagctg 720
ctgtggaagg gcgagggcgt ggtggtgatc gaggacaagg gcgacatcaa ggtggtgccc 780
cgccgcaagg ccaagatcat ccgcgactac ggcaagcaga tggccggcgc cgactgcgtg 840
gccggcggcc aggacgagga c 861

<210> 19
<211> 864
<212> DNA
<213> Artificial Sequence

<220>
<223> Description of Artificial Sequence: int.opt_C

<400> 19
ttcctggacg gcatcgacaa ggcccaggag gagcacgagc gctaccacag caactggcgc 60
gccatggcca acgagttcaa cctgcccccc atcgtggcca aggagatcgt ggccagctgc 120
gacaagtgcc agctgaaggg cgaggccatc cacggccagg tggactgcag ccccggcatc 180
tggcagctgg actgcaccca cctggagggc aagatcatcc tggtggccgt gcacgtggcc 240
agcggctaca tggaggccga ggtgatcccc gccgagaccg gccaggagac cgcctacttc 300
atcctgaagc tggccggccg ctggcccgtg aaggtgatcc acaccgacaa cggcagcaac 360
ttcaccagca ccgccgtgaa ggccgcctgc tggtgggccg gcatccagca ggagttcggc 420
atcccctaca acccccagag ccagggcgtg gtggagagca tgaacaagga gctgaagaag 480
atcatcggcc aggtgcgcga ccaggccgag cacctgaaga ccgccgtgca gatggccgtg 540
ttcatccaca acttcaagcg caagggcggc atcggcggct acagcgccgg cgagcgcatc 600
atcgacatca tcgccaccga catccagacc aaggagctgc agaagcagat catccgcatc 660
cagaacttcc gcgtgtacta ccgcgacagc cgcgacccca tctggaaggg ccccgccgag 720
ctgctgtgga agggcgaggg cgtggtggtg atcgaggaca agggcgacat caaggtggtg 780
ccccgccgca aggccaagat catccgcgac tacggcaagc agatggccgg cgccgactgc 840
gtggccggcg gccaggacga ggac 864

<210> 20
<211> 624
<212> DNA
<213> Artificial Sequence

<220>
<223> Description of Artificial Sequence:
      nef.D125G.-myr.opt.SF162

<400> 20
atggccggca agtggagcaa gcgcatgagc ggctggagcg ccgtgcgcga gcgcatgaag 60
cgcgccgagc ccgccgagcc cgccgccgac ggcgtgggcg ccgtgagccg cgacctggag 120
aagcacggcg ccatcaccag cagcaacacc gccgccaaca acgccgactg cgcctggctg 180
gaggcccagg aggacgagga cgtgggcttc cccgtgcgcc cccaggtgcc cctgcgcccc 240
atgacctaca aggccgccct ggacctgagc cacttcctga aggagaaggg cggcctggag 300
ggcctgatct acagccagaa gcgccaggac atcctggacc tgtggatcca ccacacccag 360
ggctacttcc ccggctggca gaactacacc cccggccccg gcatccgcta ccccctgacc 420
ttcggctggt gcttcaagct ggtgcccgtg gaccccgact acgtggagga ggccaacgcc 480
ggcgagaaca acagcctgct gcaccccatg agccagcacg gcatggacga ccccgagaag 540
gaggtgctgg tgtggcgctt cgacagccgc ctggccttcc accacatggc ccgcgagctg 600
caccccgagt actacaagga ctgc 624

<210> 21
<211> 570
<212> DNA
<213> Artificial Sequence

<220>
<223> Description of Artificial Sequence:

nef.D107G.-myr18.opt.SF162

<400> 21
atgaagcgcg ccgagcccgc cgagcccgcc gccgacggcg tgggcgccgt gagccgcgac 60
ctggagaagc acggcgccat caccagcagc aacaccgccg ccaacaacgc cgactgcgcc 120
tggctggagg cccaggagga cgaggacgtg ggcttccccg tgcgccccca ggtgcccctg 180
cgccccatga cctacaaggc cgccctggac ctgagccact tcctgaagga gaagggcggc 240
ctggagggcc tgatctacag ccagaagcgc caggacatcc tggacctgtg gatccaccac 300
acccagggct acttccccgg ctggcagaac tacacccccg gccccggcat ccgctacccc 360
ctgaccttcg gctggtgctt caagctggtg cccgtggacc ccgactacgt ggaggaggcc 420
aacgccggcg agaacaacag cctgctgcac cccatgagcc agcacggcat ggacgacccc 480
gagaaggagg tgctggtgtg gcgcttcgac agccgcctgg ccttccacca catggcccgc 540
gagctgcacc ccgagtacta caaggactgc 570

<210> 22
<211> 624
<212> DNA
<213> Artificial Sequence

<220>
<223> Description of Artificial Sequence:
      nef.opt.D125G.SF162

<400> 22
atgggcggca agtggagcaa gcgcatgagc ggctggagcg ccgtgcgcga gcgcatgaag 60
cgcgccgagc ccgccgagcc cgccgccgac ggcgtgggcg ccgtgagccg cgacctggag 120
aagcacggcg ccatcaccag cagcaacacc gccgccaaca acgccgactg cgcctggctg 180
gaggcccagg aggacgagga cgtgggcttc cccgtgcgcc cccaggtgcc cctgcgcccc 240
atgacctaca aggccgccct ggacctgagc cacttcctga aggagaaggg cggcctggag 300
ggcctgatct acagccagaa gcgccaggac atcctggacc tgtggatcca ccacacccag 360
ggctacttcc ccggctggca gaactacacc cccggccccg gcatccgcta ccccctgacc 420
ttcggctggt gcttcaagct ggtgcccgtg gaccccgact acgtggagga ggccaacgcc 480
ggcgagaaca acagcctgct gcaccccatg agccagcacg gcatggacga ccccgagaag 540
gaggtgctgg tgtggcgctt cgacagccgc ctggccttcc accacatggc ccgcgagctg 600
caccccgagt actacaagga ctgc 624

<210> 23
<211> 624
<212> DNA
<213> Artificial Sequence

<220>
<223> Description of Artificial Sequence: nef.opt.SF162

<400> 23
atgggcggca agtggagcaa gcgcatgagc ggctggagcg ccgtgcgcga gcgcatgaag 60
cgcgccgagc ccgccgagcc cgccgccgac ggcgtgggcg ccgtgagccg cgacctggag 120
aagcacggcg ccatcaccag cagcaacacc gccgccaaca acgccgactg cgcctggctg 180
gaggcccagg aggacgagga cgtgggcttc cccgtgcgcc cccaggtgcc cctgcgcccc 240
atgacctaca aggccgccct ggacctgagc cacttcctga aggagaaggg cggcctggag 300
ggcctgatct acagccagaa gcgccaggac atcctggacc tgtggatcca ccacacccag 360
ggctacttcc ccgactggca gaactacacc cccggccccg gcatccgcta ccccctgacc 420
ttcggctggt gcttcaagct ggtgcccgtg gaccccgact acgtggagga ggccaacgcc 480
ggcgagaaca acagcctgct gcaccccatg agccagcacg gcatggacga ccccgagaag 540

gaggtgctgg tgtggcgctt cgacagccgc ctggccttcc accacatggc ccgcgagctg 600
caccccgagt actacaagga ctgc 624

<210> 24
<211> 624
<212> DNA
<213> Artificial Sequence

<220>
<223> Description of Artificial Sequence:
      Nef_TV1_C_ZAopt

<400> 24
atgggcggca agtggagcaa gcgcagcatc gtgggctggc ccgccgtgcg cgagcgcatg 60
cgccgcaccg agcccgccgc cgagggcgtg ggcgccgcca gccaggacct ggaccgccac 120
ggcgccctga ccagcagcaa cacccccgcc accaacgagg cctgcgcctg gctgcaggcc 180
caggaggagg acggcgacgt gggcttcccc gtgcgccccc aggtgcccct gcgccccatg 240
acctacaaga gcgccgtgga cctgagcttc ttcctgaagg agaagggcgg cctggagggc 300
ctgatctaca gccgcaagcg ccaggagatc ctggacctgt gggtgtacaa cacccagggc 360
ttcttccccg actggcagaa ctacaccagc ggccccggcg tgcgcttccc cctgaccttc 420
ggctggtgct tcaagctggt gcccgtggac ccccgcgagg tgaaggaggc caacgagggc 480
gaggacaact gcctgctgca ccccatgagc cagcacggcg ccgaggacga ggaccgcgag 540
gtgctgaagt ggaagttcga cagcctgctg gcccaccgcc acatggcccg cgagctgcac 600
cccgagtact acaaggactg ctga 624

<210> 25
<211> 624
<212> DNA
<213> Artificial Sequence

<220>
<223> Description of Artificial Sequence:
      Nef_TV2_C_ZAopt

<400> 25
atgggcggca agtggagcaa gagcagcatc atcggctggc ccgaggtgcg cgagcgcatc 60
cgccgcaccc gcagcgccgc cgagggcgtg ggcagcgcca gccaggacct ggagaagcac 120
ggcgccctga ccaccagcaa caccgcccac aacaacgccg cctgcgcctg gctggaggcc 180
caggaggagg agggcgaggt gggcttcccc gtgcgccccc aggtgcccct gcgccccatg 240
acctacaagg ccgccatcga cctgagcttc ttcctgaagg agaagggcgg cctggagggc 300
ctgatctaca gcaagaagcg ccaggagatc ctggacctgt gggtgtacaa cacccagggc 360
ttcttccccg actggcagaa ctacaccccc ggccccggcg tgcgcttccc cctgaccttc 420
ggctggtact tcaagctgga gcccgtggac ccccgcgagg tggaggaggc caacgagggc 480
gagaacaact gcctgctgca ccccatgagc cagcacggca tggaggacga ggaccgcgag 540
gtgctgcgct ggaagttcga cagcaccctg gcccgccgcc acatggcccg cgagctgcac 600
cccgagtact acaaggactg ctga 624

<210> 26
<211> 624
<212> DNA
<213> Artificial Sequence

<220>
<223> Description of Artificial Sequence:

NefD124G_TV1_C_ZAopt

<400> 26
atgggcggca agtggagcaa gcgcagcatc gtgggctggc ccgccgtgcg cgagcgcatg 60
cgccgcaccg agcccgccgc cgagggcgtg ggcgccgcca gccaggacct ggaccgccac 120
ggcgccctga ccagcagcaa cacccccgcc accaacgagg cctgcgcctg gctgcaggcc 180
caggaggagg acggcgacgt gggcttcccc gtgcgccccc aggtgcccct gcgccccatg 240
acctacaaga gcgccgtgga cctgagcttc ttcctgaagg agaagggcgg cctggagggc 300
ctgatctaca gccgcaagcg ccaggagatc ctggacctgt gggtgtacaa cacccagggc 360
ttcttccccg gctggcagaa ctacaccagc ggccccggcg tgcgcttccc cctgaccttc 420
ggctggtgct tcaagctggt gcccgtggac ccccgcgagg tgaaggaggc caacgagggc 480
gaggacaact gcctgctgca ccccatgagc cagcacggcg ccgaggacga ggaccgcgag 540
gtgctgaagt ggaagttcga cagcctgctg gcccaccgcc acatggcccg cgagctgcac 600
cccgagtact acaaggactg ctga 624

<210> 27
<211> 624
<212> DNA
<213> Artificial Sequence

<220>
<223> Description of Artificial Sequence:
      NefD124G_TV2_C_ZAopt

<400> 27
atgggcggca agtggagcaa gagcagcatc atcggctggc ccgaggtgcg cgagcgcatc 60
cgccgcaccc gcagcgccgc cgagggcgtg ggcagcgcca gccaggacct ggagaagcac 120
ggcgccctga ccaccagcaa caccgcccac aacaacgccg cctgcgcctg gctggaggcc 180
caggaggagg agggcgaggt gggcttcccc gtgcgccccc aggtgcccct gcgccccatg 240
acctacaagg ccgccatcga cctgagcttc ttcctgaagg agaagggcgg cctggagggc 300
ctgatctaca gcaagaagcg ccaggagatc ctggacctgt gggtgtacaa cacccagggc 360
ttcttccccg gctggcagaa ctacaccccc ggccccggcg tgcgcttccc cctgaccttc 420
ggctggtact tcaagctgga gcccgtggac ccccgcgagg tggaggaggc caacgagggc 480
gagaacaact gcctgctgca ccccatgagc cagcacggca tggaggacga ggaccgcgag 540
gtgctgcgct ggaagttcga cagcaccctg gcccgccgcc acatggcccg cgagctgcac 600
cccgagtact acaaggactg ctga 624

<210> 28
<211> 624
<212> DNA
<213> Artificial Sequence

<220>
<223> Description of Artificial Sequence:
      NefD124G-Myr_TV1_C_ZAopt

<400> 28
atggccggca agtggagcaa gcgcagcatc gtgggctggc ccgccgtgcg cgagcgcatg 60
cgccgcaccg agcccgccgc cgagggcgtg ggcgccgcca gccaggacct ggaccgccac 120
ggcgccctga ccagcagcaa cacccccgcc accaacgagg cctgcgcctg gctgcaggcc 180
caggaggagg acggcgacgt gggcttcccc gtgcgccccc aggtgcccct gcgccccatg 240
acctacaaga gcgccgtgga cctgagcttc ttcctgaagg agaagggcgg cctggagggc 300
ctgatctaca gccgcaagcg ccaggagatc ctggacctgt gggtgtacaa cacccagggc 360
ttcttccccg gctggcagaa ctacaccagc ggccccggcg tgcgcttccc cctgaccttc 420

ggctggtgct tcaagctggt gcccgtggac ccccgcgagg tgaaggaggc caacgagggc 480
gaggacaact gcctgctgca ccccatgagc cagcacggcg ccgaggacga ggaccgcgag 540
gtgctgaagt ggaagttcga cagcctgctg gcccaccgcc acatggcccg cgagctgcac 600
cccgagtact acaaggactg ctga 624
<210> 29
<211> 570
<212> DNA
<213> Artificial Sequence

<220>
<223> Description of Artificial Sequence:
      nef.D106G.-myr19.opt_C

<400> 29
atgatccgcc gcaccgagcc cgccgccgag ggcgtgggcg ccgccagcca ggacctggac 60
aagcacggcg ccctgaccag cagcaacacc gccgccaaca acgccgactg cgcctggctg 120
gaggcccagg aggaggagga ggaggtgggc ttccccgtgc gcccccaggt gcccctgcgc 180
cccatgacct acaaggccgc cttcgacctg agcttcttcc tgaaggagaa gggcggcctg 240
gagggcctga tctacagcaa gaagcgccag gagatcctgg acctgtgggt gtaccacacc 300
cagggcttct tccccggctg gcagaactac acccccggcc ccggcgtgcg ctaccccctg 360
accttcggct ggtgcttcaa gctggtgccc gtggaccccc gcgaggtgga ggaggccaac 420
aagggcgaga acaactgcct gctgcacccc atgagccagc acggcatgga ggacgaggac 480
cgcgaggtgc tgaagtggaa gttcgacagc agcctggccc gccgccacat ggcccgcgag 540
ctgcaccccg agtactacaa ggactgcgcc 570

<210> 30
<211> 360
<212> DNA
<213> Artificial Sequence

<220>
<223> Description of Artificial Sequence:
      p15RnaseH.opt.SF2

<400> 30
tacgtggacg gcgccgccaa ccgcgagacc aagctgggca aggccggcta cgtgaccgac 60
cggggccggc agaaggtggt gagcatcgcc gacaccacca accagaagac cgagctgcag 120
gccatccacc tggccctgca ggacagcggc ctggaggtga acatcgtgac cgacagccag 180
tacgccctgg gcatcatcca ggcccagccc gacaagagcg agagcgagct ggtgagccag 240
atcatcgagc agctgatcaa gaaggagaag gtgtacctgg cctgggtgcc cgcccacaag 300
ggcatcggcg gcaacgagca ggtggacaag ctggtgagcg ccggcatccg caaggtgctg 360

<210> 31
<211> 360
<212> DNA
<213> Artificial Sequence

<220>
<223> Description of Artificial Sequence:
      p15RnaseH.opt_C

<400> 31
tacgtggacg gcgccgccaa ccgcgagacc aagatcggca aggccggcta cgtgaccgac 60
cggggccggc agaagatcgt gagcctgacc gagaccacca accagaagac cgagctgcag 120

gccatccagc tggccctgca ggacagcggc agcgaggtga acatcgtgac cgacagccag 180
tacgccctgg gcatcatcca ggcccagccc gacaagagcg agagcgagct ggtgaaccag 240
atcatcgagc agctgatcaa gaaggagaag gtgtacctga gctgggtgcc cgcccacaag 300
ggcatcggcg gcaacgagca gatcgacaag ctggtgagca agggcatccg caaggtgctc 360

<210> 32
<211> 2460
<212> DNA
<213> Artificial Sequence
<220>
<223> Description of Artificial Sequence:
      p2Pol.opt.YMWM.SF2

<400> 32
gccaccatgg ccgaggcgat gagccaggtg acgaacccgg cgaccatcat gatgcagcgc 60
ggcaacttcc gcaaccagcg gaagaccgtc aagtgcttca actgcggcaa ggagggccac 120
accgccagga actgccgcgc cccccgcaag aagggctgct ggcgctgcgg ccgcgaagga 180
caccaaatga aagattgcac tgagagacag gctaatttct tccgcgagga cctggccttc 240
ctgcagggca aggcccgcga gttcagcagc gagcagaccc gcgccaacag ccccacccgc 300
cgcgagctgc aggtgtgggg cggcgagaac aacagcctga gcgaggccgg cgccgaccgc 360
cagggcaccg tgagcttcaa cttcccccag atcaccctgt ggcagcgccc cctggtgacc 420
atcaggatcg gcggccagct caaggaggcg ctgctcgcca ccggcgccga cgacaccgtg 480
ctggaggaga tgaacctgcc cggcaagtgg aagcccaaga tgatcggcgg gatcgggggc 540
ttcatcaagg tgcggcagta cgaccagatc cccgtggaga tctgcggcca caaggccatc 600
ggcaccgtgc tggtgggccc cacccccgtg aacatcatcg gccgcaacct gctgacccag 660
atcggctgca ccctgaactt ccccatcagc cccatcgaga cggtgcccgt gaagctgaag 720
ccggggatgg acggccccaa ggtcaagcag tggcccctga ccgaggagaa gatcaaggcc 780
ctggtggaga tctgcaccga gatggagaag gagggcaaga tcagcaagat cggccccgag 840
aacccctaca acacccccgt gttcgccatc aagaagaagg acagcaccaa gtggcgcaag 900
ctggtggact tccgcgagct gaacaagcgc acccaggact tctgggaggt gcagctgggc 960
atcccccacc ccgccggcct gaagaagaag aagagcgtga ccgtgctgga cgtgggcgac 1020
gcctacttca gcgtgcccct ggacaaggac ttccgcaagt acaccgcctt caccatcccc 1080
agcatcaaca acgagacccc cggcatccgc taccagtaca acgtgctgcc ccagggctgg 1140
aagggcagcc ccgccatctt ccagagcagc atgaccaaga tcctggagcc cttccgcaag 1200
cagaaccccg acatcgtgat ctaccaggcc cccctgtacg tgggcagcga cctggagatc 1260
ggccagcacc gcaccaagat cgaggagctg cgccagcacc tgctgcgctg gggcttcacc 1320
acccccgaca agaagcacca gaaggagccc cccttcctgc ccatcgagct gcaccccgac 1380
aagtggaccg tgcagcccat catgctgccc gagaaggaca gctggaccgt gaacgacatc 1440
cagaagctgg tgggcaagct gaactgggcc agccagatct acgccggcat caaggtgaag 1500
cagctgtgca agctgctgcg cggcaccaag gccctgaccg aggtgatccc cctgaccgag 1560
gaggccgagc tggagctggc cgagaaccgc gagatcctga aggagcccgt gcacgaggtg 1620
tactacgacc ccagcaagga cctggtggcc gagatccaga agcagggcca gggccagtgg 1680
acctaccaga tctaccagga gcccttcaag aacctgaaga ccggcaagta cgcccgcatg 1740
cgcggcgccc acaccaacga cgtgaagcag ctgaccgagg ccgtgcagaa ggtgagcacc 1800
gagagcatcg tgatctgggg caagatcccc aagttcaagc tgcccatcca gaaggagacc 1860
tgggaggcct ggtggatgga gtactggcag gccacctgga tccccgagtg ggagttcgtg 1920
aacacccccc ccctggtgaa gctgtggtac cagctggaga aggagcccat cgtgggcgcc 1980
gagaccttct acgtggacgg cgccgccaac cgcgagacca agctgggcaa ggccggctac 2040
gtgaccgacc ggggccggca gaaggtggtg agcatcgccg acaccaccaa ccagaagacc 2100
gagctgcagg ccatccacct ggccctgcag gacagcggcc tggaggtgaa catcgtgacc 2160
gacagccagt acgccctggg catcatccag gcccagcccg acaagagcga gagcgagctg 2220
gtgagccaga tcatcgagca gctgatcaag aaggagaagg tgtacctggc ctgggtgccc 2280
gcccacaagg gcatcggcgg caacgagcag gtggacaagc tggtgagcgc cggcatccgc 2340
aaggtgctgt tcctgaacgg catcgatggc ggcatcgtga tctaccagta catggacgac 2400

ctgtacgtgg gcagcggcgg ccctaggatc gattaaaagc ttcccggggc tagcaccggt 2460

<210> 33
<211> 2466
<212> DNA
<213> Artificial Sequence

<220>
<223> Description of Artificial Sequence:
      p2PolInaopt.YM.SF2

<400> 33
gccaccatgg ccgaggcgat gagccaggtg acgaacccgg cgaccatcat gatgcagcgc 60
ggcaacttcc gcaaccagcg gaagaccgtc aagtgcttca actgcggcaa ggagggccac 120
accgccagga actgccgcgc cccccgcaag aagggctgct ggcgctgcgg ccgcgaagga 180
caccaaatga aagattgcac tgagagacag gctaatttct tccgcgagga cctggccttc 240
ctgcagggca aggcccgcga gttcagcagc gagcagaccc gcgccaacag ccccacccgc 300
cgcgagctgc aggtgtgggg cggcgagaac aacagcctga gcgaggccgg cgccgaccgc 360
cagggcaccg tgagcttcaa cttcccccag atcaccctgt ggcagcgccc cctggtgacc 420
atcaggatcg gcggccagct caaggaggcg ctgctcgcca ccggcgccga cgacaccgtg 480
ctggaggaga tgaacctgcc cggcaagtgg aagcccaaga tgatcggcgg gatcgggggc 540
ttcatcaagg tgcggcagta cgaccagatc cccgtggaga tctgcggcca caaggccatc 600
ggcaccgtgc tggtgggccc cacccccgtg aacatcatcg gccgcaacct gctgacccag 660
atcggctgca ccctgaactt ccccatcagc cccatcgaga cggtgcccgt gaagctgaag 720
ccggggatgg acggccccaa ggtcaagcag tggcccctga ccgaggagaa gatcaaggcc 780
ctggtggaga tctgcaccga gatggagaag gagggcaaga tcagcaagat cggccccgag 840
aacccctaca acacccccgt gttcgccatc aagaagaagg acagcaccaa gtggcgcaag 900
ctggtggact tccgcgagct gaacaagcgc acccaggact tctgggaggt gcagctgggc 960
atcccccacc ccgccggcct gaagaagaag aagagcgtga ccgtgctgga cgtgggcgac 1020
gcctacttca gcgtgcccct ggacaaggac ttccgcaagt acaccgcctt caccatcccc 1080
agcatcaaca acgagacccc cggcatccgc taccagtaca acgtgctgcc ccagggctgg 1140
aagggcagcc ccgccatctt ccagagcagc atgaccaaga tcctggagcc cttccgcaag 1200
cagaaccccg acatcgtgat ctaccaggcc cccctgtacg tgggcagcga cctggagatc 1260
ggccagcacc gcaccaagat cgaggagctg cgccagcacc tgctgcgctg gggcttcacc 1320
acccccgaca agaagcacca gaaggagccc cccttcctgt ggatgggcta cgagctgcac 1380
cccgacaagt ggaccgtgca gcccatcatg ctgcccgaga aggacagctg gaccgtgaac 1440
gacatccaga agctggtggg caagctgaac tgggccagcc agatctacgc cggcatcaag 1500
gtgaagcagc tgtgcaagct gctgcgcggc accaaggccc tgaccgaggt gatccccctg 1560
accgaggagg ccgagctgga gctggccgag aaccgcgaga tcctgaagga gcccgtgcac 1620
gaggtgtact acgaccccag caaggacctg gtggccgaga tccagaagca gggccagggc 1680
cagtggacct accagatcta ccaggagccc ttcaagaacc tgaagaccgg caagtacgcc 1740
cgcatgcgcg gcgcccacac caacgacgtg aagcagctga ccgaggccgt gcagaaggtg 1800
agcaccgaga gcatcgtgat ctggggcaag atccccaagt tcaagctgcc catccagaag 1860
gagacctggg aggcctggtg gatggagtac tggcaggcca cctggatccc cgagtgggag 1920
ttcgtgaaca ccccccccct ggtgaagctg tggtaccagc tggagaagga gcccatcgtg 1980
ggcgccgaga ccttctacgt ggacggcgcc gccaaccgcg agaccaagct gggcaaggcc 2040
ggctacgtga ccgaccgggg ccggcagaag gtggtgagca tcgccgacac caccaaccag 2100
aagaccgagc tgcaggccat ccacctggcc ctgcaggaca gcggcctgga ggtgaacatc 2160
gtgaccgaca gccagtacgc cctgggcatc atccaggccc agcccgacaa gagcgagagc 2220
gagctggtga gccagatcat cgagcagctg atcaagaagg agaaggtgta cctggcctgg 2280
gtgcccgccc acaagggcat cggcggcaac gagcaggtgg acaagctggt gagcgccggc 2340
atccgcaagg tgctgttcct gaacggcatc gatggcggca tcgtgatcta ccagtacatg 2400
gacgacctgt acgtgggcag cggcggccct aggatcgatt aaaagcttcc cggggctagc 2460
accggt 2466

<210> 34
<211> 2472
<212> DNA
<213> Artificial Sequence

<220>
<223> Description of Artificial Sequence: p2Polopt.SF2

<400> 34
gccaccatgg ccgaggcgat gagccaggtg acgaacccgg cgaccatcat gatgcagcgc 60
ggcaacttcc gcaaccagcg gaagaccgtc aagtgcttca actgcggcaa ggagggccac 120
accgccagga actgccgcgc cccccgcaag aagggctgct ggcgctgcgg ccgcgaagga 180
caccaaatga aagattgcac tgagagacag gctaatttct tccgcgagga cctggccttc 240
ctgcagggca aggcccgcga gttcagcagc gagcagaccc gcgccaacag ccccacccgc 300
cgcgagctgc aggtgtgggg cggcgagaac aacagcctga gcgaggccgg cgccgaccgc 360
cagggcaccg tgagcttcaa cttcccccag atcaccctgt ggcagcgccc cctggtgacc 420
atcaggatcg gcggccagct caaggaggcg ctgctcgaca ccggcgccga cgacaccgtg 480
ctggaggaga tgaacctgcc cggcaagtgg aagcccaaga tgatcggcgg gatcgggggc 540
ttcatcaagg tgcggcagta cgaccagatc cccgtggaga tctgcggcca caaggccatc 600
ggcaccgtgc tggtgggccc cacccccgtg aacatcatcg gccgcaacct gctgacccag 660
atcggctgca ccctgaactt ccccatcagc cccatcgaga cggtgcccgt gaagctgaag 720
ccggggatgg acggccccaa ggtcaagcag tggcccctga ccgaggagaa gatcaaggcc 780
ctggtggaga tctgcaccga gatggagaag gagggcaaga tcagcaagat cggcccccag 840
aacccctaca acacccccgt gttcgccatc aagaagaagg acagcaccaa gtggcgcaag 900
ctggtggact tccgcgagct gaacaagcgc acccaggact tctgggaggt gcagctgggc 960
atcccccacc ccgccggcct gaagaagaag aagagcgtga ccgtgctgga cgtgggcgac 1020
gcctacttca gcgtgcccct ggacaaggac ttccgcaagt acaccgcctt caccatcccc 1080
agcatcaaca acgagacccc cggcatccgc taccagtaca acgtgctgcc ccagggctgg 1140
aagggcagcc ccgccatctt ccagagcagc atgaccaaga tcctggagcc cttccgcaag 1200
cagaaccccg acatcgtgat ctaccagtac atggacgacc tgtacgtggg cagcgacctg 1260
gagatcggcc agcaccgcac caagatcgag gagctgcgcc agcacctgct gcgctggggc 1320
ttcaccaccc ccgacaagaa gcaccagaag gagcccccct tcctgtggat gggctacgag 1380
ctgcaccccg acaagtggac cgtgcagccc atcatgctgc ccgagaagga cagctggacc 1440
gtgaacgaca tccagaagct ggtgggcaag ctgaactggg ccagccagat ctacgccggc 1500
atcaaggtga agcagctgtg caagctgctg cgcggcacca aggccctgac cgaggtgatc 1560
cccctgaccg aggaggccga gctggagctg gccgagaacc gcgagatcct gaaggagccc 1620
gtgcacgagg tgtactacga ccccagcaag gacctggtgg ccgagatcca gaagcagggc 1680
cagggccagt ggacctacca gatctaccag gagcccttca agaacctgaa gaccggcaag 1740
tacgcccgca tgcgcggcgc ccacaccaac gacgtgaagc agctgaccga ggccgtgcag 1800
aaggtgagca ccgagagcat cgtgatctgg ggcaagatcc ccaagttcaa gctgcccatc 1860
cagaaggaga cctgggaggc ctggtggatg gagtactggc aggccacctg gatccccgag 1920
tgggagttcg tgaacacccc ccccctggtg aagctgtggt accagctgga gaaggagccc 1980
atcgtgggcg ccgagacctt ctacgtggac ggcgccgcca accgcgagac caagctgggc 2040
aaggccggct acgtgaccga ccgggggcgg cagaaggtgg tgagcatcgc cgacaccacc 2100
aaccagaaga ccgagctgca ggccatccac ctggccctgc aggacagcgg cctggaggtg 2160
aacatcgtga ccgacagcca gtacgccctg ggcatcatcc aggcccagcc cgacaagagc 2220
gagagcgagc tggtgagcca gatcatcgag cagctgatca agaaggagaa ggtgtacctg 2280
gcctgggtgc ccgcccacaa gggcatcggc ggcaacgagc aggtggacaa gctggtgagc 2340
gccggcatcc gcaaggtgct gttcctgaac ggcatcgatg gcggcatcgt gatctaccag 2400
tacatggacg acctgtacgt gggcagcggc ggccctagga tcgattaaaa gcttcccggg 2460
gctagcaccg gt 2472

<210> 35

<211> 3639
<212> DNA
<213> Artificial Sequence

<220>
<223> Description of Artificial Sequence:
      p2PolTatRevNef.opt.native_B

<400> 35
atggccgagg cgatgagcca ggtgacgaac ccggcgacca tcatgatgca gcgcggcaac 60
ttccgcaacc agcggaagac cgtcaagtgc ttcaactgcg gcaaggaggg ccacaccgcc 120
aggaactgcc gcgccccccg caagaagggc tgctggcgct gcggccgcga aggacaccaa 180
atgaaagatt gcactgagag acaggctaat ttcttccgcg aggacctggc cttcctgcag 240
ggcaaggccc gcgagttcag cagcgagcag acccgcgcca acagccccac ccgccgcgag 300
ctgcaggtgt ggggcggcga gaacaacagc ctgagcgagg ccggcgccga ccgccagggc 360
accgtgagct tcaacttccc ccagatcacc ctgtggcagc gccccctggt gaccatcagg 420
atcggcggcc agctcaagga ggcgctgctc gacaccggcg ccgacgacac cgtgctggag 480
gagatgaacc tgcccggcaa gtggaagccc aagatgatcg gcgggatcgg gggcttcatc 540
aaggtgcggc agtacgacca gatccccgtg gagatctgcg gccacaaggc catcggcacc 600
gtgctggtgg gccccacccc cgtgaacatc atcggccgca acctgctgac ccagatcggc 660
tgcaccctga acttccccat cagccccatc gagacggtgc ccgtgaagct gaagccgggg 720
atggacggcc ccaaggtcaa gcagtggccc ctgaccgagg agaagatcaa ggccctggtg 780
gagatctgca ccgagatgga gaaggagggc aagatcagca agatcggccc cgagaacccc 840
tacaacaccc ccgtgttcgc catcaagaag aaggacagca ccaagtggcg caagctggtg 900
gacttccgcg agctgaacaa gcgcacccag gacttctggg aggtgcagct gggcatcccc 960
caccccgccg gcctgaagaa gaagaagagc gtgaccgtgc tggacgtggg cgacgcctac 1020
ttcagcgtgc ccctggacaa ggacttccgc aagtacaccg ccttcaccat ccccagcatc 1080
aacaacgaga cccccggcat ccgctaccag tacaacgtgc tgccccaggg ctggaagggc 1140
agccccgcca tcttccagag cagcatgacc aagatcctgg agcccttccg caagcagaac 1200
cccgacatcg tgatctacca gtacatggac gacctgtacg tgggcagcga cctggagatc 1260
ggccagcacc gcaccaagat cgaggagctg cgccagcacc tgctgcgctg gggcttcacc 1320
acccccgaca agaagcacca gaaggagccc cccttcctgt ggatgggcta cgagctgcac 1380
cccgacaagt ggaccgtgca gcccatcatg ctgcccgaga aggacagctg gaccgtgaac 1440
gacatccaga agctggtggg caagctgaac tgggccagcc agatctacgc cggcatcaag 1500
gtgaagcagc tgtgcaagct gctgcgcggc accaaggccc tgaccgaggt gatccccctg 1560
accgaggagg ccgagctgga gctggccgag aaccgcgaga tcctgaagga gcccgtgcac 1620
gaggtgtact acgaccccag caaggacctg gtggccgaga tccagaagca gggccagggc 1680
cagtggacct accagatcta ccaggagccc ttcaagaacc tgaagaccgg caagtacgcc 1740
cgcatgcgcg gcgcccacac caacgacgtg aagcagctga ccgaggccgt gcagaaggtg 1800
agcaccgaga gcatcgtgat ctggggcaag atccccaagt tcaagctgcc catccagaag 1860
gagacctggg aggcctggtg gatggagtac tggcaggcca cctggatccc cgagtgggag 1920
ttcgtgaaca ccccccccct ggtgaagctg tggtaccagc tggagaagga gcccatcgtg 1980
ggcgccgaga ccttctacgt ggacggcgcc gccaaccgcg agaccaagct gggcaaggcc 2040
ggctacgtga ccgaccgggg ccggcagaag gtggtgagca tcgccgacac caccaaccag 2100
aagaccgagc tgcaggccat ccacctggcc ctgcaggaca gcggcctgga ggtgaacatc 2160
gtgaccgaca gccagtacgc cctgggcatc atccaggccc agcccgacaa gagcgagagc 2220
gagctggtga gccagatcat cgagcagctg atcaagaagg agaaggtgta cctggcctgg 2280
gtgcccgccc acaagggcat cggcggcaac gagcaggtgg acaagctggt gagcgccggc 2340
atccgcaagg tgctggaatt cgagcccgtg gacccccgcc tggagccctg gaagcacccc 2400
ggcagccagc ccaagaccgc ctgcaccaac tgctactgca agaagtgctg cttccactgc 2460
caggtgtgct tcatcaccaa gggcctgggc atcagctacg gccgcaagaa gcgccgccag 2520
cgccgccgcg ccccccccga cagcgaggtg caccaggtga gcctgcccaa gcagcccgcc 2580
agccagcccc agggcgaccc caccggcccc aaggagagca agaagaaggt ggagcgcgag 2640
accgagaccg accccgtgca ccccggggcc ggccgcagcg gcgacagcga cgaggagctg 2700

ctgcagaccg tgcgcttcat caagttcctg taccagagca accccctgcc cagccccaag 2760
ggcacccgcc aggcccgccg caaccgccgc cgccgctggc gcgagcgcca gcgccagatc 2820
cagagcatca gcgcctggat catcagcacc cacctgggcc gcagcaccga gcccgtgccc 2880
ctgcagctgc cccccctgga gcgcctgaac ctggactgca gcgaggactg cggcaccagc 2940
ggcacccagg gcgtgggcag cccccaggtg ctgggcgaga gccccgccgt gctggacagc 3000
ggcaccaagg agctcgaggg cggcaagtgg agcaagcgca tgagcggctg gagcgccgtg 3060
cgcgagcgca tgaagcgcgc cgagcccgcc gagcccgccg ccgacggcgt gggcgccgtg 3120
agccgcgacc tggagaagca cggcgccatc accagcagca acaccgccgc caacaacgcc 3180
gactgcgcct ggctggaggc ccaggaggac gaggacgtgg gcttccccgt gcgcccccag 3240
gtgcccctgc gccccatgac ctacaaggcc gccctggacc tgagccactt cctgaaggag 3300
aagggcggcc tggagggcct gatctacagc cagaagcgcc aggacatcct ggacctgtgg 3360
atccaccaca cccagggcta cttccccgac tggcagaact acacccccgg ccccggcatc 3420
cgctaccccc tgaccttcgg ctggtgcttc aagctggtgc ccgtggaccc cgactacgtg 3480
gaggaggcca acgccggcga gaacaacagc ctgctgcacc ccatgagcca gcacggcatg 3540
gacgaccccg agaaggaggt gctggtgtgg cgcttcgaca gccgcctggc cttccaccac 3600
atggcccgcg agctgcaccc cgagtactac aaggactgc 3639

<210> 36
<211> 3735
<212> DNA
<213> Artificial Sequence

<220>
<223> Description of Artificial Sequence:
      p2PolTatRevNef.opt_B

<400> 36
gccaccatgg ccgaggcgat gagccaggtg acgaacccgg cgaccatcat gatgcagcgc 60
ggcaacttcc gcaaccagcg gaagaccgtc aagtgcttca actgcggcaa ggagggccac 120
accgccagga actgccgcgc cccccgcaag aagggctgct ggcgctgcgg ccgcgaagga 180
caccaaatga aagattgcac tgagagacag gctaatttct tccgcgagga cctggccttc 240
ctgcagggca aggcccgcga gttcagcagc gagcagaccc gcgccaacag ccccacccgc 300
cgcgagctgc aggtgtgggg cggcgagaac aacagcctga gcgaggccgg cgccgaccgc 360
cagggcaccg tgagcttcaa cttcccccag atcaccctgt ggcagcgccc cctggtgacc 420
atcaggatcg gcggccagct caaggaggcg ctgctcgcca ccggcgccga cgacaccgtg 480
ctggaggaga tgaacctgcc cggcaagtgg aagcccaaga tgatcggcgg gatcgggggc 540
ttcatcaagg tgcggcagta cgaccagatc cccgtggaga tctgcggcca caaggccatc 600
ggcaccgtgc tggtgggccc cacccccgtg aacatcatcg gccgcaacct gctgacccag 660
atcggctgca ccctgaactt ccccatcagc cccatcgaga cggtgcccgt gaagctgaag 720
ccggggatgg acggccccaa ggtcaagcag tggcccctga ccgaggagaa gatcaaggcc 780
ctggtggaga tctgcaccga gatggagaag gagggcaaga tcagcaagat cggccccgag 840
aacccctaca acacccccgt gttcgccatc aagaagaagg acagcaccaa gtggcgcaag 900
ctggtggact tccgcgagct gaacaagcgc acccaggact tctgggaggt gcagctgggc 960
atcccccacc ccgccggcct gaagaagaag aagagcgtga ccgtgctgga cgtgggcgac 1020
gcctacttca gcgtgcccct ggacaaggac ttccgcaagt acaccgcctt caccatcccc 1080
agcatcaaca acgagacccc cggcatccgc taccagtaca acgtgctgcc ccagggctgg 1140
aagggcagcc ccgccatctt ccagagcagc atgaccaaga tcctggagcc cttccgcaag 1200
cagaaccccg acatcgtgat ctaccaggcc cccctgtacg tgggcagcga cctggagatc 1260
ggccagcacc gcaccaagat cgaggagctg cgccagcacc tgctgcgctg gggcttcacc 1320
acccccgaca agaagcacca gaaggagccc cccttcctgc ccatcgagct gcaccccgac 1380
aagtggaccg tgcagcccat catgctgccc gagaaggaca gctggaccgt gaacgacatc 1440
cagaagctgg tgggcaagct gaactgggcc agccagatct acgccggcat caaggtgaag 1500
cagctgtgca agctgctgcg cggcaccaag gccctgaccg aggtgatccc cctgaccgag 1560
gaggccgagc tggagctggc cgagaaccgc gagatcctga aggagcccgt gcacgaggtg 1620

tactacgacc ccagcaagga cctggtggcc gagatccaga agcagggcca gggccagtgg 1680
acctaccaga tctaccagga gcccttcaag aacctgaaga ccggcaagta cgcccgcatg 1740
cgcggcgccc acaccaacga cgtgaagcag ctgaccgagg ccgtgcagaa ggtgagcacc 1800
gagagcatcg tgatctgggg caagatcccc aagttcaagc tgcccatcca gaaggagacc 1860
tgggaggcct ggtggatgga gtactggcag gccacctgga tccccgagtg ggagttcgtg 1920
aacacccccc ccctggtgaa gctgtggtac cagctggaga aggagcccat cgtgggcgcc 1980
gagaccttct acgtggacgg cgccgccaac cgcgagacca agctgggcaa ggccggctac 2040
gtgaccgacc ggggccggca gaaggtggtg agcatcgccg acaccaccaa ccagaagacc 2100
gagctgcagg ccatccacct ggccctgcag gacagcggcc tggaggtgaa catcgtgacc 2160
gacagccagt acgccctggg catcatccag gcccagcccg acaagagcga gagcgagctg 2220
gtgagccaga tcatcgagca gctgatcaag aaggagaagg tgtacctggc ctgggtgccc 2280
gcccacaagg gcatcggcgg caacgagcag gtggacaagc tggtgagcgc cggcatccgc 2340
aaggtgctgt tcctgaacgg catcgatggc ggcatcgtga tctaccagta catggacgac 2400
ctgtacgtgg gcagcggcgg ccctagggag cccgtggacc cccgcctgga gccctggaag 2460
caccccggca gccagcccaa gaccgccggc accaactgct actgcaagaa gtgctgcttc 2520
cactgccagg tgagcttcat caccaagggc ctgggcatca gctacggccg caagaagcgc 2580
cgccagcgcc gccgcgcccc ccccgacagc gaggtgcacc aggtgagcct gcccaagcag 2640
cccgccagcc agccccaggg cgaccccacc ggccccaagg agagcaagaa gaaggtggag 2700
cgcgagaccg agaccgaccc cgtgcacccc ggggccggcc gcagcggcga cagcgacgag 2760
gagctgctgc agaccgtgcg cttcatcaag ttcctgtacc agagcaaccc cctgcccagc 2820
cccaagggca cccgccaggc cgacctgaac cgccgccgcc gctggcgcga gcgccagcgc 2880
cagatccaga gcatcagcgc ctggatcatc agcacccacc tgggccgcag caccgagccc 2940
gtgcccctgc agctgccccc cgacctgcgc ctgaacctgg actgcagcga ggactgcggc 3000
accagcggca cccagggcgt gggcagcccc caggtgctgg gcgagagccc cgccgtgctg 3060
gacagcggca ccaaggagct cgaggccggc aagtggagca agcgcatgag cggctggagc 3120
gccgtgcgcg agcgcatgaa gcgcgccgag cccgccgagc ccgccgccga cggcgtgggc 3180
gccgtgagcc gcgacctgga gaagcacggc gccatcacca gcagcaacac cgccgccaac 3240
aacgccgact gcgcctggct ggaggcccag gaggacgagg acgtgggctt ccccgtgcgc 3300
ccccaggtgc ccctgcgccc catgacctac aaggccgccc tggacctgag ccacttcctg 3360
aaggagaagg gcggcctgga gggcctgatc tacagccaga agcgccagga catcctggac 3420
ctgtggatcc accacaccca gggctacttc cccggctggc agaactacac ccccggcccc 3480
ggcatccgct accccctgac cttcggctgg tgcttcaagc tggtgcccgt ggaccccgac 3540
tacgtggagg aggccaacgc cggcgagaac aacagcctgc tgcaccccat gagccagcac 3600
ggcatggacg accccgagaa ggaggtgctg gtgtggcgct tcgacagccg cctggccttc 3660
caccacatgg cccgcgagct gcaccccgag tactacaagg actgcgatta aaagcttccc 3720
ggggctagca ccggt 3735

<210> 37
<211> 2445
<212> DNA
<213> Artificial Sequence

<220>
<223> Description of Artificial Sequence:
      p2Pol.opt.YMWM_C

<400> 37
gccaccatgg ccgaggccat gagccaggcc accagcgcca acatcctgat gcagcgcagc 60
aacttcaagg gccccaagcg catcatcaag tgcttcaact gcggcaagga gggccacatc 120
gcccgcaact gccgcgcccc ccgcaagaag ggctgctgga agtgcggcaa ggagggccac 180
cagatgaagg actgcaccga gcgccaggcc aacttcttcc gcgaggacct ggccttcccc 240
cagggcaagg cccgcgagtt ccccagcgag cagaaccgcg ccaacagccc caccagccgc 300
gagctgcagg tgcgcggcga caacccccgc agcgaggccg gcgccgagcg ccagggcacc 360
ctgaacttcc cccagatcac cctgtggcag cgccccctgg tgagcatcaa ggtgggcggc 420

cagatcaagg aggccctgct ggccaccggc gccgacgaca ccgtgctgga ggagatgagc 480
ctgcccggca agtggaagcc caagatgatc ggcggcatcg gcggcttcat caaggtgcgc 540
cagtacgacc agatcctgat cgagatctgc ggcaagaagg ccatcggcac cgtgctgatc 600
ggccccaccc ccgtgaacat catcggccgc aacatgctga cccagctggg ctgcaccctg 660
aacttcccca tcagccccat cgagaccgtg cccgtgaagc tgaagcccgg catggacggc 720
cccaaggtga agcagtggcc cctgaccgag gagaagatca aggccctgac cgccatctgc 780
gaggagatgg agaaggaggg caagatcacc aagatcggcc ccgagaaccc ctacaacacc 840
cccgtgttcg ccatcaagaa gaaggacagc accaagtggc gcaagctggt ggacttccgc 900
gagctgaaca agcgcaccca ggacttctgg gaggtgcagc tgggcatccc ccaccccgcc 960
ggcctgaaga agaagaagag cgtgaccgtg ctggacgtgg gcgacgccta cttcagcgtg 1020
cccctggacg aggacttccg caagtacacc gccttcacca tccccagcat caacaacgag 1080
acccccggca tccgctacca gtacaacgtg ctgccccagg gctggaaggg cagccccagc 1140
atcttccaga gcagcatgac caagatcctg gagcccttcc gcgcccgcaa ccccgagatc 1200
gtgatctacc aggcccccct gtacgtgggc agcgacctgg agatcggcca gcaccgcgcc 1260
aagatcgagg agctgcgcaa gcacctgctg cgctggggct tcaccacccc cgacaagaag 1320
caccagaagg agcccccctt cctgcccatc gagctgcacc ccgacaagtg gaccgtgcag 1380
cccatcgagc tgcccgagaa ggagagctgg accgtgaacg acatccagaa gctggtgggc 1440
aagctgaact gggccagcca gatctacccc ggcatcaagg tgcgccagct gtgcaagctg 1500
ctgcgcggcg ccaaggccct gaccgacatc gtgcccctga ccgaggaggc cgagctggag 1560
ctggccgaga accgcgagat cctgcgcgag cccgtgcacg gcgtgtacta cgaccccagc 1620
aaggacctgg tggccgagat ccagaagcag ggccacgacc agtggaccta ccagatctac 1680
caggagccct tcaagaacct gaagaccggc aagtacgcca agatgcgcac cgcccacacc 1740
aacgacgtga agcagctgac cgaggccgtg cagaagatcg ccatggagag catcgtgatc 1800
tggggcaaga cccccaagtt ccgcctgccc atccagaagg agacctggga gacctggtgg 1860
accgactact ggcaggccac ctggatcccc gagtgggagt tcgtgaacac cccccccctg 1920
gtgaagctgt ggtaccagct ggagaaggag cccatcatcg gcgccgagac cttctacgtg 1980
gacggcgccg ccaaccgcga gaccaagatc ggcaaggccg gctacgtgac cgaccggggc 2040
cggcagaaga tcgtgagcct gaccgagacc accaaccaga agaccgagct gcaggccatc 2100
cagctggccc tgcaggacag cggcagcgag gtgaacatcg tgaccgacag ccagtacgcc 2160
ctgggcatca tccaggccca gcccgacaag agcgagagcg agctggtgaa ccagatcatc 2220
gagcagctga tcaagaagga gaaggtgtac ctgagctggg tgcccgccca caagggcatc 2280
ggcggcaacg agcagatcga caagctggtg agcaagggca tccgcaaggt gctgttcctg 2340
gacggcatcg atggcggcat cgtgatctac cagtacatgg acgacctgta cgtgggcagc 2400
ggcggcccta ggatcgatta aaagcttccc ggggctagca ccggt 2445

<210> 38
<211> 2457
<212> DNA
<213> Artificial Sequence
<220>
<223> Description of Artificial Sequence: p2Polopt.YM_C

<400> 38
gtcgacgcca ccatggccga ggccatgagc caggccacca gcgccaacat cctgatgcag 60
cgcagcaact tcaagggccc caagcgcatc atcaagtgct tcaactgcgg caaggagggc 120
cacatcgccc gcaactgccg cgccccccgc aagaagggct gctggaagtg cggcaaggag 180
ggccaccaga tgaaggactg caccgagcgc caggccaact tcttccgcga ggacctggcc 240
ttcccccagg gcaaggcccg cgagttcccc agcgagcaga accgcgccaa cagccccacc 300
agccgcgagc tgcaggtgcg cggcgacaac ccccgcagcg aggccggcgc cgagcgccag 360
ggcaccctga acttccccca gatcaccctg tggcagcgcc ccctggtgag catcaaggtg 420
ggcggccaga tcaaggaggc cctgctggcc accggcgccg acgacaccgt gctggaggag 480
atgagcctgc ccggcaagtg gaagcccaag atgatcggcg gcatcggcgg cttcatcaag 540
gtgcgccagt acgaccagat cctgatcgag atctgcggca agaaggccat cggcaccgtg 600
ctgatcggcc ccacccccgt gaacatcatc ggccgcaaca tgctgaccca gctgggctgc 660

accctgaact tccccatcag ccccatcgag accgtgcccg tgaagctgaa gcccggcatg 720
gacggcccca aggtgaagca gtggcccctg accgaggaga agatcaaggc cctgaccgcc 780
atctgcgagg agatggagaa ggagggcaag atcaccaaga tcggccccga gaacccctac 840
aacacccccg tgttcgccat caagaagaag gacagcacca agtggcgcaa gctggtggac 900
ttccgcgagc tgaacaagcg cacccaggac ttctgggagg tgcagctggg catcccccac 960
cccgccggcc tgaagaagaa gaagagcgtg accgtgctgg acgtgggcga cgcctacttc 1020
agcgtgcccc tggacgagga cttccgcaag tacaccgcct tcaccatccc cagcatcaac 1080
aacgagaccc ccggcatccg ctaccagtac aacgtgctgc cccagggctg gaagggcagc 1140
cccagcatct tccagagcag catgaccaag atcctggagc ccttccgcgc ccgcaacccc 1200
gagatcgtga tctaccaggc ccccctgtac gtgggcagcg acctggagat cggccagcac 1260
cgcgccaaga tcgaggagct gcgcaagcac ctgctgcgct ggggcttcac cacccccgac 1320
aagaagcacc agaaggagcc ccccttcctg tggatgggct acgagctgca ccccgacaag 1380
tggaccgtgc agcccatcga gctgcccgag aaggagagct ggaccgtgaa cgacatccag 1440
aagctggtgg gcaagctgaa ctgggccagc cagatctacc ccggcatcaa ggtgcgccag 1500
ctgtgcaagc tgctgcgcgg cgccaaggcc ctgaccgaca tcgtgcccct gaccgaggag 1560
gccgagctgg agctggccga gaaccgcgag atcctgcgcg agcccgtgca cggcgtgtac 1620
tacgacccca gcaaggacct ggtggccgag atccagaagc agggccacga ccagtggacc 1680
taccagatct accaggagcc cttcaagaac ctgaagaccg gcaagtacgc caagatgcgc 1740
accgcccaca ccaacgacgt gaagcagctg accgaggccg tgcagaagat cgccatggag 1800
agcatcgtga tctggggcaa gacccccaag ttccgcctgc ccatccagaa ggagacctgg 1860
gagacctggt ggaccgacta ctggcaggcc acctggatcc ccgagtggga gttcgtgaac 1920
accccccccc tggtgaagct gtggtaccag ctggagaagg agcccatcat cggcgccgag 1980
accttctacg tggacggcgc cgccaaccgc gagaccaaga tcggcaaggc cggctacgtg 2040
accgaccggg gccggcagaa gatcgtgagc ctgaccgaga ccaccaacca gaagaccgag 2100
ctgcaggcca tccagctggc cctgcaggac agcggcagcg aggtgaacat cgtgaccgac 2160
agccagtacg ccctgggcat catccaggcc cagcccgaca agagcgagag cgagctggtg 2220
aaccagatca tcgagcagct gatcaagaag gagaaggtgt acctgagctg ggtgcccgcc 2280
cacaagggca tcggcggcaa cgagcagatc gacaagctgg tgagcaaggg catccgcaag 2340
gtgctgttcc tggacggcat cgatggcggc atcgtgatct accagtacat ggacgacctg 2400
tacgtgggca gcggcggccc taggatcgat taaaagcttc ccggggctag caccggt 2457

<210> 39
<211> 2457
<212> DNA
<213> Artificial Sequence

<220>
<223> Description of Artificial Sequence: p2Polopt_C

<400> 39
gccaccatgg ccgaggccat gagccaggcc accagcgcca acatcctgat gcagcgcagc 60
aacttcaagg gccccaagcg catcatcaag tgcttcaact gcggcaagga gggccacatc 120
gcccgcaact gccgcgcccc ccgcaagaag ggctgctgga agtgcggcaa ggagggccac 180
cagatgaagg actgcaccga gcgccaggcc aacttcttcc gcgaggacct ggccttcccc 240
cagggcaagg cccgcgagtt ccccagcgag cagaaccgcg ccaacagccc caccagccgc 300
gagctgcagg tgcgcggcga caacccccgc agcgaggccg gcgccgagcg ccagggcacc 360
ctgaacttcc cccagatcac cctgtggcag cgccccctgg tgagcatcaa ggtgggcggc 420
cagatcaagg aggccctgct ggacaccggc gccgacgaca ccgtgctgga ggagatgagc 480
ctgcccggca agtggaagcc caagatgatc ggcggcatcg gcggcttcat caaggtgcgc 540
cagtacgacc agatcctgat cgagatctgc ggcaagaagg ccatcggcac cgtgctgatc 600
ggccccaccc ccgtgaacat catcggccgc aacatgctga cccagctggg ctgcaccctg 660
aacttcccca tcagccccat cgagaccgtg cccgtgaagc tgaagcccgg catggacggc 720
cccaaggtga agcagtggcc cctgaccgag gagaagatca aggccctgac cgccatctgc 780
gaggagatgg agaaggaggg caagatcacc aagatcggcc ccgagaaccc ctacaacacc 840

cccgtgttcg ccatcaagaa gaaggacagc accaagtggc gcaagctggt ggacttccgc 900
gagctgaaca agcgcaccca ggacttctgg gaggtgcagc tgggcatccc ccaccccgcc 960
ggcctgaaga agaagaagag cgtgaccgtg ctggacgtgg gcgacgccta cttcagcgtg 1020
cccctggacg aggacttccg caagtacacc gccttcacca tccccagcat caacaacgag 1080
acccccggca tccgctacca gtacaacgtg ctgccccagg gctggaaggg cagccccagc 1140
atcttccaga gcagcatgac caagatcctg gagcccttcc gcgcccgcaa ccccgagatc 1200
gtgatctacc agtacatgga cgacctgtac gtgggcagcg acctggagat cggccagcac 1260
cgcgccaaga tcgaggagct gcgcaagcac ctgctgcgct ggggcttcac cacccccgac 1320
aagaagcacc agaaggagcc ccccttcctg tggatgggct acgagctgca ccccgacaag 1380
tggaccgtgc agcccatcga gctgcccgag aaggagagct ggaccgtgaa cgacatccag 1440
aagctggtgg gcaagctgaa ctgggccagc cagatctacc ccggcatcaa ggtgcgccag 1500
ctgtgcaagc tgctgcgcgg cgccaaggcc ctgaccgaca tcgtgcccct gaccgaggag 1560
gccgagctgg agctggccga gaaccgcgag atcctgcgcg agcccgtgca cggcgtgtac 1620
tacgacccca gcaaggacct ggtggccgag atccagaagc agggccacga ccagtggacc 1680
taccagatct accaggagcc cttcaagaac ctgaagaccg gcaagtacgc caagatgcgc 1740
accgcccaca ccaacgacgt gaagcagctg accgaggccg tgcagaagat cgccatggag 1800
agcatcgtga tctggggcaa gacccccaag ttccgcctgc ccatccagaa ggagacctgg 1860
gagacctggt ggaccgacta ctggcaggcc acctggatcc ccgagtggga gttcgtgaac 1920
accccccccc tggtgaagct gtggtaccag ctggagaagg agcccatcat cggcgccgag 1980
accttctacg tggacggcgc cgccaaccgc gagaccaaga tcggcaaggc cggctacgtg 2040
accgaccggg gccggcagaa gatcgtgagc ctgaccgaga ccaccaacca gaagaccgag 2100
ctgcaggcca tccagctggc cctgcaggac agcggcagcg aggtgaacat cgtgaccgac 2160
agccagtacg ccctgggcat catccaggcc cagcccgaca agagcgagag cgagctggtg 2220
aaccagatca tcgagcagct gatcaagaag gagaaggtgt acctgagctg ggtgcccgcc 2280
cacaagggca tcggcggcaa cgagcagatc gacaagctgg tgagcaaggg catccgcaag 2340
gtgctgttcc tggacggcat cgatggcggc atcgtgatct accagtacat ggacgacctg 2400
tacgtgggca gcggcggccc taggatcgat taaaagcttc ccggggctag caccggt 2457

<210> 40
<211> 3597
<212> DNA
<213> Artificial Sequence

<220>
<223> Description of Artificial Sequence: p2PolTatRevNef

<400> 40
gtcgacgcca ccatggagcc cgtggacccc aacctggagc cctggaacca ccccggcagc 60
cagcccaaga ccgccggcaa caagtgctac tgcaagcact gcagctacca ctgcctggtg 120
agcttccaga ccaagggcct gggcatcagc tacggccgca agaagcgccg ccagcgccgc 180
agcgcccccc ccagcagcga ggaccaccag aaccccatca gcaagcagcc cctgccccag 240
acccgcggcg accccaccgg cagcgaggag agcaagaaga aggtggagag caagaccgag 300
accgacccct tcgaccccgg ggccggccgc agcggcgaca gcgacgaggc cctgctgcag 360
gccgtgcgca tcatcaagat cctgtaccag agcaacccct accccaagcc cgagggcacc 420
cgccaggccg acctgaaccg ccgccgccgc tggcgcgccc gccagcgcca gatccacagc 480
atcagcgagc gcatcctgag cacctgcctg ggccgccccg ccgagcccgt gcccttccag 540
ctgccccccg acctgcgcct gcacatcgac tgcagcgaga gcagcggcac cagcggcacc 600
cagcagagcc agggcaccac cgagggcgtg ggcagccccc tcgaggccgg caagtggagc 660
aagagcagca tcgtgggctg gcccgccgtg cgcgagcgca tccgccgcac cgagcccgcc 720
gccgagggcg tgggcgccgc cagccaggac ctggacaagc acggcgccct gaccagcagc 780
aacaccgccg ccaacaacgc cgactgcgcc tggctggagg cccaggagga ggaggaggag 840
gtgggcttcc ccgtgcgccc ccaggtgccc ctgcgcccca tgacctacaa ggccgccttc 900
gacctgagct tcttcctgaa ggagaagggc ggcctggagg gcctgatcta cagcaagaag 960
cgccaggaga tcctggacct gtgggtgtac cacacccagg gcttcttccc cggctggcag 1020

aactacaccc ccggccccgg cgtgcgctac cccctgacct tcggctggtg cttcaagctg 1080
gtgcccgtgg acccccgcga ggtggaggag gccaacaagg gcgagaacaa ctgcctgctg 1140
caccccatga gccagcacgg catggaggac gaggaccgcg aggtgctgaa gtggaagttc 1200
gacagcagcc tggcccgccg ccacatggcc cgcgagctgc accccgagta ctacaaggac 1260
tgcgaattcg ccgaggccat gagccaggcc accagcgcca acatcctgat gcagcgcagc 1320
aacttcaagg gccccaagcg catcatcaag tgcttcaact gcggcaagga gggccacatc 1380
gcccgcaact gccgcgcccc ccgcaagaag ggctgctgga agtgcggcaa ggagggccac 1440
cagatgaagg actgcaccga gcgccaggcc aacttcttcc gcgaggacct ggccttcccc 1500
cagggcaagg cccgcgagtt ccccagcgag cagaaccgcg ccaacagccc caccagccgc 1560
gagctgcagg tgcgcggcga caacccccgc agcgaggccg gcgccgagcg ccagggcacc 1620
ctgaacttcc cccagatcac cctgtggcag cgccccctgg tgagcatcaa ggtgggcggc 1680
cagatcaagg aggccctgct ggccaccggc gccgacgaca ccgtgctgga ggagatgagc 1740
ctgcccggca agtggaagcc caagatgatc ggcggcatcg gcggcttcat caaggtgcgc 1800
cagtacgacc agatcctgat cgagatctgc ggcaagaagg ccatcggcac cgtgctgatc 1860
ggccccaccc ccgtgaacat catcggccgc aacatgctga cccagctggg ctgcaccctg 1920
aacttcccca tcagccccat cgagaccgtg cccgtgaagc tgaagcccgg catggacggc 1980
cccaaggtga agcagtggcc cctgaccgag gagaagatca aggccctgac cgccatctgc 2040
gaggagatgg agaaggaggg caagatcacc aagatcggcc ccgagaaccc ctacaacacc 2100
cccgtgttcg ccatcaagaa gaaggacagc accaagtggc gcaagctggt ggacttccgc 2160
gagctgaaca agcgcaccca ggacttctgg gaggtgcagc tgggcatccc ccaccccgcc 2220
ggcctgaaga agaagaagag cgtgaccgtg ctggacgtgg gcgacgccta cttcagcgtg 2280
cccctggacg aggacttccg caagtacacc gccttcacca tccccagcat caacaacgag 2340
acccccggca tccgctacca gtacaacgtg ctgccccagg gctggaaggg cagccccagc 2400
atcttccaga gcagcatgac caagatcctg gagcccttcc gcgcccgcaa ccccgagatc 2460
gtgatctacc aggcccccct gtacgtgggc agcgacctgg agatcggcca gcaccgcgcc 2520
aagatcgagg agctgcgcaa gcacctgctg cgctggggct tcaccacccc cgacaagaag 2580
caccagaagg agcccccctt cctgcccatc gagctgcacc ccgacaagtg gaccgtgcag 2640
cccatcgagc tgcccgagaa ggagagctgg accgtgaacg acatccagaa gctggtgggc 2700
aagctgaact gggccagcca gatctacccc ggcatcaagg tgcgccagct gtgcaagctg 2760
ctgcgcggcg ccaaggccct gaccgacatc gtgcccctga ccgaggaggc cgagctggag 2820
ctggccgaga accgcgagat cctgcgcgag cccgtgcacg gcgtgtacta cgaccccagc 2880
aaggacctgg tggccgagat ccagaagcag ggccacgacc agtggaccta ccagatctac 2940
caggagccct tcaagaacct gaagaccggc aagtacgcca agatgcgcac cgcccacacc 3000
aacgacgtga agcagctgac cgaggccgtg cagaagatcg ccatggagag catcgtgatc 3060
tggggcaaga cccccaagtt ccgcctgccc atccagaagg agacctggga gacctggtgg 3120
accgactact ggcaggccac ctggatcccc gagtgggagt tcgtgaacac cccccccctg 3180
gtgaagctgt ggtaccagct ggagaaggag cccatcatcg gcgccgagac cttctacgtg 3240
gacggcgccg ccaaccgcga gaccaagatc ggcaaggccg gctacgtgac cgaccggggc 3300
cggcagaaga tcgtgagcct gaccgagacc accaaccaga agaccgagct gcaggccatc 3360
cagctggccc tgcaggacag cggcagcgag gtgaacatcg tgaccgacag ccagtacgcc 3420
ctgggcatca tccaggccca gcccgacaag agcgagagcg agctggtgaa ccagatcatc 3480
gagcagctga tcaagaagga gaaggtgtac ctgagctggg tgcccgccca caagggcatc 3540
ggcggcaacg agcagatcga caagctggtg agcaagggca tccgcaaggt gctgtaa 3597

<210> 41
<211> 3624
<212> DNA
<213> Artificial Sequence

<220>
<223> Description of Artificial Sequence:
      p2PolTatRevNef.opt.native_C

<400> 41

gccaccatgg ccgaggccat gagccaggcc accagcgcca acatcctgat gcagcgcagc 60
aacttcaagg gccccaagcg catcatcaag tgcttcaact gcggcaagga gggccacatc 120
gcccgcaact gccgcgcccc ccgcaagaag ggctgctgga agtgcggcaa ggagggccac 180
cagatgaagg actgcaccga gcgccaggcc aacttcttcc gcgaggacct ggccttcccc 240
cagggcaagg cccgcgagtt ccccagcgag cagaaccgcg ccaacagccc caccagccgc 300
gagctgcagg tgcgcggcga caacccccgc agcgaggccg gcgccgagcg ccagggcacc 360
ctgaacttcc cccagatcac cctgtggcag cgccccctgg tgagcatcaa ggtgggcggc 420
cagatcaagg aggccctgct ggacaccggc gccgacgaca ccgtgctgga ggagatgagc 480
ctgcccggca agtggaagcc caagatgatc ggcggcatcg gcggcttcat caaggtgcgc 540
cagtacgacc agatcctgat cgagatctgc ggcaagaagg ccatcggcac cgtgctgatc 600
ggccccaccc ccgtgaacat catcggccgc aacatgctga cccagctggg ctgcaccctg 660
aacttcccca tcagccccat cgagaccgtg cccgtgaagc tgaagcccgg catggacggc 720
cccaaggtga agcagtggcc cctgaccgag gagaagatca aggccctgac cgccatctgc 780
gaggagatgg agaaggaggg caagatcacc aagatcggcc ccgagaaccc ctacaacacc 840
cccgtgttcg ccatcaagaa gaaggacagc accaagtggc gcaagctggt ggacttccgc 900
gagctgaaca agcgcaccca ggacttctgg gaggtgcagc tgggcatccc ccaccccgcc 960
ggcctgaaga agaagaagag cgtgaccgtg ctggacgtgg gcgacgccta cttcagcgtg 1020
cccctggacg aggacttccg caagtacacc gccttcacca tccccagcat caacaacgag 1080
acccccggca tccgctacca gtacaacgtg ctgccccagg gctggaaggg cagccccagc 1140
atcttccaga gcagcatgac caagatcctg gagcccttcc gcgcccgcaa ccccgagatc 1200
gtgatctacc agtacatgga cgacctgtac gtgggcagcg acctggagat cggccagcac 1260
cgcgccaaga tcgaggagct gcgcaagcac ctgctgcgct ggggcttcac cacccccgac 1320
aagaagcacc agaaggagcc ccccttcctg tggatgggct acgagctgca ccccgacaag 1380
tggaccgtgc agcccatcga gctgcccgag aaggagagct ggaccgtgaa cgacatccag 1440
aagctggtgg gcaagctgaa ctgggccagc cagatctacc ccggcatcaa ggtgcgccag 1500
ctgtgcaagc tgctgcgcgg cgccaaggcc ctgaccgaca tcgtgcccct gaccgaggag 1560
gccgagctgg agctggccga gaaccgcgag atcctgcgcg agcccgtgca cggcgtgtac 1620
tacgacccca gcaaggacct ggtggccgag atccagaagc agggccacga ccagtggacc 1680
taccagatct accaggagcc cttcaagaac ctgaagaccg gcaagtacgc caagatgcgc 1740
accgcccaca ccaacgacgt gaagcagctg accgaggccg tgcagaagat cgccatggag 1800
agcatcgtga tctggggcaa gacccccaag ttccgcctgc ccatccagaa ggagacctgg 1860
gagacctggt ggaccgacta ctggcaggcc acctggatcc ccgagtggga gttcgtgaac 1920
accccccccc tggtgaagct gtggtaccag ctggagaagg agcccatcat cggcgccgag 1980
accttctacg tggacggcgc cgccaaccgc gagaccaaga tcggcaaggc cggctacgtg 2040
accgaccggg gccggcagaa gatcgtgagc ctgaccgaga ccaccaacca gaagaccgag 2100
ctgcaggcca tccagctggc cctgcaggac agcggcagcg aggtgaacat cgtgaccgac 2160
agccagtacg ccctgggcat catccaggcc cagcccgaca agagcgagag cgagctggtg 2220
aaccagatca tcgagcagct gatcaagaag gagaaggtgt acctgagctg ggtgcccgcc 2280
cacaagggca tcggcggcaa cgagcagatc gacaagctgg tgagcaaggg catccgcaag 2340
gtgctggaat tcgagcccgt ggaccccaac ctggagccct ggaaccaccc cggcagccag 2400
cccaagaccg cctgcaacaa gtgctactgc aagcactgca gctaccactg cctggtgtgc 2460
ttccagacca agggcctggg catcagctac ggccgcaaga agcgccgcca gcgccgcagc 2520
gcccccccca gcagcgagga ccaccagaac cccatcagca agcagcccct gccccagacc 2580
cgcggcgacc ccaccggcag cgaggagagc aagaagaagg tggagagcaa gaccgagacc 2640
gaccccttcg accccggggc cggccgcagc ggcgacagcg acgaggccct gctgcaggcc 2700
gtgcgcatca tcaagatcct gtaccagagc aacccctacc ccaagcccga gggcacccgc 2760
caggcccgca agaaccgccg ccgccgctgg cgcgcccgcc agcgccagat ccacagcatc 2820
agcgagcgca tcctgagcac ctgcctgggc cgccccgccg agcccgtgcc cttccagctg 2880
ccccccatcg agcgcctgca catcgactgc agcgagagca gcggcaccag cggcacccag 2940
cagagccagg gcaccaccga gggcgtgggc agccccctcg agggcggcaa gtggagcaag 3000
agcagcatcg tgggctggcc cgccgtgcgc gagcgcatcc gccgcaccga gcccgcccgc 3060
gagggcgccg ccgagggcgc cgccgagggc gtgggcgccg ccagccagga cctggacaag 3120
cacggcgccc tgaccagcag caacaccgcc gccaacaacg ccgactgcgc ctggctggag 3180
gcccaggagg aggaggagga ggtgggcttc cccgtgcgcc cccaggtgcc cctgcgcccc 3240

atgacctaca aggccgcctt cgacctgagc ttcttcctga aggagaaggg cggcctggag 3300
ggcctgatct acagcaagaa gcgccaggag atcctggacc tgtgggtgta ccacacccag 3360
ggcttcttcc ccgactggca gaactacacc cccggccccg gcgtgcgcta ccccctgacc 3420
ttcggctggt gcttcaagct ggtgcccgtg gacccccgcg aggtggagga ggccaacaag 3480
ggcgagaaca actgcctgct gcaccccatg agccagcacg gcatggagga cgaggaccgc 3540
gaggtgctga agtggaagtt cgacagcagc ctggcccgcc gccacatggc ccgcgagctg 3600
caccccgagt actacaagga ctgc 3624

<210> 42
<211> 3607
<212> DNA
<213> Artificial Sequence

<220>
<223> Description of Artificial Sequence:
      p2PolTatRevNef.opt_C

<400> 42
gtcgacgcca ccatggccga ggccatgagc caggccacca gcgccaacat cctgatgcag 60
cgcagcaact tcaagggccc caagcgcatc atcaagtgct tcaactgcgg caaggagggc 120
cacatcgccc gcaactgccg cgccccccgc aagaagggct gctggaagtg cggcaaggag 180
ggccaccaga tgaaggactg caccgagcgc caggccaact tcttccgcga ggacctggcc 240
ttcccccagg gcaaggcccg cgagttcccc agcgagcaga accgcgccaa cagccccacc 300
agccgcgagc tgcaggtgcg cggcgacaac ccccgcagcg aggccggcgc cgagcgccag 360
ggcaccctga acttccccca gatcaccctg tggcagcgcc ccctggtgag catcaaggtg 420
ggcggccaga tcaaggaggc cctgctggcc accggcgccg acgacaccgt gctggaggag 480
atgagcctgc ccggcaagtg gaagcccaag atgatcggcg gcatcggcgg cttcatcaag 540
gtgcgccagt acgaccagat cctgatcgag atctgcggca agaaggccat cggcaccgtg 600
ctgatcggcc ccacccccgt gaacatcatc ggccgcaaca tgctgaccca gctgggctgc 660
accctgaact tccccatcag ccccatcgag accgtgcccg tgaagctgaa gcccggcatg 720
gacggcccca aggtgaagca gtggcccctg accgaggaga agatcaaggc cctgaccgcc 780
atctgcgagg agatggagaa ggagggcaag atcaccaaga tcggccccga gaacccctac 840
aacacccccg tgttcgccat caagaagaag gacagcacca agtggcgcaa gctggtggac 900
ttccgcgagc tgaacaagcg cacccaggac ttctgggagg tgcagctggg catcccccac 960
cccgccggcc tgaagaagaa gaagagcgtg accgtgctgg acgtgggcga cgcctacttc 1020
agcgtgcccc tggacgagga cttccgcaag tacaccgcct tcaccatccc cagcatcaac 1080
aacgagaccc ccggcatccg ctaccagtac aacgtgctgc cccagggctg gaagggcagc 1140
cccagcatct tccagagcag catgaccaag atcctggagc ccttccgcgc ccgcaacccc 1200
gagatcgtga tctaccaggc ccccctgtac gtgggcagcg acctggagat cggccagcac 1260
cgcgccaaga tcgaggagct gcgcaagcac ctgctgcgct ggggcttcac cacccccgac 1320
aagaagcacc agaaggagcc ccccttcctg cccatcgagc tgcaccccga caagtggacc 1380
gtgcagccca tcgagctgcc cgagaaggag agctggaccg tgaacgacat ccagaagctg 1440
gtgggcaagc tgaactgggc cagccagatc taccccggca tcaaggtgcg ccagctgtgc 1500
aagctgctgc gcggcgccaa ggccctgacc gacatcgtgc ccctgaccga ggaggccgag 1560
ctggagctgg ccgagaaccg cgagatcctg cgcgagcccg tgcacggcgt gtactacgac 1620
cccagcaagg acctggtggc cgagatccag aagcagggcc acgaccagtg gacctaccag 1680
atctaccagg agcccttcaa gaacctgaag accggcaagt acgccaagat gcgcaccgcc 1740
cacaccaacg acgtgaagca gctgaccgag gccgtgcaga agatcgccat ggagagcatc 1800
gtgatctggg gcaagacccc caagttccgc ctgcccatcc agaaggagac ctgggagacc 1860
tggtggaccg actactggca ggccacctgg atccccgagt gggagttcgt gaacaccccc 1920
cccctggtga agctgtggta ccagctggag aaggagccca tcatcggcgc cgagaccttc 1980
tacgtggacg gcgccgccaa ccgcgagacc aagatcggca aggccggcta cgtgaccgac 2040
cggggccggc agaagatcgt gagcctgacc gagaccacca accagaagac cgagctgcag 2100
gccatccagc tggccctgca ggacagcggc agcgaggtga acatcgtgac cgacagccag 2160

tacgccctgg gcatcatcca ggcccagccc gacaagagcg agagcgagct ggtgaaccag 2220
atcatcgagc agctgatcaa gaaggagaag gtgtacctga gctgggtgcc cgcccacaag 2280
ggcatcggcg gcaacgagca gatcgacaag ctggtgagca agggcatccg caaggtgctg 2340
gaattcgagc ccgtggaccc caacctggag ccctggaacc accccggcag ccagcccaag 2400
accgccggca acaagtgcta ctgcaagcac tgcagctacc actgcctggt gagcttccag 2460
accaagggcc tgggcatcag ctacggccgc aagaagcgcc gccagcgccg cagcgccccc 2520
cccagcagcg aggaccacca gaaccccatc agcaagcagc ccctgcccca gacccgcggc 2580
gaccccaccg gcagcgagga gagcaagaag aaggtggaga gcaagaccga gaccgacccc 2640
ttcgaccccg ggccggccgg cagcggcgac agcgacgagg ccctgctgca ggccgtgcgc 2700
atcatcaaga tcctgtacca gagcaacccc taccccaagc ccgagggcac ccgccaggcc 2760
gacctgaacc gccgccgccg ctggcgcgcc cgccagcgcc agatccacag catcagcgag 2820
cgcatcctga gcacctgcct gggccgcccc gccgagcccg tgcccttcca gctgcccccc 2880
gacctgcgcc tgcacatcga ctgcagcgag agcagcggca ccagcggcac ccagcagagc 2940
cagggcacca ccgagggcgt gggcagcccc ctcgaggccg gcaagtggag caagagcagc 3000
atcgtgggct ggcccgccgt gcgcgagcgc atccgccgca ccgagcccgc cgccgagggc 3060
gtgggcgccg ccagccagga cctggacaag cacggcgccc tgaccagcag caacaccgcc 3120
gccaacaacg ccgactgcgc ctggctggag gcccaggagg aggaggagga ggtgggcttc 3180
cccgtgcgcc cccaggtgcc cctgcgcccc atgacctaca aggccgcctt cgacctgagc 3240
ttcttcctga aggagaaggg cggcctggag ggcctgatct acagcaagaa gcgccaggag 3300
atcctggacc tgtgggtgta ccacacccag ggcttcttcc ccggctggca gaactacacc 3360
cccggccccg gcgtgcgcta ccccctgacc ttcggctggt gcttcaagct ggtgcccgtg 3420
gacccccgcg aggtggagga ggccaacaag ggcgagaaca actgcctgct gcaccccatg 3480
agccagcacg gcatggagga cgaggaccgc gaggtgctga agtggaagtt cgacagcagc 3540
ctggcccgcc gccacatggc ccgcgagctg caccccgagt actacaagga ctgcgcctaa 3600
atctaga 3607

<210> 43
<211> 2145
<212> DNA
<213> Artificial Sequence

<220>
<223> Description of Artificial Sequence: pol.opt.SF2

<400> 43
ttcttccgcg aggacctggc cttcctgcag ggcaaggccc gcgagttcag cagcgagcag 60
acccgcgcca acagccccac ccgccgcgag ctgcaggtgt ggggcggcga gaacaacagc 120
ctgagcgagg ccggcgccga ccgccagggc accgtgagct tcaacttccc ccagatcacc 180
ctgtggcagc gccccctggt gaccatcagg atcggcggcc agctcaagga ggcgctgctc 240
gacaccggcg ccgacgacac cgtgctggag gagatgaacc tgcccggcaa gtggaagccc 300
aagatgatcg gcgggatcgg gggcttcatc aaggtgcggc agtacgacca gatccccgtg 360
gagatctgcg gccacaaggc catcggcacc gtgctggtgg gccccacccc cgtgaacatc 420
atcggccgca acctgctgac ccagatcggc tgcaccctga acttccccat cagccccatc 480
gagacggtgc ccgtgaagct gaagccgggg atggacggcc ccaaggtcaa gcagtggccc 540
ctgaccgagg agaagatcaa ggccctggtg gagatctgca ccgagatgga gaaggagggc 600
aagatcagca agatcggccc cgagaacccc tacaacaccc ccgtgttcgc catcaagaag 660
aaggacagca ccaagtggcg caagctggtg gacttccgcg agctgaacaa gcgcacccag 720
gacttctggg aggtgcagct gggcatcccc caccccgccg gcctgaagaa gaagaagagc 780
gtgaccgtgc tggacgtggg cgacgcctac ttcagcgtgc ccctggacaa ggacttccgc 840
aagtacaccg ccttcaccat ccccagcatc aacaacgaga cccccggcat ccgctaccag 900
tacaacgtgc tgccccaggg ctggaagggc agccccgcca tcttccagag cagcatgacc 960
aagatcctgg agcccttccg caagcagaac cccgacatcg tgatctacca gtacatggac 1020
gacctgtacg tgggcagcga cctggagatc ggccagcacc gcaccaagat cgaggagctg 1080
cgccagcacc tgctgcgctg gggcttcacc acccccgaca agaagcacca gaaggagccc 1140

cccttcctgt ggatgggcta cgagctgcac cccgacaagt ggaccgtgca gcccatcatg 1200
ctgcccgaga aggacagctg gaccgtgaac gacatccaga agctggtggg caagctgaac 1260
tgggccagcc agatctacgc cggcatcaag gtgaagcagc tgtgcaagct gctgcgcggc 1320
accaaggccc tgaccgaggt gatccccctg accgaggagg ccgagctgga gctggccgag 1380
aaccgcgaga tcctgaagga gcccgtgcac gaggtgtact acgaccccag caaggacctg 1440
gtggccgaga tccagaagca gggccagggc cagtggacct accagatcta ccaggagccc 1500
ttcaagaacc tgaagaccgg caagtacgcc cgcatgcgcg gcgcccacac caacgacgtg 1560
aagcagctga ccgaggccgt gcagaaggtg agcaccgaga gcatcgtgat ctggggcaag 1620
atccccaagt tcaagctgcc catccagaag gagacctggg aggcctggtg gatggagtac 1680
tggcaggcca cctggatccc cgagtgggag ttcgtgaaca ccccccccct ggtgaagctg 1740
tggtaccagc tggagaagga gcccatcgtg ggcgccgaga ccttctacgt ggacggcgcc 1800
gccaaccgcg agaccaagct gggcaaggcc ggctacgtga ccgaccgggg ccggcagaag 1860
gtggtgagca tcgccgacac caccaaccag aagaccgagc tgcaggccat ccacctggcc 1920
ctgcaggaca gcggcctgga ggtgaacatc gtgaccgaca gccagtacgc cctgggcatc 1980
atccaggccc agcccgacaa gagcgagagc gagctggtga gccagatcat cgagcagctg 2040
atcaagaagg agaaggtgta cctggcctgg gtgcccgccc acaagggcat cggcggcaac 2100
gagcaggtgg acaagctggt gagcgccggc atccgcaagg tgctg 2145

<210> 44
<211> 3015
<212> DNA
<213> Artificial Sequence

<220>
<223> Description of Artificial Sequence:
      Pol_TV1_C_ZAopt

<400> 44
ttcttccgcg agaacctggc cttcccccag ggcgaggccc gcgagttccc ccccgagcag 60
acccgcgcca acagccccac cagccgcacc aacagcccca ccagccgcga gctgcaggtg 120
cgcggcgaca acccccgcgc cgaggagggc gagcgcgagg gcaccttcaa cttcccccag 180
atcaccctgt ggcagcgccc cctggtgagc atcaaggtgg agggccagat caaggaggcc 240
ctgctggaca ccggcgccga cgacaccgtg ctggaggaga tcgacctgcc cggcaagtgg 300
aagcccaaga tgatcggcgg catcggcggc ttcatcaagg tgcgccagta cgaccagatc 360
ctgatcgaga tctgcggcaa gaaggccatc ggcaccgtgc tggtgggccc cacccccgtg 420
aacatcatcg gccgcaacct gctgacccag ctgggctgca ccctgaactt ccccatcagc 480
cccatcgaga ccgtgcccgt gaagctgaag cccggcatgg acggccccaa ggtgaagcag 540
tggcccctga ccgaggagaa gatcaaggcc ctgaccgcca tctgcgagga gatggagaag 600
gagggcaaga tcaccaagat cggccccgac aacccctaca acacccccgt gttcgccatc 660
aagaagaagg acagcaccaa gtggcgcaag ctggtggact tccgcgagct gaacaagcgc 720
acccaggact tctgggaggt gcagctgggc atcccccacc ccgccggcct gaagaagaag 780
aagagcgtga ccgtgctgga cgtgggcgac gcctacttca gcgtgcccct ggacgagagc 840
ttccgcaagt acaccgcctt caccatcccc agcatcaaca acgagacccc cggcatccgc 900
taccagtaca acgtgctgcc ccagggctgg aagggcagcc ccgccatctt ccagagcagc 960
atgaccaaga tcctggagcc cttccgcgcc aagaaccccg acatcgtgat ctaccagtac 1020
atggacgacc tgtacgtggg cagcgacctg gagatcggcc agcaccgcgc caagatcgag 1080
gagctgcgcg agcacctgct gaagtggggc ttcaccaccc ccgacaagaa gcaccagaag 1140
gagcccccct tcctgtggat gggctacgag ctgcaccccg acaagtggac cgtgcagccc 1200
atcctgctgc ccgagaagga cagctggacc gtgaacgaca tccagaagct ggtgggcaag 1260
ctgaactggg ccagccagat ctaccccggc atcaaggtgc gccagctgtg caagctgctg 1320
cgcggcgcca aggccctgac cgacatcgtg cccctgaccg aggaggccga gctggagctg 1380
gccgagaacc gcgagatcct gcgcgagccc gtgcacggcg tgtactacga ccccagcaag 1440
gacctgatcg ccgagatcca gaagcagggc cacgagcagt ggacctacca gatctaccag 1500
gagcccttca agaacctgaa gaccggcaag tacgccaaga tgcgcaccac ccacaccaac 1560

gacgtgaagc agctgaccga ggccgtgcag aagatcgcca tggagagcat cgtgatctgg 1620
ggcaagaccc ccaagttccg cctgcccatc cagaaggaga cctgggagac ctggtggacc 1680
gactactggc aggccacctg gatccccgag tgggagttcg tgaacacccc ccccctggtg 1740
aagctgtggt accagctgga gaaggacccc atcgccggcg tggagacctt ctacgtggac 1800
ggcgccacca accgcgaggc caagatcggc aaggccggct acgtgaccga ccgcggccgc 1860
cagaagatcg tgaccctgac caacaccacc aaccagaaga ccgagctgca ggccatccag 1920
ctggccctgc aggacagcgg cagcgaggtg aacatcgtga ccgacagcca gtacgccctg 1980
ggcatcatcc aggcccagcc cgacaagagc gacagcgaga tcttcaacca gatcatcgag 2040
cagctgatca acaaggagcg catctacctg agctgggtgc ccgcccacaa gggcatcggc 2100
ggcaacgagc aggtggacaa gctggtgagc aagggcatcc gcaaggtgct gttcctggac 2160
ggcatcgaca aggcccagga ggagcacgag cgctaccaca gcaactggcg cgccatggcc 2220
aacgagttca acctgccccc catcgtggcc aaggagatcg tggccagctg cgacaagtgc 2280
cagctgaagg gcgaggccat ccacggccag gtggactgca gccccggcat ctggcagctg 2340
gactgcaccc acctggaggg caagatcatc ctggtggccg tgcacgtggc cagcggctac 2400
atggaggccg aggtgatccc cgccgagacc ggccaggaga ccgcctactt catcctgaag 2460
ctggccggcc gctggcccgt gaaggtgatc cacaccgaca acggcagcaa cttcaccagc 2520
accgccgtga aggccgcctg ctggtgggcc ggcatccagc aggagttcgg catcccctac 2580
aacccccaga gccagggcgt ggtggagagc atgaacaagg agctgaagaa gatcatcggc 2640
caggtgcgcg accaggccga gcacctgaag accgccgtgc agatggccgt gttcatccac 2700
aacttcaagc gcaagggcgg catcggcggc tacagcgccg gcgagcgcat catcgacatc 2760
atcgccaccg acatccagac caaggagctg cagaagcaga tcatccgcat ccagaacttc 2820
cgcgtgtact accgcgacag ccgcgacccc atctggaagg gccccgccga gctgctgtgg 2880
aagggcgagg gcgtggtggt gatcgaggac aagggcgaca tcaaggtggt gccccgccgc 2940
aaggccaaga tcatccgcga ctacggcaag cagatggccg gcgccgactg cgtggccggc 3000
ggccaggacg aggac 3015

<210> 45
<211> 3009
<212> DNA
<213> Artificial Sequence

<220>
<223> Description of Artificial Sequence:
      Pol_TV2_C_ZAopt

<400> 45
ttcttccgcg agaacctggc cttcccccag ggcgaggccc gcgagttccc cagcgagcag 60
acccgcgcca acagccccac cacccgcacc aacagcccca ccagccgcga gctgcaggtg 120
cagggcgaca gcgaggccgg cgccgagcgc cagggcacct tcaacttccc ccagatcacc 180
ctgtggcagc gccccctggt gagcatcaag gtggccggcc agaccaagga ggccctgctg 240
gacaccggcg ccgacgacac cgtgctggag gagatcaacc tgcccggcaa gtggaagccc 300
aagatgatcg gcggcatcgg cggcttcatc aaggtgcgcc agtacgacca gatcctgatc 360
gagatctgcg gcaagcgcgc catcggcacc gtgctggtgg gccccacccc cgtgaacatc 420
atcggccgca acctgctgac ccagctgggc tgcaccctga acttccccat cagccccatc 480
gagaccgtgc ccgtgaagct gaagcccggc atggacggcc ccaaggtgaa gcagtggccc 540
ctgaccgagg agaagatcaa ggccctgacc gagatctgcg aggagatgga gaaggagggc 600
aagatcacca agatcggccc cgagaacccc tacaacaccc ccgtgttcgc catcaagaag 660
aaggacagca ccaagtggcg caagctggtg gacttccgcg agctgaacaa gcgcacccag 720
gacttctggg aggtgcagct gggcatcccc caccccgccg gcctgaagaa gaagaagagc 780
gtgaccgtgc tggacgtggg cgacgcctac ttcagcgtgc ccctggacga gagcttccgc 840
aagtacaccg ccttcaccat ccccagcatc aacaacgaga cccccggcat ccgctaccag 900
tacaacgtgc tgccccaggg ctggaagggc agccccgcca tcttccagag cagcatgacc 960
cgcatcctgg agcccttccg cacccagaac cccgaggtgg tgatctacca gtacatggac 1020
gacctgtacg tgggcagcga cctggagatc ggccagcacc gcgccaagat cgaggagctg 1080

cgcggccacc tgctgaagtg gggcttcacc acccccgaca agaagcacca gaaggagccc 1140
cccttcctgt ggatgggcta cgagctgcac cccgacaagt ggaccgtgca gcccatccag 1200
ctgcccgaga aggagagctg gaccgtgaac gacatccaga agctggtggg caagctgaac 1260
tgggccagcc agatctaccc cggcatcaag gtgcgccagc tgtgcaagct gctgcgcggc 1320
gccaaggccc tgaccgacat cgtgcccctg accgaggagg ccgagctgga gctggccgag 1380
aaccgcgaga tcctgaagga gcccgtgcac ggcgtgtact acgaccccag caaggacctg 1440
atcgccgaga tccagaagca gggcaacgac cagtggacct accagatcta ccaggagccc 1500
ttcaagaacc tgcgcaccgg caagtacgcc aagatgcgca ccgcccacac caacgacgtg 1560
aagcagctgg ccgaggccgt gcagaagatc acccaggaga gcatcgtgat ctggggcaag 1620
acccccaagt tccgcctgcc catccccaag gagacctggg agacctggtg gagcgactac 1680
tggcaggcca cctggatccc cgagtgggag ttcgtgaaca ccccccccct ggtgaagctg 1740
tggtaccagc tggagaagga gcccatcgtg ggcgccgaga ccttctacgt ggacggcgcc 1800
gccaaccgcg agaccaagat cggcaaggcc ggctacgtga ccgacaaggg ccgccagaag 1860
gtggtgagct tcaccgagac caccaaccag aagaccgagc tgcaggccat ccagctggcc 1920
ctgcaggaca gcggccccga ggtgaacatc gtgaccgaca gccagtacgc cctgggcatc 1980
atccaggccc agcccgacaa gagcgagagc gagctggtga gccagatcat cgagcagctg 2040
atcaagaagg agaaggtgta cctgagctgg gtgcccgccc acaagggcat cggcggcaac 2100
gagcaggtgg acaagctggt gagcagcggc atccgcaagg tgctgttcct ggacggcatc 2160
gacaaggccc aggaggagca cgagaagtac cacagcaact ggcgcgccat ggccagcgag 2220
ttcaacctgc cccccatcgt ggccaaggag atcgtggcca gctgcgacaa gtgccagctg 2280
aagggcgagg ccatgcacgg ccaggtggac tgcagccccg gcatctggca gctggactgc 2340
acccacctgg agggcaagat catcctggtg gccgtgcacg tggccagcgg ctacatggag 2400
gccgaggtga tccccgccga gaccggccag gagaccgcct acttcatcct gaagctggcc 2460
ggccgctggc ccgtgaaggt gatccacacc gacaacggca gcaacttcac cagcaccgcc 2520
gtgaaggccg cctgctggtg ggccgacatc cagcgcgagt tcggcatccc ctacaacccc 2580
cagagccagg gcgtggtgga gagcatgaac aaggagctga agaagatcat cggccaggtg 2640
cgcgaccagg ccgagcacct gaagaccgcc gtgcagatgg ccgtgttcat ccacaacttc 2700
aagcgcaagg gcggcatcgg cggctacagc gccggcgagc gcatcatcga catcatcgcc 2760
agcgacatcc agaccaagga gctgcagaag cagatcatca agatccagaa cttccgcgtg 2820
tactaccgcg acagccgcga ccccatctgg aagggccccg ccaagctgct gtggaagggc 2880
gagggcgccg tggtgatcca ggacaacagc gacatcaagg tggtgccccg ccgcaaggcc 2940
aagatcatca aggactacgg caagcagatg gccggcgccg actgcgtggc cggccgccag 3000
gacgaggac 3009

<210> 46
<211> 297
<212> DNA
<213> Artificial Sequence

<220>
<223> Description of Artificial Sequence: prot.opt.SF2

<400> 46
ccccagatca ccctgtggca gcgccccctg gtgaccatca ggatcggcgg ccagctcaag 60
gaggcgctgc tcgacaccgg cgccgacgac accgtgctgg aggagatgaa cctgcccggc 120
aagtggaagc ccaagatgat cggcgggatc gggggcttca tcaaggtgcg gcagtacgac 180
cagatccccg tggagatctg cggccacaag gccatcggca ccgtgctggt gggccccacc 240
cccgtgaaca tcatcggccg caacctgctg acccagatcg gctgcaccct gaacttc 297

<210> 47
<211> 297
<212> DNA
<213> Artificial Sequence

<220>
<223> Description of Artificial Sequence:
      protIna.opt.SF2

<400> 47
ccccagatca ccctgtggca gcgccccctg gtgaccatca ggatcggcgg ccagctcaag 60
gaggcgctgc tcgccaccgg cgccgacgac accgtgctgg aggagatgaa cctgcccggc 120
aagtggaagc ccaagatgat cggcgggatc gggggcttca tcaaggtgcg gcagtacgac 180
cagatccccg tggagatctg cggccacaag gccatcggca ccgtgctggt gggccccacc 240
cccgtgaaca tcatcggccg caacctgctg acccagatcg gctgcaccct gaacttc    297

<210> 48
<211> 1971
<212> DNA
<213> Artificial Sequence

<220>
<223> Description of Artificial Sequence:
      protInaRT.YM.opt.SF2

<400> 48
ccccagatca ccctgtggca gcgccccctg gtgaccatca ggatcggcgg ccagctcaag 60
gaggcgctgc tcgccaccgg cgccgacgac accgtgctgg aggagatgaa cctgcccggc 120
aagtggaagc ccaagatgat cggcgggatc gggggcttca tcaaggtgcg gcagtacgac 180
cagatccccg tggagatctg cggccacaag gccatcggca ccgtgctggt gggccccacc 240
cccgtgaaca tcatcggccg caacctgctg acccagatcg gctgcaccct gaacttcccc 300
atcagcccca tcgagacggt gcccgtgaag ctgaagccgg ggatggacgg ccccaaggtc 360
aagcagtggc ccctgaccga ggagaagatc aaggccctgg tggagatctg caccgagatg 420
gagaaggagg gcaagatcag caagatcggc cccgagaacc cctacaacac ccccgtgttc 480
gccatcaaga agaaggacag caccaagtgg cgcaagctgg tggacttccg cgagctgaac 540
aagcgcaccc aggacttctg ggaggtgcag ctgggcatcc cccaccccgc cggcctgaag 600
aagaagaaga gcgtgaccgt gctggacgtg ggcgacgcct acttcagcgt gcccctggac 660
aaggacttcc gcaagtacac cgccttcacc atccccagca tcaacaacga gacccccggc 720
atccgctacc agtacaacgt gctgccccag ggctggaagg gcagccccgc catcttccag 780
agcagcatga ccaagatcct ggagcccttc cgcaagcaga accccgacat cgtgatctac 840
caggcccccc tgtacgtggg cagcgacctg gagatcggcc agcaccgcac caagatcgag 900
gagctgcgcc agcacctgct gcgctggggc ttcaccaccc ccgacaagaa gcaccagaag 960
gagcccccct tcctgtggat gggctacgag ctgcaccccg acaagtggac cgtgcagccc 1020
atcatgctgc ccgagaagga cagctggacc gtgaacgaca tccagaagct ggtgggcaag 1080
ctgaactggg ccagccagat ctacgccggc atcaaggtga agcagctgtg caagctgctg 1140
cgcggcacca aggccctgac cgaggtgatc cccctgaccg aggaggccga gctggagctg 1200
gccgagaacc gcgagatcct gaaggagccc gtgcacgagg tgtactacga ccccagcaag 1260
gacctggtgg ccgagatcca gaagcagggc cagggccagt ggacctacca gatctaccag 1320
gagcccttca agaacctgaa gaccggcaag tacgcccgca tgcgcggcgc ccacaccaac 1380
gacgtgaagc agctgaccga ggccgtgcag aaggtgagca ccgagagcat cgtgatctgg 1440
ggcaagatcc ccaagttcaa gctgcccatc cagaaggaga cctgggaggc ctggtggatg 1500
gagtactggc aggccacctg gatccccgag tgggagttcg tgaacacccc ccccctggtg 1560
aagctgtggt accagctgga gaaggagccc atcgtgggcg ccgagacctt ctacgtggac 1620
ggcgccgcca accgcgagac caagctgggc aaggccggct acgtgaccga ccggggccgg 1680
cagaaggtgg tgagcatcgc cgacaccacc aaccagaaga ccgagctgca ggccatccac 1740
ctggccctgc aggacagcgg cctggaggtg aacatcgtga ccgacagcca gtacgccctg 1800
ggcatcatcc aggcccagcc cgacaagagc gagagcgagc tggtgagcca gatcatcgag 1860
cagctgatca agaaggagaa ggtgtacctg gcctgggtgc ccgcccacaa gggcatcggc 1920
ggcaacgagc aggtggacaa gctggtgagc gccggcatcc gcaaggtgct g          1971

<210> 49
<211> 2262
<212> DNA
<213> Artificial Sequence

<220>
<223> Description of Artificial Sequence:
      protInaRT.YMWM.opt.SF2

<400> 49
ccccagatca ccctgtggca gcgccccctg gtgaccatca ggatcggcgg ccagctcaag 60
gaggcgctgc tcgccaccgg cgccgacgac accgtgctgg aggagatgaa cctgcccggc 120
aagtggaagc ccaagatgat cggcgggatc gggggcttca tcaaggtgcg gcagtacgac 180
cagatccccg tggagatctg cggccacaag gccatcggca ccgtgctggt gggccccacc 240
cccgtgaaca tcatcggccg caacctgctg acccagatcg gctgcaccct gaacttcccc 300
cagatcaccc tgtggcagcg ccccctggtg accatcagga tcggcggcca gctcaaggag 360
gcgctgctcg acaccggcgc cgacgacacc gtgctggagg agatgaacct gcccggcaag 420
tggaagccca agatgatcgg cgggatcggg ggcttcatca aggtgcggca gtacgaccag 480
atccccgtgg agatctgcgg ccacaaggcc atcggcaccg tgctggtggg cccacccccc 540
gtgaacatca tcggccgcaa cctgctgacc cagatcggct gcaccctgaa cttccccatc 600
agccccatcg agacggtgcc cgtgaagctg aagccgggga tggacggccc caaggtcaag 660
cagtggcccc tgaccgagga gaagatcaag gccctggtgg agatctgcac cgagatggag 720
aaggagggca agatcagcaa gatcggcccc gagaacccct acaacacccc cgtgttcgcc 780
atcaagaaga aggacagcac caagtggcgc aagctggtgg acttccgcga gctgaacaag 840
cgcacccagg acttctggga ggtgcagctg ggcatccccc accccgccgg cctgaagaag 900
aagaagagcg tgaccgtgct ggacgtgggc gacgcctact tcagcgtgcc cctggacaag 960
gacttccgca agtacaccgc cttcaccatc cccagcatca acaacgagac ccccggcatc 1020
cgctaccagt acaacgtgct gccccagggc tggaagggca gccccgccat cttccagagc 1080
agcatgacca agatcctgga gcccttccgc aagcagaacc ccgacatcgt gatctaccag 1140
gcccccctgt acgtgggcag cgacctggag atcggccagc accgcaccaa gatcgaggag 1200
ctgcgccagc acctgctgcg ctggggcttc accacccccg acaagaagca ccagaaggag 1260
ccccccttcc tgcccatcga gctgcacccc gacaagtgga ccgtgcagcc catcatgctg 1320
cccgagaagg acagctggac cgtgaacgac atccagaagc tggtgggcaa gctgaactgg 1380
gccagccaga tctacgccgg catcaaggtg aagcagctgt gcaagctgct gcgcggcacc 1440
aaggccctga ccgaggtgat ccccctgacc gaggaggccg agctggagct ggccgagaac 1500
cgcgagatcc tgaaggagcc cgtgcacgag gtgtactacg accccagcaa ggacctggtg 1560
gccgagatcc agaagcaggg ccagggccag tggacctacc agatctacca ggagcccttc 1620
aagaacctga agaccggcaa gtacgcccgc atgcgcggcg cccacaccaa cgacgtgaag 1680
cagctgaccg aggccgtgca gaaggtgagc accgagagca tcgtgatctg gggcaagatc 1740
cccaagttca agctgcccat ccagaaggag acctgggagg cctggtggat ggagtactgg 1800
caggccacct ggatccccga gtgggagttc gtgaacaccc cccccctggt gaagctgtgg 1860
taccagctgg agaaggagcc catcgtgggc gccgagacct tctacgtgga cggcgccgcc 1920
aaccgcgaga ccaagctggg caaggccggc tacgtgaccg accggggccg gcagaaggtg 1980
gtgagcatcg ccgacaccac caaccagaag accgagctgc aggccatcca cctggccctg 2040
caggacagcg gcctggaggt gaacatcgtg accgacagcc agtacgccct gggcatcatc 2100
caggcccagc ccgacaagag cgagagcgag ctggtgagcc agatcatcga gcagctgatc 2160
aagaaggaga aggtgtacct ggcctgggtg cccgcccaca agggcatcgg cggcaacgag 2220
caggtggaca agctggtgag cgccggcatc cgcaaggtgc tg 2262

<210> 50
<211> 1990
<212> DNA
<213> Artificial Sequence

<220>
<223> Description of Artificial Sequence:
      ProtInaRTmut.SF2

<400> 50
gtcgacgcca ccatgcccca gatcaccctg tggcagcgcc ccctggtgac catcaggatc 60
ggcggccagc tcaaggaggc gctgctcgcc accggcgccg acgacaccgt gctggaggag 120
atgaacctgc ccggcaagtg gaagcccaag atgatcggcg ggatcggggg cttcatcaag 180
gtgcggcagt acgaccagat ccccgtggag atctgcggcc acaaggccat cggcaccgtg 240
ctggtgggcc ccacccccgt gaacatcatc ggccgcaacc tgctgaccca gatcggctgc 300
accctgaact tccccatcag ccccatcgag acggtgcccg tgaagctgaa gccggggatg 360
gacggcccca aggtcaagca gtggcccctg accgaggaga agatcaaggc cctggtggag 420
atctgcaccg agatggagaa ggagggcaag atcagcaaga tcggccccga gaacccctac 480
aacacccccg tgttcgccat caagaagaag gacagcacca agtggcgcaa gctggtggac 540
ttccgcgagc tgaacaagcg cacccaggac ttctgggagg tgcagctggg catcccccac 600
cccgccggcc tgaagaagaa gaagagcgtg accgtgctgg acgtgggcga cgcctacttc 660
agcgtgcccc tggacaagga cttccgcaag tacaccgcct tcaccatccc cagcatcaac 720
aacgagaccc ccggcatccg ctaccagtac aacgtgctgc cccagggctg gaagggcagc 780
cccgccatct tccagagcag catgaccaag atcctggagc ccttccgcaa gcagaacccc 840
gacatcgtga tctaccaggc ccccctgtac gtgggcagcg acctggagat cggccagcac 900
cgcaccaaga tcgaggagct gcgccagcac ctgctgcgct ggggcttcac cacccccgac 960
aagaagcacc agaaggagcc ccccttcctg cccatcgagc tgcaccccga caagtggacc 1020
gtgcagccca tcatgctgcc cgagaaggac agctggaccg tgaacgacat ccagaagctg 1080
gtgggcaagc tgaactgggc cagccagatc tacgccggca tcaaggtgaa gcagctgtgc 1140
aagctgctgc gcggcaccaa ggccctgacc gaggtgatcc ccctgaccga ggaggccgag 1200
ctggagctgg ccgagaaccg cgagatcctg aaggagcccg tgcacgaggt gtactacgac 1260
cccagcaagg acctggtggc cgagatccag aagcagggcc agggccagtg gacctaccag 1320
atctaccagg agcccttcaa gaacctgaag accggcaagt acgcccgcat gcgcggcgcc 1380
cacaccaacg acgtgaagca gctgaccgag gccgtgcaga aggtgagcac cgagagcatc 1440
gtgatctggg gcaagatccc caagttcaag ctgcccatcc agaaggagac ctgggaggcc 1500
tggtggatgg agtactggca ggccacctgg atccccgagt gggagttcgt gaacaccccc 1560
cccctggtga agctgtggta ccagctggag aaggagccca tcgtgggcgc cgagaccttc 1620
tacgtggacg gcgccgccaa ccgcgagacc aagctgggca aggccggcta cgtgaccgac 1680
cggggccggc agaaggtggt gagcatcgcc gacaccacca accagaagac cgagctgcag 1740
gccatccacc tggccctgca ggacagcggc ctggaggtga acatcgtgac cgacagccag 1800
tacgccctgg gcatcatcca ggcccagccc gacaagagcg agagcgagct ggtgagccag 1860
atcatcgagc agctgatcaa gaaggagaag gtgtacctgg cctgggtgcc cgcccacaag 1920
ggcatcggcg gcaacgagca ggtggacaag ctggtgagcg ccggcatccg caaggtgctc 1980
taaatctaga                                                        1990
<210> 51
<211> 1977
<212> DNA
<213> Artificial Sequence

<220>
<223> Description of Artificial Sequence: protRT.opt.SF2

<400> 51
ccccagatca ccctgtggca gcgccccctg gtgaccatca ggatcggcgg ccagctcaag 60
gaggcgctgc tcgacaccgg cgccgacgac accgtgctgg aggagatgaa cctgcccggc 120
aagtggaagc ccaagatgat cggcgggatc gggggcttca tcaaggtgcg gcagtacgac 180
cagatccccg tggagatctg cggccacaag gccatcggca ccgtgctggt gggccccacc 240
cccgtgaaca tcatcggccg caacctgctg acccagatcg gctgcaccct gaacttcccc 300

atcagcccca tcgagacggt gcccgtgaag ctgaagccgg ggatggacgg ccccaaggtc 360
aagcagtggc ccctgaccga ggagaagatc aaggccctgg tggagatctg caccgagatg 420
gagaaggagg gcaagatcag caagatcggc cccgagaacc cctacaacac ccccgtgttc 480
gccatcaaga agaaggacag caccaagtgg cgcaagctgg tggacttccg cgagctgaac 540
aagcgcaccc aggacttctg ggaggtgcag ctgggcatcc cccaccccgc cggcctgaag 600
aagaagaaga gcgtgaccgt gctggacgtg ggcgacgcct acttcagcgt gcccctggac 660
aaggacttcc gcaagtacac cgccttcacc atccccagca tcaacaacga gacccccggc 720
atccgctacc agtacaacgt gctgccccag ggctggaagg gcagccccgc catcttccag 780
agcagcatga ccaagatcct ggagcccttc cgcaagcaga accccgacat cgtgatctac 840
cagtacatgg acgacctgta cgtgggcagc gacctggaga tcggccagca ccgcaccaag 900
atcgaggagc tgcgccagca cctgctgcgc tggggcttca ccacccccga caagaagcac 960
cagaaggagc cccccttcct gtggatgggc tacgagctgc accccgacaa gtggaccgtg 1020
cagcccatca tgctgcccga gaaggacagc tggaccgtga acgacatcca gaagctggtg 1080
ggcaagctga actgggccag ccagatctac gccggcatca aggtgaagca gctgtgcaag 1140
ctgctgcgcg gcaccaaggc cctgaccgag gtgatccccc tgaccgagga ggccgagctg 1200
gagctggccg agaaccgcga gatcctgaag gagcccgtgc acgaggtgta ctacgacccc 1260
agcaaggacc tggtggccga gatccagaag cagggccagg gccagtggac ctaccagatc 1320
taccaggagc ccttcaagaa cctgaagacc ggcaagtacg cccgcatgcg cggcgcccac 1380
accaacgacg tgaagcagct gaccgaggcc gtgcagaagg tgagcaccga gagcatcgtg 1440
atctggggca agatccccaa gttcaagctg cccatccaga aggagacctg ggaggcctgg 1500
tggatggagt actggcaggc cacctggatc cccgagtggg agttcgtgaa cacccccccc 1560
ctggtgaagc tgtggtacca gctggagaag gagcccatcg tgggcgccga gaccttctac 1620
gtggacggcg ccgccaaccg cgagaccaag ctgggcaagg ccggctacgt gaccgaccgg 1680
ggccggcaga aggtggtgag catcgccgac accaccaacc agaagaccga gctgcaggcc 1740
atccacctgg ccctgcagga cagcggcctg gaggtgaaca tcgtgaccga cagccagtac 1800
gccctgggca tcatccaggc ccagcccgac aagagcgaga gcgagctggt gagccagatc 1860
atcgagcagc tgatcaagaa ggagaaggtg tacctggcct gggtgcccgc ccacaagggc 1920
atcggcggca acgagcaggt ggacaagctg gtgagcgccg gcatccgcaa ggtgctg 1977

<210> 52
<211> 3252
<212> DNA
<213> Artificial Sequence

<220>
<223> Description of Artificial Sequence:
      ProtRT.TatRevNef.opt_B

<400> 52
atgccccaga tcaccctgtg gcagcgcccc ctggtgacca tcaggatcgg cggccagctc 60
aaggaggcgc tgctcgccac cggcgccgac gacaccgtgc tggaggagat gaacctgccc 120
ggcaagtgga agcccaagat gatcggcggg atcgggggct tcatcaaggt gcggcagtac 180
gaccagatcc ccgtggagat ctgcggccac aaggccatcg gcaccgtgct ggtgggcccc 240
acccccgtga acatcatcgg ccgcaacctg ctgacccaga tcggctgcac cctgaacttc 300
cccatcagcc ccatcgagac ggtgcccgtg aagctgaagc cggggatgga cggccccaag 360
gtcaagcagt ggcccctgac cgaggagaag atcaaggccc tggtggagat ctgcaccgag 420
atggagaagg agggcaagat cagcaagatc ggccccgaga acccctacaa cacccccgtg 480
ttcgccatca agaagaagga cagcaccaag tggcgcaagc tggtggactt ccgcgagctg 540
aacaagcgca cccaggactt ctgggaggtg cagctgggca tcccccaccc cgccggcctg 600
aagaagaaga agagcgtgac cgtgctggac gtgggcgacg cctacttcag cgtgcccctg 660
gacaaggact tccgcaagta caccgccttc accatcccca gcatcaacaa cgagaccccc 720
ggcatccgct accagtacaa cgtgctgccc cagggctgga agggcagccc cgccatcttc 780
cagagcagca tgaccaagat cctggagccc ttccgcaagc agaaccccga catcgtgatc 840
taccaggccc ccctgtacgt gggcagcgac ctggagatcg gccagcaccg caccaagatc 900

gaggagctgc gccagcacct gctgcgctgg ggcttcacca cccccgacaa gaagcaccag 960
aaggagcccc ccttcctgcc catcgagctg caccccgaca agtggaccgt gcagcccatc 1020
atgctgcccg agaaggacag ctggaccgtg aacgacatcc agaagctggt gggcaagctg 1080
aactgggcca gccagatcta cgccggcatc aaggtgaagc agctgtgcaa gctgctgcgc 1140
ggcaccaagg ccctgaccga ggtgatcccc ctgaccgagg aggccgagct ggagctggcc 1200
gagaaccgcg agatcctgaa ggagcccgtg cacgaggtgt actacgaccc cagcaaggac 1260
ctggtggccg agatccagaa gcagggccag ggccagtgga cctaccagat ctaccaggag 1320
cccttcaaga acctgaagac cggcaagtac gcccgcatgc gcggcgccca caccaacgac 1380
gtgaagcagc tgaccgaggc cgtgcagaag gtgagcaccg agagcatcgt gatctggggc 1440
aagatcccca agttcaagct gcccatccag aaggagacct gggaggcctg gtggatggag 1500
tactggcagg ccacctggat ccccgagtgg gagttcgtga acaccccccc cctggtgaag 1560
ctgtggtacc agctggagaa ggagcccatc gtgggcgccg agaccttcta cgtggacggc 1620
gccgccaacc gcgagaccaa gctgggcaag gccggctacg tgaccgaccg gggccggcag 1680
aaggtggtga gcatcgccga caccaccaac cagaagaccg agctgcaggc catccacctg 1740
gccctgcagg acagcggcct ggaggtgaac atcgtgaccg acagccagta cgccctgggc 1800
atcatccagg cccagcccga caagagcgag agcgagctgg tgagccagat catcgagcag 1860
ctgatcaaga aggagaaggt gtacctggcc tgggtgcccg cccacaaggg catcggcggc 1920
aacgagcagg tggacaagct ggtgagcgcc ggcatccgca aggtgctcga attcgagccc 1980
gtggaccccc gcctggagcc ctggaagcac cccggcagcc agcccaagac cgccggcacc 2040
aactgctact gcaagaagtg ctgcttccac tgccaggtga gcttcatcac caagggcctg 2100
ggcatcagct acggccgcaa gaagcgccgc cagcgccgcc gcgccccccc cgacagcgag 2160
gtgcaccagg tgagcctgcc caagcagccc gccagccagc cccagggcga ccccaccggc 2220
cccaaggaga gcaagaagaa ggtggagcgc gagaccgaga ccgaccccgt gcaccccggg 2280
gccggccgca gcggcgacag cgacgaggag ctgctgcaga ccgtgcgctt catcaagttc 2340
ctgtaccaga gcaaccccct gcccagcccc aagggcaccc gccaggccga cctgaaccgc 2400
cgccgccgct ggcgcgagcg ccagcgccag atccagagca tcagcgcctg gatcatcagc 2460
acccacctgg gccgcagcac cgagcccgtg cccctgcagc tgccccccga cctgcgcctg 2520
aacctggact gcagcgagga ctgcggcacc agcggcaccc agggcgtggg cagcccccag 2580
gtgctgggcg agagccccgc cgtgctggac agcggcacca aggagctcga ggccggcaag 2640
tggagcaagc gcatgagcgg ctggagcgcc gtgcgcgagc gcatgaagcg cgccgagccc 2700
gccgagcccg ccgccgacgg cgtgggcgcc gtgagccgcg acctggagaa gcacggcgcc 2760
atcaccagca gcaacaccgc cgccaacaac gccgactgcg cctggctgga ggcccaggag 2820
gacgaggacg tgggcttccc cgtgcgcccc caggtgcccc tgcgccccat gacctacaag 2880
gccgccctgg acctgagcca cttcctgaag gagaagggcg gcctggaggg cctgatctac 2940
agccagaagc gccaggacat cctggacctg tggatccacc acacccaggg ctacttcccc 3000
ggctggcaga actacacccc cggccccggc atccgctacc ccctgacctt cggctggtgc 3060
ttcaagctgg tgcccgtgga ccccgactac gtggaggagg ccaacgccgg cgagaacaac 3120
agcctgctgc accccatgag ccagcacggc atggacgacc ccgagaagga ggtgctggtg 3180
tggcgcttcg acagccgcct ggccttccac cacatggccc gcgagctgca ccccgagtac 3240
tacaaggact gc 3252

<210> 53
<211> 3264
<212> DNA
<213> Artificial Sequence

<220>
<223> Description of Artificial Sequence:
      ProtRTTatRevNef.opt_B

<400> 53
gccaccatgc cccagatcac cctgtggcag cgccccctgg tgaccatcag gatcggcggc 60
cagctcaagg aggcgctgct cgccaccggc gccgacgaca ccgtgctgga ggagatgaac 120
ctgcccggca agtggaagcc caagatgatc ggcgggatcg ggggcttcat caaggtgcgg 180

cagtacgacc agatccccgt ggagatctgc ggccacaagg ccatcggcac cgtgctggtg 240
ggccccaccc ccgtgaacat catcggccgc aacctgctga cccagatcgg ctgcaccctg 300
aacttcccca tcagccccat cgagacggtg cccgtgaagc tgaagccggg gatggacggc 360
cccaaggtca agcagtggcc cctgaccgag gagaagatca aggccctggt ggagatctgc 420
accgagatgg agaaggaggg caagatcagc aagatcggcc ccgagaaccc ctacaacacc 480
cccgtgttcg ccatcaagaa gaaggacagc accaagtggc gcaagctggt ggacttccgc 540
gagctgaaca agcgcaccca ggacttctgg gaggtgcagc tgggcatccc ccaccccgcc 600
ggcctgaaga agaagaagag cgtgaccgtg ctggacgtgg gcgacgccta cttcagcgtg 660
cccctggaca aggacttccg caagtacacc gccttcacca tccccagcat caacaacgag 720
acccccggca tccgctacca gtacaacgtg ctgccccagg gctggaaggg cagccccgcc 780
atcttccaga gcagcatgac caagatcctg gagcccttcc gcaagcagaa ccccgacatc 840
gtgatctacc aggcccccct gtacgtgggc agcgacctgg agatcggcca gcaccgcacc 900
aagatcgagg agctgcgcca gcacctgctg cgctggggct tcaccacccc cgacaagaag 960
caccagaagg agcccccctt cctgcccatc gagctgcacc ccgacaagtg gaccgtgcag 1020
cccatcatgc tgcccgagaa ggacagctgg accgtgaacg acatccagaa gctggtgggc 1080
aagctgaact gggccagcca gatctacgcc ggcatcaagg tgaagcagct gtgcaagctg 1140
ctgcgcggca ccaaggccct gaccgaggtg atccccctga ccgaggaggc cgagctggag 1200
ctggccgaga accgcgagat cctgaaggag cccgtgcacg aggtgtacta cgaccccagc 1260
aaggacctgg tggccgagat ccagaagcag ggccagggcc agtggaccta ccagatctac 1320
caggagccct tcaagaacct gaagaccggc aagtacgccc gcatgcgcgg cgcccacacc 1380
aacgacgtga agcagctgac cgaggccgtg cagaaggtga gcaccgagag catcgtgatc 1440
tggggcaaga tccccaagtt caagctgccc atccagaagg agacctggga ggcctggtgg 1500
atggagtact ggcaggccac ctggatcccc gagtgggagt tcgtgaacac cccccccctg 1560
gtgaagctgt ggtaccagct ggagaaggag cccatcgtgg gcgccgagac cttctacgtg 1620
gacggcgccg ccaaccgcga gaccaagctg ggcaaggccg gctacgtgac cgaccggggc 1680
cggcagaagg tggtgagcat cgccgacacc accaaccaga agaccgagct gcaggccatc 1740
cacctggccc tgcaggacag cggcctggag gtgaacatcg tgaccgacag ccagtacgcc 1800
ctgggcatca tccaggccca gcccgacaag agcgagagcg agctggtgag ccagatcatc 1860
gagcagctga tcaagaagga gaaggtgtac ctggcctggg tgcccgccca caagggcatc 1920
ggcggcaacg agcaggtgga caagctggtg agcgccggca tccgcaaggt gctcgaattc 1980
gagcccgtgg acccccgcct ggagccctgg aagcaccccg gcagccagcc caagaccgcc 2040
ggcaccaact gctactgcaa gaagtgctgc ttccactgcc aggtgagctt catcaccaag 2100
ggcctgggca tcagctacgg ccgcaagaag cgccgccagc gccgccgcgc cccccccgac 2160
agcgaggtgc accaggtgag cctgcccaag cagcccgcca gccagcccca gggcgacccc 2220
accggcccca aggagagcaa gaagaaggtg gagcgcgaga ccgagaccga ccccgtgcac 2280
cccggggccg gccgcagcgg cgacagcgac gaggagctgc tgcagaccgt gcgcttcatc 2340
aagttcctgt accagagcaa ccccctgccc agccccaagg gcacccgcca ggccgacctg 2400
aaccgccgcc gccgctggcg cgagcgccag cgccagatcc agagcatcag cgcctggatc 2460
atcagcaccc acctgggccg cagcaccgag cccgtgcccc tgcagctgcc ccccgacctg 2520
cgcctgaacc tggactgcag cgaggactgc ggcaccagcg gcacccaggg cgtgggcagc 2580
ccccaggtgc tgggcgagag ccccgccgtg ctggacagcg gcaccaagga gctcgaggcc 2640
ggcaagtgga gcaagcgcat gagcggctgg agcgccgtgc gcgagcgcat gaagcgcgcc 2700
gagcccgccg agcccgccgc cgacggcgtg ggcgccgtga gccgcgacct ggagaagcac 2760
ggcgccatca ccagcagcaa caccgccgcc aacaacgccg actgcgcctg gctggaggcc 2820
caggaggacg aggacgtggg cttccccgtg cgcccccagg tgcccctgcg ccccatgacc 2880
tacaaggccg ccctggacct gagccacttc ctgaaggaga agggcggcct ggagggcctg 2940
atctacagcc agaagcgcca ggacatcctg gacctgtgga tccaccacac ccagggctac 3000
ttccccggct ggcagaacta cacccccggc cccggcatcc gctaccccct gaccttcggc 3060
tggtgcttca agctggtgcc cgtggacccc gactacgtgg aggaggccaa cgccggcgag 3120
aacaacagcc tgctgcaccc catgagccag cacggcatgg acgaccccga gaaggaggtg 3180
ctggtgtggc gcttcgacag ccgcctggcc ttccaccaca tggcccgcga gctgcacccc 3240
gagtactaca aggactgcga ttaa 3264

<210> 54

<211> 1971
<212> DNA
<213> Artificial Sequence

<220>
<223> Description of Artificial Sequence:
      protInaRT.YM.opt_C

<400> 54
ccccagatca ccctgtggca gcgccccctg gtgagcatca aggtgggcgg ccagatcaag 60
gaggccctgc tggccaccgg cgccgacgac accgtgctgg aggagatgag cctgcccggc 120
aagtggaagc ccaagatgat cggcggcatc ggcggcttca tcaaggtgcg ccagtacgac 180
cagatcctga tcgagatctg cggcaagaag gccatcggca ccgtgctgat cggccccacc 240
cccgtgaaca tcatcggccg caacatgctg acccagctgg gctgcaccct gaacttcccc 300
atcagcccca tcgagaccgt gcccgtgaag ctgaagcccg gcatggacgg ccccaaggtg 360
aagcagtggc ccctgaccga ggagaagatc aaggccctga ccgccatctg cgaggagatg 420
gagaaggagg gcaagatcac caagatcggc cccgagaacc cctacaacac ccccgtgttc 480
gccatcaaga agaaggacag caccaagtgg cgcaagctgg tggacttccg cgagctgaac 540
aagcgcaccc aggacttctg ggaggtgcag ctgggcatcc cccaccccgc cggcctgaag 600
aagaagaaga gcgtgaccgt gctggacgtg ggcgacgcct acttcagcgt gcccctggac 660
gaggacttcc gcaagtacac cgccttcacc atccccagca tcaacaacga gacccccggc 720
atccgctacc agtacaacgt gctgccccag ggctggaagg gcagccccag catcttccag 780
agcagcatga ccaagatcct ggagcccttc cgcgcccgca accccgagat cgtgatctac 840
caggcccccc tgtacgtggg cagcgacctg gagatcggcc agcaccgcgc caagatcgag 900
gagctgcgca agcacctgct gcgctggggc ttcaccaccc ccgacaagaa gcaccagaag 960
gagcccccct tcctgtggat gggctacgag ctgcaccccg acaagtggac cgtgcagccc 1020
atcgagctgc ccgagaagga gagctggacc gtgaacgaca tccagaagct ggtgggcaag 1080
ctgaactggg ccagccagat ctaccccggc atcaaggtgc gccagctgtg caagctgctg 1140
cgcggcgcca aggccctgac cgacatcgtg cccctgaccg aggaggccga gctggagctg 1200
gccgagaacc gcgagatcct gcgcgagccc gtgcacggcg tgtactacga ccccagcaag 1260
gacctggtgg ccgagatcca gaagcagggc cacgaccagt ggacctacca gatctaccag 1320
gagcccttca agaacctgaa gaccggcaag tacgccaaga tgcgcaccgc ccacaccaac 1380
gacgtgaagc agctgaccga ggccgtgcag aagatcgcca tggagagcat cgtgatctgg 1440
ggcaagaccc ccaagttccg cctgcccatc cagaaggaga cctgggagac ctggtggacc 1500
gactactggc aggccacctg gatccccgag tgggagttcg tgaacacccc ccccctggtg 1560
aagctgtggt accagctgga gaaggagccc atcatcggcg ccgagacctt ctacgtggac 1620
ggcgccgcca accgcgagac caagatcggc aaggccggct acgtgaccga ccggggccgg 1680
cagaagatcg tgagcctgac cgagaccacc aaccagaaga ccgagctgca ggccatccag 1740
ctggccctgc aggacagcgg cagcgaggtg aacatcgtga ccgacagcca gtacgccctg 1800
ggcatcatcc aggcccagcc cgacaagagc gagagcgagc tggtgaacca gatcatcgag 1860
cagctgatca agaaggagaa ggtgtacctg agctgggtgc ccgcccacaa gggcatcggc 1920
ggcaacgagc agatcgacaa gctggtgagc aagggcatcc gcaaggtgct c 1971

<210> 55
<211> 1965
<212> DNA
<213> Artificial Sequence

<220>
<223> Description of Artificial Sequence:
      protInaRT.YMWM.opt_C

<400> 55
ccccagatca ccctgtggca gcgccccctg gtgagcatca aggtgggcgg ccagatcaag 60

gaggccctgc tggccaccgg cgccgacgac accgtgctgg aggagatgag cctgcccggc 120
aagtggaagc ccaagatgat cggcggcatc ggcggcttca tcaaggtgcg ccagtacgac 180
cagatcctga tcgagatctg cggcaagaag gccatcggca ccgtgctgat cggccccacc 240
cccgtgaaca tcatcggccg caacatgctg acccagctgg gctgcaccct gaacttcccc 300
atcagcccca tcgagaccgt gcccgtgaag ctgaagcccg gcatggacgg ccccaaggtg 360
aagcagtggc ccctgaccga ggagaagatc aaggccctga ccgccatctg cgaggagatg 420
gagaaggagg gcaagatcac caagatcggc cccgagaacc cctacaacac ccccgtgttc 480
gccatcaaga agaaggacag caccaagtgg cgcaagctgg tggacttccg cgagctgaac 540
aagcgcaccc aggacttctg ggaggtgcag ctgggcatcc cccaccccgc cggcctgaag 600
aagaagaaga gcgtgaccgt gctggacgtg ggcgacgcct acttcagcgt gcccctggac 660
gaggacttcc gcaagtacac cgccttcacc atccccagca tcaacaacga gacccccggc 720
atccgctacc agtacaacgt gctgccccag ggctggaagg gcagccccag catcttccag 780
agcagcatga ccaagatcct ggagcccttc cgcgcccgca accccgagat cgtgatctac 840
caggcccccc tgtacgtggg cagcgacctg gagatcggcc agcaccgcgc caagatcgag 900
gagctgcgca agcacctgct gcgctggggc ttcaccaccc ccgacaagaa gcaccagaag 960
gagcccccct tcctgcccat cgagctgcac cccgacaagt ggaccgtgca gcccatcgag 1020
ctgcccgaga aggagagctg gaccgtgaac gacatccaga agctggtggg caagctgaac 1080
tgggccagcc agatctaccc cggcatcaag gtgcgccagc tgtgcaagct gctgcgcggc 1140
gccaaggccc tgaccgacat cgtgcccctg accgaggagg ccgagctgga gctggccgag 1200
aaccgcgaga tcctgcgcga gcccgtgcac ggcgtgtact acgaccccag caaggacctg 1260
gtggccgaga tccagaagca gggccacgac cagtggacct accagatcta ccaggagccc 1320
ttcaagaacc tgaagaccgg caagtacgcc aagatgcgca ccgcccacac caacgacgtg 1380
aagcagctga ccgaggccgt gcagaagatc gccatggaga gcatcgtgat ctggggcaag 1440
acccccaagt tccgcctgcc catccagaag gagacctggg agacctggtg gaccgactac 1500
tggcaggcca cctggatccc cgagtgggag ttcgtgaaca ccccccccct ggtgaagctg 1560
tggtaccagc tggagaagga gcccatcatc ggcgccgaga ccttctacgt ggacggcgcc 1620
gccaaccgcg agaccaagat cggcaaggcc ggctacgtga ccgaccgggg ccggcagaag 1680
atcgtgagcc tgaccgagac caccaaccag aagaccgagc tgcaggccat ccagctggcc 1740
ctgcaggaca gcggcagcga ggtgaacatc gtgaccgaca gccagtacgc cctgggcatc 1800
atccaggccc agcccgacaa gagcgagagc gagctggtga accagatcat cgagcagctg 1860
atcaagaagg agaaggtgta cctgagctgg gtgcccgccc acaagggcat cggcggcaac 1920
gagcagatcg acaagctggt gagcaagggc atccgcaagg tgctc 1965

<210> 56
<211> 3234
<212> DNA
<213> Artificial Sequence

<220>
<223> Description of Artificial Sequence:
      ProtRT.TatRevNef.opt_C

<400> 56
gccaccatgc cccagatcac cctgtggcag cgccccctgg tgagcatcaa ggtgggcggc 60
cagatcaagg aggccctgct ggacaccggc gccgacgaca ccgtgctgga ggagatgagc 120
ctgcccggca agtggaagcc caagatgatc ggcggcatcg gcggcttcat caaggtgcgc 180
cagtacgacc agatcctgat cgagatctgc ggcaagaagg ccatcggcac cgtgctgatc 240
ggccccaccc ccgtgaacat catcggccgc aacatgctga cccagctggg ctgcaccctg 300
aacttcccca tcagccccat cgagaccgtg cccgtgaagc tgaagcccgg catggacggc 360
cccaaggtga agcagtggcc cctgaccgag gagaagatca aggccctgac cgccatctgc 420
gaggagatgg agaaggaggg caagatcacc aagatcggcc ccgagaaccc ctacaacacc 480
cccgtgttcg ccatcaagaa gaaggacagc accaagtggc gcaagctggt ggacttccgc 540
gagctgaaca agcgcaccca ggacttctgg gaggtgcagc tgggcatccc ccaccccgcc 600
ggcctgaaga agaagaagag cgtgaccgtg ctggacgtgg gcgacgccta cttcagcgtg 660

cccctggacg aggacttccg caagtacacc gccttcacca tccccagcat caacaacgag 720
acccccggca tccgctacca gtacaacgtg ctgccccagg gctggaaggg cagccccagc 780
atcttccaga gcagcatgac caagatcctg gagcccttcc gcgcccgcaa ccccgagatc 840
gtgatctacc aggcccccct gtacgtgggc agcgacctgg agatcggcca gcaccgcgcc 900
aagatcgagg agctgcgcaa gcacctgctg cgctggggct tcaccacccc cgacaagaag 960
caccagaagg agcccccctt cctgcccatc gagctgcacc ccgacaagtg gaccgtgcag 1020
cccatcgagc tgcccgagaa ggagagctgg accgtgaacg acatccagaa gctggtgggc 1080
aagctgaact gggccagcca gatctacccc ggcatcaagg tgcgccagct gtgcaagctg 1140
ctgcgcggcg ccaaggccct gaccgacatc gtgcccctga ccgaggaggc cgagctggag 1200
ctggccgaga accgcgagat cctgcgcgag cccgtgcacg gcgtgtacta cgaccccagc 1260
aaggacctgg tggccgagat ccagaagcag ggccacgacc agtggaccta ccagatctac 1320
caggagccct tcaagaacct gaagaccggc aagtacgcca agatgcgcac cgcccacacc 1380
aacgacgtga agcagctgac cgaggccgtg cagaagatcg ccatggagag catcgtgatc 1440
tggggcaaga cccccaagtt ccgcctgccc atccagaagg agacctggga gacctggtgg 1500
accgactact ggcaggccac ctggatcccc gagtgggagt tcgtgaacac cccccccctg 1560
gtgaagctgt ggtaccagct ggagaaggag cccatcatcg gcgccgagac cttctacgtg 1620
gacggcgccg ccaaccgcga gaccaagatc ggcaaggccg gctacgtgac cgaccggggc 1680
cggcagaaga tcgtgagcct gaccgagacc accaaccaga agaccgagct gcaggccatc 1740
cagctggccc tgcaggacag cggcagcgag gtgaacatcg tgaccgacag ccagtacgcc 1800
ctgggcatca tccaggccca gcccgacaag agcgagagcg agctggtgaa ccagatcatc 1860
gagcagctga tcaagaagga gaaggtgtac ctgagctggg tgcccgccca caagggcatc 1920
ggcggcaacg agcagatcga caagctggtg agcaagggca tccgcaaggt gctcgaattc 1980
gagcccgtgg accccaacct ggagccctgg aaccaccccg gcagccagcc caagaccgcc 2040
ggcaacaagt gctactgcaa gcactgcagc taccactgcc tggtgagctt ccagaccaag 2100
ggcctgggca tcagctacgg ccgcaagaag cgccgccagc gccgcagcgc cccccccagc 2160
agcgaggacc accagaaccc catcagcaag cagcccctgc cccagacccg cggcgacccc 2220
accggcagcg aggagagcaa gaagaaggtg gagagcaaga ccgagaccga ccccttcgac 2280
cccggggccg gccgcagcgg cgacagcgac gaggccctgc tgcaggccgt gcgcatcatc 2340
aagatcctgt accagagcaa cccctacccc aagcccgagg gcacccgcca ggccgacctg 2400
aaccgccgcc gccgctggcg cgcccgccag cgccagatcc acagcatcag cgagcgcatc 2460
ctgagcacct gcctgggccg ccccgccgag cccgtgccct tccagctgcc ccccgacctg 2520
cgcctgcaca tcgactgcag cgagagcagc ggcaccagcg gcacccagca gagccagggc 2580
accaccgagg gcgtgggcag ccccctcgag gccggcaagt ggagcaagag cagcatcgtg 2640
ggctggcccg ccgtgcgcga gcgcatccgc cgcaccgagc ccgccgccga gggcgtgggc 2700
gccgccagcc aggacctgga caagcacggc gccctgacca gcagcaacac cgccgccaac 2760
aacgccgact gcgcctggct ggaggcccag gaggaggagg aggaggtggg cttccccgtg 2820
cgcccccagg tgcccctgcg ccccatgacc tacaaggccg ccttcgacct gagcttcttc 2880
ctgaaggaga agggcggcct ggagggcctg atctacagca agaagcgcca ggagatcctg 2940
gacctgtggg tgtaccacac ccagggcttc ttccccggct ggcagaacta cacccccggc 3000
cccggcgtgc gctaccccct gaccttcggc tggtgcttca agctggtgcc cgtggacccc 3060
cgcgaggtgg aggaggccaa caagggcgag aacaactgcc tgctgcaccc catgagccag 3120
cacggcatgg aggacgagga ccgcgaggtg ctgaagtgga agttcgacag cagcctggcc 3180
cgccgccaca tggcccgcga gctgcacccc gagtactaca aggactgcgc ctaa 3234

<210> 57
<211> 348
<212> DNA
<213> Artificial Sequence

<220>
<223> Description of Artificial Sequence:
      rev.exon1_2.M5-10.opt.SF162

<400> 57

atggccggcc gcagcggcga cagcgacgag gagctgctgc agaccgtgcg cttcatcaag 60
ttcctgtacc agagcaaccc cctgcccagc cccaagggca cccgccaggc cgacctgaac 120
cgccgccgcc gctggcgcga gcgccagcgc cagatccaga gcatcagcgc ctggatcatc 180
agcacccacc tgggccgcag caccgagccc gtgcccctgc agctgccccc cgacctgcgc 240
ctgaacctgg actgcagcga ggactgcggc accagcggca cccagggcgt gggcagcccc 300
caggtgctgg gcgagagccc cgccgtgctg gacagcggca ccaaggag 348

<210> 58
<211> 348
<212> DNA
<213> Artificial Sequence

<220>
<223> Description of Artificial Sequence:
      rev.exon1_2.opt.SF162

<400> 58
atggccggcc gcagcggcga cagcgacgag gagctgctgc agaccgtgcg cttcatcaag 60
ttcctgtacc agagcaaccc cctgcccagc cccaagggca cccgccaggc ccgccgcaac 120
cgccgccgcc gctggcgcga gcgccagcgc cagatccaga gcatcagcgc ctggatcatc 180
agcacccacc tgggccgcag caccgagccc gtgcccctgc agctgccccc cctggagcgc 240
ctgaacctgg actgcagcga ggactgcggc accagcggca cccagggcgt gggcagcccc 300
caggtgctgg gcgagagccc cgccgtgctg gacagcggca ccaaggag 348

<210> 59
<211> 321
<212> DNA
<213> Artificial Sequence

<220>
<223> Description of Artificial Sequence:
      rev.exon1_2.M5-10.opt_C

<400> 59
atggccggcc gcagcggcga cagcgacgag gccctgctgc aggccgtgcg catcatcaag 60
atcctgtacc agagcaaccc ctaccccaag cccgagggca cccgccaggc cgacctgaac 120
cgccgccgcc gctggcgcgc ccgccagcgc cagatccaca gcatcagcga gcgcatcctg 180
agcacctgcc tgggccgccc cgccgagccc gtgcccttcc agctgccccc cgacctgcgc 240
ctgcacatcg actgcagcga gagcagcggc accagcggca cccagcagag ccagggcacc 300
accgagggcg tgggcagccc c 321

<210> 60
<211> 321
<212> DNA
<213> Artificial Sequence

<220>
<223> Description of Artificial Sequence: revexon1_2 TV1
      C ZAopt

<400> 60
atggccggcc gcagcggcga cagcgacgag gccctgctgc aggtggtgaa gatcatcaag 60
atcctgtacc agagccccta ccccaagccc gagggcaccc gccaggcccg ccgcaaccgc 120
cgccgccgct ggcgcgcccg ccagcgccag atccacacca tcggcgagcg catcctggtg 180

gcctgcctgg gccgcagcgc cgagcccgtg cccctgcagc tgccccccct ggagcgcctg 240
cacatcaact gcagcgaggg cagcggcacc agcggcaccc agcagagcca gggcaccacc 300
gagggcgtgg gcgaccccta a 321

<210> 61
<211> 1977
<212> DNA
<213> Artificial Sequence

<220>
<223> Description of Artificial Sequence: RT.opt.SF2
      (mutant)

<400> 61
gccaccatgc cccagatcac cctgtggcag cgccccctgg tgaccatcag gatcggcggc 60
cagctcaagg aggcgctgct cgccaccggc gccgacgaca ccgtgctgga ggagatgaac 120
ctgcccggca agtggaagcc caagatgatc ggcgggatcg ggggcttcat caaggtgcgg 180
cagtacgacc agatccccgt ggagatctgc ggccacaagg ccatcggcac cgtgctggtg 240
ggccccaccc ccgtgaacat catcggccgc aacctgctga cccagatcgg ctgcaccctg 300
aacttcccca tcagccccat cgagacggtg cccgtgaagc tgaagccggg gatggacggc 360
cccaaggtca agcagtggcc cctgaccgag gagaagatca aggccctggt ggagatctgc 420
accgagatgg agaaggaggg caagatcagc aagatcggcc ccgagaaccc ctacaacacc 480
cccgtgttcg ccatcaagaa gaaggacagc accaagtggc gcaagctggt ggacttccgc 540
gagctgaaca agcgcaccca ggacttctgg gaggtgcagc tgggcatccc ccaccccgcc 600
ggcctgaaga agaagaagag cgtgaccgtg ctggacgtgg gcgacgccta cttcagcgtg 660
cccctggaca aggacttccg caagtacacc gccttcacca tccccagcat caacaacgag 720
acccccggca tccgctacca gtacaacgtg ctgccccagg gctggaaggg cagccccgcc 780
atcttccaga gcagcatgac caagatcctg gagcccttcc gcaagcagaa ccccgacatc 840
gtgatctacc aggcccccct gtacgtgggc agcgacctgg agatcggcca gcaccgcacc 900
aagatcgagg agctgcgcca gcacctgctg cgctggggct tcaccacccc cgacaagaag 960
caccagaagg agcccccctt cctgcccatc gagctgcacc ccgacaagtg gaccgtgcag 1020
cccatcatgc tgcccgagaa ggacagctgg accgtgaacg acatccagaa gctggtgggc 1080
aagctgaact gggccagcca gatctacgcc ggcatcaagg tgaagcagct gtgcaagctg 1140
ctgcgcggca ccaaggccct gaccgaggtg atccccctga ccgaggaggc cgagctggag 1200
ctggccgaga accgcgagat cctgaaggag cccgtgcacg aggtgtacta cgaccccagc 1260
aaggacctgg tggccgagat ccagaagcag ggccagggcc agtggaccta ccagatctac 1320
caggagccct tcaagaacct gaagaccggc aagtacgccc gcatgcgcgg cgcccacacc 1380
aacgacgtga agcagctgac cgaggccgtg cagaaggtga gcaccgagag catcgtgatc 1440
tggggcaaga tccccaagtt caagctgccc atccagaagg agacctggga ggcctggtgg 1500
atggagtact ggcaggccac ctggatcccc gagtgggagt tcgtgaacac cccccccctg 1560
gtgaagctgt ggtaccagct ggagaaggag cccatcgtgg gcgccgagac cttctacgtg 1620
gacggcgccg ccaaccgcga gaccaagctg ggcaaggccg gctacgtgac cgaccggggc 1680
cggcagaagg tggtgagcat cgccgacacc accaaccaga agaccgagct gcaggccatc 1740
cacctggccc tgcaggacag cggcctggag gtgaacatcg tgaccgacag ccagtacgcc 1800
ctgggcatca tccaggccca gcccgacaag agcgagagcg agctggtgag ccagatcatc 1860
gagcagctga tcaagaagga gaaggtgtac ctggcctggg tgcccgccca caagggcatc 1920
ggcggcaacg agcaggtgga caagctggtg agcgccggca tccgcaaggt gctctaa 1977

<210> 62
<211> 1989
<212> DNA
<213> Artificial Sequence

<220>

<223> Description of Artificial Sequence: RT.opt.SF2
      (native)

<400> 62
gccaccatgc cccagatcac cctgtggcag cgccccctgg tgaccatcag gatcggcggc 60
cagctcaagg aggcgctgct cgacaccggc gccgacgaca ccgtgctgga ggagatgaac 120
ctgcccggca agtggaagcc caagatgatc ggcgggatcg ggggcttcat caaggtgcgg 180
cagtacgacc agatccccgt ggagatctgc ggccacaagg ccatcggcac cgtgctggtg 240
ggccccaccc ccgtgaacat catcggccgc aacctgctga cccagatcgg ctgcaccctg 300
aacttcccca tcagccccat cgagacggtg cccgtgaagc tgaagccggg gatggacggc 360
cccaaggtca agcagtggcc cctgaccgag gagaagatca aggccctggt ggagatctgc 420
accgagatgg agaaggaggg caagatcagc aagatcggcc ccgagaaccc ctacaacacc 480
cccgtgttcg ccatcaagaa gaaggacagc accaagtggc gcaagctggt ggacttccgc 540
gagctgaaca agcgcaccca ggacttctgg gaggtgcagc tgggcatccc ccaccccgcc 600
ggcctgaaga agaagaagag cgtgaccgtg ctggacgtgg gcgacgccta cttcagcgtg 660
cccctggaca aggacttccg caagtacacc gccttcacca tccccagcat caacaacgag 720
acccccggca tccgctacca gtacaacgtg ctgccccagg gctggaaggg cagccccgcc 780
atcttccaga gcagcatgac caagatcctg gagcccttcc gcaagcagaa ccccgacatc 840
gtgatctacc agtacatgga cgacctgtac gtgggcagcg acctggagat cggccagcac 900
cgcaccaaga tcgaggagct gcgccagcac ctgctgcgct ggggcttcac cacccccgac 960
aagaagcacc agaaggagcc ccccttcctg tggatgggct acgagctgca ccccgacaag 1020
tggaccgtgc agcccatcat gctgcccgag aaggacagct ggaccgtgaa cgacatccag 1080
aagctggtgg gcaagctgaa ctgggccagc cagatctacg ccggcatcaa ggtgaagcag 1140
ctgtgcaagc tgctgcgcgg caccaaggcc ctgaccgagg tgatccccct gaccgaggag 1200
gccgagctgg agctggccga gaaccgcgag atcctgaagg agcccgtgca cgaggtgtac 1260
tacgacccca gcaaggacct ggtggccgag atccagaagc agggccaggg ccagtggacc 1320
taccagatct accaggagcc cttcaagaac ctgaagaccg gcaagtacgc ccgcatgcgc 1380
ggcgcccaca ccaacgacgt gaagcagctg accgaggccg tgcagaaggt gagcaccgag 1440
agcatcgtga tctggggcaa gatccccaag ttcaagctgc ccatccagaa ggagacctgg 1500
gaggcctggt ggatggagta ctggcaggcc acctggatcc ccgagtggga gttcgtgaac 1560
accccccccc tggtgaagct gtggtaccag ctggagaagg agcccatcgt gggcgccgag 1620
accttctacg tggacggcgc cgccaaccgc gagaccaagc tgggcaaggc cggctacgtg 1680
accgaccggg gccggcagaa ggtggtgagc atcgccgaca ccaccaacca gaagaccgag 1740
ctgcaggcca tccacctggc cctgcaggac agcggcctgg aggtgaacat cgtgaccgac 1800
agccagtacg ccctgggcat catccaggcc cagcccgaca agagcgagag cgagctggtg 1860
agccagatca tcgagcagct gatcaagaag gagaaggtgt acctggcctg ggtgcccgcc 1920
cacaagggca tcggcggcaa cgagcaggtg gacaagctgg tgagcgccgg catccgcaag 1980
gtgctgtaa 1989

<210> 63
<211> 1693
<212> DNA
<213> Artificial Sequence

<220>
<223> Description of Artificial Sequence: RTmut.SF2

<400> 63
gtcgacgcca ccatgcccat cagccccatc gagacggtgc ccgtgaagct gaagccgggg 60
atggacggcc ccaaggtcaa gcagtggccc ctgaccgagg agaagatcaa ggccctggtg 120
gagatctgca ccgagatgga gaaggagggc aagatcagca agatcggccc cgagaacccc 180
tacaacaccc ccgtgttcgc catcaagaag aaggacagca ccaagtggcg caagctggtg 240
gacttccgcg agctgaacaa gcgcacccag gacttctggg aggtgcagct gggcatcccc 300
caccccgccg gcctgaagaa gaagaagagc gtgaccgtgc tggacgtggg cgacgcctac 360

ttcagcgtgc ccctggacaa ggacttccgc aagtacaccg ccttcaccat ccccagcatc 420
aacaacgaga cccccggcat ccgctaccag tacaacgtgc tgccccaggg ctggaagggc 480
agccccgcca tcttccagag cagcatgacc aagatcctgg agcccttccg caagcagaac 540
cccgacatcg tgatctacca ggcccccctg tacgtgggca gcgacctgga gatcggccag 600
caccgcacca agatcgagga gctgcgccag cacctgctgc gctggggctt caccaccccc 660
gacaagaagc accagaagga gccccccttc ctgcccatcg agctgcaccc cgacaagtgg 720
accgtgcagc ccatcatgct gcccgagaag gacagctgga ccgtgaacga catccagaag 780
ctggtgggca agctgaactg ggccagccag atctacgccg gcatcaaggt gaagcagctg 840
tgcaagctgc tgcgcggcac caaggccctg accgaggtga tccccctgac cgaggaggcc 900
gagctggagc tggccgagaa ccgcgagatc ctgaaggagc ccgtgcacga ggtgtactac 960
gaccccagca aggacctggt ggccgagatc cagaagcagg gccagggcca gtggacctac 1020
cagatctacc aggagccctt caagaacctg aagaccggca agtacgcccg catgcgcggc 1080
gcccacacca acgacgtgaa gcagctgacc gaggccgtgc agaaggtgag caccgagagc 1140
atcgtgatct ggggcaagat ccccaagttc aagctgccca tccagaagga gacctgggag 1200
gcctggtgga tggagtactg gcaggccacc tggatccccg agtgggagtt cgtgaacacc 1260
ccccccctgg tgaagctgtg gtaccagctg gagaaggagc ccatcgtggg cgccgagacc 1320
ttctacgtgg acggcgccgc caaccgcgag accaagctgg gcaaggccgg ctacgtgacc 1380
gaccggggcc ggcagaaggt ggtgagcatc gccgacacca ccaaccagaa gaccgagctg 1440
caggccatcc acctggccct gcaggacagc ggcctggagg tgaacatcgt gaccgacagc 1500
cagtacgccc tgggcatcat ccaggcccag cccgacaaga gcgagagcga gctggtgagc 1560
cagatcatcg agcagctgat caagaaggag aaggtgtacc tggcctgggt gcccgcccac 1620
aagggcatcg gcggcaacga gcaggtggac aagctggtga gcgccggcat ccgcaaggtg 1680
ctctaaagaa ttc 1693

<210> 64
<211> 303
<212> DNA
<213> Artificial Sequence

<220>
<223> Description of Artificial Sequence:
      tat.exon1_2.opt.C22-37.SF2

<400> 64
atggagcccg tggacccccg cctggagccc tggaagcacc ccggcagcca gcccaagacc 60
gccggcacca actgctactg caagaagtgc tgcttccact gccaggtgag cttcatcacc 120
aagggcctgg gcatcagcta cggccgcaag aagcgccgcc agcgccgccg cgcccccccc 180
gacagcgagg tgcaccaggt gagcctgccc aagcagcccg ccagccagcc ccagggcgac 240
cccaccggcc ccaaggagag caagaagaag gtggagcgcg agaccgagac cgaccccgtg 300
cac 303

<210> 65
<211> 303
<212> DNA
<213> Artificial Sequence

<220>
<223> Description of Artificial Sequence:
      tat.exon1_2.opt.C37.SF2

<400> 65
atggagcccg tggacccccg cctggagccc tggaagcacc ccggcagcca gcccaagacc 60
gcctgcacca actgctactg caagaagtgc tgcttccact gccaggtgag cttcatcacc 120
aagggcctgg gcatcagcta cggccgcaag aagcgccgcc agcgccgccg cgcccccccc 180

gacagcgagg tgcaccaggt gagcctgccc aagcagcccg ccagccagcc ccagggcgac 240
cccaccggcc ccaaggagag caagaagaag gtggagcgcg agaccgagac cgaccccgtg 300
cac 303

<210> 66
<211> 303
<212> DNA
<213> Artificial Sequence

<220>
<223> Description of Artificial Sequence:
      tat.exon1_2.opt.C22-37_C

<400> 66
atggagcccg tggaccccaa cctggagccc tggaaccacc ccggcagcca gcccaagacc 60
gccggcaaca agtgctactg caagcactgc agctaccact gcctggtgag cttccagacc 120
aagggcctgg gcatcagcta cggccgcaag aagcgccgcc agcgccgcag cgcccccccc 180
agcagcgagg accaccagaa ccccatcagc aagcagcccc tgccccagac ccgcggcgac 240
cccaccggca gcgaggagag caagaagaag gtggagagca agaccgagac cgaccccttc 300
gac 303

<210> 67
<211> 303
<212> DNA
<213> Artificial Sequence

<220>
<223> Description of Artificial Sequence:
      tat.exon1_2.opt.C37_C

<400> 67
atggagcccg tggaccccaa cctggagccc tggaaccacc ccggcagcca gcccaagacc 60
gcctgcaaca agtgctactg caagcactgc agctaccact gcctggtgag cttccagacc 120
aagggcctgg gcatcagcta cggccgcaag aagcgccgcc agcgccgcag cgcccccccc 180
agcagcgagg accaccagaa ccccatcagc aagcagcccc tgccccagac ccgcggcgac 240
cccaccggca gcgaggagag caagaagaag gtggagagca agaccgagac cgaccccttc 300
gac 303

<210> 68
<211> 306
<212> DNA
<213> Artificial Sequence

<220>
<223> Description of Artificial Sequence:
      TAT_CYS22_SF162_OPT

<400> 68
atggagcccg tggacccccg cctggagccc tggaagcacc ccggcagcca gcccaagacc 60
gccggcacca actgctactg caagaagtgc tgcttccact gccaggtgtg cttcatcacc 120
aagggcctgg gcatcagcta cggccgcaag aagcgccgcc agcgccgccg cgcccccccc 180
gacagcgagg tgcaccaggt gagcctgccc aagcagcccg ccagccagcc ccagggcgac 240
cccaccggcc ccaaggagag caagaagaag gtggagcgcg agaccgagac cgaccccgtg 300
cactag 306

<210> 69
<211> 306
<212> DNA
<213> Artificial Sequence

<220>
<223> Description of Artificial Sequence: tat_sf162_opt

<400> 69
atggagcccg tggacccccg cctggagccc tggaagcacc ccggcagcca gcccaagacc 60
gcctgcacca actgctactg caagaagtgc tgcttccact gccaggtgtg cttcatcacc 120
aagggcctgg gcatcagcta cggccgcaag aagcgccgcc agcgccgccg cgcccccccc 180
gacagcgagg tgcaccaggt gagcctgccc aagcagcccg ccagccagcc ccagggcgac 240
cccaccggcc ccaaggagag caagaagaag gtggagcgcg agaccgagac cgaccccgtg 300
cactag 306

<210> 70
<211> 309
<212> DNA
<213> Artificial Sequence

<220>
<223> Description of Artificial Sequence:
      TatC22Exon1_2_TV1_C_ZAopt

<400> 70
atggagcccg tggaccccaa gctgaagccc tggaaccacc ccggcagcca gcccaagacc 60
gccggcaaca actgcttctg caagcactgc agctaccact gcctggtgtg cttccagacc 120
aagggcctgg gcatcagcta cggccgcaag aagcgccgcc agcgccgcag cgcccccccc 180
agcggcgagg accaccagaa ccccctgagc aagcagcccc tgccccaggc ccgcggcgac 240
agcaccggca gcgaggagag caagaagaag gtggagagca agaccgagac cgacccctac 300
gactggtga 309

<210> 71
<211> 309
<212> DNA
<213> Artificial Sequence

<220>
<223> Description of Artificial Sequence: description
<400> 71
atggagcccg tggaccccaa gctgaagccc tggaaccacc ccggcagcca gcccaagacc 60
gcctgcaaca actgcttctg caagcactgc agctaccact gcctggtgtg cttccagacc 120
aagggcctgg gcatcagcta cggccgcaag aagcgccgcc agcgccgcag cgcccccccc 180
agcggcgagg accaccagaa ccccctgagc aagcagcccc tgccccaggc ccgcggcgac 240
agcaccggca gcgaggagag caagaagaag gtggagagca agaccgagac cgacccctac 300
gactggtga 309

<210> 72
<211> 1281
<212> DNA
<213> Artificial Sequence

<220>
<223> Description of Artificial Sequence: description

<400> 72
atggagcccg tggacccccg cctggagccc tggaagcacc ccggcagcca gcccaagacc 60
gcctgcacca actgctactg caagaagtgc tgcttccact gccaggtgtg cttcatcacc 120
aagggcctgg gcatcagcta cggccgcaag aagcgccgcc agcgccgccg cgcccccccc 180
gacagcgagg tgcaccaggt gagcctgccc aagcagcccg ccagccagcc ccagggcgac 240
cccaccggcc ccaaggagag caagaagaag gtggagcgcg agaccgagac cgaccccgtg 300
caccccgggg ccggccgcag cggcgacagc gacgaggagc tgctgcagac cgtgcgcttc 360
atcaagttcc tgtaccagag caaccccctg cccagcccca agggcacccg ccaggcccgc 420
cgcaaccgcc gccgccgctg gcgcgagcgc cagcgccaga tccagagcat cagcgcctgg 480
atcatcagca cccacctggg ccgcagcacc gagcccgtgc ccctgcagct gccccccctg 540
gagcgcctga acctggactg cagcgaggac tgcggcacca gcggcaccca gggcgtgggc 600
agcccccagg tgctgggcga gagccccgcc gtgctggaca gcggcaccaa ggagctcgag 660
ggcggcaagt ggagcaagcg catgagcggc tggagcgccg tgcgcgagcg catgaagcgc 720
gccgagcccg ccgagcccgc cgccgacggc gtgggcgccg tgagccgcga cctggagaag 780
cacggcgcca tcaccagcag caacaccgcc gccaacaacg ccgactgcgc ctggctggag 840
gcccaggagg acgaggacgt gggcttcccc gtgcgccccc aggtgcccct gcgccccatg 900
acctacaagg ccgccctgga cctgagccac ttcctgaagg agaagggcgg cctggagggc 960
ctgatctaca gccagaagcg ccaggacatc ctggacctgt ggatccacca cacccagggc 1020
tacttccccg actggcagaa ctacaccccc ggccccggca tccgctaccc cctgaccttc 1080
ggctggtgct tcaagctggt gcccgtggac cccgactacg tggaggaggc caacgccggc 1140
gagaacaaca gcctgctgca ccccatgagc cagcacggca tggacgaccc cgagaaggag 1200
gtgctggtgt ggcgcttcga cagccgcctg gccttccacc acatggcccg cgagctgcac 1260
cccgagtact acaaggactg c 1281

<210> 73
<211> 1281
<212> DNA
<213> Artificial Sequence

<220>
<223> Description of Artificial Sequence:
      TatRevNef.opt.SF162

<400> 73
atggagcccg tggacccccg cctggagccc tggaagcacc ccggcagcca gcccaagacc 60
gccggcacca actgctactg caagaagtgc tgcttccact gccaggtgag cttcatcacc 120
aagggcctgg gcatcagcta cggccgcaag aagcgccgcc agcgccgccg cgcccccccc 180
gacagcgagg tgcaccaggt gagcctgccc aagcagcccg ccagccagcc ccagggcgac 240
cccaccggcc ccaaggagag caagaagaag gtggagcgcg agaccgagac cgaccccgtg 300
caccccgggg ccggccgcag cggcgacagc gacgaggagc tgctgcagac cgtgcgcttc 360
atcaagttcc tgtaccagag caaccccctg cccagcccca agggcacccg ccaggccgac 420
ctgaaccgcc gccgccgctg gcgcgagcgc cagcgccaga tccagagcat cagcgcctgg 480
atcatcagca cccacctggg ccgcagcacc gagcccgtgc ccctgcagct gccccccgac 540
ctgcgcctga acctggactg cagcgaggac tgcggcacca gcggcaccca gggcgtgggc 600
agcccccagg tgctgggcga gagccccgcc gtgctggaca gcggcaccaa ggagctcgag 660
gccggcaagt ggagcaagcg catgagcggc tggagcgccg tgcgcgagcg catgaagcgc 720
gccgagcccg ccgagcccgc cgccgacggc gtgggcgccg tgagccgcga cctggagaag 780
cacggcgcca tcaccagcag caacaccgcc gccaacaacg ccgactgcgc ctggctggag 840
gcccaggagg acgaggacgt gggcttcccc gtgcgccccc aggtgcccct gcgccccatg 900
acctacaagg ccgccctgga cctgagccac ttcctgaagg agaagggcgg cctggagggc 960
ctgatctaca gccagaagcg ccaggacatc ctggacctgt ggatccacca cacccagggc 1020

tacttccccg gctggcagaa ctacaccccc ggccccggca tccgctaccc cctgaccttc 1080
ggctggtgct tcaagctggt gcccgtggac cccgactacg tggaggaggc caacgccggc 1140
gagaacaaca gcctgctgca ccccatgagc cagcacggca tggacgaccc cgagaaggag 1200
gtgctggtgt ggcgcttcga cagccgcctg gccttccacc acatggcccg cgagctgcac 1260
cccgagtact acaaggactg c 1281

<210> 74
<211> 2799
<212> DNA
<213> Artificial Sequence

<220>
<223> Description of Artificial Sequence: TatRevNefGag B

<400> 74
gccaccatgg agcccgtgga cccccgcctg gagccctgga agcaccccgg cagccagccc 60
aagaccgccg gcaccaactg ctactgcaag aagtgctgct tccactgcca ggtgagcttc 120
atcaccaagg gcctgggcat cagctacggc cgcaagaagc gccgccagcg ccgccgcgcc 180
ccccccgaca gcgaggtgca ccaggtgagc ctgcccaagc agcccgccag ccagccccag 240
ggcgacccca ccggccccaa ggagagcaag aagaaggtgg agcgcgagac cgagaccgac 300
cccgtgcacc ccggggccgg ccgcagcggc gacagcgacg aggagctgct gcagaccgtg 360
cgcttcatca agttcctgta ccagagcaac cccctgccca gccccaaggg cacccgccag 420
gccgacctga accgccgccg ccgctggcgc gagcgccagc gccagatcca gagcatcagc 480
gcctggatca tcagcaccca cctgggccgc agcaccgagc ccgtgcccct gcagctgccc 540
cccgacctgc gcctgaacct ggactgcagc gaggactgcg gcaccagcgg cacccagggc 600
gtgggcagcc cccaggtgct gggcgagagc cccgccgtgc tggacagcgg caccaaggag 660
ctcgaggccg gcaagtggag caagcgcatg agcggctgga gcgccgtgcg cgagcgcatg 720
aagcgcgccg agcccgccga gcccgccgcc gacggcgtgg gcgccgtgag ccgcgacctg 780
gagaagcacg gcgccatcac cagcagcaac accgccgcca acaacgccga ctgcgcctgg 840
ctggaggccc aggaggacga ggacgtgggc ttccccgtgc gcccccaggt gcccctgcgc 900
cccatgacct acaaggccgc cctggacctg agccacttcc tgaaggagaa gggcggcctg 960
gagggcctga tctacagcca gaagcgccag gacatcctgg acctgtggat ccaccacacc 1020
cagggctact tccccggctg gcagaactac acccccggcc ccggcatccg ctaccccctg 1080
accttcggct ggtgcttcaa gctggtgccc gtggaccccg actacgtgga ggaggccaac 1140
gccggcgaga acaacagcct gctgcacccc atgagccagc acggcatgga cgaccccgag 1200
aaggaggtgc tggtgtggcg cttcgacagc cgcctggcct tccaccacat ggcccgcgag 1260
ctgcaccccg agtactacaa ggactgcgaa ttcggcgccc gcgccagcgt gctgagcggc 1320
ggcgagctgg acaagtggga gaagatccgc ctgcgccccg gcggcaagaa gaagtacaag 1380
ctgaagcaca tcgtgtgggc cagccgcgag ctggagcgct tcgccgtgaa ccccggcctg 1440
ctggagacca gcgagggctg ccgccagatc ctgggccagc tgcagcccag cctgcagacc 1500
ggcagcgagg agctgcgcag cctgtacaac accgtggcca ccctgtactg cgtgcaccag 1560
cgcatcgacg tcaaggacac caaggaggcc ctggagaaga tcgaggagga gcagaacaag 1620
tccaagaaga aggcccagca ggccgccgcc gccgccggca ccggcaacag cagccaggtg 1680
agccagaact accccatcgt gcagaacctg cagggccaga tggtgcacca ggccatcagc 1740
ccccgcaccc tgaacgcctg ggtgaaggtg gtggaggaga aggccttcag ccccgaggtg 1800
atccccatgt tcagcgccct gagcgagggc gccacccccc aggacctgaa cacgatgttg 1860
aacaccgtgg gcggccacca ggccgccatg cagatgctga aggagaccat caacgaggag 1920
gccgccgagt gggaccgcgt gcaccccgtg cacgccggcc ccatcgcccc cggccagatg 1980
cgcgagcccc gcggcagcga catcgccggc accaccagca ccctgcagga gcagatcggc 2040
tggatgacca acaacccccc catccccgtg ggcgagatct acaagcggtg gatcatcctg 2100
ggcctgaaca agatcgtgcg gatgtacagc cccaccagca tcctggacat ccgccagggc 2160
cccaaggagc ccttccgcga ctacgtggac cgcttctaca agaccctgcg cgctgagcag 2220
gccagccagg acgtgaagaa ctggatgacc gagaccctgc tggtgcagaa cgccaacccc 2280
gactgcaaga ccatcctgaa ggctctcggc cccgcggcca ccctggagga gatgatgacc 2340

gcctgccagg gcgtgggcgg ccccggccac aaggcccgcg tgctggccga ggcgatgagc 2400
caggtgacga acccggcgac catcatgatg cagcgcggca acttccgcaa ccagcggaag 2460
accgtcaagt gcttcaactg cggcaaggag ggccacaccg ccaggaactg ccgcgccccc 2520
cgcaagaagg gctgctggcg ctgcggccgc gagggccacc agatgaagga ctgcaccgag 2580
cgccaggcca acttcctggg caagatctgg cccagctaca agggccgccc cggcaacttc 2640
ctgcagagcc gccccgagcc caccgccccc cccgaggaga gcttccgctt cggcgaggag 2700
aagaccaccc ccagccagaa gcaggagccc atcgacaagg agctgtaccc cctgaccagc 2760
ctgcgcagcc tgttcggcaa cgaccccagc agccagtaa 2799

<210> 75
<211> 5283
<212> DNA
<213> Artificial Sequence

<220>
<223> Description of Artificial Sequence:
      TatRevNefgagCpolIna B

<400> 75
gtcgacgcca ccatggagcc cgtggacccc cgcctggagc cctggaagca ccccggcagc 60
cagcccaaga ccgccggcac caactgctac tgcaagaagt gctgcttcca ctgccaggtg 120
agcttcatca ccaagggcct gggcatcagc tacggccgca agaagcgccg ccagcgccgc 180
cgcgcccccc ccgacagcga ggtgcaccag gtgagcctgc ccaagcagcc cgccagccag 240
ccccagggcg accccaccgg ccccaaggag agcaagaaga aggtggagcg cgagaccgag 300
accgaccccg tgcaccccgg ggccggccgc agcggcgaca gcgacgagga gctgctgcag 360
accgtgcgct tcatcaagtt cctgtaccag agcaaccccc tgcccagccc caagggcacc 420
cgccaggccg acctgaaccg ccgccgccgc tggcgcgagc gccagcgcca gatccagagc 480
atcagcgcct ggatcatcag cacccacctg ggccgcagca ccgagcccgt gcccctgcag 540
ctgccccccg acctgcgcct gaacctggac tgcagcgagg actgcggcac cagcggcacc 600
cagggcgtgg gcagccccca ggtgctgggc gagagccccg ccgtgctgga cagcggcacc 660
aaggagctcg aggccggcaa gtggagcaag cgcatgagcg gctggagcgc cgtgcgcgag 720
cgcatgaagc gcgccgagcc cgccgagccc gccgccgacg gcgtgggcgc cgtgagccgc 780
gacctggaga agcacggcgc catcaccagc agcaacaccg ccgccaacaa cgccgactgc 840
gcctggctgg aggcccagga ggacgaggac gtgggcttcc ccgtgcgccc ccaggtgccc 900
ctgcgcccca tgacctacaa ggccgccctg gacctgagcc acttcctgaa ggagaagggc 960
ggcctggagg gcctgatcta cagccagaag cgccaggaca tcctggacct gtggatccac 1020
cacacccagg gctacttccc cggctggcag aactacaccc ccggccccgg catccgctac 1080
cccctgacct tcggctggtg cttcaagctg gtgcccgtgg accccgacta cgtggaggag 1140
gccaacgccg gcgagaacaa cagcctgctg caccccatga gccagcacgg catggacgac 1200
cccgagaagg aggtgctggt gtggcgcttc gacagccgcc tggccttcca ccacatggcc 1260
cgcgagctgc accccgagta ctacaaggac tgcctcgagg gcgcccgcgc cagcgtgctg 1320
agcggcggcg agctggacaa gtgggagaag atccgcctgc gccccggcgg caagaagaag 1380
tacaagctga agcacatcgt gtgggccagc cgcgagctgg agcgcttcgc cgtgaacccc 1440
ggcctgctgg agaccagcga gggctgccgc cagatcctgg gccagctgca gcccagcctg 1500
cagaccggca gcgaggagct gcgcagcctg tacaacaccg tggccaccct gtactgcgtg 1560
caccagcgca tcgacgtcaa ggacaccaag gaggccctgg agaagatcga ggaggagcag 1620
aacaagtcca agaagaaggc ccagcaggcc gccgccgccg ccggcaccgg caacagcagc 1680
caggtgagcc agaactaccc catcgtgcag aacctgcagg gccagatggt gcaccaggcc 1740
atcagccccc gcaccctgaa cgcctgggtg aaggtggtgg aggagaaggc cttcagcccc 1800
gaggtgatcc ccatgttcag cgccctgagc gagggcgcca ccccccagga cctgaacacg 1860
atgttgaaca ccgtgggcgg ccaccaggcc gccatgcaga tgctgaagga gaccatcaac 1920
gaggaggccg ccgagtggga ccgcgtgcac cccgtgcacg ccggccccat cgcccccggc 1980
cagatgcgcg agccccgcgg cagcgacatc gccggcacca ccagcaccct gcaggagcag 2040
atcggctgga tgaccaacaa cccccccatc cccgtgggcg agatctacaa gcggtggatc 2100

atcctgggcc tgaacaagat cgtgcggatg tacagcccca ccagcatcct ggacatccgc 2160
cagggcccca aggagccctt ccgcgactac gtggaccgct tctacaagac cctgcgcgct 2220
gagcaggcca gccaggacgt gaagaactgg atgaccgaga ccctgctggt gcagaacgcc 2280
aaccccgact gcaagaccat cctgaaggct ctcggccccg cggccaccct ggaggagatg 2340
atgaccgcct gccagggcgt gggcggcccc ggccacaagg cccgcgtgct ggccgaggcg 2400
atgagccagg tgacgaaccc ggcgaccatc atgatgcagc gcggcaactt ccgcaaccag 2460
cggaagaccg tcaagtgctt caactgcggc aaggagggcc acaccgccag gaactgccgc 2520
gccccccgca agaagggctg ctggcgctgc ggccgcgagg gccaccagat gaaggactgc 2580
accgagcgcc aggccaactt cctgggcaag atctggccca gctacaaggg ccgccccggc 2640
aacttcctgc agagccgccc cgagcccacc gccccccccg aggagagctt ccgcttcggc 2700
gaggagaaga ccacccccag ccagaagcag gagcccatcg acaaggagct gtaccccctg 2760
accagcctgc gcagcctgtt cggcaacgac cccagcagcc agaaagaatt caaggcccgc 2820
gtgctggccg aggcgatgag ccaggtgacg aacccggcga ccatcatgat gcagcgcggc 2880
aacttccgca accagcggaa gaccgtcaag tgcttcaact gcggcaagga gggccacacc 2940
gccaggaact gccgcgcccc ccgcaagaag ggctgctggc gctgcggccg cgaaggacac 3000
caaatgaaag attgcactga gagacaggct aatttcttcc gcgaggacct ggccttcctg 3060
cagggcaagg cccgcgagtt cagcagcgag cagacccgcg ccaacagccc cacccgccgc 3120
gagctgcagg tgtggggcgg cgagaacaac agcctgagcg aggccggcgc cgaccgccag 3180
ggcaccgtga gcttcaactt cccccagatc accctgtggc agcgccccct ggtgaccatc 3240
aggatcggcg gccagctcaa ggaggcgctg ctcgccaccg gcgccgacga caccgtgctg 3300
gaggagatga acctgcccgg caagtggaag cccaagatga tcggcgggat cgggggcttc 3360
atcaaggtgc ggcagtacga ccagatcccc gtggagatct gcggccacaa ggccatcggc 3420
accgtgctgg tgggccccac ccccgtgaac atcatcggcc gcaacctgct gacccagatc 3480
ggctgcaccc tgaacttccc catcagcccc atcgagacgg tgcccgtgaa gctgaagccg 3540
gggatggacg gccccaaggt caagcagtgg cccctgaccg aggagaagat caaggccctg 3600
gtggagatct gcaccgagat ggagaaggag ggcaagatca gcaagatcgg ccccgagaac 3660
ccctacaaca cccccgtgtt cgccatcaag aagaaggaca gcaccaagtg gcgcaagctg 3720
gtggacttcc gcgagctgaa caagcgcacc caggacttct gggaggtgca gctgggcatc 3780
ccccaccccg ccggcctgaa gaagaagaag agcgtgaccg tgctggacgt gggcgacgcc 3840
tacttcagcg tgcccctgga caaggacttc cgcaagtaca ccgccttcac catccccagc 3900
atcaacaacg agacccccgg catccgctac cagtacaacg tgctgcccca gggctggaag 3960
ggcagccccg ccatcttcca gagcagcatg accaagatcc tggagccctt ccgcaagcag 4020
aaccccgaca tcgtgatcta ccaggccccc ctgtacgtgg gcagcgacct ggagatcggc 4080
cagcaccgca ccaagatcga ggagctgcgc cagcacctgc tgcgctgggg cttcaccacc 4140
cccgacaaga agcaccagaa ggagcccccc ttcctgccca tcgagctgca ccccgacaag 4200
tggaccgtgc agcccatcat gctgcccgag aaggacagct ggaccgtgaa cgacatccag 4260
aagctggtgg gcaagctgaa ctgggccagc cagatctacg ccggcatcaa ggtgaagcag 4320
ctgtgcaagc tgctgcgcgg caccaaggcc ctgaccgagg tgatccccct gaccgaggag 4380
gccgagctgg agctggccga gaaccgcgag atcctgaagg agcccgtgca cgaggtgtac 4440
tacgacccca gcaaggacct ggtggccgag atccagaagc agggccaggg ccagtggacc 4500
taccagatct accaggagcc cttcaagaac ctgaagaccg gcaagtacgc ccgcatgcgc 4560
ggcgcccaca ccaacgacgt gaagcagctg accgaggccg tgcagaaggt gagcaccgag 4620
agcatcgtga tctggggcaa gatccccaag ttcaagctgc ccatccagaa ggagacctgg 4680
gaggcctggt ggatggagta ctggcaggcc acctggatcc ccgagtggga gttcgtgaac 4740
accccccccc tggtgaagct gtggtaccag ctggagaagg agcccatcgt gggcgccgag 4800
accttctacg tggacggcgc cgccaaccgc gagaccaagc tgggcaaggc cggctacgtg 4860
accgaccggg gccggcagaa ggtggtgagc atcgccgaca ccaccaacca gaagaccgag 4920
ctgcaggcca tccacctggc cctgcaggac agcggcctgg aggtgaacat cgtgaccgac 4980
agccagtacg ccctgggcat catccaggcc cagcccgaca agagcgagag cgagctggtg 5040
agccagatca tcgagcagct gatcaagaag gagaaggtgt acctggcctg ggtgcccgcc 5100
cacaagggca tcggcggcaa cgagcaggtg gacaagctgg tgagcgccgg catccgcaag 5160
gtgctgttcc tgaacggcat cgatggcggc atcgtgatct accagtacat ggacgacctg 5220
tacgtgggca gcggcggccc taggatcgat taaaagcttc ccggggctag caccggttct 5280
aga 5283

<210> 76
<211> 4773
<212> DNA
<213> Artificial Sequence

<220>
<223> Description of Artificial Sequence:
      TatRevNefGagProtInaRTmut B

<400> 76
gccaccatgg agcccgtgga cccccgcctg gagccctgga agcaccccgg cagccagccc 60
aagaccgccg gcaccaactg ctactgcaag aagtgctgct tccactgcca ggtgagcttc 120
atcaccaagg gcctgggcat cagctacggc cgcaagaagc gccgccagcg ccgccgcgcc 180
ccccccgaca gcgaggtgca ccaggtgagc ctgcccaagc agcccgccag ccagccccag 240
ggcgacccca ccggccccaa ggagagcaag aagaaggtgg agcgcgagac cgagaccgac 300
cccgtgcacc ccggggccgg ccgcagcggc gacagcgacg aggagctgct gcagaccgtg 360
cgcttcatca agttcctgta ccagagcaac cccctgccca gccccaaggg cacccgccag 420
gccgacctga accgccgccg ccgctggcgc gagcgccagc gccagatcca gagcatcagc 480
gcctggatca tcagcaccca cctgggccgc agcaccgagc ccgtgcccct gcagctgccc 540
cccgacctgc gcctgaacct ggactgcagc gaggactgcg gcaccagcgg cacccagggc 600
gtgggcagcc cccaggtgct gggcgagagc cccgccgtgc tggacagcgg caccaaggag 660
ctcgaggccg gcaagtggag caagcgcatg agcggctgga gcgccgtgcg cgagcgcatg 720
aagcgcgccg agcccgccga gcccgccgcc gacggcgtgg gcgccgtgag ccgcgacctg 780
gagaagcacg gcgccatcac cagcagcaac accgccgcca acaacgccga ctgcgcctgg 840
ctggaggccc aggaggacga ggacgtgggc ttccccgtgc gcccccaggt gcccctgcgc 900
cccatgacct acaaggccgc cctggacctg agccacttcc tgaaggagaa gggcggcctg 960
gagggcctga tctacagcca gaagcgccag gacatcctgg acctgtggat ccaccacacc 1020
cagggctact tccccggctg gcagaactac acccccggcc ccggcatccg ctaccccctg 1080
accttcggct ggtgcttcaa gctggtgccc gtggaccccg actacgtgga ggaggccaac 1140
gccggcgaga acaacagcct gctgcacccc atgagccagc acggcatgga cgaccccgag 1200
aaggaggtgc tggtgtggcg cttcgacagc cgcctggcct tccaccacat ggcccgcgag 1260
ctgcaccccg agtactacaa ggactgcaag cttggcgccc gcgccagcgt gctgagcggc 1320
ggcgagctgg acaagtggga gaagatccgc ctgcgccccg gcggcaagaa gaagtacaag 1380
ctgaagcaca tcgtgtgggc cagccgcgag ctggagcgct tcgccgtgaa ccccggcctg 1440
ctggagacca gcgagggctg ccgccagatc ctgggccagc tgcagcccag cctgcagacc 1500
ggcagcgagg agctgcgcag cctgtacaac accgtggcca ccctgtactg cgtgcaccag 1560
cgcatcgacg tcaaggacac caaggaggcc ctggagaaga tcgaggagga gcagaacaag 1620
tccaagaaga aggcccagca ggccgccgcc gccgccggca ccggcaacag cagccaggtg 1680
agccagaact accccatcgt gcagaacctg cagggccaga tggtgcacca ggccatcagc 1740
ccccgcaccc tgaacgcctg ggtgaaggtg gtggaggaga aggccttcag ccccgaggtg 1800
atccccatgt tcagcgccct gagcgagggc gccacccccc aggacctgaa cacgatgttg 1860
aacaccgtgg gcggccacca ggccgccatg cagatgctga aggagaccat caacgaggag 1920
gccgccgagt gggaccgcgt gcaccccgtg cacgccggcc ccatcgcccc cggccagatg 1980
cgcgagcccc gcggcagcga catcgccggc accaccagca ccctgcagga gcagatcggc 2040
tggatgacca acaacccccc catccccgtg ggcgagatct acaagcggtg gatcatcctg 2100
ggcctgaaca agatcgtgcg gatgtacagc cccaccagca tcctggacat ccgccagggc 2160
cccaaggagc ccttccgcga ctacgtggac cgcttctaca agaccctgcg cgctgagcag 2220
gccagccagg acgtgaagaa ctggatgacc gagaccctgc tggtgcagaa cgccaacccc 2280
gactgcaaga ccatcctgaa ggctctcggc cccgcggcca ccctggagga gatgatgacc 2340
gcctgccagg gcgtgggcgg ccccggccac aaggcccgcg tgctggccga ggcgatgagc 2400
caggtgacga acccggcgac catcatgatg cagcgcggca acttccgcaa ccagcggaag 2460
accgtcaagt gcttcaactg cggcaaggag ggccacaccg ccaggaactg ccgcgccccc 2520
cgcaagaagg gctgctggcg ctgcggccgc gagggccacc agatgaagga ctgcaccgag 2580

cgccaggcca acttcctggg caagatctgg cccagctaca agggccgccc cggcaacttc 2640
ctgcagagcc gccccgagcc caccgccccc cccgaggaga gcttccgctt cggcgaggag 2700
aagaccaccc ccagccagaa gcaggagccc atcgacaagg agctgtaccc cctgaccagc 2760
ctgcgcagcc tgttcggcaa cgaccccagc agccagaaag aattccccca gatcaccctg 2820
tggcagcgcc ccctggtgac catcaggatc ggcggccagc tcaaggaggc gctgctcgcc 2880
accggcgccg acgacaccgt gctggaggag atgaacctgc ccggcaagtg gaagcccaag 2940
atgatcggcg ggatcggggg cttcatcaag gtgcggcagt acgaccagat ccccgtggag 3000
atctgcggcc acaaggccat cggcaccgtg ctggtgggcc ccacccccgt gaacatcatc 3060
ggccgcaacc tgctgaccca gatcggctgc accctgaact tccccatcag ccccatcgag 3120
acggtgcccg tgaagctgaa gccggggatg gacggcccca aggtcaagca gtggcccctg 3180
accgaggaga agatcaaggc cctggtggag atctgcaccg agatggagaa ggagggcaag 3240
atcagcaaga tcggccccga gaacccctac aacacccccg tgttcgccat caagaagaag 3300
gacagcacca agtggcgcaa gctggtggac ttccgcgagc tgaacaagcg cacccaggac 3360
ttctgggagg tgcagctggg catcccccac cccgccggcc tgaagaagaa gaagagcgtg 3420
accgtgctgg acgtgggcga cgcctacttc agcgtgcccc tggacaagga cttccgcaag 3480
tacaccgcct tcaccatccc cagcatcaac aacgagaccc ccggcatccg ctaccagtac 3540
aacgtgctgc cccagggctg gaagggcagc cccgccatct tccagagcag catgaccaag 3600
atcctggagc ccttccgcaa gcagaacccc gacatcgtga tctaccaggc ccccctgtac 3660
gtgggcagcg acctggagat cggccagcac cgcaccaaga tcgaggagct gcgccagcac 3720
ctgctgcgct ggggcttcac cacccccgac aagaagcacc agaaggagcc ccccttcctg 3780
cccatcgagc tgcaccccga caagtggacc gtgcagccca tcatgctgcc cgagaaggac 3840
agctggaccg tgaacgacat ccagaagctg gtgggcaagc tgaactgggc cagccagatc 3900
tacgccggca tcaaggtgaa gcagctgtgc aagctgctgc gcggcaccaa ggccctgacc 3960
gaggtgatcc ccctgaccga ggaggccgag ctggagctgg ccgagaaccg cgagatcctg 4020
aaggagcccg tgcacgaggt gtactacgac cccagcaagg acctggtggc cgagatccag 4080
aagcagggcc agggccagtg gacctaccag atctaccagg agcccttcaa gaacctgaag 4140
accggcaagt acgcccgcat gcgcggcgcc cacaccaacg acgtgaagca gctgaccgag 4200
gccgtgcaga aggtgagcac cgagagcatc gtgatctggg gcaagatccc caagttcaag 4260
ctgcccatcc agaaggagac ctgggaggcc tggtggatgg agtactggca ggccacctgg 4320
atccccgagt gggagttcgt gaacaccccc cccctggtga agctgtggta ccagctggag 4380
aaggagccca tcgtgggcgc cgagaccttc tacgtggacg gcgccgccaa ccgcgagacc 4440
aagctgggca aggccggcta cgtgaccgac cggggccggc agaaggtggt gagcatcgcc 4500
gacaccacca accagaagac cgagctgcag gccatccacc tggccctgca ggacagcggc 4560
ctggaggtga acatcgtgac cgacagccag tacgccctgg gcatcatcca ggcccagccc 4620
gacaagagcg agagcgagct ggtgagccag atcatcgagc agctgatcaa gaaggagaag 4680
gtgtacctgg cctgggtgcc cgcccacaag ggcatcggcg gcaacgagca ggtggacaag 4740
ctggtgagcg ccggcatccg caaggtgctc taa 4773

<210> 77
<211> 3636
<212> DNA
<213> Artificial Sequence

<220>
<223> Description of Artificial Sequence:
      TatRevNefp2Pol.opt_B

<400> 77
gccaccatgg agcccgtgga cccccgcctg gagccctgga agcaccccgg cagccagccc 60
aagaccgccg gcaccaactg ctactgcaag aagtgctgct tccactgcca ggtgagcttc 120
atcaccaagg gcctgggcat cagctacggc cgcaagaagc gccgccagcg ccgccgcgcc 180
ccccccgaca gcgaggtgca ccaggtgagc ctgcccaagc agcccgccag ccagccccag 240
ggcgacccca ccggccccaa ggagagcaag aagaaggtgg agcgcgagac cgagaccgac 300
cccgtgcacc ccggggccgg ccgcagcggc gacagcgacg aggagctgct gcagaccgtg 360

cgcttcatca agttcctgta ccagagcaac cccctgccca gccccaaggg cacccgccag 420
gccgacctga accgccgccg ccgctggcgc gagcgccagc gccagatcca gagcatcagc 480
gcctggatca tcagcaccca cctgggccgc agcaccgagc ccgtgcccct gcagctgccc 540
cccgacctgc gcctgaacct ggactgcagc gaggactgcg gcaccagcgg cacccagggc 600
gtgggcagcc cccaggtgct gggcgagagc cccgccgtgc tggacagcgg caccaaggag 660
ctcgaggccg gcaagtggag caagcgcatg agcggctgga gcgccgtgcg cgagcgcatg 720
aagcgcgccg agcccgccga gcccgccgcc gacggcgtgg gcgccgtgag ccgcgacctg 780
gagaagcacg gcgccatcac cagcagcaac accgccgcca acaacgccga ctgcgcctgg 840
ctggaggccc aggaggacga ggacgtgggc ttccccgtgc gcccccaggt gcccctgcgc 900
cccatgacct acaaggccgc cctggacctg agccacttcc tgaaggagaa gggcggcctg 960
gagggcctga tctacagcca gaagcgccag gacatcctgg acctgtggat ccaccacacc 1020
cagggctact tccccggctg gcagaactac acccccggcc ccggcatccg ctaccccctg 1080
accttcggct ggtgcttcaa gctggtgccc gtggaccccg actacgtgga ggaggccaac 1140
gccggcgaga acaacagcct gctgcacccc atgagccagc acggcatgga cgaccccgag 1200
aaggaggtgc tggtgtggcg cttcgacagc cgcctggcct tccaccacat ggcccgcgag 1260
ctgcaccccg agtactacaa ggactgcgaa ttcgccgagg cgatgagcca ggtgacgaac 1320
ccggcgacca tcatgatgca gcgcggcaac ttccgcaacc agcggaagac cgtcaagtgc 1380
ttcaactgcg gcaaggaggg ccacaccgcc aggaactgcc gcgccccccg caagaagggc 1440
tgctggcgct gcggccgcga aggacaccaa atgaaagatt gcactgagag acaggctaat 1500
ttcttccgcg aggacctggc cttcctgcag ggcaaggccc gcgagttcag cagcgagcag 1560
acccgcgcca acagccccac ccgccgcgag ctgcaggtgt ggggcggcga gaacaacagc 1620
ctgagcgagg ccggcgccga ccgccagggc accgtgagct tcaacttccc ccagatcacc 1680
ctgtggcagc gccccctggt gaccatcagg atcggcggcc agctcaagga ggcgctgctc 1740
gccaccggcg ccgacgacac cgtgctggag gagatgaacc tgcccggcaa gtggaagccc 1800
aagatgatcg gcgggatcgg gggcttcatc aaggtgcggc agtacgacca gatccccgtg 1860
gagatctgcg gccacaaggc catcggcacc gtgctggtgg gccccacccc cgtgaacatc 1920
atcggccgca acctgctgac ccagatcggc tgcaccctga acttccccat cagccccatc 1980
gagacggtgc ccgtgaagct gaagccgggg atggacggcc ccaaggtcaa gcagtggccc 2040
ctgaccgagg agaagatcaa ggccctggtg gagatctgca ccgagatgga gaaggagggc 2100
aagatcagca agatcggccc cgagaacccc tacaacaccc ccgtgttcgc catcaagaag 2160
aaggacagca ccaagtggcg caagctggtg gacttccgcg agctgaacaa gcgcacccag 2220
gacttctggg aggtgcagct gggcatcccc caccccgccg gcctgaagaa gaagaagagc 2280
gtgaccgtgc tggacgtggg cgacgcctac ttcagcgtgc ccctggacaa ggacttccgc 2340
aagtacaccg ccttcaccat ccccagcatc aacaacgaga cccccggcat ccgctaccag 2400
tacaacgtgc tgccccaggg ctggaagggc agccccgcca tcttccagag cagcatgacc 2460
aagatcctgg agcccttccg caagcagaac cccgacatcg tgatctacca ggcccccctg 2520
tacgtgggca gcgacctgga gatcggccag caccgcacca agatcgagga gctgcgccag 2580
cacctgctgc gctggggctt caccaccccc gacaagaagc accagaagga gccccccttc 2640
ctgcccatcg agctgcaccc cgacaagtgg accgtgcagc ccatcatgct gcccgagaag 2700
gacagctgga ccgtgaacga catccagaag ctggtgggca agctgaactg ggccagccag 2760
atctacgccg gcatcaaggt gaagcagctg tgcaagctgc tgcgcggcac caaggccctg 2820
accgaggtga tccccctgac cgaggaggcc gagctggagc tggccgagaa ccgcgagatc 2880
ctgaaggagc ccgtgcacga ggtgtactac gaccccagca aggacctggt ggccgagatc 2940
cagaagcagg gccagggcca gtggacctac cagatctacc aggagccctt caagaacctg 3000
aagaccggca agtacgcccg catgcgcggc gcccacacca acgacgtgaa gcagctgacc 3060
gaggccgtgc agaaggtgag caccgagagc atcgtgatct ggggcaagat ccccaagttc 3120
aagctgccca tccagaagga gacctgggag gcctggtgga tggagtactg gcaggccacc 3180
tggatccccg agtgggagtt cgtgaacacc ccccccctgg tgaagctgtg gtaccagctg 3240
gagaaggagc ccatcgtggg cgccgagacc ttctacgtgg acggcgccgc caaccgcgag 3300
accaagctgg gcaaggccgg ctacgtgacc gaccggggcc ggcagaaggt ggtgagcatc 3360
gccgacacca ccaaccagaa gaccgagctg caggccatcc acctggccct gcaggacagc 3420
ggcctggagg tgaacatcgt gaccgacagc cagtacgccc tgggcatcat ccaggcccag 3480
cccgacaaga gcgagagcga gctggtgagc cagatcatcg agcagctgat caagaaggag 3540
aaggtgtacc tggcctgggt gcccgcccac aagggcatcg gcggcaacga gcaggtggac 3600

aagctggtga gcgccggcat ccgcaaggtg ctgtaa 3636

<210> 78
<211> 3261
<212> DNA
<213> Artificial Sequence

<220>
<223> Description of Artificial Sequence:
      TatRevNefprotRTopt B

<400> 78
gccaccatgg agcccgtgga cccccgcctg gagccctgga agcaccccgg cagccagccc 60
aagaccgccg gcaccaactg ctactgcaag aagtgctgct tccactgcca ggtgagcttc 120
atcaccaagg gcctgggcat cagctacggc cgcaagaagc gccgccagcg ccgccgcgcc 180
ccccccgaca gcgaggtgca ccaggtgagc ctgcccaagc agcccgccag ccagccccag 240
ggcgacccca ccggccccaa ggagagcaag aagaaggtgg agcgcgagac cgagaccgac 300
cccgtgcacc ccggggccgg ccgcagcggc gacagcgacg aggagctgct gcagaccgtg 360
cgcttcatca agttcctgta ccagagcaac cccctgccca gccccaaggg cacccgccag 420
gccgacctga accgccgccg ccgctggcgc gagcgccagc gccagatcca gagcatcagc 480
gcctggatca tcagcaccca cctgggccgc agcaccgagc ccgtgcccct gcagctgccc 540
cccgacctgc gcctgaacct ggactgcagc gaggactgcg gcaccagcgg cacccagggc 600
gtgggcagcc cccaggtgct gggcgagagc cccgccgtgc tggacagcgg caccaaggag 660
ctcgaggccg gcaagtggag caagcgcatg agcggctgga gcgccgtgcg cgagcgcatg 720
aagcgcgccg agcccgccga gcccgccgcc gacggcgtgg gcgccgtgag ccgcgacctg 780
gagaagcacg gcgccatcac cagcagcaac accgccgcca acaacgccga ctgcgcctgg 840
ctggaggccc aggaggacga ggacgtgggc ttccccgtgc gcccccaggt gcccctgcgc 900
cccatgacct acaaggccgc cctggacctg agccacttcc tgaaggagaa gggcggcctg 960
gagggcctga tctacagcca gaagcgccag gacatcctgg acctgtggat ccaccacacc 1020
cagggctact tccccggctg gcagaactac acccccggcc ccggcatccg ctaccccctg 1080
accttcggct ggtgcttcaa gctggtgccc gtggaccccg actacgtgga ggaggccaac 1140
gccggcgaga acaacagcct gctgcacccc atgagccagc acggcatgga cgaccccgag 1200
aaggaggtgc tggtgtggcg cttcgacagc cgcctggcct tccaccacat ggcccgcgag 1260
ctgcaccccg agtactacaa ggactgcgaa ttcccccaga tcaccctgtg gcagcgcccc 1320
ctggtgacca tcaggatcgg cggccagctc aaggaggcgc tgctcgccac cggcgccgac 1380
gacaccgtgc tggaggagat gaacctgccc ggcaagtgga agcccaagat gatcggcggg 1440
atcgggggct tcatcaaggt gcggcagtac gaccagatcc ccgtggagat ctgcggccac 1500
aaggccatcg gcaccgtgct ggtgggcccc acccccgtga acatcatcgg ccgcaacctg 1560
ctgacccaga tcggctgcac cctgaacttc cccatcagcc ccatcgagac ggtgcccgtg 1620
aagctgaagc cggggatgga cggccccaag gtcaagcagt ggcccctgac cgaggagaag 1680
atcaaggccc tggtggagat ctgcaccgag atggagaagg agggcaagat cagcaagatc 1740
ggccccgaga acccctacaa cacccccgtg ttcgccatca agaagaagga cagcaccaag 1800
tggcgcaagc tggtggactt ccgcgagctg aacaagcgca cccaggactt ctgggaggtg 1860
cagctgggca tcccccaccc cgccggcctg aagaagaaga agagcgtgac cgtgctggac 1920
gtgggcgacg cctacttcag cgtgcccctg gacaaggact tccgcaagta caccgccttc 1980
accatcccca gcatcaacaa cgagaccccc ggcatccgct accagtacaa cgtgctgccc 2040
cagggctgga agggcagccc cgccatcttc cagagcagca tgaccaagat cctggagccc 2100
ttccgcaagc agaaccccga catcgtgatc taccaggccc ccctgtacgt gggcagcgac 2160
ctggagatcg gccagcaccg caccaagatc gaggagctgc gccagcacct gctgcgctgg 2220
ggcttcacca cccccgacaa gaagcaccag aaggagcccc ccttcctgcc catcgagctg 2280
caccccgaca agtggaccgt gcagcccatc atgctgcccg agaaggacag ctggaccgtg 2340
aacgacatcc agaagctggt gggcaagctg aactgggcca gccagatcta cgccggcatc 2400
aaggtgaagc agctgtgcaa gctgctgcgc ggcaccaagg ccctgaccga ggtgatcccc 2460
ctgaccgagg aggccgagct ggagctggcc gagaaccgcg agatcctgaa ggagcccgtg 2520

cacgaggtgt actacgaccc cagcaaggac ctggtggccg agatccagaa gcagggccag 2580
ggccagtgga cctaccagat ctaccaggag cccttcaaga acctgaagac cggcaagtac 2640
gcccgcatgc gcggcgccca caccaacgac gtgaagcagc tgaccgaggc cgtgcagaag 2700
gtgagcaccg agagcatcgt gatctggggc aagatcccca agttcaagct gcccatccag 2760
aaggagacct gggaggcctg gtggatggag tactggcagg ccacctggat ccccgagtgg 2820
gagttcgtga acaccccccc cctggtgaag ctgtggtacc agctggagaa ggagcccatc 2880
gtgggcgccg agaccttcta cgtggacggc gccgccaacc gcgagaccaa gctgggcaag 2940
gccggctacg tgaccgaccg gggccggcag aaggtggtga gcatcgccga caccaccaac 3000
cagaagaccg agctgcaggc catccacctg gccctgcagg acagcggcct ggaggtgaac 3060
atcgtgaccg acagccagta cgccctgggc atcatccagg cccagcccga caagagcgag 3120
agcgagctgg tgagccagat catcgagcag ctgatcaaga aggagaaggt gtacctggcc 3180
tgggtgcccg cccacaaggg catcggcggc aacgagcagg tggacaagct ggtgagcgcc 3240
ggcatccgca aggtgctcta a 3261

<210> 79
<211> 1275
<212> DNA
<213> Artificial Sequence

<220>
<223> Description of Artificial Sequence:
      TatRevNef.opt.native_ZA

<400> 79
atggagcccg tggaccccaa cctggagccc tggaaccacc ccggcagcca gcccaagacc 60
gcctgcaaca agtgctactg caagcactgc agctaccact gcctggtgtg cttccagacc 120
aagggcctgg gcatcagcta cggccgcaag aagcgccgcc agcgccgcag cgcccccccc 180
agcagcgagg accaccagaa ccccatcagc aagcagcccc tgccccagac ccgcggcgac 240
cccaccggca gcgaggagag caagaagaag gtggagagca agaccgagac cgaccccttc 300
gaccccgggg ccggccgcag cggcgacagc gacgaggccc tgctgcaggc cgtgcgcatc 360
atcaagatcc tgtaccagag caacccctac cccaagcccg agggcacccg ccaggcccgc 420
aagaaccgcc gccgccgctg gcgcgcccgc cagcgccaga tccacagcat cagcgagcgc 480
atcctgagca cctgcctggg ccgccccgcc gagcccgtgc ccttccagct gccccccatc 540
gagcgcctgc acatcgactg cagcgagagc agcggcacca gcggcaccca gcagagccag 600
ggcaccaccg agggcgtggg cagccccctc gagggcggca agtggagcaa gagcagcatc 660
gtgggctggc ccgccgtgcg cgagcgcatc cgccgcaccg agcccgcccg cgagggcgcc 720
gccgagggcg ccgccgaggg cgtgggcgcc gccagccagg acctggacaa gcacggcgcc 780
ctgaccagca gcaacaccgc cgccaacaac gccgactgcg cctggctgga ggcccaggag 840
gaggaggagg aggtgggctt ccccgtgcgc ccccaggtgc ccctgcgccc catgacctac 900
aaggccgcct tcgacctgag cttcttcctg aaggagaagg gcggcctgga gggcctgatc 960
tacagcaaga agcgccagga gatcctggac ctgtgggtgt accacaccca gggcttcttc 1020
cccgactggc agaactacac ccccggcccc ggcgtgcgct accccctgac cttcggctgg 1080
tgcttcaagc tggtgcccgt ggacccccgc gaggtggagg aggccaacaa gggcgagaac 1140
aactgcctgc tgcaccccat gagccagcac ggcatggagg acgaggaccg cgaggtgctg 1200
aagtggaagt tcgacagcag cctggcccgc cgccacatgg cccgcgagct gcaccccgag 1260
tactacaagg actgc 1275

<210> 80
<211> 1257
<212> DNA
<213> Artificial Sequence

<220>
<223> Description of Artificial Sequence: description

<400> 80
atggagcccg tggaccccaa cctggagccc tggaaccacc ccggcagcca gcccaagacc 60
gccggcaaca agtgctactg caagcactgc agctaccact gcctggtgag cttccagacc 120
aagggcctgg gcatcagcta cggccgcaag aagcgccgcc agcgccgcag cgcccccccc 180
agcagcgagg accaccagaa ccccatcagc aagcagcccc tgccccagac ccgcggcgac 240
cccaccggca gcgaggagag caagaagaag gtggagagca agaccgagac cgaccccttc 300
gaccccgggg ccggccgcag cggcgacagc gacgaggccc tgctgcaggc cgtgcgcatc 360
atcaagatcc tgtaccagag caacccctac cccaagcccg agggcacccg ccaggccgac 420
ctgaaccgcc gccgccgctg gcgcgcccgc cagcgccaga tccacagcat cagcgagcgc 480
atcctgagca cctgcctggg ccgccccgcc gagcccgtgc ccttccagct gccccccgac 540
ctgcgcctgc acatcgactg cagcgagagc agcggcacca gcggcaccca gcagagccag 600
ggcaccaccg agggcgtggg cagccccctc gaggccggca agtggagcaa gagcagcatc 660
gtgggctggc ccgccgtgcg cgagcgcatc cgccgcaccg agcccgccgc cgagggcgtg 720
ggcgccgcca gccaggacct ggacaagcac ggcgccctga ccagcagcaa caccgccgcc 780
aacaacgccg actgcgcctg gctggaggcc caggaggagg aggaggaggt gggcttcccc 840
gtgcgccccc aggtgcccct gcgccccatg acctacaagg ccgccttcga cctgagcttc 900
ttcctgaagg agaagggcgg cctggagggc ctgatctaca gcaagaagcg ccaggagatc 960
ctggacctgt gggtgtacca cacccagggc ttcttccccg gctggcagaa ctacaccccc 1020
ggccccggcg tgcgctaccc cctgaccttc ggctggtgct tcaagctggt gcccgtggac 1080
ccccgcgagg tggaggaggc caacaagggc gagaacaact gcctgctgca ccccatgagc 1140
cagcacggca tggaggacga ggaccgcgag gtgctgaagt ggaagttcga cagcagcctg 1200
gcccgccgcc acatggcccg cgagctgcac cccgagtact acaaggactg cgcctaa 1257

<210> 81
<211> 2742
<212> DNA
<213> Artificial Sequence

<220>
<223> Description of Artificial Sequence: TatRevNefGag C

<400> 81
gccaccatgg agcccgtgga ccccaacctg gagccctgga accaccccgg cagccagccc 60
aagaccgccg gcaacaagtg ctactgcaag cactgcagct accactgcct ggtgagcttc 120
cagaccaagg gcctgggcat cagctacggc cgcaagaagc gccgccagcg ccgcagcgcc 180
ccccccagca gcgaggacca ccagaacccc atcagcaagc agcccctgcc ccagacccgc 240
ggcgacccca ccggcagcga ggagagcaag aagaaggtgg agagcaagac cgagaccgac 300
cccttcgacc ccggggccgg ccgcagcggc gacagcgacg aggccctgct gcaggccgtg 360
cgcatcatca agatcctgta ccagagcaac ccctacccca agcccgaggg cacccgccag 420
gccgacctga accgccgccg ccgctggcgc gcccgccagc gccagatcca cagcatcagc 480
gagcgcatcc tgagcacctg cctgggccgc cccgccgagc ccgtgccctt ccagctgccc 540
cccgacctgc gcctgcacat cgactgcagc gagagcagcg gcaccagcgg cacccagcag 600
agccagggca ccaccgaggg cgtgggcagc cccctcgagg ccggcaagtg gagcaagagc 660
agcatcgtgg gctggcccgc cgtgcgcgag cgcatccgcc gcaccgagcc cgccgccgag 720
ggcgtgggcg ccgccagcca ggacctggac aagcacggcg ccctgaccag cagcaacacc 780
gccgccaaca acgccgactg cgcctggctg gaggcccagg aggaggagga ggaggtgggc 840
ttccccgtgc gcccccaggt gcccctgcgc cccatgacct acaaggccgc cttcgacctg 900
agcttcttcc tgaaggagaa gggcggcctg gagggcctga tctacagcaa gaagcgccag 960
gagatcctgg acctgtgggt gtaccacacc cagggcttct tccccggctg gcagaactac 1020
acccccggcc ccggcgtgcg ctaccccctg accttcggct ggtgcttcaa gctggtgccc 1080
gtggaccccc gcgaggtgga ggaggccaac aagggcgaga acaactgcct gctgcacccc 1140
atgagccagc acggcatgga ggacgaggac cgcgaggtgc tgaagtggaa gttcgacagc 1200
agcctggccc gccgccacat ggcccgcgag ctgcaccccg agtactacaa ggactgcgaa 1260

ttcggcgccc gcgccagcat cctgcgcggc ggcaagctgg acgcctggga gcgcatccgc 1320
ctgcgccccg gcggcaagaa gtgctacatg atgaagcacc tggtgtgggc cagccgcgag 1380
ctggagaagt tcgccctgaa ccccggcctg ctggagacca gcgagggctg caagcagatc 1440
atccgccagc tgcaccccgc cctgcagacc ggcagcgagg agctgaagag cctgttcaac 1500
accgtggcca ccctgtactg cgtgcacgag aagatcgagg tccgcgacac caaggaggcc 1560
ctggacaaga tcgaggagga gcagaacaag tgccagcaga agatccagca ggccgaggcc 1620
gccgacaagg gcaaggtgag ccagaactac cccatcgtgc agaacctgca gggccagatg 1680
gtgcaccagg ccatcagccc ccgcaccctg aacgcctggg tgaaggtgat cgaggagaag 1740
gccttcagcc ccgaggtgat ccccatgttc accgccctga gcgagggcgc caccccccag 1800
gacctgaaca cgatgttgaa caccgtgggc ggccaccagg ccgccatgca gatgctgaag 1860
gacaccatca acgaggaggc cgccgagtgg gaccgcgtgc accccgtgca cgccggcccc 1920
atcgcccccg gccagatgcg cgagccccgc ggcagcgaca tcgccggcac caccagcacc 1980
ctgcaggagc agatcgcctg gatgaccagc aaccccccca tccccgtggg cgacatctac 2040
aagcggtgga tcatcctggg cctgaacaag atcgtgcgga tgtacagccc cgtgagcatc 2100
ctggacatca agcagggccc caaggagccc ttccgcgact acgtggaccg cttcttcaag 2160
accctgcgcg ccgagcagag cacccaggag gtgaagaact ggatgaccga caccctgctg 2220
gtgcagaacg ccaaccccga ctgcaagacc atcctgcgcg ctctcggccc cggcgccagc 2280
ctggaggaga tgatgaccgc ctgccagggc gtgggcggcc ccagccacaa ggcccgcgtg 2340
ctggccgagg cgatgagcca ggccaacacc agcgtgatga tgcagaagag caacttcaag 2400
ggcccccggc gcatcgtcaa gtgcttcaac tgcggcaagg agggccacat cgcccgcaac 2460
tgccgcgccc cccgcaagaa gggctgctgg aagtgcggca aggagggcca ccagatgaag 2520
gactgcaccg agcgccaggc caacttcctg ggcaagatct ggcccagcca caagggccgc 2580
cccggcaact tcctgcagag ccgccccgag cccaccgccc cccccgccga gagcttccgc 2640
ttcgaggaga ccacccccgg ccagaagcag gagagcaagg accgcgagac cctgaccagc 2700
ctgaagagcc tgttcggcaa cgaccccctg agccaagcct aa 2742

<210> 82
<211> 5184
<212> DNA
<213> Artificial Sequence

<220>
<223> Description of Artificial Sequence:
      TatRevNefgagCpolIna C

<400> 82
gccaccatgg agcccgtgga ccccaacctg gagccctgga accaccccgg cagccagccc 60
aagaccgccg gcaacaagtg ctactgcaag cactgcagct accactgcct ggtgagcttc 120
cagaccaagg gcctgggcat cagctacggc cgcaagaagc gccgccagcg ccgcagcgcc 180
ccccccagca gcgaggacca ccagaacccc atcagcaagc agcccctgcc ccagacccgc 240
ggcgacccca ccggcagcga ggagagcaag aagaaggtgg agagcaagac cgagaccgac 300
cccttcgacc ccggggccgg ccgcagcggc gacagcgacg aggccctgct gcaggccgtg 360
cgcatcatca agatcctgta ccagagcaac ccctacccca agcccgaggg cacccgccag 420
gccgacctga accgccgccg ccgctggcgc gcccgccagc gccagatcca cagcatcagc 480
gagcgcatcc tgagcacctg cctgggccgc cccgccgagc ccgtgccctt ccagctgccc 540
cccgacctgc gcctgcacat cgactgcagc gagagcagcg gcaccagcgg cacccagcag 600
agccagggca ccaccgaggg cgtgggcagc cccctcgagg ccggcaagtg gagcaagagc 660
agcatcgtgg gctggcccgc cgtgcgcgag cgcatccgcc gcaccgagcc cgccgccgag 720
ggcgtgggcg ccgccagcca ggacctggac aagcacggcg ccctgaccag cagcaacacc 780
gccgccaaca acgccgactg cgcctggctg gaggcccagg aggaggagga ggaggtgggc 840
ttccccgtgc gcccccaggt gcccctgcgc cccatgacct acaaggccgc cttcgacctg 900
agcttcttcc tgaaggagaa gggcggcctg gagggcctga tctacagcaa gaagcgccag 960
gagatcctgg acctgtgggt gtaccacacc cagggcttct tccccggctg gcagaactac 1020
acccccggcc ccggcgtgcg ctaccccctg accttcggct ggtgcttcaa gctggtgccc 1080

gtggaccccc gcgaggtgga ggaggccaac aagggcgaga acaactgcct gctgcacccc 1140
atgagccagc acggcatgga ggacgaggac cgcgaggtgc tgaagtggaa gttcgacagc 1200
agcctggccc gccgccacat ggcccgcgag ctgcaccccg agtactacaa ggactgcctc 1260
gagggcgccc gcgccagcat cctgcgcggc ggcaagctgg acgcctggga gcgcatccgc 1320
ctgcgccccg gcggcaagaa gtgctacatg atgaagcacc tggtgtgggc cagccgcgag 1380
ctggagaagt tcgccctgaa ccccggcctg ctggagacca gcgagggctg caagcagatc 1440
atccgccagc tgcaccccgc cctgcagacc ggcagcgagg agctgaagag cctgttcaac 1500
accgtggcca ccctgtactg cgtgcacgag aagatcgagg tccgcgacac caaggaggcc 1560
ctggacaaga tcgaggagga gcagaacaag tgccagcaga agatccagca ggccgaggcc 1620
gccgacaagg gcaaggtgag ccagaactac cccatcgtgc agaacctgca gggccagatg 1680
gtgcaccagg ccatcagccc ccgcaccctg aacgcctggg tgaaggtgat cgaggagaag 1740
gccttcagcc ccgaggtgat ccccatgttc accgccctga gcgagggcgc caccccccag 1800
gacctgaaca cgatgttgaa caccgtgggc ggccaccagg ccgccatgca gatgctgaag 1860
gacaccatca acgaggaggc cgccgagtgg gaccgcgtgc accccgtgca cgccggcccc 1920
atcgcccccg gccagatgcg cgagccccgc ggcagcgaca tcgccggcac caccagcacc 1980
ctgcaggagc agatcgcctg gatgaccagc aaccccccca tccccgtggg cgacatctac 2040
aagcggtgga tcatcctggg cctgaacaag atcgtgcgga tgtacagccc cgtgagcatc 2100
ctggacatca agcagggccc caaggagccc ttccgcgact acgtggaccg cttcttcaag 2160
accctgcgcg ccgagcagag cacccaggag gtgaagaact ggatgaccga caccctgctg 2220
gtgcagaacg ccaaccccga ctgcaagacc atcctgcgcg ctctcggccc cggcgccagc 2280
ctggaggaga tgatgaccgc ctgccagggc gtgggcggcc ccagccacaa ggcccgcgtg 2340
ctggccgagg cgatgagcca ggccaacacc agcgtgatga tgcagaagag caacttcaag 2400
ggcccccggc gcatcgtcaa gtgcttcaac tgcggcaagg agggccacat cgcccgcaac 2460
tgccgcgccc cccgcaagaa gggctgctgg aagtgcggca aggagggcca ccagatgaag 2520
gactgcaccg agcgccaggc caacttcctg ggcaagatct ggcccagcca caagggccgc 2580
cccggcaact tcctgcagag ccgccccgag cccaccgccc cccccgccga gagcttccgc 2640
ttcgaggaga ccacccccgg ccagaagcag gagagcaagg accgcgagac cctgaccagc 2700
ctgaagagcc tgttcggcaa cgaccccctg agccaagaat tcgccgaggc catgagccag 2760
gccaccagcg ccaacatcct gatgcagcgc agcaacttca agggccccaa gcgcatcatc 2820
aagtgcttca actgcggcaa ggagggccac atcgcccgca actgccgcgc cccccgcaag 2880
aagggctgct ggaagtgcgg caaggagggc caccagatga aggactgcac cgagcgccag 2940
gccaacttct tccgcgagga cctggccttc ccccagggca aggcccgcga gttccccagc 3000
gagcagaacc gcgccaacag ccccaccagc cgcgagctgc aggtgcgcgg cgacaacccc 3060
cgcagcgagg ccggcgccga gcgccagggc accctgaact tcccccagat caccctgtgg 3120
cagcgccccc tggtgagcat caaggtgggc ggccagatca aggaggccct gctggccacc 3180
ggcgccgacg acaccgtgct ggaggagatg agcctgcccg gcaagtggaa gcccaagatg 3240
atcggcggca tcggcggctt catcaaggtg cgccagtacg accagatcct gatcgagatc 3300
tgcggcaaga aggccatcgg caccgtgctg atcggcccca cccccgtgaa catcatcggc 3360
cgcaacatgc tgacccagct gggctgcacc ctgaacttcc ccatcagccc catcgagacc 3420
gtgcccgtga agctgaagcc cggcatggac ggccccaagg tgaagcagtg gcccctgacc 3480
gaggagaaga tcaaggccct gaccgccatc tgcgaggaga tggagaagga gggcaagatc 3540
accaagatcg gccccgagaa cccctacaac acccccgtgt tcgccatcaa gaagaaggac 3600
agcaccaagt ggcgcaagct ggtggacttc cgcgagctga acaagcgcac ccaggacttc 3660
tgggaggtgc agctgggcat cccccacccc gccggcctga agaagaagaa gagcgtgacc 3720
gtgctggacg tgggcgacgc ctacttcagc gtgcccctgg acgaggactt ccgcaagtac 3780
accgccttca ccatccccag catcaacaac gagacccccg gcatccgcta ccagtacaac 3840
gtgctgcccc agggctggaa gggcagcccc agcatcttcc agagcagcat gaccaagatc 3900
ctggagccct tccgcgcccg caaccccgag atcgtgatct accaggcccc cctgtacgtg 3960
ggcagcgacc tggagatcgg ccagcaccgc gccaagatcg aggagctgcg caagcacctg 4020
ctgcgctggg gcttcaccac ccccgacaag aagcaccaga aggagccccc cttcctgccc 4080
atcgagctgc accccgacaa gtggaccgtg cagcccatcg agctgcccga gaaggagagc 4140
tggaccgtga acgacatcca gaagctggtg ggcaagctga actgggccag ccagatctac 4200
cccggcatca aggtgcgcca gctgtgcaag ctgctgcgcg gcgccaaggc cctgaccgac 4260
atcgtgcccc tgaccgagga ggccgagctg gagctggccg agaaccgcga gatcctgcgc 4320

gagcccgtgc acggcgtgta ctacgacccc agcaaggacc tggtggccga gatccagaag 4380
cagggccacg accagtggac ctaccagatc taccaggagc ccttcaagaa cctgaagacc 4440
ggcaagtacg ccaagatgcg caccgcccac accaacgacg tgaagcagct gaccgaggcc 4500
gtgcagaaga tcgccatgga gagcatcgtg atctggggca agacccccaa gttccgcctg 4560
cccatccaga aggagacctg ggagacctgg tggaccgact actggcaggc cacctggatc 4620
cccgagtggg agttcgtgaa cacccccccc ctggtgaagc tgtggtacca gctggagaag 4680
gagcccatca tcggcgccga gaccttctac gtggacggcg ccgccaaccg cgagaccaag 4740
atcggcaagg ccggctacgt gaccgaccgg ggccggcaga agatcgtgag cctgaccgag 4800
accaccaacc agaagaccga gctgcaggcc atccagctgg ccctgcagga cagcggcagc 4860
gaggtgaaca tcgtgaccga cagccagtac gccctgggca tcatccaggc ccagcccgac 4920
aagagcgaga gcgagctggt gaaccagatc atcgagcagc tgatcaagaa ggagaaggtg 4980
tacctgagct gggtgcccgc ccacaagggc atcggcggca acgagcagat cgacaagctg 5040
gtgagcaagg gcatccgcaa ggtgctgttc ctggacggca tcgatggcgg catcgtgatc 5100
taccagtaca tggacgacct gtacgtgggc agcggcggcc ctaggatcga ttaaaagctt 5160
cccggggcta gcaccggttc taga 5184

<210> 83
<211> 4713
<212> DNA
<213> Artificial Sequence

<220>
<223> Description of Artificial Sequence:
      TatRevNefGagProtInaRTmut C

<400> 83
gccaccatgg agcccgtgga ccccaacctg gagccctgga accaccccgg cagccagccc 60
aagaccgccg gcaacaagtg ctactgcaag cactgcagct accactgcct ggtgagcttc 120
cagaccaagg gcctgggcat cagctacggc cgcaagaagc gccgccagcg ccgcagcgcc 180
ccccccagca gcgaggacca ccagaacccc atcagcaagc agcccctgcc ccagacccgc 240
ggcgacccca ccggcagcga ggagagcaag aagaaggtgg agagcaagac cgagaccgac 300
cccttcgacc ccggggccgg ccgcagcggc gacagcgacg aggccctgct gcaggccgtg 360
cgcatcatca agatcctgta ccagagcaac ccctacccca agcccgaggg cacccgccag 420
gccgacctga accgccgccg ccgctggcgc gcccgccagc gccagatcca cagcatcagc 480
gagcgcatcc tgagcacctg cctgggccgc cccgccgagc ccgtgccctt ccagctgccc 540
cccgacctgc gcctgcacat cgactgcagc gagagcagcg gcaccagcgg cacccagcag 600
agccagggca ccaccgaggg cgtgggcagc cccctcgagg ccggcaagtg gagcaagagc 660
agcatcgtgg gctggcccgc cgtgcgcgag cgcatccgcc gcaccgagcc cgccgccgag 720
ggcgtgggcg ccgccagcca ggacctggac aagcacggcg ccctgaccag cagcaacacc 780
gccgccaaca acgccgactg cgcctggctg gaggcccagg aggaggagga ggaggtgggc 840
ttccccgtgc gcccccaggt gcccctgcgc cccatgacct acaaggccgc cttcgacctg 900
agcttcttcc tgaaggagaa gggcggcctg gagggcctga tctacagcaa gaagcgccag 960
gagatcctgg acctgtgggt gtaccacacc cagggcttct tccccggctg gcagaactac 1020
acccccggcc ccggcgtgcg ctaccccctg accttcggct ggtgcttcaa gctggtgccc 1080
gtggaccccc gcgaggtgga ggaggccaac aagggcgaga acaactgcct gctgcacccc 1140
atgagccagc acggcatgga ggacgaggac cgcgaggtgc tgaagtggaa gttcgacagc 1200
agcctggccc gccgccacat ggcccgcgag ctgcaccccg agtactacaa ggactgcaag 1260
cttggcgccc gcgccagcat cctgcgcggc ggcaagctgg acgcctggga gcgcatccgc 1320
ctgcgccccg gcggcaagaa gtgctacatg atgaagcacc tggtgtgggc cagccgcgag 1380
ctggagaagt tcgccctgaa ccccggcctg ctggagacca gcgagggctg caagcagatc 1440
atccgccagc tgcaccccgc cctgcagacc ggcagcgagg agctgaagag cctgttcaac 1500
accgtggcca ccctgtactg cgtgcacgag aagatcgagg tccgcgacac caaggaggcc 1560
ctggacaaga tcgaggagga gcagaacaag tgccagcaga agatccagca ggccgaggcc 1620
gccgacaagg gcaaggtgag ccagaactac cccatcgtgc agaacctgca gggccagatg 1680

gtgcaccagg ccatcagccc ccgcaccctg aacgcctggg tgaaggtgat cgaggagaag 1740
gccttcagcc ccgaggtgat ccccatgttc accgccctga gcgagggcgc caccccccag 1800
gacctgaaca cgatgttgaa caccgtgggc ggccaccagg ccgccatgca gatgctgaag 1860
gacaccatca acgaggaggc cgccgagtgg gaccgcgtgc accccgtgca cgccggcccc 1920
atcgcccccg gccagatgcg cgagccccgc ggcagcgaca tcgccggcac caccagcacc 1980
ctgcaggagc agatcgcctg gatgaccagc aaccccccca tccccgtggg cgacatctac 2040
aagcggtgga tcatcctggg cctgaacaag atcgtgcgga tgtacagccc cgtgagcatc 2100
ctggacatca agcagggccc caaggagccc ttccgcgact acgtggaccg cttcttcaag 2160
accctgcgcg ccgagcagag cacccaggag gtgaagaact ggatgaccga caccctgctg 2220
gtgcagaacg ccaaccccga ctgcaagacc atcctgcgcg ctctcggccc cggcgccagc 2280
ctggaggaga tgatgaccgc ctgccagggc gtgggcggcc ccagccacaa ggcccgcgtg 2340
ctggccgagg cgatgagcca ggccaacacc agcgtgatga tgcagaagag caacttcaag 2400
ggcccccggc gcatcgtcaa gtgcttcaac tgcggcaagg agggccacat cgcccgcaac 2460
tgccgcgccc cccgcaagaa gggctgctgg aagtgcggca aggagggcca ccagatgaag 2520
gactgcaccg agcgccaggc caacttcctg ggcaagatct ggcccagcca caagggccgc 2580
cccggcaact tcctgcagag ccgccccgag cccaccgccc cccccgccga gagcttccgc 2640
ttcgaggaga ccacccccgg ccagaagcag gagagcaagg accgcgagac cctgaccagc 2700
ctgaagagcc tgttcggcaa cgaccccctg agccagaaag aattccccca gatcaccctg 2760
tggcagcgcc ccctggtgag catcaaggtg ggcggccaga tcaaggaggc cctgctggcc 2820
accggcgccg acgacaccgt gctggaggag atgagcctgc ccggcaagtg gaagcccaag 2880
atgatcggcg gcatcggcgg cttcatcaag gtgcgccagt acgaccagat cctgatcgag 2940
atctgcggca agaaggccat cggcaccgtg ctgatcggcc ccacccccgt gaacatcatc 3000
ggccgcaaca tgctgaccca gctgggctgc accctgaact tccccatcag ccccatcgag 3060
accgtgcccg tgaagctgaa gcccggcatg gacggcccca aggtgaagca gtggcccctg 3120
accgaggaga agatcaaggc cctgaccgcc atctgcgagg agatggagaa ggagggcaag 3180
atcaccaaga tcggccccga gaacccctac aacacccccg tgttcgccat caagaagaag 3240
gacagcacca agtggcgcaa gctggtggac ttccgcgagc tgaacaagcg cacccaggac 3300
ttctgggagg tgcagctggg catcccccac cccgccggcc tgaagaagaa gaagagcgtg 3360
accgtgctgg acgtgggcga cgcctacttc agcgtgcccc tggacgagga cttccgcaag 3420
tacaccgcct tcaccatccc cagcatcaac aacgagaccc ccggcatccg ctaccagtac 3480
aacgtgctgc cccagggctg gaagggcagc cccagcatct tccagagcag catgaccaag 3540
atcctggagc ccttccgcgc ccgcaacccc gagatcgtga tctaccaggc ccccctgtac 3600
gtgggcagcg acctggagat cggccagcac cgcgccaaga tcgaggagct gcgcaagcac 3660
ctgctgcgct ggggcttcac cacccccgac aagaagcacc agaaggagcc ccccttcctg 3720
cccatcgagc tgcaccccga caagtggacc gtgcagccca tcgagctgcc cgagaaggag 3780
agctggaccg tgaacgacat ccagaagctg gtgggcaagc tgaactgggc cagccagatc 3840
taccccggca tcaaggtgcg ccagctgtgc aagctgctgc gcggcgccaa ggccctgacc 3900
gacatcgtgc ccctgaccga ggaggccgag ctggagctgg ccgagaaccg cgagatcctg 3960
cgcgagcccg tgcacggcgt gtactacgac cccagcaagg acctggtggc cgagatccag 4020
aagcagggcc acgaccagtg gacctaccag atctaccagg agcccttcaa gaacctgaag 4080
accggcaagt acgccaagat gcgcaccgcc cacaccaacg acgtgaagca gctgaccgag 4140
gccgtgcaga agatcgccat ggagagcatc gtgatctggg gcaagacccc caagttccgc 4200
ctgcccatcc agaaggagac ctgggagacc tggtggaccg actactggca ggccacctgg 4260
atccccgagt gggagttcgt gaacaccccc cccctggtga agctgtggta ccagctggag 4320
aaggagccca tcatcggcgc cgagaccttc tacgtggacg gcgccgccaa ccgcgagacc 4380
aagatcggca aggccggcta cgtgaccgac cggggccggc agaagatcgt gagcctgacc 4440
gagaccacca accagaagac cgagctgcag gccatccagc tggccctgca ggacagcggc 4500
agcgaggtga acatcgtgac cgacagccag tacgccctgg gcatcatcca ggcccagccc 4560
gacaagagcg agagcgagct ggtgaaccag atcatcgagc agctgatcaa gaaggagaag 4620
gtgtacctga gctgggtgcc cgcccacaag ggcatcggcg gcaacgagca gatcgacaag 4680
ctggtgagca agggcatccg caaggtgctc taa 4713

<210> 84
<211> 3231

<212> DNA
<213> Artificial Sequence

<220>
<223> Description of Artificial Sequence:
      TatRevNefProtRT opt C

<400> 84
gccaccatgg agcccgtgga ccccaacctg gagccctgga accaccccgg cagccagccc 60
aagaccgccg gcaacaagtg ctactgcaag cactgcagct accactgcct ggtgagcttc 120
cagaccaagg gcctgggcat cagctacggc cgcaagaagc gccgccagcg ccgcagcgcc 180
ccccccagca gcgaggacca ccagaacccc atcagcaagc agcccctgcc ccagacccgc 240
ggcgacccca ccggcagcga ggagagcaag aagaaggtgg agagcaagac cgagaccgac 300
cccttcgacc ccggggccgg ccgcagcggc gacagcgacg aggccctgct gcaggccgtg 360
cgcatcatca agatcctgta ccagagcaac ccctacccca agcccgaggg cacccgccag 420
gccgacctga accgccgccg ccgctggcgc gcccgccagc gccagatcca cagcatcagc 480
gagcgcatcc tgagcacctg cctgggccgc cccgccgagc ccgtgccctt ccagctgccc 540
cccgacctgc gcctgcacat cgactgcagc gagagcagcg gcaccagcgg cacccagcag 600
agccagggca ccaccgaggg cgtgggcagc cccctcgagg ccggcaagtg gagcaagagc 660
agcatcgtgg gctggcccgc cgtgcgcgag cgcatccgcc gcaccgagcc cgccgccgag 720
ggcgtgggcg ccgccagcca ggacctggac aagcacggcg ccctgaccag cagcaacacc 780
gccgccaaca acgccgactg cgcctggctg gaggcccagg aggaggagga ggaggtgggc 840
ttccccgtgc gcccccaggt gcccctgcgc cccatgacct acaaggccgc cttcgacctg 900
agcttcttcc tgaaggagaa gggcggcctg gagggcctga tctacagcaa gaagcgccag 960
gagatcctgg acctgtgggt gtaccacacc cagggcttct tccccggctg gcagaactac 1020
acccccggcc ccggcgtgcg ctaccccctg accttcggct ggtgcttcaa gctggtgccc 1080
gtggaccccc gcgaggtgga ggaggccaac aagggcgaga acaactgcct gctgcacccc 1140
atgagccagc acggcatgga ggacgaggac cgcgaggtgc tgaagtggaa gttcgacagc 1200
agcctggccc gccgccacat ggcccgcgag ctgcaccccg agtactacaa ggactgcgaa 1260
ttcccccaga tcaccctgtg gcagcgcccc ctggtgagca tcaaggtggg cggccagatc 1320
aaggaggccc tgctggacac cggcgccgac gacaccgtgc tggaggagat gagcctgccc 1380
ggcaagtgga agcccaagat gatcggcggc atcggcggct tcatcaaggt gcgccagtac 1440
gaccagatcc tgatcgagat ctgcggcaag aaggccatcg gcaccgtgct gatcggcccc 1500
acccccgtga acatcatcgg ccgcaacatg ctgacccagc tgggctgcac cctgaacttc 1560
cccatcagcc ccatcgagac cgtgcccgtg aagctgaagc ccggcatgga cggccccaag 1620
gtgaagcagt ggcccctgac cgaggagaag atcaaggccc tgaccgccat ctgcgaggag 1680
atggagaagg agggcaagat caccaagatc ggccccgaga acccctacaa cacccccgtg 1740
ttcgccatca agaagaagga cagcaccaag tggcgcaagc tggtggactt ccgcgagctg 1800
aacaagcgca cccaggactt ctgggaggtg cagctgggca tcccccaccc cgccggcctg 1860
aagaagaaga agagcgtgac cgtgctggac gtgggcgacg cctacttcag cgtgcccctg 1920
gacgaggact tccgcaagta caccgccttc accatcccca gcatcaacaa cgagaccccc 1980
ggcatccgct accagtacaa cgtgctgccc cagggctgga agggcagccc cagcatcttc 2040
cagagcagca tgaccaagat cctggagccc ttccgcgccc gcaaccccga gatcgtgatc 2100
taccaggccc ccctgtacgt gggcagcgac ctggagatcg gccagcaccg cgccaagatc 2160
gaggagctgc gcaagcacct gctgcgctgg ggcttcacca cccccgacaa gaagcaccag 2220
aaggagcccc ccttcctgcc catcgagctg caccccgaca agtggaccgt gcagcccatc 2280
gagctgcccg agaaggagag ctggaccgtg aacgacatcc agaagctggt gggcaagctg 2340
aactgggcca gccagatcta ccccggcatc aaggtgcgcc agctgtgcaa gctgctgcgc 2400
ggcgccaagg ccctgaccga catcgtgccc ctgaccgagg aggccgagct ggagctggcc 2460
gagaaccgcg agatcctgcg cgagcccgtg cacggcgtgt actacgaccc cagcaaggac 2520
ctggtggccg agatccagaa gcagggccac gaccagtgga cctaccagat ctaccaggag 2580
cccttcaaga acctgaagac cggcaagtac gccaagatgc gcaccgccca caccaacgac 2640
gtgaagcagc tgaccgaggc cgtgcagaag atcgccatgg agagcatcgt gatctggggc 2700
aagaccccca agttccgcct gcccatccag aaggagacct gggagacctg gtggaccgac 2760

tactggcagg ccacctggat ccccgagtgg gagttcgtga acaccccccc cctggtgaag 2820
ctgtggtacc agctggagaa ggagcccatc atcggcgccg agaccttcta cgtggacggc 2880
gccgccaacc gcgagaccaa gatcggcaag gccggctacg tgaccgaccg gggccggcag 2940
aagatcgtga gcctgaccga gaccaccaac cagaagaccg agctgcaggc catccagctg 3000
gccctgcagg acagcggcag cgaggtgaac atcgtgaccg acagccagta cgccctgggc 3060
atcatccagg cccagcccga caagagcgag agcgagctgg tgaaccagat catcgagcag 3120
ctgatcaaga aggagaaggt gtacctgagc tgggtgcccg cccacaaggg catcggcggc 3180
aacgagcaga tcgacaagct ggtgagcaag ggcatccgca aggtgctcta a 3231

<210> 85
<211> 576
<212> DNA
<213> Artificial Sequence

<220>
<223> Description of Artificial Sequence: vif.opt.SF2

<400> 85
atggagaacc gctggcaggt gatgatcgtg tggcaggtgg accgcatgcg catccgcacc 60
tggaagagcc tggtgaagca ccacatgtac atcagcaaga aggccaaggg ctggttctac 120
cgccaccact acgagagcac ccacccccgc gtgagcagcg aggtgcacat ccccctgggc 180
gacgccaagc tggtgatcac cacctactgg ggcctgcaca ccggcgagcg cgagtggcac 240
ctgggccagg gcgtggccat cgagtggcgc aagaagaagt acagcaccca ggtggacccc 300
ggcctggccg accagctgat ccacctgcac tacttcgact gcttcagcga gagcgccatc 360
aagaacgcca tcctgggcta ccgcgtgagc ccccgctgcg agtaccaggc cggccacaac 420
aaggtgggca gcctgcagta cctggccctg gccgccctga tcacccccaa gaagaccaag 480
ccccccctgc ccagcgtgaa gaagctgacc gaggaccgct ggaacaagcc ccagaagacc 540
aagggccacc gcggcagcca caccatgaac ggccac 576

<210> 86
<211> 291
<212> DNA
<213> Artificial Sequence

<220>
<223> Description of Artificial Sequence: vpr.opt.SF2

<400> 86
atggagcagg cccccgagga ccagggcccc cagcgcgagc cctacaacga gtggaccctg 60
gagctgctgg aggagctgaa gcgcgaggcc gtgcgccact tcccccgccc ctggctgcac 120
agcctgggcc agtacatcta cgagacctac ggcgacacct gggccggcgt ggaggccatc 180
atccgcatcc tgcagcagct gctgttcatc cacttccgca tcggctgcca gcacagccgc 240
atcggcatca tccagcagcg ccgcgcccgc cgcaacggcg ccagccgcag c 291

<210> 87
<211> 243
<212> DNA
<213> Artificial Sequence

<220>
<223> Description of Artificial Sequence: vpu.opt.SF162

<400> 87
atgcagcccc tgcagatcct ggccatcgtg gccctggtgg tggccgccat catcgccatc 60

gtggtgtgga ccatcgtgta catcgagtac cgcaagatcc tgcgccagcg caagatcgac 120
cgcctgatcg accgcatcac cgagcgcgcc gaggacagcg gcaacgagag cgagggcgac 180
caggaggagc tgagcgccct ggtggagcgc ggccacctgg ccccctggga cgtggacgac 240
ctg 243

<210> 88
<211> 579
<212> DNA
<213> Artificial Sequence

<220>
<223> Description of Artificial Sequence:
      Vif_TV1_C_ZAopt

<400> 88
atggagaacc gctggcaggt gctgatcgtg tggcaggtgg accgcatgaa gatccgcgcc 60
tggaacagcc tggtgaagca ccacatgtac atcagccgcc gcgccagcgg ctgggtgtac 120
cgccaccact tcgagagccg ccaccccaag gtgagcagcg aggtgcacat ccccctgggc 180
gacgcccgcc tggtgatcaa gacctactgg ggcctgcaga ccggcgagcg cgactggcac 240
ctgggccacg gcgtgagcat cgagtggcgc ctgcgcgagt acagcaccca ggtggacccc 300
gacctggccg accagctgat ccacatgcac tacttcgact gcttcaccga gagcgccatc 360
cgccaggcca tcctgggcca catcgtgttc ccccgctgcg actaccaggc cggccacaag 420
aaggtgggca gcctgcagta cctggccctg accgccctga tcaagcccaa gaagcgcaag 480
ccccccctgc ccagcgtgcg caagctggtg gaggaccgct ggaacgaccc ccagaagacc 540
cgcggccgcc gcggcaacca caccatgaac ggccactag 579

<210> 89
<211> 579
<212> DNA
<213> Artificial Sequence

<220>
<223> Description of Artificial Sequence: description

<400> 89
atggagaacc gctggcaggt gctgatcgtg tggcaggtgg accgcatgaa gatccgcacc 60
tggcacagcc tggtgaagca ccacatgtac gtgagccgcc gcgccgacgg ctggttctac 120
cgccaccact acgagagccg ccaccccaag gtgagcagcg aggtgcacat ccccctgggc 180
gacgcccgcc tggtgatcaa gacctactgg ggcctgcaga ccggcgagcg cgcctggcac 240
ctgggccacg gcgtgagcat cgagtggcgc ctgcgccgct acagcaccca ggtggacccc 300
gacctgaccg accagctgat ccacatgcac tacttcgact gcttcgccga gagcgccatc 360
cgcaaggcca tcctgggcca gatcgtgagc cccaagtgcg actaccaggc cggccacaac 420
aaggtgggca gcctgcagta cctggccctg accgccctga tcaagcccaa gaagatcaag 480
ccccccctgc ccagcgtgcg caagctggtg gaggaccgct ggaacaagcc ccagaagacc 540
cgcggccgcc gcggcaacca caccatgaac ggccactag 579

<210> 90
<211> 288
<212> DNA
<213> Artificial Sequence

<220>
<223> Description of Artificial Sequence:
      Vpr_TV1_C_ZAopt

<400> 90
atggagcgcc cccccgagga ccagggcccc cagcgcgagc cctacaacga gtggaccctg 60
gagatcctgg aggagctgaa gcaggaggcc gtgcgccact tcccccgccc ctggctgcac 120
agcctgggcc agtacatcta cgagacctac ggcgacacct ggaccggcgt ggaggccatc 180
atccgcgtgc tgcagcagct gctgttcatc cacttccgca tcggctgcca gcacagccgc 240
atcggcatcc tgcgccagcg ccgcgcccgc aacggcgcca gccgcagc 288

<210> 91
<211> 288
<212> DNA
<213> Artificial Sequence

<220>
<223> Description of Artificial Sequence:
      Vpr_TV2_C_ZAopt

<400> 91
atggagcagg cccccgagga ccagggcccc cagcgcgagc cctacaacga gtggaccctg 60
gagctgctgg aggagctgaa gcaggaggcc gtgcgccact tcccccgccc ctggctgcac 120
aacctgggcc agcacatcta cgagacctac ggcgacacct ggaccggcgt ggaggccatc 180
atccgcatcc tgcagcagct gctgttcatc cacttccgca tcggctgcca ccacagccgc 240
atcggcatcc tgcgccagcg ccgcgcccgc aacggcgcca accgcagc 288

<210> 92
<211> 267
<212> DNA
<213> Artificial Sequence

<220>
<223> Description of Artificial Sequence:
      Vpu_TV1_C_ZAopt

<400> 92
atggtgagcc tgagcctgtt caagggcgtg gactaccgcc tgggcgtggg cgccctgatc 60
gtggccctga tcatcgccat catcgtgtgg accatcgcct acatcgagta ccgcaagctg 120
gtgcgccaga agaagatcga ctggctgatc aagcgcatcc gcgagcgcgc cgaggacagc 180
ggcaacgaga gcgacggcga caccgaggag ctgagcacca tggtggacat gggccacctg 240
cgcctgctgg acgccaacga cctgtaa 267

<210> 93
<211> 261
<212> DNA
<213> Artificial Sequence

<220>
<223> Description of Artificial Sequence:
      Vpu_TV2_C_ZAopt

<400> 93
atgctggacc tgaccgcccg catcgacagc cgcctgggca tcggcgccct gatcgtggcc 60
ctgatcatcg ccatcatcgt gtggaccatc gtgtacatcg agtaccgcaa gctggtgcgc 120
cagcgcaaga tcgactggct ggtgaagcgc atccgcgagc gcgccgagga cagcggcaac 180
gagagcgagg gcgacaccga ggagctgagc accctggtgg acatgggcca cctgcgcctg 240

ctggacgcca acgacgtgta a                                                261